# EUROPEAN PATENT APPLICATION

(11) **EP 4 198 030 A1**
(43) Date of publication of application: **21.06.2023**
(21) Application number: 21855598.5
(22) Date of filing: 12.08.2021
(51) Int. Cl.: C07D 401/04, C07D 401/14, C07D 405/04, C07D 405/14, A61K 31/517, A61P 35/00, A61P 37/00

(54) **IMMUNOREGULATORY COMPOUND AND ANTITUMOR APPLICATION THEREOF**

(30) Priority: 14.08.2020 CN 202010817385
(71) Applicant: Shanghaitech University, Shanghai 201210 (CN)
(72) Inventor: YANG, Xiaobao, Shanghai 201210 (CN); JIANG, Biao, Shanghai 201210 (CN); LIU, Linyi, Shanghai 201210 (CN); REN, Chaowei, Shanghai 201210 (CN); QIU, Xing, Shanghai 201210 (CN)
(74) Representative: J A Kemp LLP
(86) International application number: PCT/CN2021/112236
(87) International publication number: WO 2022/033548

(57) **Abstract**

The present disclosure provides compounds of formula (I) or or salts, enantiomers, stereoisomers, solvates, or polymorphs thereof, and applications thereof, and pharmaceutical compositions comprising, as an active ingredient, the compound of Formula (I) or a salt, enantiomer, stereoisomer, solvate, or polymorph thereof, and applications of the pharmaceutical compositions. The compounds can effectively prevent and/or treat diseases or disorders associated with TNF-α.

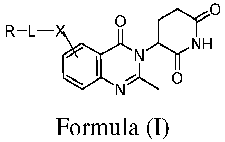

## Description

### Technical Field

The present disclosure relates to compounds of Formula (I) or a salt, an enantiomer, a stereoisomer, a solvate, or a polymorph thereof and applications thereof, especially their application in the prevention and/or treatment of a disease or disorder associated with TNF-α.

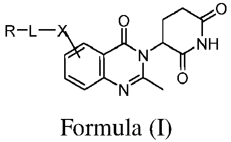

### Background

Tumor necrosis factor alpha (TNF-α) is an important pro-inflammatory cytokine involved in immune and inflammatory response of the body. Research shows that TNF-α can not only participate in the occurrence and development of inflammation, but also promote the formation of tumor. The over-expression of TNF-α and the over-activation of related signals are related to the pathological mechanism of many diseases. Thus, blocking TNF-α at various levels may potentially treat diseases or conditions associated with TNF-α. As immunomodulators, Lenalidomide and Pomalidomide can significantly reduce the level of TNF-α through cereblon E3 ligase and inhibit the secretion of other pro-inflammatory factors, and have anti-tumor and immunomodulatory activities (Lopez-Girona A, et al. Leukemia, 2012; Liu D, et al. Gen Comp Endocrinol, 2016; 228:1). Currently, Lenalidomide has been approved for the treatment of multiple myeloma, myelodysplastic syndrome and mantle cell lymphoma, while Pomalidomide is applicable to patients with multiple myeloma who have received at least two drugs (including Lenalidomide, Bortezomib) in the past and recently undergone treatment or progressed within 60 days after completion of treatment. In addition, in clinical trials, Lenalidomide and Pomalidomide can be used alone or in combination with other therapeutic drugs for the treatment of other diseases such as lymphomas, thyroid cancer, leukemia, melanoma, lung cancer.

Avadomide (CC-122) is a new generation of effective oral immunoregulator. Avadomide can modulate the activity of cereblon E3 ligase, leading to more significant degradation of IKZF1 and IKZF 3, thereby down-regulating TNF-α. Thus, Avadomide can have anti-tumor and immunomodulatory activities. At present, phase II clinical trials (NCT02406742, NCT02859324, NCT03834623) have been conducted to assess therapeutic efficacy of Avadomide on a variety of related tumors.

Although several domide derivatives compounds have been approved for clinical use or entered in clinical trials, the occurrence of side effects and drug resistance limits the clinical application of these drugs. Therefore, there is a need for novel and optimized domide derivatives compounds to meet societal needs.

### Summary of Invention

Therefore, in one aspect, the present disclosure provides a compound of Formula (I): 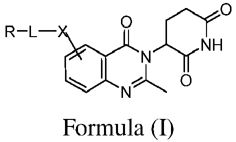 or a salt, an enantiomer, a stereoisomer, a solvate, or polymorph thereof, in which
X, R, and L and all substituents are as defined in the Detailed Description of the Invention. The following compound is excluded from the compounds of Formula (I) of the the present disclosure:

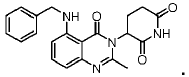

The present disclosure also provides a pharmaceutical composition comprising the compound of Formula (I) or a pharmaceutically acceptable salt, an enantiomer, a stereoisomer, a solvate, or polymorph thereof, and at least one pharmaceutically acceptable carrier.

The present disclosure also provides the compound of Formula (I), or a pharmaceutically acceptable salt, an enantiomer, a diastereomer, a solvate, or polymorph thereof for use as a medicament.

The present disclosure also provides the compound of Formula (I), or a pharmaceutically acceptable salt, an enantiomer, a diastereomer, a solvate, or polymorph thereof or the pharmaceutical composition for use in the prevention or treatment of a disease or disorder associated with TNF-α.

The present disclosure also provides the use of the compound of Formula (I) or a pharmaceutically acceptable salt, an enantiomer, a stereoisomer, a solvate, or polymorph thereof or the pharmaceutical composition for the manufacture of a medicament for the treatment or prevention of a disease or disorder associated with TNF-α.

The present disclosure also provides a method for the treatment or prevention of a disease or disorder associated with TNF-α, comprising administering to a subject a therapeutically effective amount of the compound of Formula (I), or a pharmaceutically acceptable salt, an enantiomer, a stereoisomer, a solvate, or polymorph thereof, or the pharmaceutical composition of the present disclosure.

### Brief Description of Drawings

Figure 1 shows degradation effects of some compounds of the present invention on IKZF1/3. It can be seen from the gray depth that the compounds of the present invention can significantly degrade IKZF1/3.
Figure 2 shows inhibition effects of some compounds of the present invention on the expression of TNF-α. It can be found from the strength of inhibition that the compounds of the present invention can greatly inhibit the expression of TNF-α.
Figure 3 shows anti-proliferation effect of compound SIAIS355035 of the present invention on a variety of selected cell lines.
Figure 4-5 show degradation effects of the compounds of the present disclosure on IKZF1/3. It can be found from the gray depth that the compounds of the present invention can significantly degrade IKZF1/3.

### Detailed Description of the Invention

In one aspect, the present disclosure provides a compound of Formula (I): 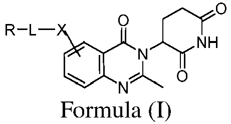 or a salt, an enantiomer, a diastereomer, a solvate, or a polymorph thereof, in which
X represents N(Rₐ₁), O or S, where Rₐ₁ represents H or C₁₋₃ alkyl (e.g., methyl or ethyl);
L represents:
   optionally substituted linear or branched alkylene, wherein said linear or branched alkylene is optionally interrupted one or more times by one or more groups selected from the group consisting of: optionally substituted arylene, optionally substituted heteroarylene or any combination thereof; or
   Formula -Lₐ₂₋Lₐ₁₋-^{∗}, where Lₐ₁ represents optionally substituted linear or branched alkylene, Lₐ₂ represents optionally substituted cycloalkylene, optionally substituted arylene, optionally substituted heterocyclylene or optionally substituted heteroarylene, and symbol * indicates the point of attachment to X; and
R represents NRₐ₇Rₐ₈, C(O)NRₐ₉Rₐ₁₀, N(Rₐ₁₁)C(O)Rₐ₁₂, N(Rₐ₁₃)C(O)NRₐ₁₄Rₐ₁₅, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, C₂₋₆ alkynylene-Rₐ₂₉, C₂₋₆ alkenylene-Rₐ₃₀, optionally substituted cycloalkyl, optionally substituted aryl, optionally substituted heterocyclyl or optionally substituted heteroaryl,
   wherein Rₐ₇, Rₐ₈, Rₐ₉, Rₐ₁₀, Rₐ₁₄, Rₐ₁₅, Rₐ₁₆, Rₐ₁₇, Rₐ₂₁, Rₐ₂₂, Rₐ₂₉, and Rₐ₃₀ each independently represent H, optionally substituted linear or branched alkyl, optionally substituted cycloalkyl, optionally substituted aryl, optionally substituted heterocyclyl or optionally substituted heteroaryl;
   Rₐ₁₁, Rₐ₁₃, and Rₐ₂₃ each independently represent H or C₁₋₃ alkyl; and
   Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched alkyl, optionally substituted cycloalkyl, optionally substituted aryl, optionally substituted heterocyclyl or optionally substituted heteroaryl;
   or
X represents alkynylene (e.g., C₂₋₈ alkynylene) or alkenylene (e.g., C₂₋₈ alkenylene); and
L represents:
   optionally substituted linear or branched alkylene, wherein said linear or branched alkylene is optionally interrupted one or more times by one or more groups selected from the group consisting of: optionally substituted arylene, optionally substituted heteroarylene or any combination thereof; or
   Formula -L_{b2}-L_{b1}-#, where L_{b1} represents optionally substituted cycloalkylene, optionally substituted arylene, optionally substituted heterocyclylene or optionally substituted heteroarylene, L_{b2} represents optionally substituted linear or branched C₁₋₄₀ alkylene, and symbol # indicates the point of attachment to X; and
R represents NR_{b7}R_{b8}, C(O)NR_{b9}R_{b10}, N(R_{b11})C(O)R_{b12}, N(R_{b13})C(O)NR_{b14}R_{b15}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, C₂₋₆ alkynylene-R_{b29}, C₂₋₆ alkenylene-R_{b30}, optionally substituted cycloalkyl, optionally substituted aryl, optionally substituted heterocyclyl or optionally substituted heteroaryl,
   wherein R_{b7}, R_{b8}, R_{b9}, R_{b10}, R_{b14}, R_{b15}, R_{b16}, R_{b17}, R_{b21}, R_{b22}, R_{b29}, and R_{b30} each independently represent H, optionally substituted linear or branched alkyl, optionally substituted cycloalkyl, optionally substituted aryl, optionally substituted heterocyclyl or optionally substituted heteroaryl;
   R_{b11}, R_{b13}, and R_{b23} each independently represent H or C₁₋₃ alkyl; and
   R_{b12}, R_{b18}, R_{b19}, R_{b20}, R_{b24}, R_{b25}, R_{b26}, R_{b27}, and R_{b28} each independently represent optionally substituted linear or branched alkyl, optionally substituted cycloalkyl, optionally substituted aryl, optionally substituted heterocyclyl or optionally substituted heteroaryl;
   or
X represents a bond; and
L represents optionally substituted linear or branched alkylene, wherein said linear or branched alkylene is optionally interrupted one or more times by one or more groups selected from the group consisting of: optionally substituted arylene, optionally substituted heteroarylene or any combination thereof, wherein R_{c1}, R_{c2}, and R_{c3} each independently represent H or C₁₋₃ alkyl; and
R represents NR_{c4}R_{c5}, C(O)NR_{c6}R_{c7}, N(R_{c8})C(O)R_{c9}, N(R_{c10})C(O)NR_{c11}R_{c12}, OR_{c13}, SR_{c14}, C(O)R_{c15}, OC(O)R_{c16}, C(O)OR_{c17}, S(O)₂NR_{c18}R_{c19}, N(R_{c20})S(O)₂R_{c21}, S(O)R_{c22}, S(O)₂R_{c23}, S(O)₂OR_{c24}, OS(O)₂R_{c25}, C₂₋₆ alkynylene-R_{c26}, C₂₋₆ alkenylene-R_{c27}, optionally substituted C₅₋₂₀ cycloalkyl, or optionally substituted C₅₋₂₀ heterocyclyl,
   wherein R_{c8}, R_{c10}, and R_{c20} each independently represent H or C₁₋₃ alkyl;
   R_{c4}, R_{c6}, R_{c11}, R_{c18}, R_{c26}, and R_{c27} each independently represent H, optionally substituted linear or branched C₁₋₁₀ alkyl, optionally substituted cycloalkyl, optionally substituted aryl, optionally substituted heterocyclyl or optionally substituted heteroaryl; and
   R_{c5}, R_{c7}, R_{c9}, R_{c12}, R_{c13}, R_{c14}, R_{c15}, R_{c16}, R_{c17}, R_{c19}, R_{c21}, R_{c22}, R_{c23}, R_{c24}, and R_{c25} each independently represent optionally substituted C₅₋₂₀ cycloalkyl or optionally substituted C₅₋₂₀ heterocyclyl.

Herein, the following compound is excluded from the compounds of Formula (I) of the the present disclosure:

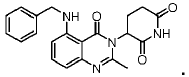

In one embodiment of the present disclosure, X represents O.

In one embodiment of the present disclosure, X represents S.

In one embodiment of the present disclosure, X represents N(Rₐ₁), wherein Rₐ₁ represents H or C₁₋₃ alkyl (e.g., methyl, ethyl, or propyl). In one embodiment of the present disclosure, X represents NH or N(CH₃).

In one embodiment of the present disclosure, X represents a bond.

In one embodiment of the present disclosure, X represents alkenylene (e.g., C₂₋₆ alkenylene, e.g., vinylene).

In one embodiment of the present disclosure, X represents alkynylene (e.g., C₂₋₆ alkynylene, e.g., ethynylene).

In one embodiment of the present disclosure, X represents N(Rₐ₁), O or S, where Rₐ₁ represents H or C₁₋₃ alkyl; and
L represents linear or branched C₁₋₃ alkylene optionally substituted by a substituent selected from the group consisting of halogen, hydroxyl, amino or any combination thereof; and
R represents:
   NRₐ₇Rₐ₈, wherein Rₐ₇ is H or C₁₋₃ alkyl, and Rₐ₈ is phenyl optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; or
   benzo[d][1,3]dioxolyl, tetrahydropyranyl, pyridyl or phenyl, said benzo[d][1,3]dioxolyl, tetrahydropyranyl, pyridyl and phenyl are each independently optionally substituted by a substituent selected from the group consisting of halogen, hydroxyl, mercapto, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)- or any combination thereof;
   or
L represents Formula -Lₐ₂₋Lₐ₁₋-^{∗}, where Lₐ₁ represents linear or branched C₁₋₄₀ alkylene optionally substituted by a substituent selected from the group consisting of halogen, hydroxyl, amino or any combination thereof, Lₐ₂ represents optionally substituted C₃₋₁₅ cycloalkylene or optionally substituted C₅₋₁₅ arylene, wherein said C₃₋₁₅ cycloalkylene is optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)- or any combination thereof, and said C₅₋₁₅ arylene is optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof, and symbol ^{∗} indicates the point of attachment to X;
   and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, or OS(O)₂Rₐ₂₈,
   wherein Rₐ₁₆ and Rₐ₁₇ each independently represent H, optionally substituted cycloalkyl, or optionally substituted heterocyclyl;
   Rₐ₉, Rₐ₁₁, Rₐ₂₁, and Rₐ₂₃ each independently represent H or C₁₋₃ alkyl; and
   Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted cycloalkyl or optionally substituted heterocyclyl;
   or
L represents linear or branched alkylene interrupted one or more times (such as 1-10, 1-8, 1-6, 1-5, 1-3, 1-2 times, or once, which are not restricted in principle, or are automatically limited by the size of building unit referenced herein, or can vary as required) by one or more (such as 1-10, 1-8, 1-6, 1-5, 1-4, 1-3, 1-2, or 1, which are not restricted in principle, or are automatically limited by the size of building unit referenced herein, or can vary as required) groups selected from the group consisting of: optionally substituted arylene, optionally substituted heteroarylene or any combination thereof, wherein said linear or branched alkylene is optionally substituted by a substituent selected from the group consisting of halogen, hydroxyl, amino or any combination thereof;
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted cycloalkyl, or optionally substituted heterocyclyl;
   wherein Rₐ₇, Rₐ₈, Rₐ₉, Rₐ₁₀, Rₐ₁₆, Rₐ₁₇, Rₐ₂₁ and Rₐ₂₂ each independently represent H, optionally substituted linear or branched alkyl, optionally substituted cycloalkyl or optionally substituted heterocyclyl;
   Rₐ₁₁ and Rₐ₂₃ each independently represent H or C₁₋₃ alkyl; and
   Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched alkyl, optionally substituted cycloalkyl or optionally substituted heterocyclyl;
   or
X represents alkynylene or alkenylene; and
L represents:
   linear or branched alkylene interrupted one or more times (such as 1-10, 1-8, 1-6, 1-5, 1-3, 1-2 times, or once, which are not restricted in principle, or are automatically limited by the size of building unit referenced herein, or can vary as required) by one or more (such as 1-10, 1-8, 1-6, 1-5, 1-4, 1-3, 1-2, or 1, which are not restricted in principle, or are automatically limited by the size of building unit referenced herein, or can vary as required) groups selected from the group consisting of: optionally substituted arylene, optionally substituted heteroarylene or any combination thereof, wherein said linear or branched alkylene is optionally substituted by a substituent selected from the group consisting of halogen, hydroxyl, amino or any combination thereof; or
   Formula -L_{b2}-L_{b1}-#, where L_{b1} represents optionally substituted C₅₋₁₅ arylene or optionally substituted C₅₋₁₅ heteroarylene, wherein said C₅₋₁₅ arylene and C₅₋₁₅ heteroarylene are each independently optionally substituted by one or more (such as 1-5, 1-4, 1-3, 1-2, or 1, which are not restricted in principle, or are automatically limited by the size of building unit referenced herein, or can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; L_{b2} represents optionally substituted linear or branched C₁₋₄₀ alkylene, and symbol # indicates the point of attachment to X; and
R represents NR_{b7}R_{b8}, C(O)NR_{b9}R_{b10}, N(R_{b11})C(O)R_{b12}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, optionally substituted cycloalkyl or optionally substituted heterocyclyl,
   wherein R_{b7}, R_{b8}, R_{b9}, R_{b10}, R_{b16}, R_{b17}, R_{b21} and R_{b22} each independently represent H, optionally substituted linear or branched alkyl, optionally substituted cycloalkyl or optionally substituted heterocyclyl;
   R_{b11} and R_{b13} each independently represent H or C₁₋₃ alkyl; and
   R_{b12}, R_{b18}, R_{b19}, R_{b20}, R_{b24}, R_{b25}, R_{b26}, R_{b27}, and R_{b28} each independently represent optionally substituted linear or branched alkyl, optionally substituted cycloalkyl or optionally substituted heterocyclyl;
   or
X represents a bond; and
L represents linear or branched alkylene interrupted one or more times (such as 1-10, 1-8, 1-6, 1-5, 1-3, 1-2 times, or once, which are not restricted in principle, or are automatically limited by the size of building unit referenced herein, or can vary as required) by one or more (such as 1-10, 1-8, 1-6, 1-5, 1-4, 1-3, 1-2, or 1, which are not restricted in principle, or are automatically limited by the size of building unit referenced herein, or can vary as required) groups selected from the group consisting of: optionally substituted arylene, optionally substituted heteroarylene or any combination thereof, wherein said linear or branched alkylene is optionally substituted by a substituent selected from the group consisting of halogen, hydroxyl, amino or any combination thereof; and
R represents NR_{c4}R_{c5}, C(O)NR_{c6}R_{c7}, N(R_{c8})C(O)R_{c9}, OR_{c13}, SR_{c14}, C(O)R_{c15}, OC(O)R_{c16}, C(O)OR_{c17}, S(O)₂NR_{c18}R_{c19}, N(R_{c20})S(O)₂R_{c21}, S(O)R_{c22}, S(O)₂R_{c23}, S(O)₂OR_{c24}, OS(O)₂R_{c25}, optionally substituted C₅₋₂₀ cycloalkyl, or optionally substituted C₅₋₂₀ heterocyclyl,
   wherein R_{c8} and R_{c20} each independently represent H or C₁₋₃ alkyl;
   R_{c4}, R_{c6}, and R_{c18} each independently represent H, optionally substituted linear or branched C₁₋₁₀ alkyl, optionally substituted cycloalkyl, optionally substituted aryl, optionally substituted heterocyclyl or optionally substituted heteroaryl; and
   R_{c5}, R_{c7}, R_{c9}, R_{c13}, R_{c14}, R_{c15}, R_{c16}, R_{c17}, R_{c19}, R_{c21}, R_{c22}, R_{c23}, R_{c24}, and R_{c25} each independently represent optionally substituted C₅₋₂₀ cycloalkyl or optionally substituted C₅₋₂₀ heterocyclyl.

In one embodiment of the present disclosure, X represents N(Rₐ₁), O or S, where Rₐ₁ represents H or C₁₋₃ alkyl; and
L represents linear or branched C₁₋₃ alkylene optionally substituted by a substituent selected from the group consisting of halogen, hydroxyl, amino or any combination thereof; and
R represents:
   NRₐ₇Rₐ₈, wherein Rₐ₇ is H or C₁₋₃ alkyl, and Rₐ₈ is phenyl optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; or
   benzo[d][1,3]dioxolyl, tetrahydropyranyl, pyridyl or phenyl, said benzo[d][1,3]dioxolyl, tetrahydropyranyl, pyridyl and phenyl are each independently optionally substituted by a substituent selected from the group consisting of halogen, hydroxyl, mercapto, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)- or any combination thereof;
   or
L represents Formula -Lₐ₂₋Lₐ₁₋-^{∗}, where Lₐ₁ represents linear or branched C₁₋₄₀ alkylene optionally substituted by a substituent selected from the group consisting of halogen, hydroxyl, amino or any combination thereof, Lₐ₂ represents optionally substituted C₃₋₁₅ cycloalkylene or optionally substituted C₅₋₁₅ arylene, wherein said C₃₋₁₅ cycloalkylene is optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)- or any combination thereof, and said C₅₋₁₅ arylene is optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof, and symbol ^{∗} indicates the point of attachment to X; and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, or OS(O)₂Rₐ₂₈,
   wherein Rₐ₁₆ and Rₐ₁₇ each independently represent H, optionally substituted C₃₋₂₀ cycloalkyl, or optionally substituted C₃₋₂₀ heterocyclyl;
   Rₐ₉, Rₐ₁₁, Rₐ₂₁, and Rₐ₂₃ each independently represent H or C₁₋₃ alkyl; and
   Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl;
   or
L represents linear or branched C₂₋₄₀ alkylene interrupted one or more times (such as 1-10, 1-8, 1-6, 1-5, 1-3, 1-2 times, or once, which are not restricted in principle, or are automatically limited by the size of building unit referenced herein, or can vary as required) by one or more (such as 1-10, 1-8, 1-6, 1-5, 1-4, 1-3, 1-2, or 1, which are not restricted in principle, or are automatically limited by the size of building unit referenced herein, or can vary as required) groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof;
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted cycloalkyl, or optionally substituted heterocyclyl;
   wherein Rₐ₇, Rₐ₈, Rₐ₉, Rₐ₁₀, Rₐ₁₆, Rₐ₁₇, Rₐ₂₁ and Rₐ₂₂ each independently represent H, optionally substituted linear or branched C₁₋₁₀ alkyl, optionally substituted C₃₋₂₀ cycloalkyl or optionally substituted C₃₋₂₀ heterocyclyl;
   Rₐ₁₁ and Rₐ₂₃ each independently represent H or C₁₋₃ alkyl; and
   Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
   or
X represents C₂₋₆ alkynylene or C₂₋₆ alkenylene; and
L represents:
   linear or branched C₂₋₄₀ alkylene interrupted one or more times (such as 1-10, 1-8, 1-6, 1-5, 1-3, 1-2 times, or once, which are not restricted in principle, or are automatically limited by the size of building unit referenced herein, or can vary as required) by one or more (such as 1-10, 1-8, 1-6, 1-5, 1-4, 1-3, 1-2, or 1, which are not restricted in principle, or are automatically limited by the size of building unit referenced herein, or can vary as required) groups selected from the group consisting of: optionally substituted C₅₋₂₀ arylene, optionally substituted C₅₋₂₀ heteroarylene or any combination thereof, wherein said linear or branched C₂₋₄₀ alkylene is optionally substituted by a substituent selected from the group consisting of halogen, hydroxyl, amino or any combination thereof; or
   Formula -L_{b2}-L_{b1}-#, where L_{b1} represents optionally substituted C₅₋₁₅ arylene or optionally substituted C₅₋₁₅ heteroarylene, wherein said C₅₋₁₅ arylene and C₅₋₁₅ heteroarylene are each independently optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; L_{b2} represents optionally substituted linear or branched C₁₋₄₀ alkylene, and symbol # indicates the point of attachment to X; and
R represents NR_{b7}R_{b8}, C(O)NR_{b9}R_{b10}, N(R_{b11})C(O)R_{b12}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, optionally substituted C₃₋₂₀ cycloalkyl or optionally substituted C₃₋₂₀ heterocyclyl,
   wherein R_{b7}, R_{b8}, R_{b9}, R_{b10}, R_{b16}, R_{b17}, R_{b21} and R_{b22} each independently represent H, optionally substituted linear or branched C₁₋₁₀ alkyl, optionally substituted C₃₋₂₀ cycloalkyl or optionally substituted C₃₋₂₀ heterocyclyl;
   R_{b11} and R_{b13} each independently represent H or C₁₋₃ alkyl; and
   R_{b12}, R_{b18}, R_{b19}, R_{b20}, R_{b24}, R_{b25}, R_{b26}, R_{b27}, and R_{b28} each independently represent optionally substituted linear or branched C₁₋₁₀ alkyl, optionally substituted C₃₋₂₀ cycloalkyl or optionally substituted C₃₋₂₀ heterocyclyl;
   or
X represents a bond; and
L represents linear or branched C₂₋₄₀ alkylene interrupted one or more times (such as 1-10, 1-8, 1-6, 1-5, 1-3, 1-2 times, or once, which are not restricted in principle, or are automatically limited by the size of building unit referenced herein, or can vary as required) by one or more (such as 1-10, 1-8, 1-6, 1-5, 1-4, 1-3, 1-2, or 1, which are not restricted in principle, or are automatically limited by the size of building unit referenced herein, or can vary as required) groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof; and
R represents NR_{c4}R_{c5}, C(O)NR_{c6}R_{c7}, N(R_{c8})C(O)R_{c9}, OR_{c13}, SR_{c14}, C(O)R_{c15}, OC(O)R_{c16}, C(O)OR_{c17}, S(O)₂NR_{c18}R_{c19}, N(R_{c20})S(O)₂R_{c21}, S(O)R_{c22}, S(O)₂R_{c23}, S(O)₂OR_{c24}, OS(O)₂R_{c25}, optionally substituted C₅₋₂₀ cycloalkyl, or optionally substituted C₅₋₂₀ heterocyclyl,
   wherein R_{c8} and R_{c20} each independently represent H or C₁₋₃ alkyl;
   R_{c4}, R_{c6}, and R_{c18} each independently represent H, optionally substituted linear or branched C₁₋₁₀ alkyl, optionally substituted C₃₋₂₀ cycloalkyl, optionally substituted C₅₋₂₀ aryl, optionally substituted C₃₋₂₀ heterocyclyl or optionally substituted C₅₋₂₀ heteroaryl; and
   R_{c5}, R_{c7}, R_{c9}, R_{c13}, R_{c14}, R_{c15}, R_{c16}, R_{c17}, R_{c19}, R_{c21}, R_{c22}, R_{c23}, R_{c24}, and R_{c25} each independently represent optionally substituted C₅₋₂₀ cycloalkyl or optionally substituted C₅₋₂₀ heterocyclyl.

In one embodiment of the present disclosure, X represents N(Rₐ₁), O or S, where Rₐ₁ represents H or C₁₋₃ alkyl; and
L represents linear or branched C₁₋₃ alkylene optionally substituted by a substituent selected from the group consisting of halogen, hydroxyl, amino or any combination thereof; and
R represents:
   NRₐ₇Rₐ₈, wherein Rₐ₇ is H or C₁₋₃ alkyl, and Rₐ₈ is phenyl optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; or
   benzo[d][1,3]dioxolyl, tetrahydropyranyl, pyridyl or phenyl, said benzo[d][1,3]dioxolyl, tetrahydropyranyl, pyridyl and phenyl are each independently optionally substituted by a substituent selected from the group consisting of halogen, hydroxyl, mercapto, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)- or any combination thereof.

In one embodiment of the present disclosure, X represents N(Rₐ₁), O or S, where Rₐ₁ represents H or C₁₋₃ alkyl; and
L represents Formula -Lₐ₂₋Lₐ₁₋-^{∗}, where Lₐ₁ represents linear or branched C₁₋₄₀ alkylene optionally substituted by a substituent selected from the group consisting of halogen, hydroxyl, amino or any combination thereof, Lₐ₂ represents optionally substituted C₃₋₁₅ cycloalkylene or optionally substituted C₅₋₁₅ arylene, wherein said C₃₋₁₅ cycloalkylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)- or any combination thereof, and said C₅₋₁₅ arylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof, and symbol ^{∗} indicates the point of attachment to X; and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, or OS(O)₂Rₐ₂₈,
   wherein Rₐ₁₆ and Rₐ₁₇ each independently represent H, optionally substituted C₃₋₂₀ cycloalkyl, or optionally substituted C₃₋₂₀ heterocyclyl;
   Rₐ₉, Rₐ₁₁, Rₐ₂₁, and Rₐ₂₃ each independently represent H or C₁₋₃ alkyl; and
   Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl.

In one embodiment of the present disclosure, X represents N(Rₐ₁), O or S, where Rₐ₁ represents H or C₁₋₃ alkyl; and
L represents linear or branched C₂₋₄₀ alkylene interrupted one or more times (such as 1-10, 1-8, 1-6, 1-5, 1-3, 1-2 times, or once, which can vary as required) by one or more (such as 1-10, 1-8, 1-6, 1-5, 1-4, 1-3, 1-2, or 1, which can vary as required) groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and each C₅₋₁₅ arylene and each C₅₋₁₅ heteroarylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)- or any combination thereof;
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted C₃₋₂₀ cycloalkyl, or optionally substituted C₃₋₂₀ heterocyclyl,
   wherein Rₐ₇, Rₐ₈, Rₐ₉, Rₐ₁₀, Rₐ₁₆, Rₐ₁₇, Rₐ₂₁ and Rₐ₂₂ are the same or different and each independently represent H, optionally substituted linear or branched C₁₋₁₀ alkyl, optionally substituted C₃₋₂₀ cycloalkyl or optionally substituted C₃₋₂₀ heterocyclyl;
   Rₐ₁₁ and Rₐ₂₃ each independently represent H or C₁₋₃ alkyl; and
   Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
   wherein said linear or branched C₁₋₁₀ alkyl is optionally substituted by one or more (such as 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof;
   each C₃₋₂₀ cycloalkyl and each C₃₋₂₀ heterocyclyl is independently optionally substituted by one or more (such as 1-5, 1-4, 1-3, 1-2, or 1, which can vary as required) substituents selected from the group consisting of halogen, hydroxyl, mercapto, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and
L represents:
   linear or branched C₂₋₄₀ alkylene interrupted one or more times (such as 1-10, 1-8, 1-6, 1-5, 1-3, 1-2 times, or once, which can vary as required) by one or more (such as 1-10, 1-9, 1-8, 1-7, 1-6, 1-5, 1-4, 1-3, 1-2, or 1, which can vary as required) groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and wherein said C₅₋₁₅ arylene and C₅₋₁₅ heteroarylene are each independently optionally substituted by one or more (such as 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)- or any combination thereof; or
   Formula -L_{b2}-L_{b1}-#, where L_{b1} represents optionally substituted C₅₋₁₅ arylene or optionally substituted C₅₋₁₅ heteroarylene, wherein said C₅₋₁₅ arylene and C₅₋₁₅ heteroarylene are each independently optionally substituted by one or more (such as 1-3) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; L_{b2} represents linear or branched C₁₋₄₀ alkylene optionally substituted by a substituent selected from the group consisting of halogen, hydroxyl, amino or any combination thereof, and symbol # indicates the point of attachment to X; and
R represents NR_{b7}R_{b8}, C(O)NR_{b9}R_{b10}, N(R_{b11})C(O)R_{b12}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, optionally substituted C₃₋₂₀ cycloalkyl or optionally substituted C₃₋₂₀ heterocyclyl,
   wherein R_{b7}, R_{b8}, R_{b9}, R_{b10}, Rₐ₂₁ and R_{b22} are the same or different and each independently represent H, optionally substituted linear or branched C₁₋₁₀ alkyl, optionally substituted C₃₋₂₀ cycloalkyl or optionally substituted C₃₋₂₀ heterocyclyl;
   R_{b16} and R_{b17} each independently represent H, optionally substituted linear or branched C₁₋₁₀ alkyl, optionally substituted C₃₋₂₀ cycloalkyl or optionally substituted C₃₋₂₀ heterocyclyl; R_{b11} and R_{b23} each independently represent H or C₁₋₃ alkyl; and
   R_{b12}, R_{b18}, R_{b19}, R_{b20}, R_{b24}, R_{b25}, R_{b26}, R_{b27}, and R_{b28} each independently represent optionally substituted linear or branched C₁₋₁₀ alkyl, optionally substituted C₃₋₂₀ cycloalkyl or optionally substituted C₃₋₂₀ heterocyclyl; and
   wherein said linear or branched C₁₋₁₀ alkyl is optionally substituted by one or more (such as 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof;
   each C₃₋₂₀ cycloalkyl and each C₃₋₂₀ heterocyclyl is each independently optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents a bond; and
L represents linear or branched C₂₋₄₀ alkylene interrupted one or more times (such as 1-10, 1-8, 1-6, 1-5, 1-3, 1-2 times, or once, which can vary as required) by one or more (such as 1-10, 1-9, 1-8, 1-7, 1-6, 1-5, 1-4, 1-3, 1-2, or 1, which can vary as required) groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and said C₅₋₁₅ arylene and C₅₋₁₅ heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)- or any combination thereof; and
R represents NR_{c4}R_{c5}, C(O)NR_{c6}R_{c7}, N(R_{c8})C(O)R_{c9}, OR_{c13}, SR_{c14}, C(O)R_{c15}, OC(O)R_{c16}, C(O)OR_{c17}, S(O)₂NR_{c18}R_{c19}, N(R_{c20})S(O)₂R_{c21}, S(O)R_{c22}, S(O)₂R_{c23}, S(O)₂OR_{c24}, OS(O)₂R_{c25}, optionally substituted C₅₋₂₀ cycloalkyl, or optionally substituted C₅₋₂₀ heterocyclyl,
   wherein R_{c8} and R_{c20} each independently represent H or C₁₋₃ alkyl;
   R_{c4}, R_{c6}, and R_{c18} each independently represent H, optionally substituted linear or branched C₁₋₁₀ alkyl, optionally substituted C₃₋₂₀ cycloalkyl, optionally substituted C₅₋₂₀ aryl, optionally substituted C₃₋₂₀ heterocyclyl or optionally substituted C₅₋₂₀ heteroaryl; and
   R_{c5}, R_{c7}, R_{c9}, R_{c13}, R_{c14}, R_{c15}, R_{c16}, R_{c17}, R_{c19}, R_{c21}, R_{c22}, R_{c23}, R_{c24}, and R_{c25} each independently represent optionally substituted C₅₋₂₀ cycloalkyl or optionally substituted C₅₋₂₀ heterocyclyl;
   wherein said linear or branched C₁₋₁₀ alkyl is optionally substituted by one or more (such as 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof;
   each C₃₋₂₀ cycloalkyl and each C₃₋₂₀ heterocyclyl is independently optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof; and
   each C₅₋₂₀ aryl and each C₅₋₂₀ heteroaryl is independently optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)-, optionally substituted C₆₋₂₀ aryl or any combination thereof, wherein said C₆₋₂₀ aryl is optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, or any combination thereof.

In one embodiment of the present disclosure, X represents N(Rₐ₁), O or S, where Rₐ₁ represents H or C₁₋₃ alkyl; and L represents the following groups optionally substituted by a substituent selected from the group consisting of halogen, hydroxyl, amino or any combination thereof:
-CH₂-; -(CH₂)₂-; -CH₂-CH(CH₃)-; or -(CH₂)₃-;
R represents:
NRₐ₇Rₐ₈, wherein Rₐ₇ is H or C₁₋₃ alkyl, and Rₐ₈ is phenyl optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; or
benzo[d][1,3]dioxolyl, tetrahydropyranyl, pyridyl or phenyl, said benzo[d][1,3]dioxolyl, tetrahydropyranyl, pyridyl and phenyl are each independently optionally substituted by a substituent selected from the group consisting of halogen, hydroxyl, mercapto, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)- or any combination thereof.

In one embodiment of the present disclosure, X represents N(Rₐ₁), O or S, where Rₐ₁ represents H or C₁₋₃ alkyl; and
L represents the group represented by the following formula:
   -(CH₂)ₙ₁-C₅₋₁₅ arylene-(CH₂)ₙ₂-^{∗∗}, or -(CH₂)ₙ₁-C₅₋₁₅ heteroarylene-(CH₂)ₙ₂-^{∗∗}, wherein symbol ^{∗∗} indicates the point of attachment to X;
   wherein n1 and n2 each independently represent an integer of 1, 2, 3, 4, 5, 6, 7, 8, 9, 10, 11, 12, 13, 14, 15, 16, 17, 18, 19, or 20; and
   said C₅₋₁₅ arylene and C₅₋₁₅ heteroarylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof (in a sub-embodiment, said C₅₋₁₅ heteroarylene includes, but is not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzo[d][1,3]dioxolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene and imidazo[2,1-b]thiazolylene. In a sub-embodiment, said C₅₋₁₅ arylene includes, but is not limited to, phenylene and naphthylene); and
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted C₃₋₂₀ cycloalkyl, or optionally substituted C₃₋₂₀ heterocyclyl,
   wherein Rₐ₇, Rₐ₈, Rₐ₉, Rₐ₁₀, Rₐ₁₆, Rₐ₁₇, Rₐ₂₁ and Rₐ₂₂ are the same or different and each independently represent H, optionally substituted linear or branched C₁₋₁₀ alkyl, optionally substituted C₃₋₂₀ cycloalkyl or optionally substituted C₃₋₂₀ heterocyclyl;
   Rₐ₁₁ and Rₐ₂₃ each independently represent H or C₁₋₃ alkyl; and
   Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
   wherein said linear or branched C₁₋₁₀ alkyl is optionally substituted by one or more (such as 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof; and
   each C₃₋₂₀ cycloalkyl and each C₃₋₂₀ heterocyclyl is independently optionally substituted by one or more (such as 1-5, 1-4, 1-3, 1-2, or 1, which can vary as required) substituents selected from the group consisting of halogen, hydroxyl, mercapto, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents N(Rₐ₁), O or S, where Rₐ₁ represents H or C₁₋₃ alkyl; and
L represents:
   -CH₂-phenylene-CH₂-^{∗∗}, -CH₂-phenylene-(CH₂)₂-^{∗∗}, -CH₂-phenylene-(CH₂)₃-^{∗∗}, -CH₂-phenylene-(CH₂)₄-^{∗∗}, -CH₂-phenylene-(CH₂)₅-^{∗∗}, -CH₂-phenylene-(CH₂)₆-^{∗∗}, -CH₂-phenylene-(CH₂)₇-^{∗∗}, -CH₂-phenylene-(CH₂)₈-^{∗∗}, -(CH₂)₂-phenylene-CH₂-^{∗∗}, -(CH₂)₂-phenylene-(CH₂)₂-^{∗∗}, -(CH₂)₂-phenylene-(CH₂)₃-^{∗∗}, -(CH₂)₂-phenylene-(CH₂)₄-^{∗∗}, -(CH₂)₂-phenylene-(CH₂)₅-^{∗∗}, -(CH₂)₂-phenylene-(CH₂)₆-^{∗∗}, -(CH₂)₂-phenylene-(CH₂)₇-^{∗∗}, -(CH₂)₂-phenylene-(CH₂)₈-^{∗∗}, -(CH₂)₃-phenylene-CH₂-^{∗∗}, -(CH₂)₃-phenylene-(CH₂)₂-^{∗∗}, -(CH₂)₃-phenylene-(CH₂)₃-^{∗∗}, -(CH₂)₃-phenylene-(CH₂)₄-^{∗∗}, -(CH₂)₃-phenylene-(CH₂)₅-^{∗∗}, -(CH₂)₃-phenylene-(CH₂)₆-^{∗∗}, -(CH₂)₃-phenylene-(CH₂)₇-^{∗∗}, -(CH₂)₃-phenylene-(CH₂)₈-^{∗∗}, -(CH₂)₄-phenylene-CH₂-^{∗∗}, -(CH₂)₄-phenylene-(CH₂)₂-^{∗∗}, -(CH₂)₄-phenylene-(CH₂)₃-^{∗∗}, -(CH₂)₄-phenylene-(CH₂)₄-^{∗∗}, -(CH₂)₄-phenylene-(CH₂)₃-^{∗∗}, -(CH₂)₄-phenylene-(CH₂)₆-^{∗∗}, -(CH₂)₄-phenylene-(CH₂)₇-^{∗∗}, -(CH₂)₄-phenylene-(CH₂)₈-^{∗∗}, -(CH₂)₅-phenylene-CH₂-^{∗∗}, -(CH₂)₅-phenylene-(CH₂)₂-^{∗∗}, -(CH₂)₅-phenylene-(CH₂)₃-^{∗∗}, -(CH₂)₅-phenylene-(CH₂)₄-^{∗∗}, -(CH₂)₅-phenylene-(CH₂)₅-^{∗∗}, -(CH₂)₆-phenylene-CH₂-^{∗∗}, -(CH₂)₆-phenylene-(CH₂)₂-^{∗∗}, -(CH₂)₆-phenylene-(CH₂)₃-^{∗∗}, -(CH₂)₆-phenylene-(CH₂)₄-^{∗∗}, -(CH₂)₆-phenylene-(CH₂)₅-^{∗∗}, -(CH₂)₆-phenylene-(CH₂)₆-^{∗∗}, -(CH₂)₇-phenylene-CH₂-^{∗∗}, -(CH₂)₇-phenylene-(CH₂)₂-^{∗∗}, -(CH₂)₇-phenylene-(CH₂)₃-^{∗∗}, -(CH₂)₇-phenylene-(CH₂)₄-^{∗∗}, -(CH₂)₇-phenylene-(CH₂)₅-^{∗∗}, -(CH₂)₇-phenylene-(CH₂)₆-^{∗∗}, -(CH₂)₈-phenylene-CH₂-^{∗∗}, -(CH₂)₈-phenylene-(CH₂)₂-^{∗∗}, -(CH₂)₈-phenylene-(CH₂)₃-^{∗∗}, -(CH₂)₈-phenylene-(CH₂)₄-^{∗∗}, -(CH₂)₈-phenylene-(CH₂)₅-^{∗∗}, -(CH₂)₈-phenylene-(CH₂)₆-^{∗∗}, -(CH₂)₈-phenylene-(CH₂)₇-^{∗∗}, -(CH₂)₈-phenylene-(CH₂)₈-^{∗∗}, -CH₂-pyridylene-CH₂-^{∗∗}, -CH₂-pyridylene-(CH₂)₂-^{∗∗}, -CH₂-pyridylene-(CH₂)₃-^{∗∗}, -CH₂-pyridylene-(CH₂)₄-^{∗∗}, -CH₂-pyridylene-(CH₂)₅-^{∗∗}, -CH₂-pyridylene-(CH₂)₆-^{∗∗}, -CH₂-pyridylene-(CH₂)₇-^{∗∗}, -CH₂-pyridylene-(CH₂)₈-^{∗∗}, -(CH₂)₂-pyridylene-CH₂-^{∗∗}, -(CH₂)₂-pyridylene-(CH₂)₂-^{∗∗}, -(CH₂)₂-pyridylene-(CH₂)₃-^{∗∗}, -(CH₂)₂-pyridylene-(CH₂)₄-^{∗∗}, -(CH₂)₂-pyridylene-(CH₂)₅-^{∗∗}, -(CH₂)₂-pyridylene-(CH₂)₆-^{∗∗}, -(CH₂)₂-pyridylene-(CH₂)₇-^{∗∗}, -(CH₂)₂-pyridylene-(CH₂)₈-^{∗∗}, -(CH₂)₃-pyridylene-CH₂-^{∗∗}, -(CH₂)₃-pyridylene-(CH₂)₂-^{∗∗}, -(CH₂)₃-pyridylene-(CH₂)₃-^{∗∗}, -(CH₂)₃-pyridylene-(CH₂)₄-^{∗∗}, -(CH₂)₃-pyridylene-(CH₂)₅-^{∗∗}, -(CH₂)₃-pyridylene-(CH₂)₆-^{∗∗}, -(CH₂)₃-pyridylene-(CH₂)₇-^{∗∗}, -(CH₂)₃-pyridylene-(CH₂)₈-^{∗∗}, -(CH₂)₄-pyridylene-CH₂-^{∗∗}, -(CH₂)₄-pyridylene-(CH₂)₂-^{∗∗}, -(CH₂)₄-pyridylene-(CH₂)₃-^{∗∗}, -(CH₂)₄-pyridylene-(CH₂)₄-^{∗∗}, -(CH₂)₄-pyridylene-(CH₂)₅-^{∗∗}, -(CH₂)₄-pyridylene-(CH₂)₆-^{∗∗}, -(CH₂)₄-pyridylene-(CH₂)₇-^{∗∗}, -(CH₂)₄-pyridylene-(CH₂)₈-^{∗∗}, -(CH₂)₅-pyridylene-CH₂-^{∗∗}, -(CH₂)₅-pyridylene-(CH₂)₂-^{∗∗}, -(CH₂)₅-pyridylene-(CH₂)₃-^{∗∗}, -(CH₂)₅-pyridylene-(CH₂)₄-^{∗∗}, -(CH₂)₅-pyridylene-(CH₂)₅-^{∗∗}, -(CH₂)₆-pyridylene-CH₂-^{∗∗}, -(CH₂)₆-pyridylene-(CH₂)₂-^{∗∗}, -(CH₂)₆-pyridylene-(CH₂)₃-^{∗∗}, -(CH₂)₆-pyridylene-(CH₂)₄-^{∗∗}, -(CH₂)₆-pyridylene-(CH₂)₅-^{∗∗}, -(CH₂)₆-pyridylene-(CH₂)₆-^{∗∗}, -(CH₂)₇-pyridylene-CH₂-^{∗∗}, -(CH₂)₇-pyridylene-(CH₂)₂-^{∗∗}, -(CH₂)₇-pyridylene-(CH₂)₃-^{∗∗}, -(CH₂)₇-pyridylene-(CH₂)₄-^{∗∗}, -(CH₂)₇-pyridylene-(CH₂)₅-^{∗∗}, -(CH₂)₇-pyridylene-(CH₂)₆-^{∗∗}, -(CH₂)₈-pyridylene-CH₂-^{∗∗}, -(CH₂)₈-pyridylene-(CH₂)₂-^{∗∗}, -(CH₂)₈-pyridylene-(CH₂)₃-^{∗∗}, -(CH₂)₈-pyridylene-(CH₂)₄-^{∗∗}, -(CH₂)₈-pyridylene-(CH₂)₅-^{∗∗}, -(CH₂)₈-pyridylene-(CH₂)₆-^{∗∗}, -(CH₂)₈-pyridylene-(CH₂)₇-^{∗∗}, -(CH₂)₈-pyridylene-(CH₂)₈-^{∗∗}, -CH₂-thiazolylene-CH₂-^{∗∗}, -CH₂-thiazolylene-(CH₂)₂-^{∗∗}, -CH₂-thiazolylene-(CH₂)₃-^{∗∗}, -CH₂-thiazolylene-(CH₂)₄-^{∗∗}, -CH₂-thiazolylene-(CH₂)₅-^{∗∗}, -CH₂-thiazolylene-(CH₂)₆-^{∗∗}, -CH₂-thiazolylene-(CH₂)₇-^{∗∗}, -CH₂-thiazolylene-(CH₂)₈-^{∗∗}, -(CH₂)₂-thiazolylene-CH₂-^{∗∗}, -(CH₂)₂-thiazolylene-(CH₂)₂-^{∗∗}, -(CH₂)₂-thiazolylene-(CH₂)₃-^{∗∗}, -(CH₂)₂-thiazolylene-(CH₂)₄-^{∗∗}, -(CH₂)₂-thiazolylene-(CH₂)₅-^{∗∗},-(CH₂)₂-thiazolylene-(CH₂)₆-^{∗∗}, -(CH₂)₂-thiazolylene-(CH₂)₇-^{∗∗}, -(CH₂)₂-thiazolylene-(CH₂)₈-^{∗∗}, -(CH₂)₃-thiazolylene-CH₂-^{∗∗}, -(CH₂)₃-thiazolylene-(CH₂)₂-^{∗∗}, -(CH₂)₃-thiazolylene-(CH₂)₃-^{∗∗}, -(CH₂)₃-thiazolylene-(CH₂)₄-^{∗∗}, -(CH₂)₃-thiazolylene-(CH₂)₅-^{∗∗}, -(CH₂)₃-thiazolylene-(CH₂)₆-^{∗∗}, -(CH₂)₃-thiazolylene-(CH₂)₇-^{∗∗}, -(CH₂)₃-thiazolylene-(CH₂)₈-^{∗∗}, -(CH₂)₄-thiazolylene-CH₂-^{∗∗}, -(CH₂)₄-thiazolylene-(CH₂)₂-^{∗∗}, -(CH₂)₄-thiazolylene-(CH₂)₃-^{∗∗}, -(CH₂)₄-thiazolylene-(CH₂)₄-^{∗∗}, -(CH₂)₄-thiazolylene-(CH₂)₅-^{∗∗}, -(CH₂)₄-thiazolylene-(CH₂)₆-^{∗∗}, -(CH₂)₄-thiazolylene-(CH₂)₇-^{∗∗}, -(CH₂)₄-thiazolylene-(CH₂)₈-^{∗∗}, -(CH₂)₅-thiazolylene-CH₂-^{∗∗}, -(CH₂)₅-thiazolylene-(CH₂)₂-^{∗∗}, -(CH₂)₅-thiazolylene-(CH₂)₃-^{∗∗}, -(CH₂)₅-thiazolylene-(CH₂)₄-^{∗∗},-(CH₂)₅-thiazolylene-(CH₂)₅-^{∗∗}, -(CH₂)₆-thiazolylene-CH₂-^{∗∗}, -(CH₂)₆-thiazolylene-(CH₂)₂-^{∗∗},-(CH₂)₆-thiazolylene-(CH₂)₃-^{∗∗}, -(CH₂)₆-thiazolylene-(CH₂)₄-^{∗∗}, -(CH₂)₆-thiazolylene-(CH₂)₅-^{∗∗}, -(CH₂)₆-thiazolylene-(CH₂)₆-^{∗∗}, -(CH₂)₇-thiazolylene-CH₂-^{∗∗}, -(CH₂)₇-thiazolylene-(CH₂)₂-^{∗∗}, -(CH₂)₇-thiazolylene-(CH₂)₃-^{∗∗}, -(CH₂)₇-thiazolylene-(CH₂)₄-^{∗∗}, -(CH₂)₇-thiazolylene-(CH₂)₅-^{∗∗}, -(CH₂)₇-thiazolylene-(CH₂)₆-^{∗∗}, -(CH₂)₈-thiazolylene-CH₂-^{∗∗}, -(CH₂)₈-thiazolylene-(CH₂)₂-^{∗∗}, -(CH₂)₈-thiazolylene-(CH₂)₃-^{∗∗}, -(CH₂)₈-thiazolylene-(CH₂)₄-^{∗∗}, -(CH₂)₈-thiazolylene-(CH₂)₅-^{∗∗}, -(CH₂)₈-thiazolylene-(CH₂)₆-^{∗∗}, -(CH₂)₈-thiazolylene-(CH₂)₇-^{∗∗}, or -(CH₂)₈-thiazolylene-(CH₂)₈-^{∗∗}, wherein symbol ** indicates the point of attachment to X;
   wherein said phenylene, pyridylene, and thiazolylene are each independently optionally substituted by one or more (such as 1-4, 1-3, 1-2, or 1) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof, and
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted C₃₋₂₀ cycloalkyl, or optionally substituted C₃₋₂₀ heterocyclyl;
   wherein Rₐ₇, Rₐ₈, Rₐ₉, Rₐ₁₀, Rₐ₁₆, Rₐ₁₇, Rₐ₂₁ and Rₐ₂₂ are the same or different and each independently represent H, optionally substituted linear or branched C₁₋₁₀ alkyl, optionally substituted C₃₋₂₀ cycloalkyl or optionally substituted C₃₋₂₀ heterocyclyl;
   Rₐ₁₁ and Rₐ₂₃ each independently represent H or C₁₋₃ alkyl; and
   Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
   wherein said linear or branched C₁₋₁₀ alkyl is optionally substituted by one or more (such as 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof; and
   each C₃₋₂₀ cycloalkyl and each C₃₋₂₀ heterocyclyl is independently optionally substituted by one or more (such as 1-5, 1-4, 1-3, 1-2, or 1, which can vary as required) substituents selected from the group consisting of halogen, hydroxyl, mercapto, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents N(Rₐ₁), O or S, where Rₐ₁ represents H or C₁₋₃ alkyl; and L represents: 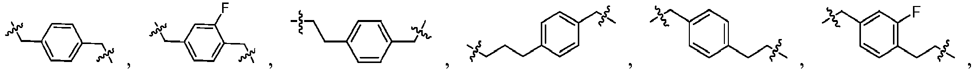 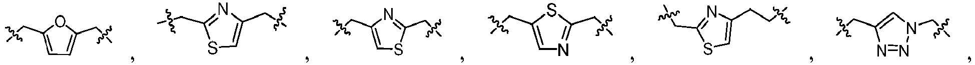 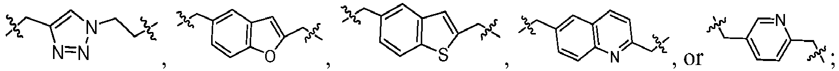 and R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted C₃₋₂₀ cycloalkyl, or optionally substituted C₃₋₂₀ heterocyclyl,
wherein Rₐ₇, Rₐ₈, Rₐ₉, Rₐ₁₀, Rₐ₁₆, Rₐ₁₇, Rₐ₂₁ and Rₐ₂₂ are the same or different and each independently represent H, optionally substituted linear or branched C₁₋₁₀ alkyl, optionally substituted C₃₋₂₀ cycloalkyl or optionally substituted C₃₋₂₀ heterocyclyl;
Rₐ₁₁ and Rₐ₂₃ each independently represent H or C₁₋₃ alkyl; and
Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
wherein said linear or branched C₁₋₁₀ alkyl is optionally substituted by one or more (such as 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof; and
each C₃₋₂₀ cycloalkyl and each C₃₋₂₀ heterocyclyl is independently optionally substituted by one or more (such as 1-5, 1-4, 1-3, 1-2, or 1, which can vary as required) substituents selected from the group consisting of halogen, hydroxyl, mercapto, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents N(Rₐ₁), O or S, where Rₐ₁ represents H or C₁₋₃ alkyl; and
L represents the group represented by the following formula:
   -C₃₋₁₅cycloalkylene-(CH₂)₁₋₄₀-^{∗}, or -C₅₋₁₅ arylene-(CH₂)₁₋₄₀-^{∗}, wherein symbol ^{∗} indicates the point of attachment to X; and
   wherein said C₃₋₁₅cycloalkylene is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, or any combination thereof, and said C₅₋₁₅ arylene is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof (in a sub-embodiment, said C₅₋₁₅ arylene includes, but is not limited to, phenylene and naphthylene. In a sub-embodiment, said C₃₋₁₅cycloalkylene includes, but is not limited to, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene, adamantanylene, noradamantanylene, bornylene, bicyclo[2.2.1]heptylene, and bicyclo[2.2.1]heptenylene); and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, or OS(O)₂Rₐ₂₈,
   wherein Rₐ₁₆ and Rₐ₁₇ are each independently represent H, optionally substituted C₃₋₂₀ cycloalkyl or optionally substituted C₃₋₂₀ heterocyclyl;
   Rₐ₉, Rₐ₁₁, Rₐ₂₁ and Rₐ₂₃ each independently represent H or C₁₋₃ alkyl; and
   Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl.

In one embodiment of the present disclosure, X represents N(Rₐ₁), O or S, where Rₐ₁ represents H or C₁₋₃ alkyl; and L represents:
-cyclohexylene-CH₂-^{∗}, -cyclohexylene-(CH₂)₂-^{∗}, -cyclohexylene-(CH₂)₃-^{∗}, -cyclohexylene-(CH₂)₄-^{∗}, -cyclohexylene-(CH₂)₃-^{∗}, -cyclohexylene-(CH₂)₆-^{∗}, -cyclohexylene-(CH₂)₇-^{∗}, -cyclohexylene-(CH₂)₈-^{∗}, -cyclohexylene-(CH₂)₉-^{∗}, -cyclohexylene-(CH₂)₁₀-^{∗}, -cyclohexylene-(CH₂)₁₁-^{∗},-cyclohexylene-(CH₂)₁₂-^{∗}, -phenylene-CH₂-^{∗}, -phenylene-(CH₂)₂-^{∗}, -phenylene-(CH₂)₃-^{∗},-phenylene-(CH₂)₄-^{∗}, -phenylene-(CH₂)₅-^{∗}, -phenylene-(CH₂)₆-^{∗}, -phenylene-(CH₂)₇-^{∗},-phenylene-(CH₂)₈-^{∗}, -phenylene-(CH₂)₉-^{∗}, -phenylene-(CH₂)₁₀-^{∗}, -phenylene-(CH₂)₁₁-^{∗}, or-phenylene-(CH₂)₁₂-^{∗}, wherein symbol * indicates the point of attachment to X; and
   wherein said cyclohexylene is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, or any combination thereof; and said phenylene is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof; and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₃, S(O)₂Rₐ₂₆, S(O)₂ORₐ₂₇, or OS(O)₂Rₐ₂₈,
   wherein Rₐ₁₆ and Rₐ₁₇ are each independently represent H, optionally substituted C₃₋₂₀ cycloalkyl or optionally substituted C₃₋₂₀ heterocyclyl;
   Rₐ₉, Rₐ₁₁, Rₐ₂₁ and Rₐ₂₃ each independently represent H or C₁₋₃ alkyl; and
   Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl.

In one embodiment of the present disclosure, X represents N(Rₐ₁), O or S, where Rₐ₁ represents H or C₁₋₃ alkyl; and L represents: 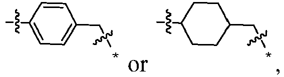 wherein symbol * indicates the point of attachment to X; and R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, or OS(O)₂Rₐ₂₈,
wherein Rₐ₁₆ and Rₐ₁₇ are each independently represent H, optionally substituted C₃₋₂₀ cycloalkyl or optionally substituted C₃₋₂₀ heterocyclyl;
Rₐ₉, Rₐ₁₁, Rₐ₂₁ and Rₐ₂₃ each independently represent H or C₁₋₃ alkyl; and
Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and
L represents:
   linear or branched C₂₋₄₀ alkylene interrupted one or more times (such as 1-10, 1-8, 1-6, 1-5, 1-3, 1-2 times, or once, which can vary as required) by one or more (such as 1-10, 1-9, 1-8, 1-7, 1-6, 1-5, 1-4, 1-3, 1-2, or 1, which can vary as required) groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and wherein said C₅₋₁₅ arylene and C₅₋₁₅ heteroarylene are each independently optionally substituted by one or more (such as 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)- or any combination thereof; and
R represents NR_{b7}R_{b8}, C(O)NR_{b9}R_{b10}, N(R_{b11})C(O)R_{b12}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, optionally substituted C₃₋₂₀ cycloalkyl or optionally substituted C₃₋₂₀ heterocyclyl,
   wherein R_{b7}, R_{b8}, R_{b9}, R_{b10}, Rₐ₂₁ and R_{b22} are the same or different and each independently represent H, optionally substituted linear or branched C₁₋₁₀ alkyl, optionally substituted C₃₋₂₀ cycloalkyl or optionally substituted C₃₋₂₀ heterocyclyl;
   R_{b6} and R_{b17} each independently represent H, optionally substituted linear or branched C₁₋₁₀ alkyl, optionally substituted C₃₋₂₀ cycloalkyl or optionally substituted C₃₋₂₀ heterocyclyl;
   R_{b11} and R_{b23} each independently represent H or C₁₋₃ alkyl; and
   R_{b12}, R_{b18}, R_{b19}, R_{b20}, R_{b24}, R_{b25}, R_{b26}, R_{b27}, and R_{b28} each independently represent optionally substituted linear or branched C₁₋₁₀ alkyl, optionally substituted C₃₋₂₀ cycloalkyl or optionally substituted C₃₋₂₀ heterocyclyl; and
   wherein said linear or branched C₁₋₁₀ alkyl is optionally substituted by one or more (such as 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof;
   each C₃₋₂₀ cycloalkyl and each C₃₋₂₀ heterocyclyl is each independently optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and
L represents:
   Formula -L_{b2}-L_{b1}-#, where L_{b1} represents optionally substituted C₅₋₁₅ arylene or optionally substituted C₅₋₁₅ heteroarylene, wherein said C₅₋₁₅ arylene and C₅₋₁₅ heteroarylene are each independently optionally substituted by one or more (such as 1-3) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; L_{b2} represents linear or branched C₁₋₄₀ alkylene optionally substituted by a substituent selected from the group consisting of halogen, hydroxyl, amino or any combination thereof, and symbol # indicates the point of attachment to X; and
R represents NR_{b7}R_{b8}, C(O)NR_{b9}R_{b10}, N(R_{b11})C(O)R_{b12}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, optionally substituted C₃₋₂₀ cycloalkyl or optionally substituted C₃₋₂₀ heterocyclyl,
   wherein R_{b7}, R_{b8}, R_{b9}, R_{b10}, Rₐ₂₁ and R_{b22} are the same or different and each independently represent H, optionally substituted linear or branched C₁₋₁₀ alkyl, optionally substituted C₃₋₂₀ cycloalkyl or optionally substituted C₃₋₂₀ heterocyclyl;
   R_{b16} and R_{b17} each independently represent H, optionally substituted linear or branched C₁₋₁₀ alkyl, optionally substituted C₃₋₂₀ cycloalkyl or optionally substituted C₃₋₂₀ heterocyclyl;
   R_{b11} and R_{b23} each independently represent H or C₁₋₃ alkyl; and
   R_{b12}, R_{b18}, R_{b19}, R_{b20}, R_{b24}, R_{b25}, R_{b26}, R_{b27}, and R_{b28} each independently represent optionally substituted linear or branched C₁₋₁₀ alkyl, optionally substituted C₃₋₂₀ cycloalkyl or optionally substituted C₃₋₂₀ heterocyclyl; and
   wherein said linear or branched C₁₋₁₀ alkyl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof;
   each C₃₋₂₀ cycloalkyl and each C₃₋₂₀ heterocyclyl is each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and
L represents the group represented by the following formula:
-(CH₂)ₙ₁-C₅₋₁₅arylene-(CH₂)ₙ₂-^{∗∗}, or -(CH₂)ₙ₁-C₅₋₁₅heteroarylene-(CH₂)ₙ₂-^{∗∗}, wherein symbol ^{∗∗} indicates the point of attachment to X;
wherein n1 and n2 each independently represent an integer of 1, 2, 3, 4, 5, 6, 7, 8, 9, 10, 11, 12, 13, 14, 15, 16, 17, 18, 19, or 20; and
said C₅₋₁₅ arylene and C₅₋₁₅ heteroarylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof. In a sub-embodiment, said C₅₋₁₅ heteroarylene includes, but is not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzo[d][1,3]dioxolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene and imidazo[2,1-b]thiazolylene. In a sub-embodiment, said C₅₋₁₅ arylene includes, but is not limited to, phenylene and naphthylene.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and L represents:
-CH₂-phenylene-CH₂-^{∗∗}, -CH₂-phenylene-(CH₂)₂-^{∗∗}, -CH₂-phenylene-(CH₂)₃-^{∗∗}, -CH₂-phenylene-(CH₂)₄-^{∗∗}, -CH₂-phenylene-(CH₂)₅-^{∗∗}, -CH₂-phenylene-(CH₂)₆-^{∗∗}, -CH₂-phenylene-(CH₂)₇-^{∗∗}, -CH₂-phenylene-(CH₂)₈-^{∗∗}, -(CH₂)₂-phenylene-CH₂-^{∗∗}, -(CH₂)₂-phenylene-(CH₂)₂-^{∗∗}, -(CH₂)₂-phenylene-(CH₂)₃-^{∗∗}, -(CH₂)₂-phenylene-(CH₂)₄-^{∗∗}, -(CH₂)₂-phenylene-(CH₂)₅-^{∗∗}, -(CH₂)₂-phenylene-(CH₂)₆-^{∗∗}, -(CH₂)₂-phenylene-(CH₂)₇-^{∗∗}, -(CH₂)₂-phenylene-(CH₂)₈-^{∗∗}, -(CH₂)₃-phenylene-CH₂-^{∗∗}, -(CH₂)₃-phenylene-(CH₂)₂-^{∗∗}, -(CH₂)₃-phenylene-(CH₂)₃-^{∗∗}, -(CH₂)₃-phenylene-(CH₂)₄-^{∗∗}, -(CH₂)₃-phenylene-(CH₂)₅-^{∗∗}, -(CH₂)₃-phenylene-(CH₂)₆-^{∗∗}, -(CH₂)₃-phenylene-(CH₂)₇-^{∗∗}, -(CH₂)₃-phenylene-(CH₂)₈-^{∗∗}, -(CH₂)₄-phenylene-CH₂-^{∗∗}, -(CH₂)₄-phenylene-(CH₂)₂-^{∗∗}, -(CH₂)₄-phenylene-(CH₂)₃-^{∗∗}, -(CH₂)₄-phenylene-(CH₂)₄-^{∗∗}, -(CH₂)₄-phenylene-(CH₂)₅-^{∗∗}, -(CH₂)₄-phenylene-(CH₂)₆-^{∗∗}, -(CH₂)₄-phenylene-(CH₂)₇-^{∗∗}, -(CH₂)₄-phenylene-(CH₂)₈-^{∗∗}, -(CH₂)₅-phenylene-CH₂-^{∗∗}, -(CH₂)₅-phenylene-(CH₂)₂-^{∗∗}, -(CH₂)₅-phenylene-(CH₂)₃-^{∗∗}, -(CH₂)₅-phenylene-(CH₂)₄-^{∗∗}, -(CH₂)₅-phenylene-(CH₂)₅-^{∗∗}, -(CH₂)₆-phenylene-CH₂-^{∗∗}, -(CH₂)₆-phenylene-(CH₂)₂-^{∗∗}, -(CH₂)₆-phenylene-(CH₂)₃-^{∗∗}, -(CH₂)₆-phenylene-(CH₂)₄-^{∗∗}, -(CH₂)₆-phenylene-(CH₂)₅-^{∗∗}, -(CH₂)₆-phenylene-(CH₂)₆-^{∗∗}, -(CH₂)₇-phenylene-CH₂-^{∗∗}, -(CH₂)₇-phenylene-(CH₂)₂-^{∗∗}, -(CH₂)₇-phenylene-(CH₂)₃-^{∗∗}, -(CH₂)₇-phenylene-(CH₂)₄-^{∗∗}, -(CH₂)₇-phenylene-(CH₂)₅-^{∗∗}, -(CH₂)₇-phenylene-(CH₂)₆-^{∗∗}, -(CH₂)₈-phenylene-CH₂-^{∗∗}, -(CH₂)₈-phenylene-(CH₂)₂-^{∗∗}, -(CH₂)₈-phenylene-(CH₂)₃-^{∗∗}, -(CH₂)₈-phenylene-(CH₂)₄-^{∗∗}, -(CH₂)₈-phenylene-(CH₂)₅-^{∗∗}, -(CH₂)₈-phenylene-(CH₂)₆-^{∗∗}, -(CH₂)₈-phenylene-(CH₂)₇-^{∗∗}, -(CH₂)₈-phenylene-(CH₂)₈-^{∗∗}, -CH₂-pyridylene-CH₂-^{∗∗}, -CH₂-pyridylene-(CH₂)₂-^{∗∗}, -CH₂-pyridylene-(CH₂)₃-^{∗∗}, -CH₂-pyridylene-(CH₂)₄-^{∗∗}, -CH₂-pyridylene-(CH₂)₅-^{∗∗}, -CH₂-pyridylene-(CH₂)₆-^{∗∗}, -CH₂-pyridylene-(CH₂)₇-^{∗∗}, -CH₂-pyridylene-(CH₂)₈-^{∗∗}, -(CH₂)₂-pyridylene-CH₂-^{∗∗}, -(CH₂)₂-pyridylene-(CH₂)₂-^{∗∗}, -(CH₂)₂-pyridylene-(CH₂)₃-^{∗∗}, -(CH₂)₂-pyridylene-(CH₂)₄-^{∗∗}, -(CH₂)₂-pyridylene-(CH₂)₅-^{∗∗}, -(CH₂)₂-pyridylene-(CH₂)₆-^{∗∗}, -(CH₂)₂-pyridylene-(CH₂)₇-^{∗∗}, -(CH₂)₂-pyridylene-(CH₂)₈-^{∗∗}, -(CH₂)₃-pyridylene-CH₂-^{∗∗}, -(CH₂)₃-pyridylene-(CH₂)₂-^{∗∗}, -(CH₂)₃-pyridylene-(CH₂)₃-^{∗∗}, -(CH₂)₃-pyridylene-(CH₂)₄-^{∗∗}, -(CH₂)₃-pyridylene-(CH₂)₅-^{∗∗}, -(CH₂)₃-pyridylene-(CH₂)₆-^{∗∗}, -(CH₂)₃-pyridylene-(CH₂)₇-^{∗∗}, -(CH₂)₃-pyridylene-(CH₂)₈-^{∗∗}, -(CH₂)₄-pyridylene-CH₂-^{∗∗}, -(CH₂)₄-pyridylene-(CH₂)₂-^{∗∗}, -(CH₂)₄-pyridylene-(CH₂)₃-^{∗∗}, -(CH₂)₄-pyridylene-(CH₂)₄-^{∗∗}, -(CH₂)₄-pyridylene-(CH₂)₅-^{∗∗}, -(CH₂)₄-pyridylene-(CH₂)₆-^{∗∗}, -(CH₂)₄-pyridylene-(CH₂)₇-^{∗∗}, -(CH₂)₄-pyridylene-(CH₂)₈-^{∗∗}, -(CH₂)₅-pyridylene-CH₂-^{∗∗}, -(CH₂)₅-pyridylene-(CH₂)₂-^{∗∗}, -(CH₂)₅-pyridylene-(CH₂)₃-^{∗∗}, -(CH₂)₅-pyridylene-(CH₂)₄-^{∗∗}, -(CH₂)₅-pyridylene-(CH₂)₅-^{∗∗}, -(CH₂)₆-pyridylene-CH₂-^{∗∗}, -(CH₂)₆-pyridylene-(CH₂)₂-^{∗∗}, -(CH₂)₆-pyridylene-(CH₂)₃-^{∗∗}, -(CH₂)₆-pyridylene-(CH₂)₄-^{∗∗}, -(CH₂)₆-pyridylene-(CH₂)₅-^{∗∗}, -(CH₂)₆-pyridylene-(CH₂)₆-^{∗∗}, -(CH₂)₇-pyridylene-CH₂-^{∗∗}, -(CH₂)₇-pyridylene-(CH₂)₂-^{∗∗}, -(CH₂)₇-pyridylene-(CH₂)₃-^{∗∗}, -(CH₂)₇-pyridylene-(CH₂)₄-^{∗∗}, -(CH₂)₇-pyridylene-(CH₂)₅-^{∗∗}, -(CH₂)₇-pyridylene-(CH₂)₆-^{∗∗}, -(CH₂)₈-pyridylene-CH₂-^{∗∗}, -(CH₂)₈-pyridylene-(CH₂)₂-^{∗∗}, -(CH₂)₈-pyridylene-(CH₂)₃-^{∗∗}, -(CH₂)₈-pyridylene-(CH₂)₄-^{∗∗}, -(CH₂)₈-pyridylene-(CH₂)₅-^{∗∗}, -(CH₂)₈-pyridylene-(CH₂)₆-^{∗∗}, -(CH₂)₈-pyridylene-(CH₂)₇-^{∗∗}, -(CH₂)₈-pyridylene-(CH₂)₈-^{∗∗}, -CH₂-thiazolylene-CH₂-^{∗∗}, -CH₂-thiazolylene-(CH₂)₂-^{∗∗}, -CH₂-thiazolylene-(CH₂)₃-^{∗∗}, -CH₂-thiazolylene-(CH₂)₄-^{∗∗}, -CH₂-thiazolylene-(CH₂)₃-^{∗∗}, -CH₂-thiazolylene-(CH₂)₆-^{∗∗}, -CH₂-thiazolylene-(CH₂)₇-^{∗∗}, -CH₂-thiazolylene-(CH₂)₈-^{∗∗}, -(CH₂)₂-thiazolylene-CH₂-^{∗∗}, -(CH₂)₂-thiazolylene-(CH₂)₂-^{∗∗}, -(CH₂)₂-thiazolylene-(CH₂)₃-^{∗∗}, -(CH₂)₂-thiazolylene-(CH₂)₄-^{∗∗}, -(CH₂)₂-thiazolylene-(CH₂)₅-^{∗∗},-(CH₂)₂-thiazolylene-(CH₂)₆-^{∗∗}, -(CH₂)₂-thiazolylene-(CH₂)₇-^{∗∗}, -(CH₂)₂-thiazolylene-(CH₂)₈-^{∗∗}, -(CH₂)₃-thiazolylene-CH₂-^{∗∗}, -(CH₂)₃-thiazolylene-(CH₂)₂-^{∗∗}, -(CH₂)₃-thiazolylene-(CH₂)₃-^{∗∗}, -(CH₂)₃-thiazolylene-(CH₂)₄-^{∗∗}, -(CH₂)₃-thiazolylene-(CH₂)₅-^{∗∗}, -(CH₂)₃-thiazolylene-(CH₂)₆-^{∗∗}, -(CH₂)₃-thiazolylene-(CH₂)₇-^{∗∗}, -(CH₂)₃-thiazolylene-(CH₂)₈-^{∗∗}, -(CH₂)₄-thiazolylene-CH₂-^{∗∗}, -(CH₂)₄-thiazolylene-(CH₂)₂-^{∗∗}, -(CH₂)₄-thiazolylene-(CH₂)₃-^{∗∗}, -(CH₂)₄-thiazolylene-(CH₂)₄-^{∗∗}, -(CH₂)₄-thiazolylene-(CH₂)₅-^{∗∗}, -(CH₂)₄-thiazolylene-(CH₂)₆-^{∗∗}, -(CH₂)₄-thiazolylene-(CH₂)₇-^{∗∗}, -(CH₂)₄-thiazolylene-(CH₂)₈-^{∗∗}, -(CH₂)₅-thiazolylene-CH₂-^{∗∗}, -(CH₂)₅-thiazolylene-(CH₂)₂-^{∗∗}, -(CH₂)₅-thiazolylene-(CH₂)₃-^{∗∗}, -(CH₂)₅-thiazolylene-(CH₂)₄-^{∗∗},-(CH₂)₅-thiazolylene-(CH₂)₅-^{∗∗}, -(CH₂)₆-thiazolylene-CH₂-^{∗∗}, -(CH₂)₆-thiazolylene-(CH₂)₂-^{∗∗},-(CH₂)₆-thiazolylene-(CH₂)₃-^{∗∗}, -(CH₂)₆-thiazolylene-(CH₂)₄-^{∗∗}, -(CH₂)₆-thiazolylene-(CH₂)₅-^{∗∗}, -(CH₂)₆-thiazolylene-(CH₂)₆-^{∗∗}, -(CH₂)₇-thiazolylene-CH₂-^{∗∗}, -(CH₂)₇-thiazolylene-(CH₂)₂-^{∗∗}, -(CH₂)₇-thiazolylene-(CH₂)₃-^{∗∗}, -(CH₂)₇-thiazolylene-(CH₂)₄-^{∗∗}, -(CH₂)₇-thiazolylene-(CH₂)₅-^{∗∗}, -(CH₂)₇-thiazolylene-(CH₂)₆-^{∗∗}, -(CH₂)₈-thiazolylene-CH₂-^{∗∗}, -(CH₂)₈-thiazolylene-(CH₂)₂-^{∗∗}, -(CH₂)₈-thiazolylene-(CH₂)₃-^{∗∗}, -(CH₂)₈-thiazolylene-(CH₂)₄-^{∗∗}, -(CH₂)₈-thiazolylene-(CH₂)₅-^{∗∗}, -(CH₂)₈-thiazolylene-(CH₂)₆-^{∗∗}, -(CH₂)₈-thiazolylene-(CH₂)₇-^{∗∗}, or -(CH₂)₈-thiazolylene-(CH₂)₈-^{∗∗}, wherein symbol ^{∗∗} indicates the point of attachment to X;
wherein said phenylene, pyridylene, and thiazolylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and L represents:

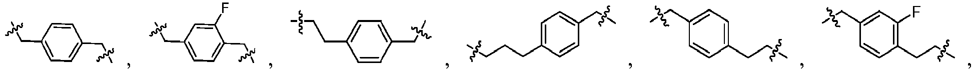

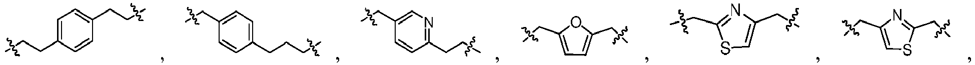

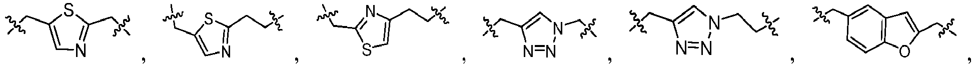

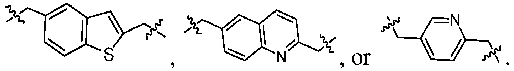

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and L represents the groups represented by the following formula:
-(CH₂)₁₋₄₀-C₅₋₁₅arylene-#, or -(CH₂)₁₋₄₀-C₅₋₁₅heteroarylene-#, wherein symbol # indicates the point of attachment to X;
wherein said C₅₋₁₅arylene and C₅₋₁₅heteroarylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof. In a sub-embodiment, said C₅₋₁₅ arylene includes, but is not limited to, phenylene and naphthylene.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and L represents:
CH₂-phenylene-#, (CH₂)₂-phenylene-#, (CH₂)₃-phenylene-#, (CH₂)₄-phenylene-#, (CH₂)₅-phenylene-#, (CH₂)₆-phenylene-#, (CH₂)₇-phenylene-#, (CH₂)₈-phenylene-#, (CH₂)₉-phenylene-#, (CH₂)₁₀-phenylene-#, (CH₂)₁₁-phenylene-#, (CH₂)₁₂-phenylene-#, -CH₂-pyridylene-#, -(CH₂)₂-pyridylene-#, -(CH₂)₃-pyridylene-#, -(CH₂)₄-pyridylene-#, -(CH₂)₅-pyridylene-#, -(CH₂)₆-pyridylene-#, -(CH₂)₇-pyridylene-#, -(CH₂)₈-pyridylene-#, -(CH₂)₉-pyridylene-#, -(CH₂)₁₀-pyridylene-#, -(CH₂)₁₁-pyridylene-#, -(CH₂)₁₂-pyridylene-#, -CH₂-thiazolylene-#, -(CH₂)₂-thiazolylene-#, -(CH₂)₃-thiazolylene-#, -(CH₂)₄-thiazolylene-#, -(CH₂)₅-thiazolylene-#, -(CH₂)₆-thiazolylene-#, -(CH₂)₇-thiazolylene-#, -(CH₂)₈-thiazolylene-#, -(CH₂)₉-thiazolylene-#, - (CH₂)₁₀-thiazolylene-#, -(CH₂)₁₁-thiazolylene-#, or -(CH₂)₁₂-thiazolylene-#, wherein symbol # indicates the point of attachment to X; and
wherein said phenylene, pyridylene, and thiazolylene are each independently optionally substituted by one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and
L represents: 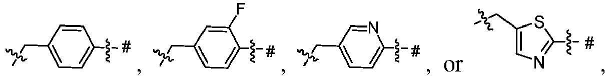 wherein symbol # indicates the point of attachment to X.

In one embodiment of the present disclosure, X represents a bond; and L represents:
linear or branched C₂₋₄₀ alkylene interrupted one or more times (such as 1-10, 1-8, 1-6, 1-5, 1-3, 1-2 times, or once, which are not restricted in principle, or are automatically limited by the size of building unit referenced herein, or can vary as required) by one or more (such as 1-10, 1-9, 1-8, 1-7, 1-6, 1-5, 1-4, 1-3, 1-2, or 1, which are not restricted in principle, or are automatically limited by the size of building unit referenced herein, or can vary as required) groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof; and said C₅₋₁₅arylene and C₅₋₁₅heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof; and
R represents NR_{c4}R_{c5}, C(O)NR_{c6}R_{c7}, N(R_{c8})C(O)R_{c9}, OR_{c13}, SR_{c14}, C(O)R_{c15}, OC(O)R_{c16}, C(O)OR_{c17}, S(O)₂NR_{c18}R_{c19}, N(R_{c20})S(O)₂R_{c21}, S(O)R_{c22}, S(O)₂R_{c23}, S(O)₂OR_{c24}, OS(O)₂R_{c25}, optionally substituted C₅₋₂₀ cycloalkyl, or optionally substituted C₅₋₂₀ heterocyclyl,
   wherein R_{c8} and R_{c20} each independently represent H or C₁₋₃ alkyl;
   R_{c4}, R_{c6}, and R_{c18} each independently represent H, optionally substituted linear or branched C₁₋₁₀ alkyl, optionally substituted C₃₋₂₀ cycloalkyl, optionally substituted C₅₋₂₀ aryl, optionally substituted C₃₋₂₀ heterocyclyl or optionally substituted C₅₋₂₀ heteroaryl; and
   R_{c5}, R_{c7}, R_{c9}, R_{c13}, R_{c14}, R_{c15}, R_{c16}, R_{c17}, R_{c19}, R_{c21}, R_{c22}, R_{c23}, R_{c24}, and R_{c25} each independently represent optionally substituted C₅₋₂₀ cycloalkyl or optionally substituted C₅₋₂₀ heterocyclyl;
   wherein said linear or branched C₁₋₁₀ alkyl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof;
   each C₃₋₂₀ cycloalkyl and each C₃₋₂₀ heterocyclyl is each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof; and
   each C₅₋₂₀ aryl and each C₅₋₂₀ heteroaryl is each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)-, optionally substituted C₆₋₂₀ aryl or any combination thereof, wherein said C₆₋₂₀ aryl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, or any combination thereof.

In one embodiment of the present disclosure, X represents a bond; and L represents:
-(CH₂)ₙ₁-C₅₋₁₅ arylene-(CH₂)ₙ₂-^{∗∗}, or -(CH₂)ₙ₁-C₅₋₁₅ heteroarylene-(CH₂)ₙ₂-^{∗∗}, wherein symbol ** indicates the point of attachment to X;
wherein n1 and n2 each independently represent an integer of 1, 2, 3, 4, 5, 6, 7, 8, 9, 10, 11, 12, 13, 14, 15, 16, 17, 18, 19, or 20; and
said C₅₋₁₅ arylene and C₅₋₁₅ heteroarylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof. In a sub-embodiment, said C₅₋₁₅ heteroarylene includes, but is not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzo[d][1,3]dioxolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene and imidazo[2,1-b]thiazolylene. In a sub-embodiment, said C₅₋₁₅ arylene includes, but is not limited to, phenylene and naphthylene.

In one embodiment of the present disclosure, X represents a bond; and L represents: -CH₂-phenylene-CH₂-^{∗∗}, -CH₂-phenylene-(CH₂)₂-^{∗∗}, -CH₂-phenylene-(CH₂)₃-^{∗∗}, -CH₂-phenylene-(CH₂)₄-^{∗∗}, -CH₂-phenylene-(CH₂)₃-^{∗∗}, -CH₂-phenylene-(CH₂)₆-^{∗∗}, -CH₂-phenylene-(CH₂)₇-^{∗∗}, - CH₂-phenylene-(CH₂)₈-^{∗∗}, -(CH₂)₂-phenylene-CH₂-^{∗∗}, -(CH₂)₂-phenylene-(CH₂)₂-^{∗∗}, -(CH₂)₂-phenylene-(CH₂)₃-^{∗∗}, -(CH₂)₂-phenylene-(CH₂)₄-^{∗∗}, -(CH₂)₂-phenylene-(CH₂)₅-^{∗∗}, -(CH₂)₂-phenylene-(CH₂)₆-^{∗∗}, -(CH₂)₂-phenylene-(CH₂)₇-^{∗∗}, -(CH₂)₂-phenylene-(CH₂)₈-^{∗∗}, -(CH₂)₃-phenylene-CH₂-^{∗∗}, -(CH₂)₃-phenylene-(CH₂)₂-^{∗∗}, -(CH₂)₃-phenylene-(CH₂)₃-^{∗∗}, -(CH₂)₃-phenylene-(CH₂)₄-^{∗∗}, -(CH₂)₃-phenylene-(CH₂)₅-^{∗∗}, -(CH₂)₃-phenylene-(CH₂)₆-^{∗∗}, -(CH₂)₃-phenylene-(CH₂)₇-^{∗∗}, -(CH₂)₃-phenylene-(CH₂)₈-^{∗∗}, -(CH₂)₄-phenylene-CH₂-^{∗∗}, -(CH₂)₄-phenylene-(CH₂)₂-^{∗∗}, - (CH₂)₄-phenylene-(CH₂)₃-^{∗∗}, -(CH₂)₄-phenylene-(CH₂)₄-^{∗∗}, -(CH₂)₄-phenylene-(CH₂)₅-^{∗∗}, -(CH₂)₄-phenylene-(CH₂)₆-^{∗∗}, -(CH₂)₄-phenylene-(CH₂)₇-^{∗∗}, -(CH₂)₄-phenylene-(CH₂)₈-^{∗∗}, -(CH₂)₅-phenylene-CH₂-^{∗∗}, -(CH₂)₅-phenylene-(CH₂)₂-^{∗∗}, -(CH₂)₅-phenylene-(CH₂)₃-^{∗∗}, -(CH₂)₅-phenylene-(CH₂)₄-^{∗∗}, -(CH₂)₅-phenylene-(CH₂)₅-^{∗∗}, -(CH₂)₆-phenylene-CH₂-^{∗∗}, -(CH₂)₆-phenylene-(CH₂)₂-^{∗∗}, -(CH₂)₆-phenylene-(CH₂)₃-^{∗∗}, -(CH₂)₆-phenylene-(CH₂)₄-^{∗∗}, -(CH₂)₆-phenylene-(CH₂)₅-^{∗∗}, -(CH₂)₆-phenylene-(CH₂)₆-^{∗∗}, -(CH₂)₇-phenylene-CH₂-^{∗∗}, -(CH₂)₇-phenylene-(CH₂)₂-^{∗∗}, -(CH₂)₇-phenylene-(CH₂)₃-^{∗∗}, -(CH₂)₇-phenylene-(CH₂)₄-^{∗∗}, -(CH₂)₇-phenylene-(CH₂)₅-^{∗∗}, -(CH₂)₇-phenylene-(CH₂)₆-^{∗∗}, -(CH₂)₈-phenylene-CH₂-^{∗∗}, -(CH₂)₈-phenylene-(CH₂)₂-^{∗∗}, -(CH₂)₈-phenylene-(CH₂)₃-^{∗∗}, - (CH₂)₈-phenylene-(CH₂)₄-^{∗∗}, -(CH₂)₈-phenylene-(CH₂)₅-^{∗∗}, -(CH₂)₈-phenylene-(CH₂)₆-^{∗∗}, -(CH₂)₈-phenylene-(CH₂)₇-^{∗∗}, -(CH₂)₈-phenylene-(CH₂)₈-^{∗∗}, -CH₂-pyridylene-CH₂-^{∗∗}, -CH₂-pyridylene-(CH₂)₂-^{∗∗}, -CH₂-pyridylene-(CH₂)₃-^{∗∗}, -CH₂-pyridylene-(CH₂)₄-^{∗∗}, -CH₂-pyridylene-(CH₂)₅-^{∗∗}, - CH₂-pyridylene-(CH₂)₆-^{∗∗}, -CH₂-pyridylene-(CH₂)₇-^{∗∗}, -CH₂-pyridylene-(CH₂)₈-^{∗∗}, -(CH₂)₂-pyridylene-CH₂-^{∗∗}, -(CH₂)₂-pyridylene-(CH₂)₂-^{∗∗}, -(CH₂)₂-pyridylene-(CH₂)₃-^{∗∗}, -(CH₂)₂-pyridylene-(CH₂)₄-^{∗∗}, -(CH₂)₂-pyridylene-(CH₂)₅-^{∗∗}, -(CH₂)₂-pyridylene-(CH₂)₆-^{∗∗}, -(CH₂)₂-pyridylene-(CH₂)₇-^{∗∗}, -(CH₂)₂-pyridylene-(CH₂)₈-^{∗∗}, -(CH₂)₃-pyridylene-CH₂-^{∗∗}, -(CH₂)₃-pyridylene-(CH₂)₂-^{∗∗}, -(CH₂)₃-pyridylene-(CH₂)₃-^{∗∗}, -(CH₂)₃-pyridylene-(CH₂)₄-^{∗∗}, -(CH₂)₃-pyridylene-(CH₂)₅-^{∗∗}, -(CH₂)₃-pyridylene-(CH₂)₆-^{∗∗}, -(CH₂)₃-pyridylene-(CH₂)₇-^{∗∗}, -(CH₂)₃-pyridylene-(CH₂)₈-^{∗∗}, -(CH₂)₄-pyridylene-CH₂-^{∗∗}, -(CH₂)₄-pyridylene-(CH₂)₂-^{∗∗}, -(CH₂)₄-pyridylene-(CH₂)₃-^{∗∗}, -(CH₂)₄-pyridylene-(CH₂)₄-^{∗∗}, -(CH₂)₄-pyridylene-(CH₂)₅-^{∗∗}, -(CH₂)₄-pyridylene-(CH₂)₆-^{∗∗}, -(CH₂)₄-pyridylene-(CH₂)₇-^{∗∗}, -(CH₂)₄-pyridylene-(CH₂)₈-^{∗∗}, -(CH₂)₅-pyridylene-CH₂-^{∗∗}, -(CH₂)₅-pyridylene-(CH₂)₂-^{∗∗}, -(CH₂)₅-pyridylene-(CH₂)₃-^{∗∗}, -(CH₂)₅-pyridylene-(CH₂)₄-^{∗∗}, -(CH₂)₅-pyridylene-(CH₂)₅-^{∗∗}, -(CH₂)₆-pyridylene-CH₂-^{∗∗}, -(CH₂)₆-pyridylene-(CH₂)₂-^{∗∗}, -(CH₂)₆-pyridylene-(CH₂)₃-^{∗∗}, -(CH₂)₆-pyridylene-(CH₂)₄-^{∗∗}, -(CH₂)₆-pyridylene-(CH₂)₅-^{∗∗}, -(CH₂)₆-pyridylene-(CH₂)₆-^{∗∗}, -(CH₂)₇-pyridylene-CH₂-^{∗∗}, -(CH₂)₇-pyridylene-(CH₂)₂-^{∗∗}, -(CH₂)₇-pyridylene-(CH₂)₃-^{∗∗}, -(CH₂)₇-pyridylene-(CH₂)₄-^{∗∗}, -(CH₂)₇-pyridylene-(CH₂)₅-^{∗∗}, -(CH₂)₇-pyridylene-(CH₂)₆-^{∗∗}, -(CH₂)₈-pyridylene-CH₂-^{∗∗}, -(CH₂)₈-pyridylene-(CH₂)₂-^{∗∗}, -(CH₂)₈-pyridylene-(CH₂)₃-^{∗∗}, -(CH₂)₈-pyridylene-(CH₂)₄-^{∗∗}, -(CH₂)₈-pyridylene-(CH₂)₅-^{∗∗}, -(CH₂)₈-pyridylene-(CH₂)₆-^{∗∗}, -(CH₂)₈-pyridylene-(CH₂)₇-^{∗∗}, -(CH₂)₈-pyridylene-(CH₂)₈-^{∗∗}, -CH₂-thiazolylene-CH₂-^{∗∗}, -CH₂-thiazolylene-(CH₂)₂-^{∗∗}, -CH₂-thiazolylene-(CH₂)₃-^{∗∗}, -CH₂-thiazolylene-(CH₂)₄-^{∗∗}, -CH₂-thiazolylene-(CH₂)₃-^{∗∗}, -CH₂-thiazolylene-(CH₂)₆-^{∗∗}, -CH₂-thiazolylene-(CH₂)₇-^{∗∗}, -CH₂-thiazolylene-(CH₂)₈-^{∗∗}, -(CH₂)₂-thiazolylene-CH₂-^{∗∗}, -(CH₂)₂-thiazolylene-(CH₂)₂-^{∗∗}, -(CH₂)₂-thiazolylene-(CH₂)₃-^{∗∗}, -(CH₂)₂-thiazolylene-(CH₂)₄-^{∗∗}, -(CH₂)₂-thiazolylene-(CH₂)₅-^{∗∗}, -(CH₂)₂-thiazolylene-(CH₂)₆-^{∗∗}, -(CH₂)₂-thiazolylene-(CH₂)₇-^{∗∗}, -(CH₂)₂-thiazolylene-(CH₂)₈-^{∗∗}, -(CH₂)₃-thiazolylene-CH₂-^{∗∗}, -(CH₂)₃-thiazolylene-(CH₂)₂-^{∗∗}, -(CH₂)₃-thiazolylene-(CH₂)₃-^{∗∗}, -(CH₂)₃-thiazolylene-(CH₂)₄-^{∗∗}, -(CH₂)₃-thiazolylene-(CH₂)₃-^{∗∗}, -(CH₂)₃-thiazolylene-(CH₂)₆-^{∗∗}, -(CH₂)₃-thiazolylene-(CH₂)₇-^{∗∗}, -(CH₂)₃-thiazolylene-(CH₂)₈-^{∗∗}, -(CH₂)₄-thiazolylene-CH₂-^{∗∗}, -(CH₂)₄-thiazolylene-(CH₂)₂-^{∗∗}, -(CH₂)₄-thiazolylene-(CH₂)₃-^{∗∗}, -(CH₂)₄-thiazolylene-(CH₂)₄-^{∗∗}, -(CH₂)₄-thiazolylene-(CH₂)₅-^{∗∗}, -(CH₂)₄-thiazolylene-(CH₂)₆-^{∗∗}, -(CH₂)₄-thiazolylene-(CH₂)₇-^{∗∗}, -(CH₂)₄-thiazolylene-(CH₂)₈-^{∗∗}, -(CH₂)₅-thiazolylene-CH₂-^{∗∗}, -(CH₂)₅-thiazolylene-(CH₂)₂-^{∗∗}, -(CH₂)₅-thiazolylene-(CH₂)₃-^{∗∗}, -(CH₂)₅-thiazolylene-(CH₂)₄-^{∗∗}, -(CH₂)₅-thiazolylene-(CH₂)₃-^{∗∗}, -(CH₂)₆-thiazolylene-CH₂-^{∗∗}, -(CH₂)₆-thiazolylene-(CH₂)₂-^{∗∗}, -(CH₂)₆-thiazolylene-(CH₂)₃-^{∗∗}, -(CH₂)₆-thiazolylene-(CH₂)₄-^{∗∗}, -(CH₂)₆-thiazolylene-(CH₂)₅-^{∗∗}, -(CH₂)₆-thiazolylene-(CH₂)₆-^{∗∗}, -(CH₂)₇-thiazolylene-CH₂-^{∗∗}, -(CH₂)₇-thiazolylene-(CH₂)₂-^{∗∗}, -(CH₂)₇-thiazolylene-(CH₂)₃-^{∗∗}, -(CH₂)₇-thiazolylene-(CH₂)₄-^{∗∗}, -(CH₂)₇-thiazolylene-(CH₂)₅-^{∗∗}, -(CH₂)₇-thiazolylene-(CH₂)₆-^{∗∗}, -(CH₂)₈-thiazolylene-CH₂-^{∗∗}, -(CH₂)₈-thiazolylene-(CH₂)₂-^{∗∗}, -(CH₂)₈-thiazolylene-(CH₂)₃-^{∗∗}, -(CH₂)₈-thiazolylene-(CH₂)₄-^{∗∗}, -(CH₂)₈-thiazolylene-(CH₂)₅-^{∗∗}, -(CH₂)₈-thiazolylene-(CH₂)₆-^{∗∗}, -(CH₂)₈-thiazolylene-(CH₂)₇-^{∗∗}, or -(CH₂)₈-thiazolylene-(CH₂)₈-^{∗∗}, wherein symbol ** indicates the point of attachment to X;
wherein said phenylene, pyridylene, and thiazolylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof.

In one embodiment of the present disclosure, X represents a bond; and L represents:

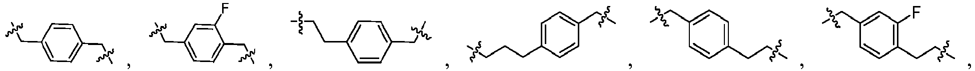

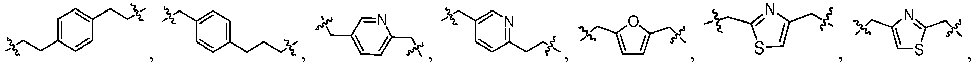

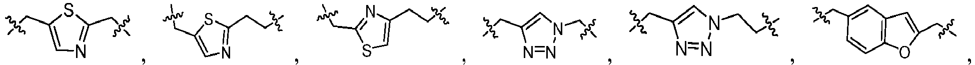

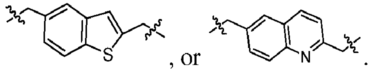

In one embodiment of the present disclosure, X represents N(Rₐ₁), O or S, where Rₐ₁ represents H or C₁₋₃ alkyl; and
L represents linear or branched C₂₋₄₀ alkylene interrupted one or more times (such as 1-10, 1-8, 1-6, 1-5, 1-3, 1-2 times, or once, which can vary as required) by one or more groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and each C₅₋₁₅ arylene and each C₅₋₁₅ heteroarylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)- or any combination thereof; and
R represents NRₐ₇Rₐ₈, wherein Rₐ₇ and Rₐ₈ each independently represent:
   H; or
   methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, or decyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, mercapto, C₁₋₃ alkoxy, C₁₋₃ alkylamino, C₁₋₃ alkyl-NHC(O)- or any combination thereof; or
   adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
   noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
   cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
   p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 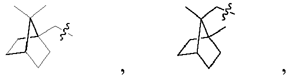 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

In one embodiment of the present disclosure, X represents N(Rₐ₁), O or S, where Rₐ₁ represents H or C₁₋₃ alkyl; and when R represents ORₐ₁₆, Rₐ₁₆ represents:
H; or
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 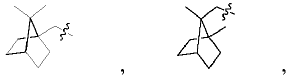 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

In one embodiment of the present disclosure, X represents N(Rₐ₁), O or S, where Rₐ₁ represents H or C₁₋₃ alkyl; and
L represents linear or branched C₂₋₄₀ alkylene interrupted one or more times (such as 1-10, 1-8, 1-6, 1-5, 1-3, 1-2 times, or once, which can vary as required) by one or more (such as 1-10, 1-9, 1-8, 1-7, 1-6, 1-5, 1-4, 1-3, 1-2, or 1, which can vary as required) groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and each C₅₋₁₅ arylene and each C₅₋₁₅ heteroarylene is independently optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)- or any combination thereof; and
R represents azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are independently optionally substituted by one or more substituents selected from the group consisting of:
   halogen, hydroxyl, mercapto, oxo, cyano, amino, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)-, optionally substituted phenyl or any combination thereof, wherein said optionally substituted phenyl is optionally substituted by a substituent selected from the group consisting of halogen, hydroxyl, amino, cyano, mercapto, C₁₋₃ alkyl, or any combination thereof.

In one embodiment of the present disclosure, X represents N(Rₐ₁), O or S, where Rₐ₁ represents H or C₁₋₃ alkyl; and
L represents Formula -Lₐ₂₋Lₐ₁₋-^{∗}, where Lₐ₁ represents linear or branched C₁₋₄₀ alkylene optionally substituted by a substituent selected from the group consisting of halogen, hydroxyl, amino or any combination thereof, Lₐ₂ represents optionally substituted C₃₋₁₅ cycloalkylene or optionally substituted C₅₋₁₅ arylene, wherein said C₃₋₁₅ cycloalkylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)- or any combination thereof, and said C₅₋₁₅ arylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof, and symbol * indicates the point of attachment to X; and
R represents:
   C(O)NRₐ₉Rₐ₁₀, N(Rₐ₁₁)C(O)Rₐ₁₂, C(O)Rₐ₁₈, OC(O)Rₐ₁₉, C(O)ORₐ₂₀, S(O)₂NRₐ₂₁Rₐ₂₂ or N(Rₐ₂₃)S(O)₂Rₐ₂₄,
   wherein Rₐ₉, Rₐ₁₁, Rₐ₂₁, and Rₐ₂₃ each independently represent H or C₁₋₃ alkyl; and
   Rₐ₁₀, Rₐ₁₂, Rₐ₁₈, Rₐ₁₉, Rₐ₂₀, Rₐ₂₂ and Rₐ₂₄ each independently represent the following groups:
      adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, adamantan-10-yl, noradamantan-1-yl, noradamantan-2-yl, noradamantan-3-yl, noradamantan-4-yl, noradamantan-5-yl, noradamantan-6-yl, noradamantan-7-yl, noradamantan-8-yl, noradamantan-9-yl, cyclopropyl, cyclobutyl, cyclopentyl, cyclopentenyl, cyclohexyl, cyclohexenyl, cycloheptyl, cyclooctyl, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, C₇₋₁₁azaspirocycloalkyl, p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 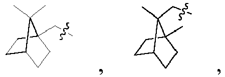 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl,
      wherein the groups are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, amino, C₁₋₃ alkyl, halogenated C₁₋₃ alkyl, C₁₋₃ alkoxy, C₁₋₃ alkyl substituted by hydroxy, C₁₋₃ alkyl substituted by NH₂, C₁₋₃ alkylamino, C₁₋₃ alkyl-NHC(O)- or any combination thereof.

In one embodiment of the present disclosure, X represents N(Rₐ₁), O or S, where Rₐ₁ represents H or C₁₋₃ alkyl; and
L represents linear or branched C₂₋₄₀ alkylene interrupted one or more times (such as 1-10, 1-8, 1-6, 1-5, 1-3, 1-2 times, or once, which can vary as required) by one or more (such as 1-10, 1-9, 1-8, 1-7, 1-6, 1-5, 1-4, 1-3, 1-2, or 1, which can vary as required) groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and each C₅₋₁₅ arylene and each C₅₋₁₅ heteroarylene is independently optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)- or any combination thereof; and
R represents:
   C(O)NRₐ₉Rₐ₁₀, N(Rₐ₁₁)C(O)Rₐ₁₂, C(O)Rₐ₁₈, OC(O)Rₐ₁₉, C(O)ORₐ₂₀, S(O)₂NRₐ₂₁Rₐ₂₂ or N(Rₐ₂₃)S(O)₂Rₐ₂₄,
   wherein Rₐ₉, Rₐ₁₁, Rₐ₂₁, and Rₐ₂₃ each independently represent H or C₁₋₃ alkyl; and
   Rₐ₁₀, Rₐ₁₂, Rₐ₁₈, Rₐ₁₉, Rₐ₂₀, Rₐ₂₂ and Rₐ₂₄ each independently represent the following groups:
      adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, adamantan-10-yl, noradamantan-1-yl, noradamantan-2-yl, noradamantan-3-yl, noradamantan-4-yl, noradamantan-5-yl, noradamantan-6-yl, noradamantan-7-yl, noradamantan-8-yl, noradamantan-9-yl, cyclopropyl, cyclobutyl, cyclopentyl, cyclopentenyl, cyclohexyl, cyclohexenyl, cycloheptyl, cyclooctyl, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, C₇₋₁₁azaspirocycloalkyl, p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 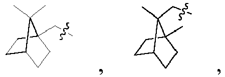 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl,
      wherein the groups are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, amino, C₁₋₃ alkyl, halogenated C₁₋₃ alkyl, C₁₋₃ alkoxy, C₁₋₃ alkyl substituted by hydroxy, C₁₋₃ alkyl substituted by NH₂, C₁₋₃ alkylamino, C₁₋₃ alkyl-NHC(O)- or any combination thereof.

In one embodiment of the present disclosure, X represents N(Rₐ₁), O or S, where Rₐ₁ represents H or C₁₋₃ alkyl; and
L represents linear or branched C₁₋₃ alkylene optionally substituted by a substituent selected from the group consisting of halogen, hydroxyl, amino or any combination thereof; and
R represents: 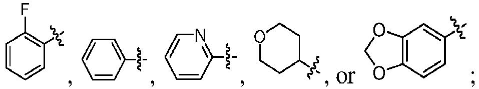 wherein the groups are optionally substituted by a substituent selected from the group consisting of halogen, hydroxyl, mercapto, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)- or any combination thereof.

In one embodiment of the present disclosure, X represents N(Rₐ₁), O or S, where Rₐ₁ represents H or C₁₋₃ alkyl; and
L represents Formula -Lₐ₂₋Lₐ₁₋-^{∗}, where Lₐ₁ represents linear or branched C₁₋₄₀ alkylene optionally substituted by a substituent selected from the group consisting of halogen, hydroxyl, amino or any combination thereof, Lₐ₂ represents optionally substituted C₃₋₁₅ cycloalkylene or optionally substituted C₅₋₁₅ arylene, wherein said C₃₋₁₅ cycloalkylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)- or any combination thereof, and said C₅₋₁₅ arylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof, and symbol * indicates the point of attachment to X; and
R represents:
   OH,

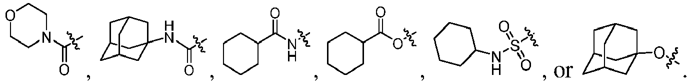

In one embodiment of the present disclosure, X represents N(Rₐ₁), O or S, where Rₐ₁ represents H or C₁₋₃ alkyl; and
L represents linear or branched C₂₋₄₀ alkylene interrupted one or more times by one or more groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and each C₅₋₁₅ arylene and each C₅₋₁₅ heteroarylene is independently optionally substituted by one or more (such as 1-3) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)- or any combination thereof; and
R represents:
   OH, N(CH₃)₂, N(CH₂CH₃)₂,

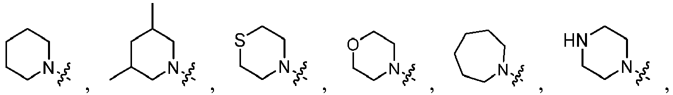

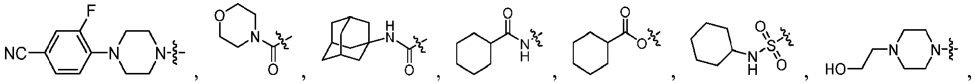

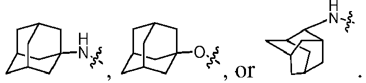

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and R represents NR_{b7}R_{b8}, wherein R_{b7} and R_{b8} are the same or different and each independently represent:
H, or
methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, or decyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, mercapto, C₁₋₃ alkoxy, C₁₋₃ alkylamino, C₁₋₃ alkyl-NHC(O)- or any combination thereof; or
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 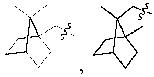 , 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and R represents OR_{b16}, wherein R_{b16} represents:
H, or
methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, or decyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, mercapto, C₁₋₃ alkoxy, C₁₋₃ alkylamino, C₁₋₃ alkyl-NHC(O)- or any combination thereof; or
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 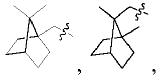 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and R represents:
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are independently optionally substituted by one or more (such as 1-10, 1-9, 1-8, 1-7, 1-6, 1-5, 1-4, 1-3, 1-2, or 1, which can vary as required) substituents selected from the group consisting of:
halogen, hydroxyl, mercapto, oxo, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)-, optionally substituted phenyl or any combination thereof, wherein said optionally substituted phenyl is optionally substituted by one or more (such as 1-5, 1-4, 1-3, 1-2, or 1, which can vary as required) substituents selected from the group consisting of halogen, hydroxyl, amino, cyano, mercapto, C₁₋₃ alkyl, or any combination thereof.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and R represents:
C(O)NR_{b9}R_{b10}, N(R_{b11})C(O)R_{b12}, C(O)R_{b18}, OC(O)R_{b19}, C(O)OR_{b20}, S(O)₂NR_{b21}R_{b22} or N(R_{b23})S(O)₂R_{b24},
wherein R_{b9}, R_{b11}, R_{b21} and R_{b23} each independently represent H or C₁₋₃ alkyl; and
R_{b10}, R_{b12}, R_{b18}, R_{b19}, R_{b20}, R_{b22}, and R_{b24} each independently represent the following groups:
   adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, adamantan-10-yl, noradamantan-1-yl, noradamantan-2-yl, noradamantan-3-yl, noradamantan-4-yl, noradamantan-5-yl, noradamantan-6-yl, noradamantan-7-yl, noradamantan-8-yl, noradamantan-9-yl, cyclopropyl, cyclobutyl, cyclopentyl, cyclopentenyl, cyclohexyl, cyclohexenyl, cycloheptyl, cyclooctyl, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, C₇₋₁₁azaspirocycloalkyl, p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 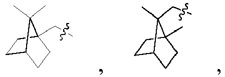 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl,
   wherein the groups are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and R represents:
OH, N(CH₃)₂, N(CH₂CH₃)₂,

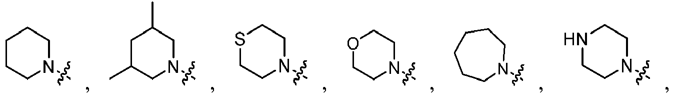

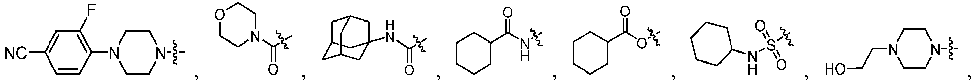

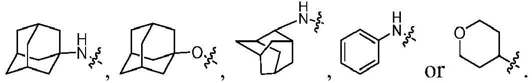

In one embodiment of the present disclosure, X represents a bond; and R represents NR_{c4}R_{c5}, wherein
R_{c4} represents H or C₁₋₃ alkyl;
R _{c5} represents:
   adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
   noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
   cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
   p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 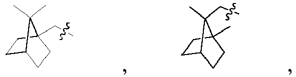 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

In one embodiment of the present disclosure, X represents a bond; and R represents OR_{c13}, wherein R_{c13} represents:
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 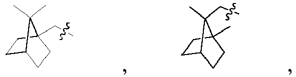 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

In one embodiment of the present disclosure, X represents a bond; and R represents:
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are optionally substituted by one or more (such as 1-10, 1-9, 1-8, 1-7, 1-6, 1-5, 1-4, 1-3, 1-2, or 1, which can vary as required) substituents selected from the group consisting of:
halogen, hydroxyl, mercapto, oxo, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)-, or any combination thereof.

In one embodiment of the present disclosure, X represents a bond; and R represents:
C(O)NR_{c6}R_{c7}, N(R_{c8})C(O)R_{c9}, C(O)R_{c15}, OC(O)R_{c16}, C(O)OR_{c17}, S(O)₂NR_{c18}R_{c19} or N(R_{c20})S(O)₂R_{c21},
wherein R_{c6}, R_{c8}, R_{c18} and R_{c20} each independently represent H or C₁₋₃ alkyl; and
R_{c7}, R_{c9}, R_{c15}, R_{c16}, R_{c17}, R_{c19}, and R_{c21} each independently represent the following groups:
   adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, adamantan-10-yl, noradamantan-1-yl, noradamantan-2-yl, noradamantan-3-yl, noradamantan-4-yl, noradamantan-5-yl, noradamantan-6-yl, noradamantan-7-yl, noradamantan-8-yl, noradamantan-9-yl, cyclopropyl, cyclobutyl, cyclopentyl, cyclopentenyl, cyclohexyl, cyclohexenyl, cycloheptyl, cyclooctyl, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, C₇₋₁₁azaspirocycloalkyl, p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 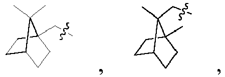 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl,
   wherein the groups are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof.

In one embodiment of the present disclosure, X represents a bond; and R represents:

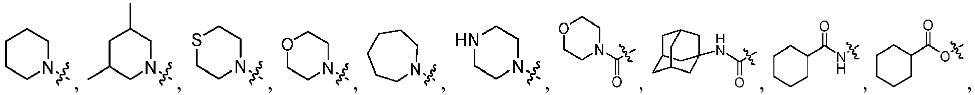

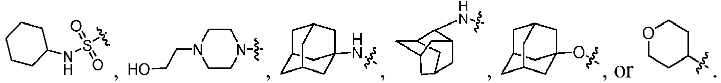

In one embodiment of the present disclosure, X represents N(Rₐ₁), O or S, where Rₐ₁ represents H or C₁₋₃ alkyl; and R-L- in formula (I) represents:

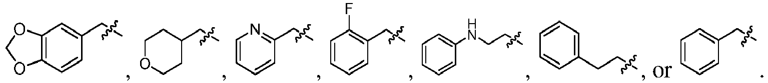

In one embodiment of the present disclosure, X represents N(Rₐ₁), O, S, C₂₋₆ alkynylene (such as C₂₋₄ alkynylene, e.g., ethynylene or butynylene) or C₂₋₆ alkenylene (such as C₂₋₄ alkenylene, e.g., vinylene or butenylene), where Rₐ₁ represents H or C₁₋₃ alkyl; and
L represents:
   linear or branched alkylene (such as C₂₋₄₀ alkylene, C₂₋₃₀ alkylene, C₂₋₂₀ alkylene, C₂₋₁₅ alkylene, or C₂₋₁₀ alkylene) interrupted one or more times (such as 1-10, 1-8, 1-6, 1-5, 1-3, 1-2 times, or once, which can vary as required) by one or more (such as 1-10, 1-9, 1-8, 1-7, 1-6, 1-5, 1-4, 1-3, 1-2, or 1, which can vary as required) groups selected from the group consisting of: optionally substituted arylene (such as C₅₋₁₅ arylene), optionally substituted heteroarylene (such as C₅₋₁₅ heteroarylene) or any combination thereof, wherein said linear or branched alkylene is optionally substituted by a substituent selected from the group consisting of halogen, hydroxyl, amino or any combination thereof, and wherein said arylene (such as C₅₋₁₅ arylene) and said heteroarylene (such as C₅₋₁₅ heteroarylene) are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)- or any combination thereof; and
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted C₃₋₂₀ cycloalkyl or optionally substituted C₃₋₂₀ heterocyclyl,
   wherein Rₐ₇, Rₐ₈, Rₐ₉, Rₐ₁₀, Rₐ₁₆, Rₐ₁₇, Rₐ₂₁ and R_{b22} each independently represent H, optionally substituted linear or branched C₁₋₁₀ alkyl, optionally substituted C₃₋₂₀ cycloalkyl or optionally substituted C₃₋₂₀ heterocyclyl;
   R_{b11} and R_{b23} each independently represent H or C₁₋₃ alkyl; and
   Rₐ₁₂, Rₐ₁₈, Rₐ₁₉, Rₐ₂₀, Rₐ₂₄, Rₐ₂₅, Rₐ₂₆, Rₐ₂₇, and R_{b28} each independently represent optionally substituted linear or branched C₁₋₁₀ alkyl, optionally substituted C₃₋₂₀ cycloalkyl or optionally substituted C₃₋₂₀ heterocyclyl;
   wherein said linear or branched C₁₋₁₀ alkyl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof;
   each C₃₋₂₀ cycloalkyl and each C₃₋₂₀ heterocyclyl is each independently optionally substituted by one or more (such as 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents N(Rₐ₁), O, S, C₂₋₆ alkynylene (such as C₂₋₄ alkynylene, e.g., ethynylene or butynylene) or C₂₋₆ alkenylene (such as C₂₋₄ alkenylene, e.g., vinylene or butenylene), where Rₐ₁ represents H or C₁₋₃ alkyl; and L represents the groups represented by the following formula:
-(CH₂)ₙ₁-C₅₋₁₅arylene-(CH₂)ₙ₂-^{∗∗}, or -(CH₂)ₙ₁-C₅₋₁₅heteroarylene-(CH₂)ₙ₂-^{∗∗}, wherein symbol ** indicates the point of attachment to X;
wherein n1 and n2 each independently represent an integer of 1, 2, 3, 4, 5, 6, 7, 8, 9, 10, 11, 12, 13, 14, 15, 16, 17, 18, 19, or 20; and
said C₅₋₁₅ arylene and C₅₋₁₅ heteroarylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof (wherein in a sub-embodiment, said C₅₋₁₅ heteroarylene includes, but is not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzo[d][1,3]dioxolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene and imidazo[2,1-b]thiazolylene; in a sub-embodiment, said C₅₋₁₅ arylene includes, but is not limited to, phenylene and naphthylene); and
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted C₃₋₂₀ cycloalkyl, or optionally substituted C₃₋₂₀ heterocyclyl,
wherein Rₐ₇, Rₐ₈, Rₐ₉, Rₐ₁₀, Rₐ₁₆, Rₐ₁₇, Rₐ₂₁ and Rₐ₂₂ represent H, optionally substituted linear or branched C₁₋₁₀ alkyl, optionally substituted C₃₋₂₀ cycloalkyl or optionally substituted C₃₋₂₀ heterocyclyl;
Rₐ₁₁ and Rₐ₂₃ each independently represent H or C₁₋₃ alkyl; and
Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
wherein said linear or branched C₁₋₁₀ alkyl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof; and
each C₃₋₂₀ cycloalkyl and each C₃₋₂₀ heterocyclyl is independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents N(Rₐ₁), O, S, C₂₋₆ alkynylene (such as C₂₋₄ alkynylene, e.g., ethynylene or butynylene) or C₂₋₆ alkenylene (such as C₂₋₄ alkenylene, e.g., vinylene or butenylene), where Rₐ₁ represents H or C₁₋₃ alkyl; and L represents:
-CH₂-phenylene-CH₂-^{∗∗}, -CH₂-phenylene-(CH₂)₂-^{∗∗}, -CH₂-phenylene-(CH₂)₃-^{∗∗}, -CH₂-phenylene-(CH₂)₄-^{∗∗}, -CH₂-phenylene-(CH₂)₅-^{∗∗}, -CH₂-phenylene-(CH₂)₆-^{∗∗}, -CH₂-phenylene-(CH₂)₇-^{∗∗}, -CH₂-phenylene-(CH₂)₈-^{∗∗}, -(CH₂)₂-phenylene-CH₂-^{∗∗}, -(CH₂)₂-phenylene-(CH₂)₂-^{∗∗}, -(CH₂)₂-phenylene-(CH₂)₃-^{∗∗}, -(CH₂)₂-phenylene-(CH₂)₄-^{∗∗}, - (CH₂)₂-phenylene-(CH₂)₅-^{∗∗}, -(CH₂)₂-phenylene-(CH₂)₆-^{∗∗}, -(CH₂)₂-phenylene-(CH₂)₇-^{∗∗}, -(CH₂)₂-phenylene-(CH₂)₈-^{∗∗}, -(CH₂)₃-phenylene-CH₂-^{∗∗}, -(CH₂)₃-phenylene-(CH₂)₂-^{∗∗}, -(CH₂)₃-phenylene-(CH₂)₃-^{∗∗}, -(CH₂)₃-phenylene-(CH₂)₄-^{∗∗}, -(CH₂)₃-phenylene-(CH₂)₅-^{∗∗}, -(CH₂)₃-phenylene-(CH₂)₆-^{∗∗}, -(CH₂)₃-phenylene-(CH₂)₇-^{∗∗}, -(CH₂)₃-phenylene-(CH₂)₈-^{∗∗}, -(CH₂)₄-phenylene-CH₂-^{∗∗}, -(CH₂)₄-phenylene-(CH₂)₂-^{∗∗}, -(CH₂)₄-phenylene-(CH₂)₃-^{∗∗}, -(CH₂)₄-phenylene-(CH₂)₄-^{∗∗}, -(CH₂)₄-phenylene-(CH₂)₅-^{∗∗}, - (CH₂)₄-phenylene-(CH₂)₆-^{∗∗}, -(CH₂)₄-phenylene-(CH₂)₇-^{∗∗}, -(CH₂)₄-phenylene-(CH₂)₈-^{∗∗}, -(CH₂)₅-phenylene-CH₂-^{∗∗}, -(CH₂)₅-phenylene-(CH₂)₂-^{∗∗}, -(CH₂)₅-phenylene-(CH₂)₃-^{∗∗}, -(CH₂)₅-phenylene-(CH₂)₄-^{∗∗}, -(CH₂)₅-phenylene-(CH₂)₅-^{∗∗}, -(CH₂)₆-phenylene-CH₂-^{∗∗}, -(CH₂)₆-phenylene-(CH₂)₂-^{∗∗}, -(CH₂)₆-phenylene-(CH₂)₃-^{∗∗}, -(CH₂)₆-phenylene-(CH₂)₄-^{∗∗}, -(CH₂)₆-phenylene-(CH₂)₃-^{∗∗}, -(CH₂)₆-phenylene-(CH₂)₆-^{∗∗}, -(CH₂)₇-phenylene-CH₂-^{∗∗}, -(CH₂)₇-phenylene-(CH₂)₂-^{∗∗}, -(CH₂)₇-phenylene-(CH₂)₃-^{∗∗}, -(CH₂)₇-phenylene-(CH₂)₄-^{∗∗}, -(CH₂)₇-phenylene-(CH₂)₅-^{∗∗}, -(CH₂)₇-phenylene-(CH₂)₆-^{∗∗}, - (CH₂)₈-phenylene-CH₂-^{∗∗}, -(CH₂)₈-phenylene-(CH₂)₂-^{∗∗}, -(CH₂)₈-phenylene-(CH₂)₃-^{∗∗}, - (CH₂)₈-phenylene-(CH₂)₄-^{∗∗}, -(CH₂)₈-phenylene-(CH₂)₅-^{∗∗}, -(CH₂)₈-phenylene-(CH₂)₆-^{∗∗}, -(CH₂)₈-phenylene-(CH₂)₇-^{∗∗}, -(CH₂)₈-phenylene-(CH₂)₈-^{∗∗}, -CH₂-pyridylene-CH₂-^{∗∗}, -CH₂-pyridylene-(CH₂)₂-^{∗∗}, -CH₂-pyridylene-(CH₂)₃-^{∗∗}, -CH₂-pyridylene-(CH₂)₄-^{∗∗}, -CH₂-pyridylene-(CH₂)₃-^{∗∗}, -CH₂-pyridylene-(CH₂)₆-^{∗∗}, -CH₂-pyridylene-(CH₂)₇-^{∗∗}, - CH₂-pyridylene-(CH₂)₈-^{∗∗}, -(CH₂)₂-pyridylene-CH₂-^{∗∗}, -(CH₂)₂-pyridylene-(CH₂)₂-^{∗∗}, - (CH₂)₂-pyridylene-(CH₂)₃-^{∗∗}, -(CH₂)₂-pyridylene-(CH₂)₄-^{∗∗}, -(CH₂)₂-pyridylene-(CH₂)₅-^{∗∗}, -(CH₂)₂-pyridylene-(CH₂)₆-^{∗∗}, -(CH₂)₂-pyridylene-(CH₂)₇-^{∗∗}, -(CH₂)₂-pyridylene-(CH₂)₈-^{∗∗}, -(CH₂)₃-pyridylene-CH₂-^{∗∗}, -(CH₂)₃-pyridylene-(CH₂)₂-^{∗∗}, -(CH₂)₃-pyridylene-(CH₂)₃-^{∗∗}, -(CH₂)₃-pyridylene-(CH₂)₄-^{∗∗}, -(CH₂)₃-pyridylene-(CH₂)₅-^{∗∗}, - (CH₂)₃-pyridylene-(CH₂)₆-^{∗∗}, -(CH₂)₃-pyridylene-(CH₂)₇-^{∗∗}, -(CH₂)₃-pyridylene-(CH₂)₈-^{∗∗}, -(CH₂)₄-pyridylene-CH₂-^{∗∗}, -(CH₂)₄-pyridylene-(CH₂)₂-^{∗∗}, -(CH₂)₄-pyridylene-(CH₂)₃-^{∗∗}, -(CH₂)₄-pyridylene-(CH₂)₄-^{∗∗}, -(CH₂)₄-pyridylene-(CH₂)₅-^{∗∗}, -(CH₂)₄-pyridylene-(CH₂)₆-^{∗∗}, -(CH₂)₄-pyridylene-(CH₂)₇-^{∗∗}, -(CH₂)₄-pyridylene-(CH₂)₈-^{∗∗}, - (CH₂)₅-pyridylene-CH₂-^{∗∗}, -(CH₂)ₛ-pyridylene-(CH₂)₂-^{∗∗}, -(CH₂)₅-pyridylene-(CH₂)₃-^{∗∗}, -(CH₂)₅-pyridylene-(CH₂)₄-^{∗∗}, -(CH₂)₅-pyridylene-(CH₂)₅-^{∗∗}, -(CH₂)₆-pyridylene-CH₂-^{∗∗}, -(CH₂)₆-pyridylene-(CH₂)₂-^{∗∗}, -(CH₂)₆-pyridylene-(CH₂)₃-^{∗∗}, -(CH₂)₆-pyridylene-(CH₂)₄-^{∗∗}, -(CH₂)₆-pyridylene-(CH₂)₅-^{∗∗}, -(CH₂)₆-pyridylene-(CH₂)₆-^{∗∗}, -(CH₂)₇-pyridylene-CH₂-^{∗∗}, -(CH₂)₇-pyridylene-(CH₂)₂-^{∗∗}, -(CH₂)₇-pyridylene-(CH₂)₃-^{∗∗}, -(CH₂)₇-pyridylene-(CH₂)₄-^{∗∗}, -(CH₂)₇-pyridylene-(CH₂)₅-^{∗∗}, -(CH₂)₇-pyridylene-(CH₂)₆-^{∗∗}, - (CH₂)₈-pyridylene-CH₂-^{∗∗}, -(CH₂)₈-pyridylene-(CH₂)₂-^{∗∗}, -(CH₂)₈-pyridylene-(CH₂)₃-^{∗∗}, -(CH₂)₈-pyridylene-(CH₂)₄-^{∗∗}, -(CH₂)₈-pyridylene-(CH₂)₅-^{∗∗}, -(CH₂)₈-pyridylene-(CH₂)₆-^{∗∗}, -(CH₂)₈-pyridylene-(CH₂)₇-^{∗∗}, -(CH₂)₈-pyridylene-(CH₂)₈-^{∗∗}, -CH₂-thiazolylene-CH₂-^{∗∗}, -CH₂-thiazolylene-(CH₂)₂-^{∗∗}, -CH₂-thiazolylene-(CH₂)₃-^{∗∗}, -CH₂-thiazolylene-(CH₂)₄-^{∗∗}, -CH₂-thiazolylene-(CH₂)₃-^{∗∗}, -CH₂-thiazolylene-(CH₂)₆-^{∗∗}, -CH₂-thiazolylene-(CH₂)₇-^{∗∗}, -CH₂-thiazolylene-(CH₂)₈-^{∗∗}, -(CH₂)₂-thiazolylene-CH₂-^{∗∗}, - (CH₂)₂-thiazolylene-(CH₂)₂-^{∗∗}, -(CH₂)₂-thiazolylene-(CH₂)₃-^{∗∗}, -(CH₂)₂-thiazolylene-(CH₂)₄-^{∗∗}, -(CH₂)₂-thiazolylene-(CH₂)₅-^{∗∗}, -(CH₂)₂-thiazolylene-(CH₂)₆-^{∗∗}, -(CH₂)₂-thiazolylene-(CH₂)₇-^{∗∗}, -(CH₂)₂-thiazolylene-(CH₂)₈-^{∗∗}, -(CH₂)₃-thiazolylene-CH₂-^{∗∗}, - (CH₂)₃-thiazolylene-(CH₂)₂-^{∗∗}, -(CH₂)₃-thiazolylene-(CH₂)₃-^{∗∗}, -(CH₂)₃-thiazolylene-(CH₂)₄-^{∗∗}, -(CH₂)₃-thiazolylene-(CH₂)₅-^{∗∗}, -(CH₂)₃-thiazolylene-(CH₂)₆-^{∗∗}, -(CH₂)₃-thiazolylene-(CH₂)₇-^{∗∗}, -(CH₂)₃-thiazolylene-(CH₂)₈-^{∗∗}, -(CH₂)₄-thiazolylene-CH₂-^{∗∗}, - (CH₂)₄-thiazolylene-(CH₂)₂-^{∗∗}, -(CH₂)₄-thiazolylene-(CH₂)₃-^{∗∗}, -(CH₂)₄-thiazolylene-(CH₂)₄-^{∗∗}, -(CH₂)₄-thiazolylene-(CH₂)₅-^{∗∗}, -(CH₂)₄-thiazolylene-(CH₂)₆-^{∗∗}, -(CH₂)₄-thiazolylene-(CH₂)₇-^{∗∗}, -(CH₂)₄-thiazolylene-(CH₂)₈-^{∗∗}, -(CH₂)₅-thiazolylene-CH₂-^{∗∗}, - (CH₂)₅-thiazolylene-(CH₂)₂-^{∗∗}, -(CH₂)₅-thiazolylene-(CH₂)₃-^{∗∗}, -(CH₂)₅-thiazolylene-(CH₂)₄-^{∗∗}, -(CH₂)₃-thiazolylene-(CH₂)₃-^{∗∗}, -(CH₂)₆-thiazolylene-CH₂-^{∗∗}, -(CH₂)₆-thiazolylene-(CH₂)₂-^{∗∗}, -(CH₂)₆-thiazolylene-(CH₂)₃-^{∗∗}, -(CH₂)₆-thiazolylene-(CH₂)₄-^{∗∗}, -(CH₂)₆-thiazolylene-(CH₂)₅-^{∗∗}, -(CH₂)₆-thiazolylene-(CH₂)₆-^{∗∗}, -(CH₂)₇-thiazolylene-CH₂-^{∗∗}, -(CH₂)₇-thiazolylene-(CH₂)₂-^{∗∗}, -(CH₂)₇-thiazolylene-(CH₂)₃-^{∗∗}, -(CH₂)₇-thiazolylene-(CH₂)₄-^{∗∗}, -(CH₂)₇-thiazolylene-(CH₂)₅-^{∗∗}, -(CH₂)₇-thiazolylene-(CH₂)₆-^{∗∗}, -(CH₂)₈-thiazolylene-CH₂-^{∗∗}, -(CH₂)₈-thiazolylene-(CH₂)₂-^{∗∗}, -(CH₂)₈-thiazolylene-(CH₂)₃-^{∗∗}, -(CH₂)₈-thiazolylene-(CH₂)₄-^{∗∗}, -(CH₂)₈-thiazolylene-(CH₂)₅-^{∗∗}, -(CH₂)₈-thiazolylene-(CH₂)₆-^{∗∗}, -(CH₂)₈-thiazolylene-(CH₂)₇-^{∗∗}, or -(CH₂)₈-thiazolylene-(CH₂)₈-^{∗∗}, wherein symbol ** indicates the point of attachment to X;
wherein said phenylene, pyridylene, and thiazolylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof;
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted C₃₋₂₀ cycloalkyl, or optionally substituted C₃₋₂₀ heterocyclyl,
wherein Rₐ₇, Rₐ₈, Rₐ₉, Rₐ₁₀, Rₐ₁₆, Rₐ₁₇, Rₐ₂₁ and Rₐ₂₂ each independently represent H, optionally substituted linear or branched C₁₋₁₀ alkyl, optionally substituted C₃₋₂₀ cycloalkyl or optionally substituted C₃₋₂₀ heterocyclyl;
Rₐ₁₁ and Rₐ₂₃ each independently represent H or C₁₋₃ alkyl; and
Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
wherein said linear or branched C₁₋₁₀ alkyl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof; and
said C₃₋₂₀ cycloalkyl and said C₃₋₂₀ heterocyclyl are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents N(Rₐ₁), O, S, C₂₋₆ alkynylene (such as C₂₋₄ alkynylene, e.g., ethynylene or butynylene) or C₂₋₆ alkenylene (such as C₂₋₄ alkenylene, e.g., vinylene or butenylene), where Rₐ₁ represents H or C₁₋₃ alkyl; and L represents:
-CH₂-phenylene-CH₂-^{∗∗}, -CH₂-phenylene-(CH₂)₂-^{∗∗}, -CH₂-phenylene-(CH₂)₃-^{∗∗}, -CH₂-phenylene-(CH₂)₄-^{∗∗}, -CH₂-phenylene-(CH₂)₅-^{∗∗}, -CH₂-phenylene-(CH₂)₆-^{∗∗}, -CH₂-phenylene-(CH₂)₇-^{∗∗}, -CH₂-phenylene-(CH₂)₈-^{∗∗}, -(CH₂)₂-phenylene-CH₂-^{∗∗}, -(CH₂)₂-phenylene-(CH₂)₂-^{∗∗}, -(CH₂)₂-phenylene-(CH₂)₃-^{∗∗}, -(CH₂)₂-phenylene-(CH₂)₄-^{∗∗}, - (CH₂)₂-phenylene-(CH₂)₅-^{∗∗}, -(CH₂)₂-phenylene-(CH₂)₆-^{∗∗}, -(CH₂)₂-phenylene-(CH₂)₇-^{∗∗}, -(CH₂)₂-phenylene-(CH₂)₈-^{∗∗}, -(CH₂)₃-phenylene-CH₂-^{∗∗}, -(CH₂)₃-phenylene-(CH₂)₂-^{∗∗}, -(CH₂)₃-phenylene-(CH₂)₃-^{∗∗}, -(CH₂)₃-phenylene-(CH₂)₄-^{∗∗}, -(CH₂)₃-phenylene-(CH₂)₅-^{∗∗}, -(CH₂)₃-phenylene-(CH₂)₆-^{∗∗}, -(CH₂)₃-phenylene-(CH₂)₇-^{∗∗}, -(CH₂)₃-phenylene-(CH₂)₈-^{∗∗}, -(CH₂)₄-phenylene-CH₂-^{∗∗}, -(CH₂)₄-phenylene-(CH₂)₂-^{∗∗}, -(CH₂)₄-phenylene-(CH₂)₃-^{∗∗}, -(CH₂)₄-phenylene-(CH₂)₄-^{∗∗}, -(CH₂)₄-phenylene-(CH₂)₅-^{∗∗}, - (CH₂)₄-phenylene-(CH₂)₆-^{∗∗}, -(CH₂)₄-phenylene-(CH₂)₇-^{∗∗}, -(CH₂)₄-phenylene-(CH₂)₈-^{∗∗}, -(CH₂)₅-phenylene-CH₂-^{∗∗}, -(CH₂)₅-phenylene-(CH₂)₂-^{∗∗}, -(CH₂)₅-phenylene-(CH₂)₃-^{∗∗}, -(CH₂)₅-phenylene-(CH₂)₄-^{∗∗}, -(CH₂)₅-phenylene-(CH₂)₅-^{∗∗}, -(CH₂)₆-phenylene-CH₂-^{∗∗}, -(CH₂)₆-phenylene-(CH₂)₂-^{∗∗}, -(CH₂)₆-phenylene-(CH₂)₃-^{∗∗}, -(CH₂)₆-phenylene-(CH₂)₄-^{∗∗}, -(CH₂)₆-phenylene-(CH₂)₅-^{∗∗}, -(CH₂)₆-phenylene-(CH₂)₆-^{∗∗}, -(CH₂)₇-phenylene-CH₂-^{∗∗}, -(CH₂)₇-phenylene-(CH₂)₂-^{∗∗}, -(CH₂)₇-phenylene-(CH₂)₃-^{∗∗}, -(CH₂)₇-phenylene-(CH₂)₄-^{∗∗}, -(CH₂)₇-phenylene-(CH₂)₅-^{∗∗}, -(CH₂)₇-phenylene-(CH₂)₆-^{∗∗}, - (CH₂)₈-phenylene-CH₂-^{∗∗}, -(CH₂)₈-phenylene-(CH₂)₂-^{∗∗}, -(CH₂)₈-phenylene-(CH₂)₃-^{∗∗}, - (CH₂)₈-phenylene-(CH₂)₄-^{∗∗}, -(CH₂)₈-phenylene-(CH₂)₅-^{∗∗}, -(CH₂)₈-phenylene-(CH₂)₆-^{∗∗}, -(CH₂)₈-phenylene-(CH₂)₇-^{∗∗}, -(CH₂)₈-phenylene-(CH₂)₈-^{∗∗}, -CH₂-pyridylene-CH₂-^{∗∗}, -CH₂-pyridylene-(CH₂)₂-^{∗∗}, -CH₂-pyridylene-(CH₂)₃-^{∗∗}, -CH₂-pyridylene-(CH₂)₄-^{∗∗}, -CH₂-pyridylene-(CH₂)₃-^{∗∗}, -CH₂-pyridylene-(CH₂)₆-^{∗∗}, -CH₂-pyridylene-(CH₂)₇-^{∗∗}, - CH₂-pyridylene-(CH₂)₈-^{∗∗}, -(CH₂)₂-pyridylene-CH₂-^{∗∗}, -(CH₂)₂-pyridylene-(CH₂)₂-^{∗∗}, - (CH₂)₂-pyridylene-(CH₂)₃-^{∗∗}, -(CH₂)₂-pyridylene-(CH₂)₄-^{∗∗}, -(CH₂)₂-pyridylene-(CH₂)₅-^{∗∗}, -(CH₂)₂-pyridylene-(CH₂)₆-^{∗∗}, -(CH₂)₂-pyridylene-(CH₂)₇-^{∗∗}, -(CH₂)₂-pyridylene-(CH₂)₈-^{∗∗}, -(CH₂)₃-pyridylene-CH₂-^{∗∗}, -(CH₂)₃-pyridylene-(CH₂)₂-^{∗∗}, -(CH₂)₃-pyridylene-(CH₂)₃-^{∗∗}, -(CH₂)₃-pyridylene-(CH₂)₄-^{∗∗}, -(CH₂)₃-pyridylene-(CH₂)₅-^{∗∗}, - (CH₂)₃-pyridylene-(CH₂)₆-^{∗∗}, -(CH₂)₃-pyridylene-(CH₂)₇-^{∗∗}, -(CH₂)₃-pyridylene-(CH₂)₈-^{∗∗}, -(CH₂)₄-pyridylene-CH₂-^{∗∗}, -(CH₂)₄-pyridylene-(CH₂)₂-^{∗∗}, -(CH₂)₄-pyridylene-(CH₂)₃-^{∗∗}, -(CH₂)₄-pyridylene-(CH₂)₄-^{∗∗}, -(CH₂)₄-pyridylene-(CH₂)₅-^{∗∗}, -(CH₂)₄-pyridylene-(CH₂)₆-^{∗∗}, -(CH₂)₄-pyridylene-(CH₂)₇-^{∗∗}, -(CH₂)₄-pyridylene-(CH₂)₈-^{∗∗}, - (CH₂)₅-pyridylene-CH₂-^{∗∗}, -(CH₂)₅-pyridylene-(CH₂)₂-^{∗∗}, -(CH₂)₅-pyridylene-(CH₂)₃-^{∗∗}, -(CH₂)₅-pyridylene-(CH₂)₄-^{∗∗}, -(CH₂)₅-pyridylene-(CH₂)₅-^{∗∗}, -(CH₂)₆-pyridylene-CH₂-^{∗∗}, -(CH₂)₆-pyridylene-(CH₂)₂-^{∗∗}, -(CH₂)₆-pyridylene-(CH₂)₃-^{∗∗}, -(CH₂)₆-pyridylene-(CH₂)₄-^{∗∗}, -(CH₂)₆-pyridylene-(CH₂)₅-^{∗∗}, -(CH₂)₆-pyridylene-(CH₂)₆-^{∗∗}, -(CH₂)₇-pyridylene-CH₂-^{∗∗}, -(CH₂)₇-pyridylene-(CH₂)₂-^{∗∗}, -(CH₂)₇-pyridylene-(CH₂)₃-^{∗∗}, -(CH₂)₇-pyridylene-(CH₂)₄-^{∗∗}, -(CH₂)₇-pyridylene-(CH₂)₅-^{∗∗}, -(CH₂)₇-pyridylene-(CH₂)₆-^{∗∗}, - (CH₂)₈-pyridylene-CH₂-^{∗∗}, -(CH₂)₈-pyridylene-(CH₂)₂-^{∗∗}, -(CH₂)₈-pyridylene-(CH₂)₃-^{∗∗}, -(CH₂)₈-pyridylene-(CH₂)₄-^{∗∗}, -(CH₂)₈-pyridylene-(CH₂)₅-^{∗∗}, -(CH₂)₈-pyridylene-(CH₂)₆-^{∗∗}, -(CH₂)₈-pyridylene-(CH₂)₇-^{∗∗}, -(CH₂)₈-pyridylene-(CH₂)₈-^{∗∗}, -CH₂-thiazolylene-CH₂-^{∗∗}, -CH₂-thiazolylene-(CH₂)₂-^{∗∗}, -CH₂-thiazolylene-(CH₂)₃-^{∗∗}, -CH₂-thiazolylene-(CH₂)₄-^{∗∗}, -CH₂-thiazolylene-(CH₂)₅-^{∗∗}, -CH₂-thiazolylene-(CH₂)₆-^{∗∗}, -CH₂-thiazolylene-(CH₂)₇-^{∗∗}, -CH₂-thiazolylene-(CH₂)₈-^{∗∗}, -(CH₂)₂-thiazolylene-CH₂-^{∗∗}, - (CH₂)₂-thiazolylene-(CH₂)₂-^{∗∗}, -(CH₂)₂-thiazolylene-(CH₂)₃-^{∗∗}, -(CH₂)₂-thiazolylene-(CH₂)₄-^{∗∗}, -(CH₂)₂-thiazolylene-(CH₂)₅-^{∗∗}, -(CH₂)₂-thiazolylene-(CH₂)₆-^{∗∗}, -(CH₂)₂-thiazolylene-(CH₂)₇-^{∗∗}, -(CH₂)₂-thiazolylene-(CH₂)₈-^{∗∗}, -(CH₂)₃-thiazolylene-CH₂-^{∗∗}, - (CH₂)₃-thiazolylene-(CH₂)₂-^{∗∗}, -(CH₂)₃-thiazolylene-(CH₂)₃-^{∗∗}, -(CH₂)₃-thiazolylene-(CH₂)₄-^{∗∗}, -(CH₂)₃-thiazolylene-(CH₂)₅-^{∗∗}, -(CH₂)₃-thiazolylene-(CH₂)₆-^{∗∗}, -(CH₂)₃-thiazolylene-(CH₂)₇-^{∗∗}, -(CH₂)₃-thiazolylene-(CH₂)₈-^{∗∗}, -(CH₂)₄-thiazolylene-CH₂-^{∗∗}, - (CH₂)₄-thiazolylene-(CH₂)₂-^{∗∗}, -(CH₂)₄-thiazolylene-(CH₂)₃-^{∗∗}, -(CH₂)₄-thiazolylene-(CH₂)₄-^{∗∗}, -(CH₂)₄-thiazolylene-(CH₂)₅-^{∗∗}, -(CH₂)₄-thiazolylene-(CH₂)₆-^{∗∗}, -(CH₂)₄-thiazolylene-(CH₂)₇-^{∗∗}, -(CH₂)₄-thiazolylene-(CH₂)₈-^{∗∗}, -(CH₂)₅-thiazolylene-CH₂-^{∗∗}, - (CH₂)₅-thiazolylene-(CH₂)₂-^{∗∗}, -(CH₂)₅-thiazolylene-(CH₂)₃-^{∗∗}, -(CH₂)₅-thiazolylene-(CH₂)₄-^{∗∗}, -(CH₂)₅-thiazolylene-(CH₂)₅-^{∗∗}, -(CH₂)₆-thiazolylene-CH₂-^{∗∗}, -(CH₂)₆-thiazolylene-(CH₂)₂-^{∗∗}, -(CH₂)₆-thiazolylene-(CH₂)₃-^{∗∗}, -(CH₂)₆-thiazolylene-(CH₂)₄-^{∗∗}, -(CH₂)₆-thiazolylene-(CH₂)₅-^{∗∗}, -(CH₂)₆-thiazolylene-(CH₂)₆-^{∗∗}, -(CH₂)₇-thiazolylene-CH₂-^{∗∗}, -(CH₂)₇-thiazolylene-(CH₂)₂-^{∗∗}, -(CH₂)₇-thiazolylene-(CH₂)₃-^{∗∗}, -(CH₂)₇-thiazolylene-(CH₂)₄-^{∗∗}, -(CH₂)₇-thiazolylene-(CH₂)₅-^{∗∗}, -(CH₂)₇-thiazolylene-(CH₂)₆-^{∗∗}, -(CH₂)₈-thiazolylene-CH₂-^{∗∗}, -(CH₂)₈-thiazolylene-(CH₂)₂-^{∗∗}, -(CH₂)₈-thiazolylene-(CH₂)₃-^{∗∗}, -(CH₂)₈-thiazolylene-(CH₂)₄-^{∗∗}, -(CH₂)₈-thiazolylene-(CH₂)₅-^{∗∗}, -(CH₂)₈-thiazolylene-(CH₂)₆-^{∗∗}, -(CH₂)₈-thiazolylene-(CH₂)₇-^{∗∗}, or -(CH₂)₈-thiazolylene-(CH₂)₈-^{∗∗}, wherein symbol ** indicates the point of attachment to X;
wherein said phenylene, pyridylene, and thiazolylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof; and
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇ or OS(O)₂Rₐ₂₈,
wherein Rₐ₇ and Rₐ₈ each independently represent H, optionally substituted linear or branched C₁₋₁₀ alkyl, optionally substituted C₃₋₂₀ cycloalkyl or optionally substituted C₃₋₂₀ heterocyclyl;
Rₐ₉, Rₐ₁₁, Rₐ₂₁ 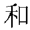 Rₐ₂₃ each independently represent H or C₁₋₃ alkyl; and
Rₐ₁₀, Rₐ₁₂, Rₐ₁₆, Rₐ₁₇, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl;
wherein examples of C₃₋₂₀ cycloalkyl include (but are not limited to):
   adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino or any combination thereof;
   noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, or any combination thereof;
   cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, or any combination thereof; and
   p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, or 1,7,7-trimethylbicyclo[2.2.1]heptan-6-yl, 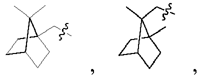 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, bicyclo[2.2.1]hept-5-en-7-yl, which are optionally substituted by one or more (such as 1-10, 1-9, 1-8, 1-7, 1-6, 1-5, 1-4, 1-3, 1-2, or 1, which can vary as required) substituents selected from the group consisting of:
      halogen, hydroxyl, mercapto, oxo, cyano, C₁₋₃ alkyl, halogenated C₁₋₃ alkyl, C₁₋₃ alkyl substituted by hydroxy, C₁₋₃ alkoxy, C₁₋₃ alkylamino, optionally substituted phenyl or any combination thereof, wherein said optionally substituted phenyl is optionally substituted by a substituent selected from the group consisting of halogen, hydroxyl, amino, cyano, mercapto, C₁₋₃ alkyl, or any combination thereof;
and wherein examples of C₃₋₂₀ heterocyclyl include (but are not limited to): azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are independently optionally substituted by one or more (such as 1-10, 1-9, 1-8, 1-7, 1-6, 1-5, 1-4, 1-3, 1-2, or 1, which can vary as required) substituents selected from the group consisting of:
   halogen, hydroxyl, mercapto, oxo, cyano, C₁₋₃ alkyl, halogenated C₁₋₃ alkyl, C₁₋₃ alkyl substituted by hydroxy, C₁₋₃ alkoxy, C₁₋₃ alkylamino, optionally substituted phenyl or any combination thereof, wherein said optionally substituted phenyl is optionally substituted by a substituent selected from the group consisting of halogen, hydroxyl, amino, cyano, mercapto, C₁₋₃ alkyl, or any combination thereof.

In one embodiment of the present disclosure, X represents N(Rₐ₁), O, S, C₂₋₆ alkynylene (such as C₂₋₄ alkynylene, e.g., ethynylene or butynylene) or C₂₋₆ alkenylene (such as C₂₋₄ alkenylene, e.g., vinylene or butenylene), where Rₐ₁ represents H or C₁₋₃ alkyl; and L represents:
-CH₂-phenylene-CH₂-^{∗∗}, -CH₂-phenylene-(CH₂)₂-^{∗∗}, -CH₂-phenylene-(CH₂)₃-^{∗∗}, -CH₂-phenylene-(CH₂)₄-^{∗∗}, -CH₂-phenylene-(CH₂)₅-^{∗∗}, -CH₂-phenylene-(CH₂)₆-^{∗∗}, -CH₂-phenylene-(CH₂)₇-^{∗∗}, -CH₂-phenylene-(CH₂)₈-^{∗∗}, -(CH₂)₂-phenylene-CH₂-^{∗∗}, -(CH₂)₂-phenylene-(CH₂)₂-^{∗∗}, -(CH₂)₂-phenylene-(CH₂)₃-^{∗∗}, -(CH₂)₂-phenylene-(CH₂)₄-^{∗∗}, - (CH₂)₂-phenylene-(CH₂)₅-^{∗∗}, -(CH₂)₂-phenylene-(CH₂)₆-^{∗∗}, -(CH₂)₂-phenylene-(CH₂)₇-^{∗∗}, -(CH₂)₂-phenylene-(CH₂)₈-^{∗∗}, -(CH₂)₃-phenylene-CH₂-^{∗∗}, -(CH₂)₃-phenylene-(CH₂)₂-^{∗∗}, -(CH₂)₃-phenylene-(CH₂)₃-^{∗∗}, -(CH₂)₃-phenylene-(CH₂)₄-^{∗∗}, -(CH₂)₃-phenylene-(CH₂)₃-^{∗∗}, -(CH₂)₃-phenylene-(CH₂)₆-^{∗∗}, -(CH₂)₃-phenylene-(CH₂)₇-^{∗∗}, -(CH₂)₃-phenylene-(CH₂)₈-^{∗∗}, -(CH₂)₄-phenylene-CH₂-^{∗∗}, -(CH₂)₄-phenylene-(CH₂)₂-^{∗∗}, -(CH₂)₄-phenylene-(CH₂)₃-^{∗∗}, -(CH₂)₄-phenylene-(CH₂)₄-^{∗∗}, -(CH₂)₄-phenylene-(CH₂)₅-^{∗∗}, - (CH₂)₄-phenylene-(CH₂)₆-^{∗∗}, -(CH₂)₄-phenylene-(CH₂)₇-^{∗∗}, -(CH₂)₄-phenylene-(CH₂)₈-^{∗∗}, -(CH₂)₅-phenylene-CH₂-^{∗∗}, -(CH₂)₅-phenylene-(CH₂)₂-^{∗∗}, -(CH₂)₅-phenylene-(CH₂)₃-^{∗∗}, -(CH₂)₅-phenylene-(CH₂)₄-^{∗∗}, -(CH₂)₅-phenylene-(CH₂)₅-^{∗∗}, -(CH₂)₆-phenylene-CH₂-^{∗∗}, -(CH₂)₆-phenylene-(CH₂)₂-^{∗∗}, -(CH₂)₆-phenylene-(CH₂)₃-^{∗∗}, -(CH₂)₆-phenylene-(CH₂)₄-^{∗∗}, -(CH₂)₆-phenylene-(CH₂)₅-^{∗∗}, -(CH₂)₆-phenylene-(CH₂)₆-^{∗∗}, -(CH₂)₇-phenylene-CH₂-^{∗∗}, -(CH₂)₇-phenylene-(CH₂)₂-^{∗∗}, -(CH₂)₇-phenylene-(CH₂)₃-^{∗∗}, -(CH₂)₇-phenylene-(CH₂)₄-^{∗∗}, -(CH₂)₇-phenylene-(CH₂)₅-^{∗∗}, -(CH₂)₇-phenylene-(CH₂)₆-^{∗∗}, - (CH₂)₈-phenylene-CH₂-^{∗∗}, -(CH₂)₈-phenylene-(CH₂)₂-^{∗∗}, -(CH₂)₈-phenylene-(CH₂)₃-^{∗∗}, - (CH₂)₈-phenylene-(CH₂)₄-^{∗∗}, -(CH₂)₈-phenylene-(CH₂)₅-^{∗∗}, -(CH₂)₈-phenylene-(CH₂)₆-^{∗∗}, -(CH₂)₈-phenylene-(CH₂)₇-^{∗∗}, -(CH₂)₈-phenylene-(CH₂)₈-^{∗∗}, -CH₂-pyridylene-CH₂-^{∗∗}, -CH₂-pyridylene-(CH₂)₂-^{∗∗}, -CH₂-pyridylene-(CH₂)₃-^{∗∗}, -CH₂-pyridylene-(CH₂)₄-^{∗∗}, -CH₂-pyridylene-(CH₂)₃-^{∗∗}, -CH₂-pyridylene-(CH₂)₆-^{∗∗}, -CH₂-pyridylene-(CH₂)₇-^{∗∗}, - CH₂-pyridylene-(CH₂)₈-^{∗∗}, -(CH₂)₂-pyridylene-CH₂-^{∗∗}, -(CH₂)₂-pyridylene-(CH₂)₂-^{∗∗}, - (CH₂)₂-pyridylene-(CH₂)₃-^{∗∗}, -(CH₂)₂-pyridylene-(CH₂)₄-^{∗∗}, -(CH₂)₂-pyridylene-(CH₂)₅-^{∗∗}, -(CH₂)₂-pyridylene-(CH₂)₆-^{∗∗}, -(CH₂)₂-pyridylene-(CH₂)₇-^{∗∗}, -(CH₂)₂-pyridylene-(CH₂)₈-^{∗∗}, -(CH₂)₃-pyridylene-CH₂-^{∗∗}, -(CH₂)₃-pyridylene-(CH₂)₂-^{∗∗}, -(CH₂)₃-pyridylene-(CH₂)₃-^{∗∗}, -(CH₂)₃-pyridylene-(CH₂)₄-^{∗∗}, -(CH₂)₃-pyridylene-(CH₂)₅-^{∗∗}, - (CH₂)₃-pyridylene-(CH₂)₆-^{∗∗}, -(CH₂)₃-pyridylene-(CH₂)₇-^{∗∗}, -(CH₂)₃-pyridylene-(CH₂)₈-^{∗∗}, -(CH₂)₄-pyridylene-CH₂-^{∗∗}, -(CH₂)₄-pyridylene-(CH₂)₂-^{∗∗}, -(CH₂)₄-pyridylene-(CH₂)₃-^{∗∗}, -(CH₂)₄-pyridylene-(CH₂)₄-^{∗∗}, -(CH₂)₄-pyridylene-(CH₂)₅-^{∗∗}, -(CH₂)₄-pyridylene-(CH₂)₆-^{∗∗}, -(CH₂)₄-pyridylene-(CH₂)₇-^{∗∗}, -(CH₂)₄-pyridylene-(CH₂)₈-^{∗∗}, - (CH₂)₅-pyridylene-CH₂-^{∗∗}, -(CH₂)₅-pyridylene-(CH₂)₂-^{∗∗}, -(CH₂)₅-pyridylene-(CH₂)₃-^{∗∗}, -(CH₂)₅-pyridylene-(CH₂)₄-^{∗∗}, -(CH₂)₅-pyridylene-(CH₂)₅-^{∗∗}, -(CH₂)₆-pyridylene-CH₂-^{∗∗}, -(CH₂)₆-pyridylene-(CH₂)₂-^{∗∗}, -(CH₂)₆-pyridylene-(CH₂)₃-^{∗∗}, -(CH₂)₆-pyridylene-(CH₂)₄-^{∗∗}, -(CH₂)₆-pyridylene-(CH₂)₅-^{∗∗}, -(CH₂)₆-pyridylene-(CH₂)₆-^{∗∗}, -(CH₂)₇-pyridylene-CH₂-^{∗∗}, -(CH₂)₇-pyridylene-(CH₂)₂-^{∗∗}, -(CH₂)₇-pyridylene-(CH₂)₃-^{∗∗}, -(CH₂)₇-pyridylene-(CH₂)₄-^{∗∗}, -(CH₂)₇-pyridylene-(CH₂)₅-^{∗∗}, -(CH₂)₇-pyridylene-(CH₂)₆-^{∗∗}, - (CH₂)₈-pyridylene-CH₂-^{∗∗}, -(CH₂)₈-pyridylene-(CH₂)₂-^{∗∗}, -(CH₂)₈-pyridylene-(CH₂)₃-^{∗∗}, -(CH₂)₈-pyridylene-(CH₂)₄-^{∗∗}, -(CH₂)₈-pyridylene-(CH₂)₅-^{∗∗}, -(CH₂)₈-pyridylene-(CH₂)₆-^{∗∗}, -(CH₂)₈-pyridylene-(CH₂)₇-^{∗∗}, -(CH₂)₈-pyridylene-(CH₂)₈-^{∗∗}, -CH₂-thiazolylene-CH₂-^{∗∗}, -CH₂-thiazolylene-(CH₂)₂-^{∗∗}, -CH₂-thiazolylene-(CH₂)₃-^{∗∗}, -CH₂-thiazolylene-(CH₂)₄-^{∗∗}, -CH₂-thiazolylene-(CH₂)₅-^{∗∗}, -CH₂-thiazolylene-(CH₂)₆-^{∗∗}, -CH₂-thiazolylene-(CH₂)₇-^{∗∗}, -CH₂-thiazolylene-(CH₂)₈-^{∗∗}, -(CH₂)₂-thiazolylene-CH₂-^{∗∗}, - (CH₂)₂-thiazolylene-(CH₂)₂-^{∗∗}, -(CH₂)₂-thiazolylene-(CH₂)₃-^{∗∗}, -(CH₂)₂-thiazolylene-(CH₂)₄-^{∗∗}, -(CH₂)₂-thiazolylene-(CH₂)₅-^{∗∗}, -(CH₂)₂-thiazolylene-(CH₂)₆-^{∗∗}, -(CH₂)₂-thiazolylene-(CH₂)₇-^{∗∗}, -(CH₂)₂-thiazolylene-(CH₂)₈-^{∗∗}, -(CH₂)₃-thiazolylene-CH₂-^{∗∗}, - (CH₂)₃-thiazolylene-(CH₂)₂-^{∗∗}, -(CH₂)₃-thiazolylene-(CH₂)₃-^{∗∗}, -(CH₂)₃-thiazolylene-(CH₂)₄-^{∗∗}, -(CH₂)₃-thiazolylene-(CH₂)₅-^{∗∗}, -(CH₂)₃-thiazolylene-(CH₂)₆-^{∗∗}, -(CH₂)₃-thiazolylene-(CH₂)₇-^{∗∗}, -(CH₂)₃-thiazolylene-(CH₂)₈-^{∗∗}, -(CH₂)₄-thiazolylene-CH₂-^{∗∗}, - (CH₂)₄-thiazolylene-(CH₂)₂-^{∗∗}, -(CH₂)₄-thiazolylene-(CH₂)₃-^{∗∗}, -(CH₂)₄-thiazolylene-(CH₂)₄-^{∗∗}, -(CH₂)₄-thiazolylene-(CH₂)₅-^{∗∗}, -(CH₂)₄-thiazolylene-(CH₂)₆-^{∗∗}, -(CH₂)₄-thiazolylene-(CH₂)₇-^{∗∗}, -(CH₂)₄-thiazolylene-(CH₂)₈-^{∗∗}, -(CH₂)₅-thiazolylene-CH₂-^{∗∗}, - (CH₂)₅-thiazolylene-(CH₂)₂-^{∗∗}, -(CH₂)₅-thiazolylene-(CH₂)₃-^{∗∗}, -(CH₂)₅-thiazolylene-(CH₂)₄-^{∗∗}, -(CH₂)₃-thiazolylene-(CH₂)₃-^{∗∗}, -(CH₂)₆-thiazolylene-CH₂-^{∗∗}, -(CH₂)₆-thiazolylene-(CH₂)₂-^{∗∗}, -(CH₂)₆-thiazolylene-(CH₂)₃-^{∗∗}, -(CH₂)₆-thiazolylene-(CH₂)₄-^{∗∗}, -(CH₂)₆-thiazolylene-(CH₂)₅-^{∗∗}, -(CH₂)₆-thiazolylene-(CH₂)₆-^{∗∗}, -(CH₂)₇-thiazolylene-CH₂-^{∗∗}, -(CH₂)₇-thiazolylene-(CH₂)₂-^{∗∗}, -(CH₂)₇-thiazolylene-(CH₂)₃-^{∗∗}, -(CH₂)₇-thiazolylene-(CH₂)₄-^{∗∗}, -(CH₂)₇-thiazolylene-(CH₂)₅-^{∗∗}, -(CH₂)₇-thiazolylene-(CH₂)₆-^{∗∗}, -(CH₂)₈-thiazolylene-CH₂-^{∗∗}, -(CH₂)₈-thiazolylene-(CH₂)₂-^{∗∗}, -(CH₂)₈-thiazolylene-(CH₂)₃-^{∗∗}, -(CH₂)₈-thiazolylene-(CH₂)₄-^{∗∗}, -(CH₂)₈-thiazolylene-(CH₂)₅-^{∗∗}, -(CH₂)₈-thiazolylene-(CH₂)₆-^{∗∗}, -(CH₂)₈-thiazolylene-(CH₂)₇-^{∗∗}, or -(CH₂)₈-thiazolylene-(CH₂)₈-^{∗∗}, wherein symbol ^{∗∗} indicates the point of attachment to X;
wherein said phenylene, pyridylene, and thiazolylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof; and
R represents:
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)-, or any combination thereof;
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)-, or any combination thereof;
cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)-, or any combination thereof; or
p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, or 1,7,7-trimethylbicyclo[2.2.1]heptan-6-yl, 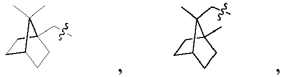 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl;
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are optionally substituted by one or more substituent selected from the group consisting of:
   halogen, hydroxyl, mercapto, oxo, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)-, optionally substituted phenyl or any combination thereof, wherein said optionally substituted phenyl is optionally substituted by a substituent selected from the group consisting of halogen, hydroxyl, amino, cyano, mercapto, C₁₋₃ alkyl, or any combination thereof.

In one embodiment of the present disclosure, X represents N(Rₐ₁), O, S, C₂₋₆ alkynylene (such as C₂₋₄ alkynylene, e.g., ethynylene or butynylene) or C₂₋₆ alkenylene (such as C₂₋₄ alkenylene, e.g., vinylene or butenylene), where Rₐ₁ represents H or C₁₋₃ alkyl; and R-L- in formula (I) represents:

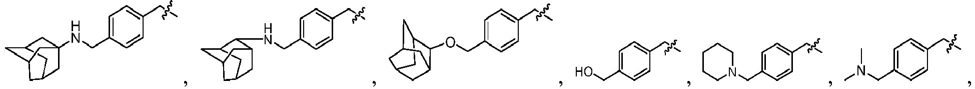

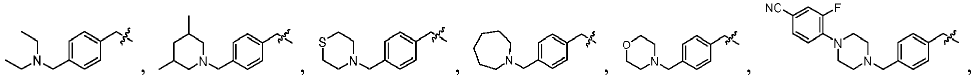

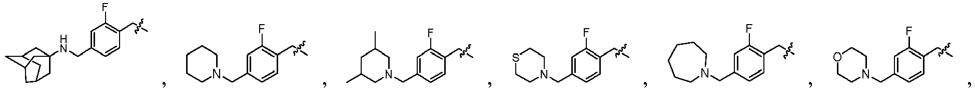

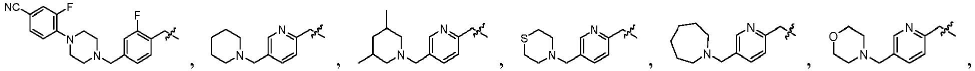

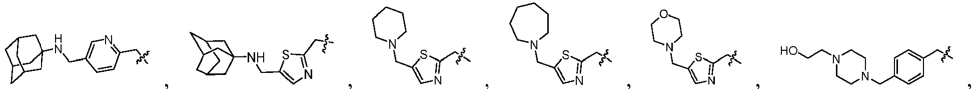

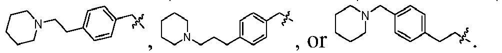

In one embodiment of the present disclosure, X represents N(Rₐ₁), O, or S, where Rₐ₁ represents H or C₁₋₃ alkyl; and
L represents the groups represented by the following formula:
   -C₃₋₁₅cycloalkylene-(CH₂)₁₋₄₀-^{∗}, or -C₅₋₁₅arylene-(CH₂)₁₋₄₀-^{∗}, wherein symbol * indicates the point of attachment to X; and
   wherein said C₃₋₁₅cycloalkylene is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, or any combination thereof, and said C₅₋₁₅ arylene is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof (wherein, e.g., in a sub-embodiment, said C₅₋₁₅ arylene includes, but is not limited to, phenylene and naphthylene; in a sub-embodiment, said C₃₋₁₅cycloalkylene includes, but is not limited to, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, C₅₋₁₅ spiro-cycloalkylene, adamantanylene, noradamantanylene, bornylene, bicyclo[2.2.1]heptylene, and bicyclo[2.2.1]heptenylene); and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇ or OS(O)₂Rₐ₂₈,
   wherein Rₐ₉, Rₐ₁₁, Rₐ₂₁, and Rₐ₂₃ each independently represent H or C₁₋₃ alkyl; and
   Rₐ₁₀, Rₐ₁₂, Rₐ₁₆, Rₐ₁₇, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl;
wherein said C₃₋₂₀ cycloalkyl and C₃₋₂₀ heterocyclyl are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents N(Rₐ₁), O, or S, where Rₐ₁ represents H or C₁₋₃ alkyl; and L represents:
-cyclohexylene-CH₂-^{∗}, -cyclohexylene-(CH₂)₂-^{∗}, -cyclohexylene-(CH₂)₃-^{∗}, -cyclohexylene-(CH₂)₄-^{∗}, -cyclohexylene-(CH₂)₃-^{∗}, -cyclohexylene-(CH₂)₆-^{∗}, -cyclohexylene-(CH₂)₇-^{∗}, -cyclohexylene-(CH₂)₈-^{∗}, -cyclohexylene-(CH₂)₉-^{∗}, -cyclohexylene-(CH₂)₁₀-^{∗}, -cyclohexylene-(CH₂)₁₁-^{∗}, - cyclohexylene-(CH₂)₁₂-^{∗}, -phenylene-CH₂-^{∗}, -phenylene-(CH₂)₂-^{∗}, -phenylene-(CH₂)₃-^{∗}, - phenylene-(CH₂)₄-^{∗}, -phenylene-(CH₂)₃-^{∗}, -phenylene-(CH₂)₆-^{∗}, -phenylene-(CH₂)₇-^{∗}, - phenylene-(CH₂)₈-^{∗}, -phenylene-(CH₂)₉-^{∗}, -phenylene-(CH₂)₁₀-^{∗}, -phenylene-(CH₂)₁₁-^{∗}, or - phenylene-(CH₂)₁₂-^{∗}, wherein symbol * indicates the point of attachment to X; and
   wherein said cyclohexylene is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, or any combination thereof, and said phenylene is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof;
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇ or OS(O)₂Rₐ₂₈,
   wherein Rₐ₉, Rₐ₁₁, Rₐ₂₁ and Rₐ₂₃ each independently represent H or C₁₋₃ alkyl; and
   Rₐ₁₀, Rₐ₁₂, Rₐ₁₆, Rₐ₁₇, Rₐ₁₈, Rₐ₁₉, Rₐ₂₀, Rₐ₂₂, Rₐ₂₄, Rₐ₂₅, Rₐ₂₆, Rₐ₂₇ and Rₐ₂₈ each independently represent:
      adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, or any combination thereof; or
      noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, or any combination thereof; or
      cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, or any combination thereof; or
      p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, or 1,7,7-trimethylbicyclo[2.2.1]heptan-6-yl, 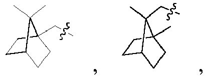 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, bicyclo[2.2.1]hept-5-en-7-yl, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are independently optionally substituted by one or more (such as 1-10, 1-9, 1-8, 1-6, 1-5, 1-4, 1-3, 1-2, or 1, which can vary as required) substituents selected from the group consisting of:
      halogen, hydroxyl, mercapto, oxo, cyano, C₁₋₃ alkyl, halogenated C₁₋₃ alkyl, C₁₋₃ alkyl substituted by hydroxy, C₁₋₃ alkoxy, C₁₋₃ alkylamino, optionally substituted phenyl or any combination thereof, wherein said optionally substituted phenyl is optionally substituted by a substituent selected from the group consisting of halogen, hydroxyl, amino, cyano, mercapto, C₁₋₃ alkyl, or any combination thereof.

In one embodiment of the present disclosure, X represents N(Rₐ₁), O, or S, where Rₐ₁ represents H or C₁₋₃ alkyl; and R-L- in formula (I) represents:

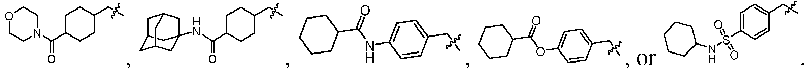

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and L represents the groups represented by the following formula:
-(CH₂)₁₋₄₀-C₅₋₁₅arylene-#, or -(CH2)₁₋₄₀-C₅₋₁₅heteroarylene-#, wherein symbol # indicates the point of attachment to X;
   wherein said C₅₋₁₅arylene and C₅₋₁₅heteroarylene are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof (wherein for example in a sub-embodiment, said C₅₋₁₅ arylene includes, but is not limited to, phenylene and naphthylene; in a sub-embodiment, said C₅₋₁₅heteroarylene includes, but is not limited to, pyridylene and thiazolylene); and
R represents:
   NR_{b7}R_{b8}, C(O)NR_{b9}R_{b10}, N(R_{b11})C(O)R_{b12}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, optionally substituted C₃₋₂₀ cycloalkyl or optionally substituted C₃₋₂₀ heterocyclyl,
   wherein R_{b7}, R_{b8}, R_{b9}, R_{b10}, Rₐ₂₁ and R_{b22} are the same or different and each independently represent H, optionally substituted linear or branched C₁₋₁₀ alkyl, optionally substituted C₃₋₂₀ cycloalkyl or optionally substituted C₃₋₂₀ heterocyclyl;
   R_{b16}, R_{b17}, R_{b29} and R_{b30} each independently represent H, optionally substituted linear or branched C₁₋₁₀ alkyl, optionally substituted C₃₋₂₀ cycloalkyl or optionally substituted C₃₋₂₀ heterocyclyl;
   R_{b11} and R_{b23} each independently represent H or C₁₋₃ alkyl; and
   R_{b12}, R_{b18}, R_{b19}, R_{b20}, R_{b24}, R_{b25}, R_{b26}, R_{b27}, and R_{b28} each independently represent optionally substituted linear or branched C₁₋₁₀ alkyl, optionally substituted C₃₋₂₀ cycloalkyl or optionally substituted C₃₋₂₀ heterocyclyl; and
   wherein said linear or branched C₁₋₁₀ alkyl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkoxy, C₁₋₃ alkylamino, C₁₋₃ alkyl-NHC(O)- or any combination thereof;
   said C₃₋₂₀ cycloalkyl and C₃₋₂₀ heterocyclyl are each independently optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and L represents the group represented by the following formula:
-CH₂-phenylene-#, -(CH₂)₂-phenylene-#, -(CH₂)₃-phenylene-#, -(CH₂)₄-phenylene-#, -(CH₂)₅-phenylene-#, -(CH₂)₆-phenylene-#, -(CH₂)₇-phenylene-#, -(CH₂)₈-phenylene-#, -(CH₂)₉-phenylene-#, -(CH₂)₁₀-phenylene-#, -(CH₂)₁₁-phenylene-#, -(CH₂)₁₂-phenylene-#, -CH₂-pyridylene-#, -(CH₂)₂-pyridylene-#, -(CH₂)₃-pyridylene-#, -(CH₂)₄-pyridylene-#, -(CH₂)₅-pyridylene-#, -(CH₂)₆-pyridylene-#, -(CH₂)₇-pyridylene-#, -(CH₂)₈-pyridylene-#, -(CH₂)₉-pyridylene-#, -(CH₂)₁₀-pyridylene-#, -(CH₂)₁₁-pyridylene-#, -(CH₂)₁₂-pyridylene-#, -CH₂-thiazolylene-#, -(CH₂)₂-thiazolylene-#, -(CH₂)₃-thiazolylene-#, -(CH₂)₄-thiazolylene-#, -(CH₂)₅-thiazolylene-#, -(CH₂)₆-thiazolylene-#, -(CH₂)₇-thiazolylene-#, -(CH₂)₈-thiazolylene-#, -(CH₂)₉-thiazolylene-#, -(CH₂)₁₀-thiazolylene-#, -(CH₂)₁₁-thiazolylene-#, or -(CH₂)₁₂-thiazolylene-#,
   wherein symbol # indicates the point of attachment to X; and
   wherein said phenylene, pyridylene and thiazolylene are each independently optionally substituted by e.g., one or more (such as 1-5, 1-4, 1-3, 1-2, or 1, which can vary as required) substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof; and
R represents:
   adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
   noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
   cyclopropyl, cyclobutyl, cyclopentyl, cyclopentenyl, cyclohexyl, cyclohexenyl, cycloheptyl, or cyclooctyl, which are optionally substituted by one or more (such as 1-10, 1-9, 1-8, 1-7, 1-6, 1-5, 1-4, 1-3, 1-2, or 1, which can vary as required) substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
   p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, or 1,7,7-trimethylbicyclo[2.2.1]heptan-6-yl, 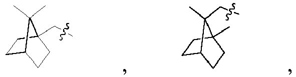 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl; or
   azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are optionally substituted by one or more (such as 1-10, 1-9, 1-8, 1-7, 1-6, 1-5, 1-4, 1-3, 1-2, or 1, which can vary as required) substituents selected from the group consisting of:
   halogen, hydroxyl, mercapto, oxo, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)-, optionally substituted phenyl or any combination thereof, wherein said optionally substituted phenyl is optionally substituted by a substituent selected from the group consisting of halogen, hydroxyl, amino, cyano, mercapto, C₁₋₃ alkyl, or any combination thereof.

In one embodiment of the present disclosure, X represents C₂₋₆ alkynylene (such as C₂₋₄ alkynylene, e.g., ethynylene or butynylene) or C₂₋₆ alkenylene (such as C₂₋₄ alkenylene, e.g., vinylene or butenylene); and R-L- in formula (I) represents:

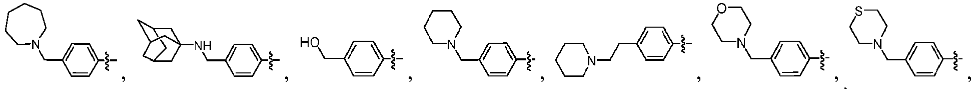

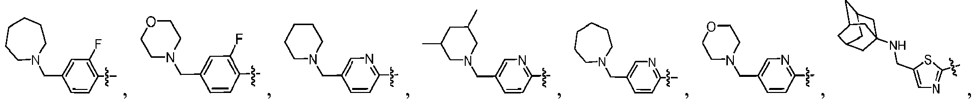

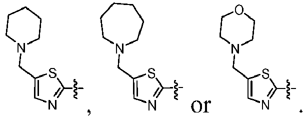

In one embodiment of the present disclosure, the compound of Formula (I) is also a compound of Formula (Ia): 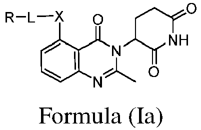 wherein X, L and R are as defined in Formula (I) above, and also as defined in various embodiments thereof.

In one embodiment of the present disclosure, the compound of Formula (I) is also a compound of Formula (Ib): 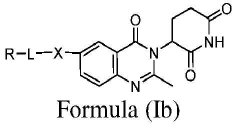 wherein X, L and R are as defined in Formula (I) above, and also as defined in various embodiments thereof.

In one embodiment of the present disclosure, the compound of Formula (I) is also a compound of Formula (Ic): 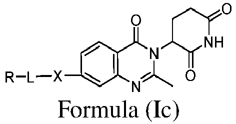 wherein X, L and R are as defined in Formula (I) above, and also as defined in various embodiments thereof.

In one embodiment of the present disclosure, the compound of Formula (I) is also a compound of Formula (Id): 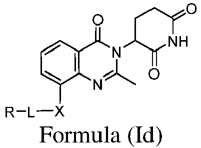 wherein X, L and R are as defined in Formula (I) above, and also as defined in various embodiments thereof.

Particularly preferred are the compounds of the present invention and their salts (especially pharmaceutically acceptable salts, such as hydrochloride, etc.), enantiomers, stereoisomers, solvates, or polymorphs thereof in Table 1:

**Table 1. The compounds of the present invention**

| Compound No. | Structure of the compounds | The compound's name |
|---|---|---|
| SIAIS2641 77 | 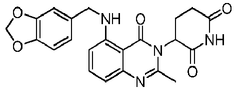 | 3-(5-((benzo[d] [1,3]dioxol-5-ylmethyl)amino)-2-methyl-4-oxoquinazolin-3(4H)-yl)piperidine-2,6-dione |
| SIAIS2640 66 | 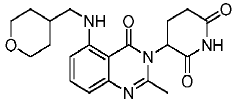 | 3-(2-methyl-4-oxo-5-(((tetrahydro-2H-pyran-4-yl)methyl)amino)quinazolin-3(4H)-yl)piperidine-2,6-dione |
| SIAIS2641 20 | 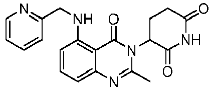 | 3-(2-methyl-4-oxo-5-((pyridin-2-ylmethyl)amino)quinazolin-3(4H)-yl)piperidine-2,6-dione |
| SIAIS2641 23 | 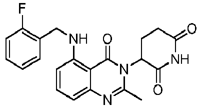 | 3-(5-((2-fluorobenzyl)amino)-2-methyl-4-oxoquinazolin-3(4H)-yl)piperidine-2,6-dione |
| SIAIS2641 56 | 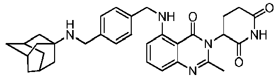 | 3-(5-((4-(((1R,3R,SS)-adamantan-1-ylamino)methyl)benzyl)amino)-2-methyl-4-oxoquinazolin-3(4H)-yl)piperidine-2,6-dione |
| SIAIS2641 72 | 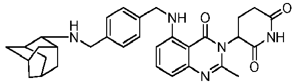 | 3-(5-((4-(((1R,2s,3S,5r)-adamantan-2-ylamino)methyl)benzyl)amino)-2-methyl-4-oxoquinazolin-3(4H)-yl)piperidine-2,6-dione |
| | 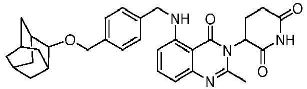 | 3-(5-((4-((adamantan-2-yloxy)methyl)benzyl)amino)-2-methyl-4-oxoquinazolin-3(4H)-yl)piperidine-2,6-dione |
| SIAIS3130 55 | 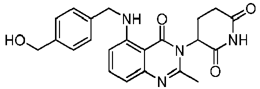 | 3-(5-((4-(hydroxymethyl)benzyl)amino)-2-methyl-4-oxoquinazolin-3(4H)-yl)piperidine-2,6-dione |
| SIAIS3130 69 | 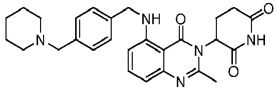 | 3-(2-methyl-4-oxo-5-((4-(piperidin-1- ylmethyl)benzyl)amino)quinazolin-3(4H)-yl)piperidine-2,6-dione |
| | 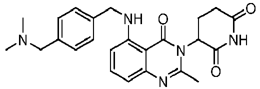 | 3-(5-((4-((dimethylamino)methyl)benzyl)amino)-2-methyl-4-oxoquinazolin-3(4H)-yl)piperidine-2,6-dione |
| | 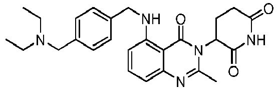 | 3-(5-((4-((diethylamino)methyl)benzyl)amino)-2-methyl-4-oxoquinazolin-3(4H)-yl)piperidine-2,6-dione |
| SIAIS3130 75 | 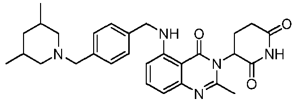 | 3-(5-((4-((3,5-dimethylpiperidin-1-yl)methyl)benzyl)amino)-2-methyl-4-oxoquinazolin-3(4H)-yl)piperidine-2,6-dione |
| SIAIS3130 74 | 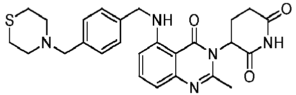 | 3-(2-methyl-4-oxo-5-((4-(thiomorpholinomethyl)benzyl)amino)quinazolin-3(4H)-yl)piperidine-2,6-dione |
| SIAIS3130 87 | 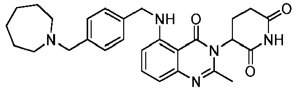 | 3-(5-((4-(azepan-1-ylmethyl)benzyl)amino)-2-methyl-4-oxoquinazolin-3(4H)-yl)piperidine-2,6-dione |
| SIAIS2640 67 | 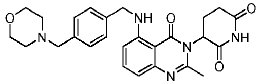 | 3-(2-methyl-5-((4-(morpholinomethyl)benzyl)amino)-4-oxoquinazolin-3(4H)-yl)piperidine-2,6-dione |
| | 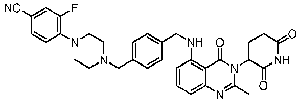 | 4-(4-(4-(((3-(2,6-dioxopiperidin-3-yl)-2-methyl-4-oxo-3,4-dihydroquinazolin-5-yl)amino)methyl)benzyl)piperazin-1-yl)-3-fluorobenzonitrile |
| SIAIS2640 97 | 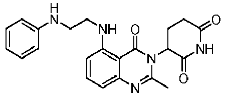 | 3-(2-methyl-4-oxo-5-((2-(phenylamino)ethyl)amino)quinazolin-3(4H)-yl)piperidine-2,6-dione |
| SIAIS2641 03 | 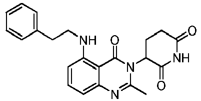 | 3-(2-methyl-4-oxo-5-(phenethylamino)quinazolin-3(4H)-yl)piperidine-2,6-dione |
| SIAIS3550 49 | 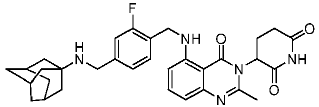 | 3-(5-((4-((adamantan-1-ylamino)methyl)-2-fluorobenzyl)amino)-2-methyl-4-oxoquinazolin-3(4H)-yl)piperidine-2,6-dione |
| | 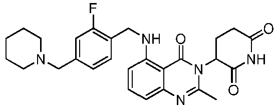 | 3-(5-((2-fluoro-4-(piperidin-1-ylmethyl)benzyl)amino)-2-methyl-4-oxoquinazolin-3(4H)-yl)piperidine-2,6-dione |
| | 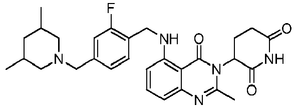 | 3-(5-((4-((3,5-dimethylpiperidin-1-yl)methyl)-2-fluorobenzyl)amino)-2-methyl-4-oxoquinazolin-3(4H)-yl)piperidine-2,6-dione |
| | 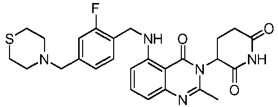 | 3-(5-((2-fluoro-4-(thiomorpholinomethyl)benzyl)amino)-2-methyl-4-oxoquinazolin-3(4H)-yl)piperidine-2,6-dione |
| SIAIS3550 35 | 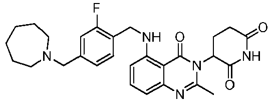 | 3-(5-((4-(azepan-1-ylmethyl)-2-fluorobenzyl)amino)-2-methyl-4-oxoquinazolin-3(4H)-yl)piperidine-2,6-dione |
| | 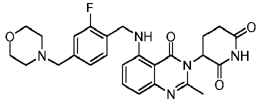 | 3-(5-((2-fluoro-4-(morpholinomethyl)benzyl)amino)-2-methyl-4-oxoquinazolin-3(4H)-yl)piperidine-2,6-dione |
| | 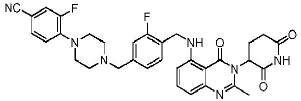 | 4-(4-(4-(((3-(2,6-dioxopiperidin-3-yl)-2-methyl-4-oxo-3,4-dihydroquinazolin-5-yl)amino)methyl)-3-fluorobenzyl)piperazin-1-yl)-3-fluorobenzonitrile |
| | 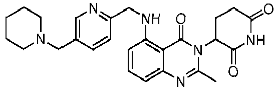 | 3-(2-methyl-4-oxo-5-(((5-(piperidin-1-ylmethyl)pyridin-2-yl)methyl)amino)quinazolin-3(4H)-yl)piperidine-2,6-dione |
| | 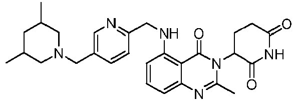 | 3-(5-(((5-((3,5-dimethylpiperidin-1-yl)methyl)pyridin-2-yl)methyl)amino)-2-methyl-4-oxoquinazolin-3(4H)-yl)piperidine-2,6-dione |
| | 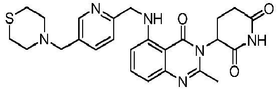 | 3-(2-methyl-4-oxo-5-(((5-(thiomorpholinomethyl)pyridin-2-yl)methyl)amino)quinazolin-3(4H)-yl)piperidine-2,6-dione |
| SIAIS3550 62 | 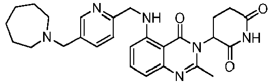 | 3-(5-(((5-(azepan-1-ylmethyl)pyridin-2-yl)methyl)amino)-2-methyl-4-oxoquinazolin-3(4H)-yl)piperidine-2,6-dione |
| SIAIS3550 68 | 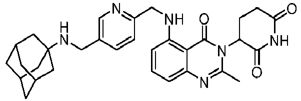 | 3-(5-(((5-(((adamantan-1-yl)amino)methyl)pyridin-2-yl)methyl)amino)-2-methyl-4-oxoquinazolin-3(4H)-yl)piperidine-2,6-dione |
| | 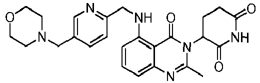 | 3-(2-methyl-5-(((5-(morpholinomethyl)pyridin-2-yl)methyl)amino)-4-oxoquinazolin-3(4H)-yl)piperidine-2,6-dione |
| | 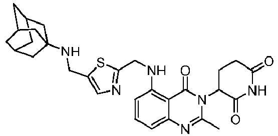 | 3-(5-(((5-((adamantan-1-ylamino)methyl)thiazol-2-yl)methyl)amino)-2-methyl-4-oxoquinazolin-3(4H)-yl)piperidine-2,6-dione |
| | 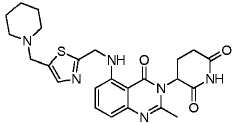 | 3-(2-methyl-4-oxo-5-(((5-(piperidin-1-ylmethyl)thiazol-2-yl)methyl)amino)quinazolin-3(4H)-yl)piperidine-2,6-dione |
| | 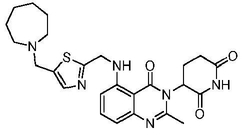 | 3-(5-(((5-(azepan-1-ylmethyl)thiazol-2-yl)methyl)amino)-2-methyl-4-oxoquinazolin-3(4H)-yl)piperidine-2,6-dione |
| | 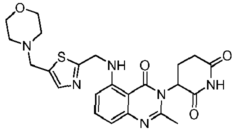 | 3-(2-methyl-5-(((5-(morpholinomethyl)thiazol-2-yl)methyl)amino)-4-oxoquinazolin-3(4H)-yl)piperidine-2,6-dione |
| SIAIS3130 67 | 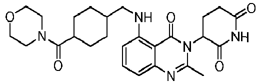 | 3-(2-methyl-5-(((4-(morpholine-4-carbonyl)cyclohexyl)methyl)amino)-4-oxoquinazolin-3(4H)-yl)piperidine-2,6-dione |
| SIAIS3130 68 | 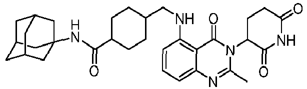 | N-((3s,5s,7s)-adamantan-1-yl)-4-(((3-(2,6-dioxopiperidin-3-yl)-2-methyl-4-oxo-3,4-dihydroquinazolin-5-yl)amino)methyl)cyclohexanecarboxamide |
| | 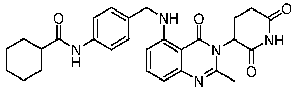 | N-(4-(((3-(2,6-dioxopiperidin-3-yl)-2-methyl-4-oxo-3,4-dihydroquinazolin-5-yl)amino)methyl)phenyl)cyclohexanecarboxamide |
| | 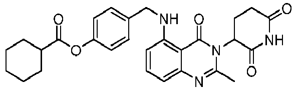 | 4-(((3-(2,6-dioxopiperidin-3-yl)-2-methyl-4-oxo-3,4-dihydroquinazolin-5-yl)amino)methyl)phenyl cyclohexanecarboxylate |
| | 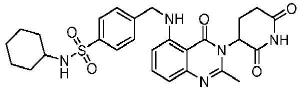 | N-cyclohexyl-4-(((3-(2,6-dioxopiperidin-3-yl)-2-methyl-4-oxo-3,4-dihydroquinazolin-5-yl)amino)methyl)benzenesulfonamide |
| | 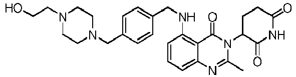 | 3-(5-((4-((4-(2-hydroxyethyl)piperazin-1-yl)methyl)benzyl)amino)-2-methyl-4-oxoquinazolin-3(4H)-yl)piperidine-2,6-dione |
| | 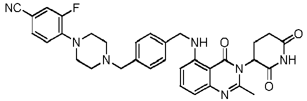 | 4-(4-(4-(((3-(2,6-dioxopiperidin-3-yl)-2-methyl-4-oxo-3,4-dihydroquinazolin-5-yl)amino)methyl)benzyl)piperazin-1-yl)-3-fluorobenzonitrile |
| | 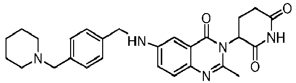 | 3-(2-methyl-4-oxo-6-((4-(piperidin-1- ylmethyl)benzyl)amino)quinazolin-3(4H)-yl)piperidine-2,6-dione |
| | 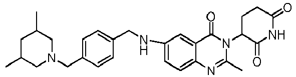 | 3-(6-((4-((3,5-dimethylpiperidin-1-yl)methyl)benzyl)amino)-2-methyl-4-oxoquinazolin-3(4H)-yl)piperidine-2,6-dione |
| | 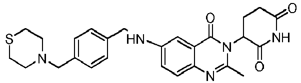 | 3-(2-methyl-4-oxo-6-((4-(thiomorpholinomethyl)benzyl)amino)quinazolin-3(4H)-yl)piperidine-2,6-dione |
| SIAIS3550 28 | 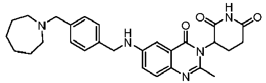 | 3-(6-((4-(azepan-1-ylmethyl)benzyl)amino)-2-methyl-4-oxoquinazolin-3(4H)-yl)piperidine-2,6-dione |
| | 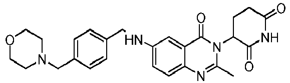 | 3-(2-methyl-6-((4-(morpholinomethyl)benzyl)amino)-4-oxoquinazolin-3(4H)-yl)piperidine-2,6-dione |
| | 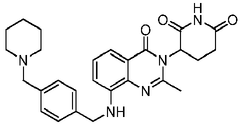 | 3-(2-methyl-4-oxo-8-((4-(piperidin-1-ylmethyl)benzyl)amino)quinazolin-3(4H)-yl)piperidine-2,6-dione |
| | 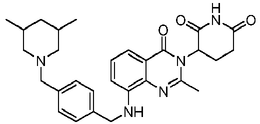 | 3-(8-((4-((3,5-dimethylpiperidin-1-yl)methyl)benzyl)amino)-2-methyl-4-oxoquinazolin-3(4H)-yl)piperidine-2,6-dione |
| | 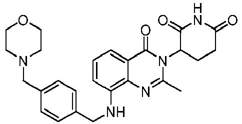 | 3-(2-methyl-8-((4-(morpholinomethyl)benzyl)amino)-4-oxoquinazolin-3(4H)-yl)piperidine-2,6-dione |
| SIAIS3550 65 | 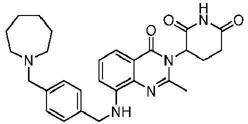 | 3-(8-((4-(azepan-1-ylmethyl)benzyl)amino)-2-methyl-4-oxoquinazolin-3(4H)-yl)piperidine-2,6-dione |
| | 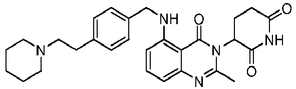 | 3-(2-methyl-4-oxo-5-((4-(2-(piperidin-1-yl)ethyl)benzyl)amino)quinazolin-3(4H)-yl)piperidine-2,6-dione |
| | 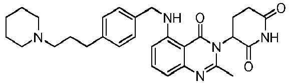 | 3-(2-methyl-4-oxo-5-((4-(3-(piperidin-1-yl)propyl)benzyl)amino)quinazolin-3(4H)-yl)piperidine-2,6-dione |
| | 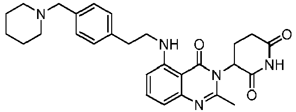 | 3-(2-methyl-4-oxo-5-((4-(piperidin-1- ylmethyl)phenethyl)amino)quinazolin-3(4H)-yl)piperidine-2,6-dione |
| | 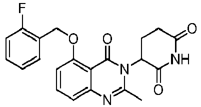 | 3-(5-((2-fluorobenzyl)oxy)-2-methyl-4-oxoquinazolin-3(4H)-yl)piperidine-2,6-dione |
| | 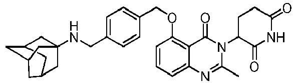 | 3-(5-((4-((adamantan-1-ylamino)methyl)benzyl)oxy)-2-methyl-4-oxoquinazolin-3(4H)-yl)piperidine-2,6-dione |
| | 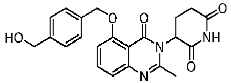 | 3-(5-((4-(hydroxymethyl)benzyl)oxy)-2-methyl-4-oxoquinazolin-3(4H)-yl)piperidine-2,6-dione |
| | 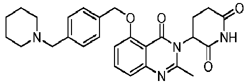 | 3-(2-methyl-4-oxo-5-((4-(piperidin-1-ylmethyl)benzyl)oxy)quinazolin-3(4H)-yl)piperidine-2,6-dione |
| | 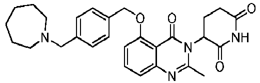 | 3-(5-((4-(azepan-1-ylmethyl)benzyl)oxy)-2-methyl-4-oxoquinazolin-3(4H)-yl)piperidine-2,6-dione |
| | 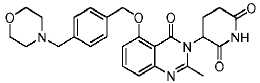 | 3-(2-methyl-5-((4-(morpholinomethyl)benzyl)oxy)-4-oxoquinazolin-3(4H)-yl)piperidine-2,6-dione |
| | 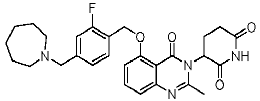 | 3-(5-((4-(azepan-1-ylmethyl)-2-fluorobenzyl)oxy)-2-methyl-4-oxoquinazolin-3(4H)-yl)piperidine-2,6-dione |
| | 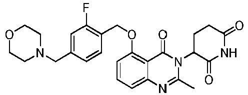 | 3-(5-((2-fluoro-4-(morpholinomethyl)benzyl)oxy)-2-methyl-4-oxoquinazolin-3(4H)-yl)piperidine-2,6-dione |
| | 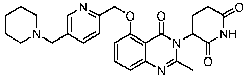 | 3-(2-methyl-4-oxo-5-((5-(piperidin-1-ylmethyl)pyridin-2-yl)methoxy)quinazolin-3(4H)-yl)piperidine-2,6-dione |
| | 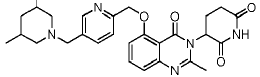 | 3-(5-((5-((3,5-dimethylpiperidin-1-yl)methyl)pyridin-2-yl)methoxy)-2-methyl-4-oxoquinazolin-3(4H)-yl)piperidine-2,6-dione |
| | 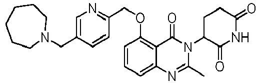 | 3-(5-((5-(azepan-1-ylmethyl)pyridin-2-yl)methoxy)-2-methyl-4-oxoquinazolin-3(4H)-yl)piperidine-2,6-dione |
| | 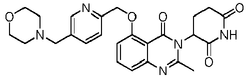 | 3-(2-methyl-5-((5-(morpholinomethyl)pyridin-2-yl)methoxy)-4-oxoquinazolin-3(4H)-yl)piperidine-2,6-dione |
| | 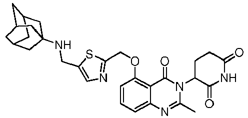 | 3-(5-((5-((adamantan-1-ylamino)methyl)thiazol-2-yl)methoxy)-2-methyl-4-oxoquinazolin-3(4H)-yl)piperidine-2,6-dione |
| | 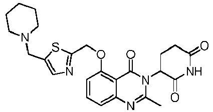 | 3-(2-methyl-4-oxo-5-((5-(piperidin-1-ylmethyl)thiazol-2-yl)methoxy)quinazolin-3(4H)-yl)piperidine-2,6-dione |
| | 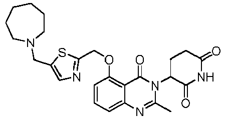 | 3-(5-((5-(azepan-1-ylmethyl)thiazol-2-yl)methoxy)-2-methyl-4-oxoquinazolin-3(4H)-yl)piperidine-2,6-dione |
| | 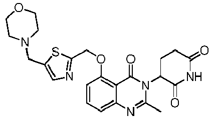 | 3-(2-methyl-5-((5-(morpholinomethyl)thiazol-2-yl)methoxy)-4-oxoquinazolin-3(4H)-yl)piperidine-2,6-dione |
| | 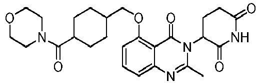 | 3-(2-methyl-5-((4-(morpholine-4-carbonyl)cyclohexyl)methoxy)-4-oxoquinazolin-3(4H)-yl)piperidine-2,6-dione |
| | 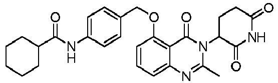 | N-(4-(((3-(2,6-dioxopiperidin-3-yl)-2-methyl-4-oxo-3,4-dihydroquinazolin-5-yl)oxy)methyl)phenyl)cyclohexanecarboxamide |
| | 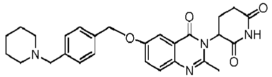 | 3-(2-methyl-4-oxo-6-((4-(piperidin-1- ylmethyl)benzyl)oxy)quinazolin-3(4H)-yl)piperidine-2,6-dione |
| | 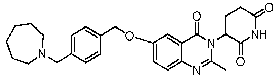 | 3-(6-((4-(azepan-1-ylmethyl)benzyl)oxy)-2-methyl-4-oxoquinazolin-3(4H)-yl)piperidine-2,6-dione |
| | 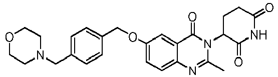 | 3-(2-methyl-6-((4-(morpholinomethyl)benzyl)oxy)-4-oxoquinazolin-3(4H)-yl)piperidine-2,6-dione |
| | 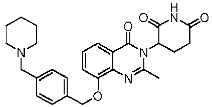 | 3-(2-methyl-4-oxo-8-((4-(piperidin-1-ylmethyl)benzyl)oxy)quinazolin-3(4H)-yl)piperidine-2,6-dione |
| | 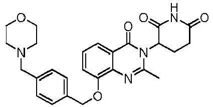 | 3-(2-methyl-8-((4-(morpholinomethyl)benzyl)oxy)-4-oxoquinazolin-3(4H)-yl)piperidine-2,6-dione |
| | 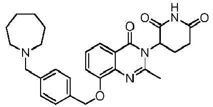 | 3-(8-((4-(azepan-1-ylmethyl)benzyl)oxy)-2-methyl-4-oxoquinazolin-3(4H)-yl)piperidine-2,6-dione |
| | 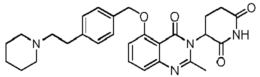 | 3-(2-methyl-4-oxo-5-((4-(2-(piperidin-1-yl)ethyl)benzyl)oxy)quinazolin-3(4H)-yl)piperidine-2,6-dione |
| | 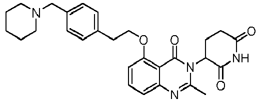 | 3-(2-methyl-4-oxo-5-(4-(piperidin-1-ylmethyl)phenethoxy)quinazolin-3(4H)-yl)piperidine-2,6-dione |
| SIAIS3130 34 | 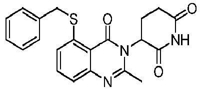 | 3-(5-(benzylthio)-2-methyl-4-oxoquinazolin-3(4H)-yl)piperidine-2,6-dione |
| | 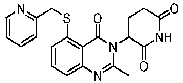 | 3-(2-methyl-4-oxo-5-((pyridin-2-ylmethyl)thio)quinazolin-3(4H)-yl)piperidine-2,6-dione |
| | 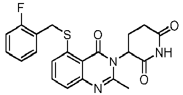 | 3-(5-((2-fluorobenzyl)thio)-2-methyl-4-oxoquinazolin-3(4H)-yl)piperidine-2,6-dione |
| | 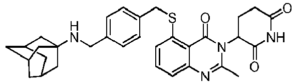 | 3-(5-((4-((adamantan-1-ylamino)methyl)benzyl)thio)-2-methyl-4-oxoquinazolin-3(4H)-yl)piperidine-2,6-dione |
| | 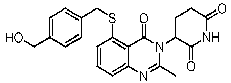 | 3-(5-((4-(hydroxymethyl)benzyl)thio)-2-methyl-4-oxoquinazolin-3(4H)-yl)piperidine-2,6-dione |
| | 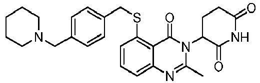 | 3-(2-methyl-4-oxo-5-((4-(piperidin-1-ylmethyl)benzyl)thio)quinazolin-3(4H)-yl)piperidine-2,6-dione |
| | 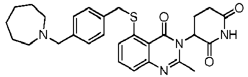 | 3-(5-((4-(azepan-1-ylmethyl)benzyl)thio)-2-methyl-4-oxoquinazolin-3(4H)-yl)piperidine-2,6-dione |
| | 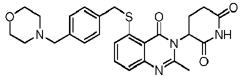 | 3-(2-methyl-5-((4-(morpholinomethyl)benzyl)thio)-4-oxoquinazolin-3(4H)-yl)piperidine-2,6-dione |
| | 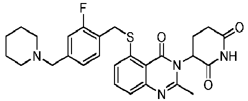 | 3-(5-((2-fluoro-4-(piperidin-1-ylmethyl)benzyl)thio)-2-methyl-4-oxoquinazolin-3(4H)-yl)piperidine-2,6-dione |
| | 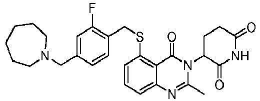 | 3-(5-((4-(azepan-1-ylmethyl)-2-fluorobenzyl)thio)-2-methyl-4-oxoquinazolin-3(4H)-yl)piperidine-2,6-dione |
| | 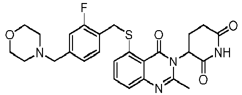 | 3-(5-((2-fluoro-4-(morpholinomethyl)benzyl)thio)-2-methyl-4-oxoquinazolin-3(4H)-yl)piperidine-2,6-dione |
| | 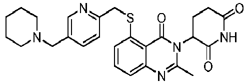 | 3-(2-methyl-4-oxo-5-(((5-(piperidin-1-ylmethyl)pyridin-2-yl)methyl)thio)quinazolin-3(4H)-yl)piperidine-2,6-dione |
| | 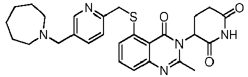 | 3-(5-(((5-(azepan-1-ylmethyl)pyridin-2-yl)methyl)thio)-2-methyl-4-oxoquinazolin-3(4H)-yl)piperidine-2,6-dione |
| | 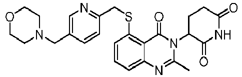 | 3-(2-methyl-5-(((5-(morpholinomethyl)pyridin-2-yl)methyl)thio)-4-oxoquinazolin-3(4H)-yl)piperidine-2,6-dione |
| | 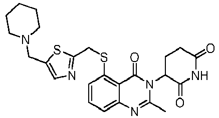 | 3-(2-methyl-4-oxo-5-(((5-(piperidin-1-ylmethyl)thiazol-2-yl)methyl)thio)quinazolin-3(4H)-yl)piperidine-2,6-dione |
| | 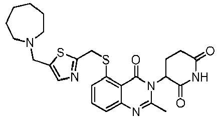 | 3-(5-(((5-(azepan-1-ylmethyl)thiazol-2-yl)methyl)thio)-2-methyl-4-oxoquinazolin-3(4H)-yl)piperidine-2,6-dione |
| | 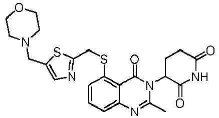 | 3-(2-methyl-5-(((5-(morpholinomethyl)thiazol-2-yl)methyl)thio)-4-oxoquinazolin-3(4H)-yl)piperidine-2,6-dione |
| | 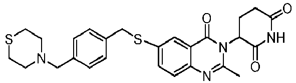 | 3-(2-methyl-4-oxo-6-((4-(thiomorpholinomethyl)benzyl)thio)quinazolin-3(4H)-yl)piperidine-2,6-dione |
| | 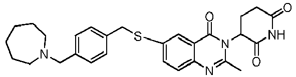 | 3-(6-((4-(azepan-1-ylmethyl)benzyl)thio)-2-methyl-4-oxoquinazolin-3(4H)-yl)piperidine-2,6-dione |
| | 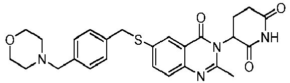 | 3-(2-methyl-6-((4-(morpholinomethyl)benzyl)thio)-4-oxoquinazolin-3(4H)-yl)piperidine-2,6-dione |
| | 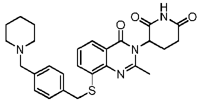 | 3-(2-methyl-4-oxo-8-((4-(piperidin-1-ylmethyl)benzyl)thio)quinazolin-3(4H)-yl)piperidine-2,6-dione |
| | 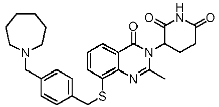 | 3-(8-((4-(azepan-1-ylmethyl)benzyl)thio)-2-methyl-4-oxoquinazolin-3(4H)-yl)piperidine-2,6-dione |
| | 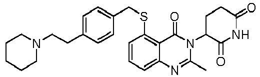 | 3-(2-methyl-4-oxo-5-((4-(2-(piperidin-1-yl)ethyl)benzyl)thio)quinazolin-3(4H)-yl)piperidine-2,6-dione |
| | 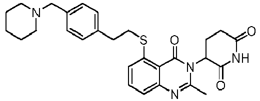 | 3-(2-methyl-4-oxo-5-((4-(piperidin-1 - ylmethyl)phenethyl)thio)quinazolin-3(4H)-yl)piperidine-2,6-dione |
| | 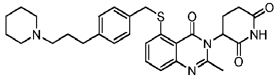 | 3-(2-methyl-4-oxo-5-((4-(3-(piperidin-1-yl)propyl)benzyl)thio)quinazolin-3(4H)-yl)piperidine-2,6-dione |
| | 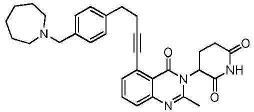 | 3-(5-(4-(4-(azepan-1 -ylmethyl)phenyl)but-1-yn-1- yl)-2-methyl-4-oxoquinazolin-3(4H)-yl)piperidine-2,6-dione |
| | 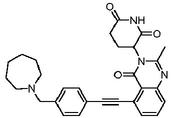 | 3-(5-((4-(azepan-1-ylmethyl)phenyl)ethynyl)-2-methyl-4-oxoquinazolin-3(4H)-yl)piperidine-2,6-dione |
| | 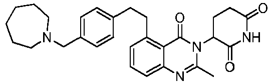 | 3-(5-(4-(azepan-1-ylmethyl)phenethyl)-2-methyl-4-oxoquinazolin-3(4H)-yl)piperidine-2,6-dione |
| | 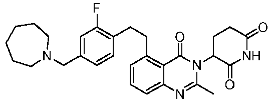 | 3-(5-(4-(azepan-1-ylmethyl)-2-fluorophenethyl)-2-methyl-4-oxoquinazolin-3(4H)-yl)piperidine-2,6-dione |
| | 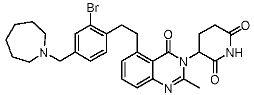 | 3-(5-(4-(azepan-1-ylmethyl)-2-bromophenethyl)-2-methyl-4-oxoquinazolin-3(4H)-yl)piperidine-2,6-dione |
| | 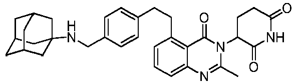 | 3-(5-(4-((adamantan-1-ylamino)methyl)phenethyl)-2-methyl-4-oxoquinazolin-3(4H)-yl)piperidine-2,6-dione |
| | 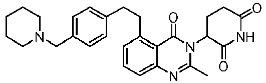 | 3-(2-methyl-4-oxo-5-(4-(piperidin-1-ylmethyl)phenethyl)quinazolin-3(4H)-yl)piperidine-2,6-dione |
| | 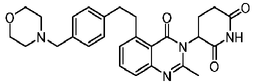 | 3-(2-methyl-5-(4-(morpholinomethyl)phenethyl)-4-oxoquinazolin-3(4H)-yl)piperidine-2,6-dione |
| | 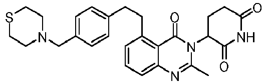 | 3-(2-methyl-4-oxo-5-(4-(thiomorpholinomethyl)phenethyl)quinazolin-3(4H)-yl)piperidine-2,6-dione |
| | 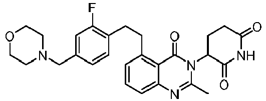 | 3-(5-(2-fluoro-4-(morpholinomethyl)phenethyl)-2-methyl-4-oxoquinazolin-3(4H)-yl)piperidine-2,6-dione |
| | 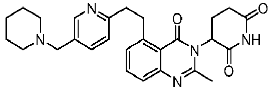 | 3-(2-methyl-4-oxo-5-(2-(5-(piperidin-1-ylmethyl)pyridin-2-yl)ethyl)quinazolin-3(4H)-yl)piperidine-2,6-dione |
| | 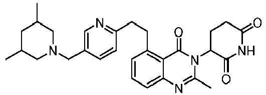 | 3-(5-(2-(5-((3,5-dimethylpiperidin-1-yl)methyl)pyridin-2-yl)ethyl)-2-methyl-4-oxoquinazolin-3(4H)-yl)piperidine-2,6-dione |
| | 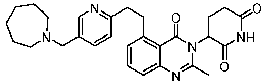 | 3-(5-(2-(5-(azepan-1-ylmethyl)pyridin-2-yl)ethyl)-2-methyl-4-oxoquinazolin-3(4H)-yl)piperidine-2,6-dione |
| | 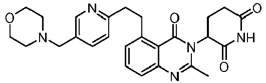 | 3-(2-methyl-5-(2-(5-(morpholinomethyl)pyridin-2-yl)ethyl)-4-oxoquinazolin-3(4H)-yl)piperidine-2,6-dione |
| | 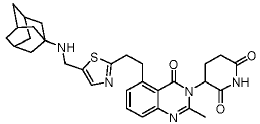 | 3-(5-(2-(5-((((1R,3R,SS)-adamantan-1-yl)amino)methyl)thiazol-2-yl)ethyl)-2-methyl-4-oxoquinazolin-3(4H)-yl)piperidine-2,6-dione |
| | 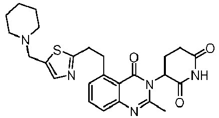 | 3-(2-methyl-4-oxo-5-(2-(5-(piperidin-1-ylmethyl)thiazol-2-yl)ethyl)quinazolin-3(4H)-yl)piperidine-2,6-dione |
| | 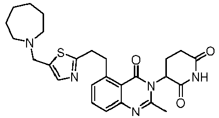 | 3-(5-(2-(5-(azepan-1-ylmethyl)thiazol-2-yl)ethyl)-2-methyl-4-oxoquinazolin-3(4H)-yl)piperidine-2,6-dione |
| | 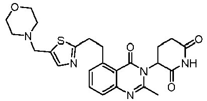 | 3-(2-methyl-5-(2-(5-(morpholinomethyl)thiazol-2-yl)ethyl)-4-oxoquinazolin-3(4H)-yl)piperidine-2,6-dione |
| | 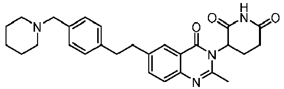 | 3-(2-methyl-4-oxo-6-(4-(piperidin-1-ylmethyl)phenethyl)quinazolin-3(4H)-yl)piperidine-2,6-dione |
| | 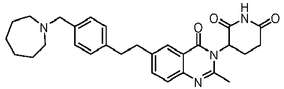 | 3-(6-(4-(azepan-1-ylmethyl)phenethyl)-2-methyl-4-oxoquinazolin-3(4H)-yl)piperidine-2,6-dione |
| | 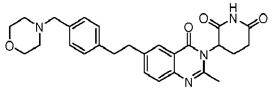 | 3-(2-methyl-6-(4-(morpholinomethyl)phenethyl)-4-oxoquinazolin-3(4H)-yl)piperidine-2,6-dione |
| | 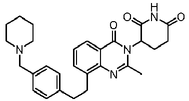 | 3-(2-methyl-4-oxo-8-(4-(piperidin-1-ylmethyl)phenethyl)quinazolin-3(4H)-yl)piperidine-2,6-dione |
| | 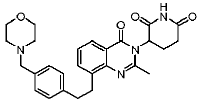 | 3-(2-methyl-8-(4-(morpholinomethyl)phenethyl)-4-oxoquinazolin-3(4H)-yl)piperidine-2,6-dione |
| | 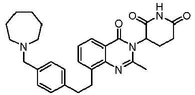 | 3-(8-(4-(azepan-1-ylmethyl)phenethyl)-2-methyl-4-oxoquinazolin-3(4H)-yl)piperidine-2,6-dione |
| | 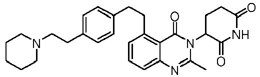 | 3-(2-methyl-4-oxo-5-(4-(2-(piperidin-1-yl)ethyl)phenethyl)quinazolin-3(4H)-yl)piperidine-2,6-dione |
| | 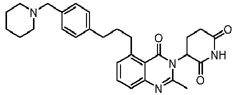 | 3-(2-methyl-4-oxo-5-(3-(4-(piperidin-1-ylmethyl)phenyl)propyl)quinazolin-3(4H)-yl)piperidine-2,6-dione |
| | 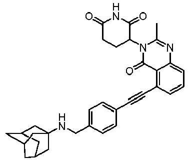 | 3-(5-((4-((((1R,3R,SS)-adamantan-1-yl)amino)methyl)phenyl)ethynyl)-2-methyl-4-oxoquinazolin-3(4H)-yl)piperidine-2,6-dione |
| | 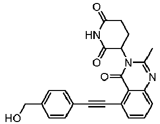 | 3-(5-((4-(hydroxymethyl)phenyl)ethynyl)-2-methyl-4-oxoquinazolin-3(4H)-yl)piperidine-2,6-dione |
| | 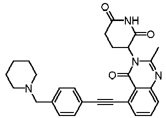 | 3-(2-methyl-4-oxo-5-((4-(piperidin-1-ylmethyl)phenyl)ethynyl)quinazolin-3(4H)-yl)piperidine-2,6-dione |
| | 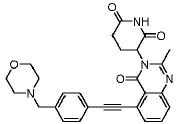 | 3-(2-methyl-5-((4-(morpholinomethyl)phenyl)ethynyl)-4-oxoquinazolin-3(4H)-yl)piperidine-2,6-dione |
| | 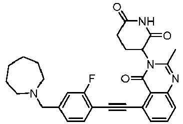 | 3-(5-((4-(azepan-1-ylmethyl)-2-fluorophenyl)ethynyl)-2-methyl-4-oxoquinazolin-3(4H)-yl)piperidine-2,6-dione |
| | 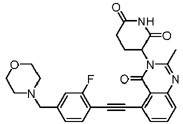 | 3-(5-((2-fluoro-4-(morpholinomethyl)phenyl)ethynyl)-2-methyl-4-oxoquinazolin-3(4H)-yl)piperidine-2,6-dione |
| | 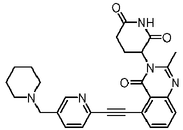 | 3-(2-methyl-4-oxo-5-((5-(piperidin-1-ylmethyl)pyridin-2-yl)ethynyl)quinazolin-3(4H)-yl)piperidine-2,6-dione |
| | 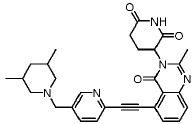 | 3-(5-((5-((3,5-dimethylpiperidin-1-yl)methyl)pyridin-2-yl)ethynyl)-2-methyl-4-oxoquinazolin-3(4H)-yl)piperidine-2,6-dione |
| | 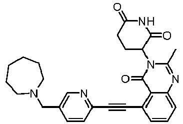 | 3-(5-((5-(azepan-1-ylmethyl)pyridin-2-yl)ethynyl)-2-methyl-4-oxoquinazolin-3(4H)-yl)piperidine-2,6-dione |
| | 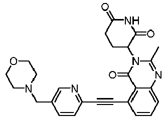 | 3-(2-methyl-5-((5-(morpholinomethyl)pyridin-2-yl)ethynyl)-4-oxoquinazolin-3(4H)-yl)piperidine-2,6-dione |
| | 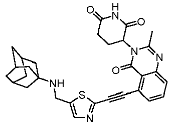 | 3-(5-((5-((((1R,3R,SS)-adamantan-1-yl)amino)methyl)thiazol-2-yl)ethynyl)-2-methyl-4-oxoquinazolin-3(4H)-yl)piperidine-2,6-dione |
| | 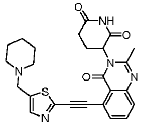 | 3-(2-methyl-4-oxo-5-((5-(piperidin-1-ylmethyl)thiazol-2-yl)ethynyl)quinazolin-3(4H)-yl)piperidine-2,6-dione |
| | 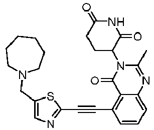 | 3-(5-((5-(azepan-1-ylmethyl)thiazol-2-yl)ethynyl)-2-methyl-4-oxoquinazolin-3(4H)-yl)piperidine-2,6-dione |
| | 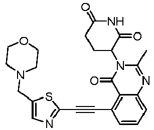 | 3-(2-methyl-5-((5-(morpholinomethyl)thiazol-2-yl)ethynyl)-4-oxoquinazolin-3(4H)-yl)piperidine-2,6-dione |
| | 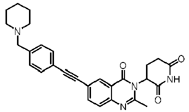 | 3-(2-methyl-4-oxo-6-((4-(piperidin-1-ylmethyl)phenyl)ethynyl)quinazolin-3(4H)-yl)piperidine-2,6-dione |
| | 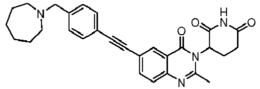 | 3-(6-((4-(azepan-1-ylmethyl)phenyl)ethynyl)-2-methyl-4-oxoquinazolin-3(4H)-yl)piperidine-2,6-dione |
| | 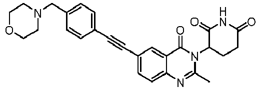 | 3-(2-methyl-6-((4-(morpholinomethyl)phenyl)ethynyl)-4-oxoquinazolin-3(4H)-yl)piperidine-2,6-dione |
| | 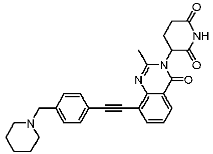 | 3-(2-methyl-4-oxo-8-((4-(piperidin-1-ylmethyl)phenyl)ethynyl)quinazolin-3(4H)-yl)piperidine-2,6-dione |
| | 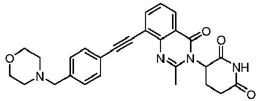 | 3-(2-methyl-8-((4-(morpholinomethyl)phenyl)ethynyl)-4-oxoquinazolin-3(4H)-yl)piperidine-2,6-dione |
| | 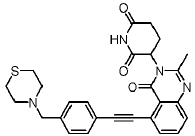 | 3-(2-methyl-4-oxo-5-((4-(thiomorpholinomethyl)phenyl)ethynyl)quinazolin -3(4H)-yl)piperidine-2,6-dione |
| | 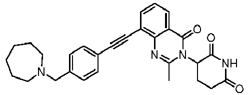 | 3-(8-((4-(azepan-1-ylmethyl)phenyl)ethynyl)-2-methyl-4-oxoquinazolin-3(4H)-yl)piperidine-2,6-dione |
| | 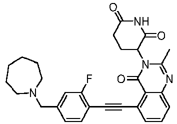 | 3-(5-((4-(azepan-1-ylmethyl)-2-fluorophenyl)ethynyl)-2-methyl-4-oxoquinazolin-3(4H)-yl)piperidine-2,6-dione |
| | 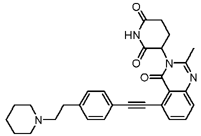 | 3-(2-methyl-4-oxo-5-((4-(2-(piperidin-1-yl)ethyl)phenyl)ethynyl)quinazolin-3(4H)-yl)piperidine-2,6-dione |
| | 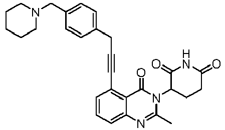 | 3-(2-methyl-4-oxo-5-(3-(4-(piperidin-1-ylmethyl)phenyl)prop-1-yn-1-yl)quinazolin-3(4H)-yl)piperidine-2,6-dione |
| | 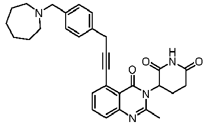 | 3-(5-(3-(4-(azepan-1-ylmethyl)phenyl)prop-1-yn-1-yl)-2-methyl-4-oxoquinazolin-3(4H)-yl)piperidine-2,6-dione |
| | 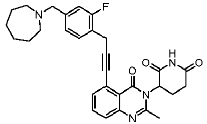 | 3-(5-(3-(4-(azepan-1-ylmethyl)-2-fluorophenyl)prop-1-yn-1-yl)-2-methyl-4-oxoquinazolin-3(4H)-yl)piperidine-2,6-dione |
| | 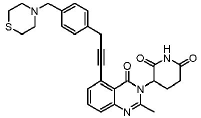 | 3-(2-methyl-4-oxo-5-(3-(4-(thiomorpholinomethyl)phenyl)prop-1-yn-1-yl)quinazolin-3(4H)-yl)piperidine-2,6-dione |
| | 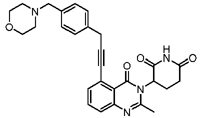 | 3-(2-methyl-5-(3-(4-(morpholinomethyl)phenyl)prop-1-yn-1-yl)-4-oxoquinazolin-3(4H)-yl)piperidine-2,6-dione |

It is to be understood that the compounds of Formula (I), (Ia), (Ib), (Ic), (Id) of the present invention may have a stereo configuration and thus can be in more than one stereoisomeric form. The present invention also relates to compounds having a stereo configuration in substantially pure isomeric form, e.g., greater than about 90% enantiomeric/diastereomeric excess ("ee"), such as about 95% ee or 97% ee, or greater than 99% ee, and mixtures thereof, including racemic mixtures. These isomers can be prepared by using asymmetric synthesis (e.g., by using chiral intermediates) or by chiral resolution.

In another aspect, the present invention also provides a pharmaceutical composition comprising, as an active ingredient, the compound of Formula (I) of the present invention or pharmaceutically acceptable salts, racemates, enantiomers, diastereomers, solvates, or polymorphs thereof, and a pharmaceutically acceptable carrier.

The pharmaceutical composition of the present invention further comprises at least one additional therapeutic agent for the treatment or prevention of a disease or disorder associated with TNF-α. The additional therapeutic agent can be combined with the compound of Formula (I) of the present invention to treat diseases or disorders associated with TNF-α, including but not limited to chemotherapeutic agents, immunotherapy agents, gene therapy agents and the like. In one embodiment, the diseases or disorders associated with TNF-α is selected from the group consisting of: tumors (or cancer), infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, Unverricht Syndrome, acute liver failure, or diabetes. The diseases or disorders associated with TNF-α include, but are not limited to:
tumors (or cancers), including: e.g., myeloma, such as multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, anemia associated with leukemia, acute myeloid leukemia (AML); lymphoma, including diffuse large B-cell lymphoma, non-Hodgkin's lymphoma, anaplastic lymphoma, anaplastic large cell lymphoma, lymphoma CD20 positive, mantle cell lymphoma, primary lymphoma, B-cell lymphoma, recurrent B-cell non-Hodgkin's lymphoma, recurrent diffuse large B-cell lymphoma, recurrent mediastinal (thymus) large B-cell, primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; inflammatory myofibroblastoma; colorectal cancer; brain glioma; astroblastoma; oophoroma; bronchial carcinoma; prostate cancer; breast cancer, including triple negative breast cancer, sporadic breast cancer and Cowden's disease patients; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial carcinoma; brain cancer; oral cancer; sarcoma, including rhabdomyosarcoma, various lipogenic tumors, Ewing's sarcoma/primitive neuroectodermal tumors (Ewing/PNETs), and smooth muscle sarcoma;
autoimmune diseases, including, e.g., rheumatoid arthritis; autoimmune encephalomyelitis; ankylosing spondylitis; psoriasis; systemic lupus erythematosus; recurrent oral ulcer; multiple sclerosis; Kawasaki disease;
infectious diseases, including, e.g., COVID-19 novel coronavirus infection, septic shock, bacterial meningitis, cerebral malaria, acquired immune deficiency syndrome (AIDS), sepsis syndrome, tuberculosis;
inflammatory diseases, including, e.g., pneumonia, osteoarthritis, synovitis, systemic inflammatory response syndrome, airway inflammation, bronchitis, inflammatory bowel disease including Crohn's disease and ulcerative colitis;
multiple organ dysfunction syndrome, including, e.g., multiple organ failure due to cachexia and septic shock; and
Unverricht Syndrome; asthma; chronic obstructive pulmonary disease; hemorrhagic shock; organ (including kidney, heart, lung) or tissue transplant rejection; diabetes; sarcoidosis; adult respiratory distress syndrome; congestive heart failure; myocardial infarction; and acute liver failure.

The pharmaceutical composition of the present invention comprising, as an active ingredient, the compound of Formula (I) of the present invention or a pharmaceutically acceptable salt thereof can be formulated into any suitable formulations such as sprays, patches, tablets, capsules, dragees, troches, powders, granules, powder injections, or liquid formulations (such as suspensions, solutions, emulsions, or syrups), or conventional injectable dosage forms such as lyophilized compositions and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). The compounds of Formula (I) can also be formulated into conventional, dispersible, chewable, oral disintegrating, or rapidly dissolving formulations as required by those skilled in the art.

In another aspect, the present invention provides the compound of Formula (I), or pharmaceutically acceptable salts, racemates, enantiomers, stereoisomers, solvates, or polymorphs thereof for use as a medicament.

In another aspect, the present invention provides the compound of Formula (I), or pharmaceutically acceptable salts, racemates, enantiomers, stereoisomers, solvates, or polymorphs thereof for use in the prevention and/or treatment of diseases or disorders associated with TNF-α. In one embodiment, the diseases or disorders associated with TNF-α are selected from the group consisting of tumors (or cancer), infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, Unverricht Syndrome, acute liver failure, or diabetes. The diseases or disorders associated with TNF-α include, but are not limited to, tumors (or cancers), including: e.g., myeloma, such as multiple myeloma, plasma cell myeloma, smoldering myeloma, smoldering multiple myeloma; transplantation-related cancer; myelofibrosis; bone marrow disease; myelodysplastic syndrome (MDS); previously treated myelodysplastic syndrome; neutropenia; leukemia, including acute myeloid leukemia, chronic myelogenous leukemia, B-cell chronic lymphocytic leukemia, acute myeloid leukemia (AML); anemia associated with leukemia; lymphoma, including diffuse large B-cell lymphoma, non-Hodgkin's lymphoma, anaplastic lymphoma, anaplastic large cell lymphoma, lymphoma CD20 positive, mantle cell lymphoma, primary lymphoma, B-cell lymphoma, recurrent B-cell non-Hodgkin's lymphoma, recurrent diffuse large B-cell lymphoma, recurrent mediastinal (thymus) large B-cell, primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; inflammatory myofibroblastoma; colorectal cancer; brain glioma; astroblastoma; oophoroma; bronchial carcinoma; prostate cancer; breast cancer, including triple negative breast cancer, sporadic breast cancer and Cowden's disease patients; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial carcinoma; brain cancer; oral cancer; sarcoma, including rhabdomyosarcoma, various lipogenic tumors, Ewing's sarcoma/primitive neuroectodermal tumors (Ewing/PNETs), and smooth muscle sarcoma; autoimmune diseases, including, e.g., rheumatoid arthritis; autoimmune encephalomyelitis; ankylosing spondylitis; psoriasis; systemic lupus erythematosus; recurrent oral ulcer; multiple sclerosis; Kawasaki disease; infectious diseases, including, e.g., bacterial meningitis, cerebral malaria, acquired immune deficiency syndrome (AIDS), COVID-19 novel coronavirus infection, septic shock, sepsis syndrome, tuberculosis; inflammatory diseases, including, e.g., pneumonia, osteoarthritis, synovitis, systemic inflammatory response syndrome, airway inflammation, bronchitis, inflammatory bowel disease including Crohn's disease and ulcerative colitis; chronic obstructive pulmonary disease; asthma; multiple organ dysfunction syndrome, including, e.g., multiple organ failure due to cachexia and septic shock; Unverricht Syndrome; hemorrhagic shock; organ (including kidney, heart, lung) or tissue transplant rejection; diabetes; sarcoidosis; adult respiratory distress syndrome; congestive heart failure; myocardial infarction; or acute liver failure.

In another aspect, the present invention provides the use of the compound of Formula (I) or pharmaceutically acceptable salts, racemates, enantiomers, stereoisomers, solvates, or polymorphs thereof, for the manufacture of a medicament for the prevention and/or treatment of diseases or disorders associated with TNF-α. In one embodiment, the diseases or disorders associated with TNF-α are selected from the group consisting of tumors (or cancer), infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, Unverricht Syndrome, acute liver failure, or diabetes. The diseases or disorders associated with TNF-α include, but are not limited to, tumors (or cancers), including: e.g., myeloma, such as multiple myeloma, plasma cell myeloma, smoldering myeloma, smoldering multiple myeloma; transplantation-related cancer; myelofibrosis; bone marrow disease; myelodysplastic syndrome (MDS); previously treated myelodysplastic syndrome; neutropenia; leukemia, including acute myeloid leukemia, chronic myelogenous leukemia, B-cell chronic lymphocytic leukemia, acute myeloid leukemia (AML); anemia associated with leukemia; lymphoma, including diffuse large B-cell lymphoma, non-Hodgkin's lymphoma, anaplastic lymphoma, anaplastic large cell lymphoma, lymphoma CD20 positive, mantle cell lymphoma, primary lymphoma, B-cell lymphoma, recurrent B-cell non-Hodgkin's lymphoma, recurrent diffuse large B-cell lymphoma, recurrent mediastinal (thymus) large B-cell, primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; inflammatory myofibroblastoma; colorectal cancer; brain glioma; astroblastoma; oophoroma; bronchial carcinoma; prostate cancer; breast cancer, including triple negative breast cancer, sporadic breast cancer and Cowden's disease patients; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial carcinoma; brain cancer; oral cancer; sarcoma, including rhabdomyosarcoma, various lipogenic tumors, Ewing's sarcoma/primitive neuroectodermal tumors (Ewing/PNETs), and smooth muscle sarcoma; autoimmune diseases, including, e.g., rheumatoid arthritis; autoimmune encephalomyelitis; ankylosing spondylitis; psoriasis; systemic lupus erythematosus; recurrent oral ulcer; multiple sclerosis; Kawasaki disease; infectious diseases, including, e.g., bacterial meningitis, cerebral malaria, acquired immune deficiency syndrome (AIDS), COVID-19 novel coronavirus infection, septic shock, sepsis syndrome, tuberculosis; inflammatory diseases, including, e.g., pneumonia, osteoarthritis, synovitis, systemic inflammatory response syndrome, airway inflammation, bronchitis, inflammatory bowel disease including Crohn's disease and ulcerative colitis; chronic obstructive pulmonary disease; asthma; multiple organ dysfunction syndrome, including, e.g., multiple organ failure due to cachexia and septic shock; Unverricht Syndrome; hemorrhagic shock; organ (including kidney, heart, lung) or tissue transplant rejection; diabetes; sarcoidosis; adult respiratory distress syndrome; congestive heart failure; myocardial infarction; or acute liver failure.

In another aspect, the present invention also provides a method for treating or preventing diseases or disorders associated with TNF-α, comprising administering to a subject a therapeutically effective amount of the compound of Formula (I) of the present invention or a pharmaceutically acceptable salt, racemate, enantiomer, stereoisomer, solvate, or polymorph thereof, or the pharmaceutical composition of the present invention. In one embodiment, the diseases or disorders associated with TNF-α are selected from the group consisting of tumors (or cancer), infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, Unverricht Syndrome, acute liver failure, or diabetes. The diseases or disorders associated with TNF-α include, but are not limited to, tumors (or cancers), including: e.g., myeloma, such as multiple myeloma, plasma cell myeloma, smoldering myeloma, smoldering multiple myeloma; transplantation-related cancer; myelofibrosis; bone marrow disease; myelodysplastic syndrome (MDS); previously treated myelodysplastic syndrome; neutropenia; leukemia, including acute myeloid leukemia, chronic myelogenous leukemia, B-cell chronic lymphocytic leukemia, acute myeloid leukemia (AML); anemia associated with leukemia; lymphoma, including diffuse large B-cell lymphoma, non-Hodgkin's lymphoma, anaplastic lymphoma, anaplastic large cell lymphoma, lymphoma CD20 positive, mantle cell lymphoma, primary lymphoma, B-cell lymphoma, recurrent B-cell non-Hodgkin's lymphoma, recurrent diffuse large B-cell lymphoma, recurrent mediastinal (thymus) large B-cell, primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; inflammatory myofibroblastoma; colorectal cancer; brain glioma; astroblastoma; oophoroma; bronchial carcinoma; prostate cancer; breast cancer, including triple negative breast cancer, sporadic breast cancer and Cowden's disease patients; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial carcinoma; brain cancer; oral cancer; sarcoma, including rhabdomyosarcoma, various lipogenic tumors, Ewing's sarcoma/primitive neuroectodermal tumors (Ewing/PNETs), and smooth muscle sarcoma; autoimmune diseases, including, e.g., rheumatoid arthritis; autoimmune encephalomyelitis; ankylosing spondylitis; psoriasis; systemic lupus erythematosus; recurrent oral ulcer; multiple sclerosis; Kawasaki disease; infectious diseases, including, e.g., bacterial meningitis, cerebral malaria, acquired immune deficiency syndrome (AIDS), COVID-19 novel coronavirus infection, septic shock, sepsis syndrome, tuberculosis; inflammatory diseases, including, e.g., pneumonia, osteoarthritis, synovitis, systemic inflammatory response syndrome, airway inflammation, bronchitis, inflammatory bowel disease including Crohn's disease and ulcerative colitis; chronic obstructive pulmonary disease; asthma; multiple organ dysfunction syndrome, including, e.g., multiple organ failure due to cachexia and septic shock; Unverricht Syndrome; hemorrhagic shock; organ (including kidney, heart, lung) or tissue transplant rejection; diabetes; sarcoidosis; adult respiratory distress syndrome; congestive heart failure; myocardial infarction; or acute liver failure.

In the method for treating or preventing diseases or disorders associated with TNF-α of the present invention, the compound of Formula (I) of the present invention, or a pharmaceutically acceptable salt, racemate, enantiomer, stereoisomer, solvate, or polymorph thereof, or said pharmaceutical composition is administered to the subject by at least one mode of administration selected from the group consisting of: nasal, inhalation, topical, oral, oral mucosal, rectal, pleural, peritoneal, vaginal, intramuscular, subcutaneous, transdermal, epidural, intrathecal, and intravenous administration.

### Definition

Unless otherwise specified, the following words, phrases and symbols used herein generally have the meanings as described below.

In general, the nomenclature used herein (such as the IUPAC nomenclature) and the laboratory procedures described below (including those used in cell culture, organic chemistry, analytical chemistry, and pharmacology, etc.) are those well known and commonly used in the art. Unless otherwise defined, all scientific and technical terms used herein in connection with the present disclosure described herein have the same meaning as commonly understood by one skill in the art. In addition, the word "a" or "an" when used in conjunction with the term "comprising" or nouns in the claims and/or the specification may mean "one", but also be consistent with the meaning of "one or more", "at least one", and "one or more than one". Similarly, the terms "another" or "other" can mean at least a second or more. Unless clearly defined otherwise, the singular forms "a," "an" and "the" used herein specifically also encompass the plural forms of the terms to which they refer.

It is to be understood that whenever the terms "comprising" or "containing" are used herein to describe various aspects, other similar aspects recited by "consisting of" and/or "consisting essentially of" are also provided.

The term "about" used herein refers to approximately, roughly, nearly, or around. When the term "about" is used in conjunction with a numerical range, it modifies that range by extending the boundaries above and below the stated numerical value. For example, the term "about" can modify a numerical value above and below the stated value by an upward or downward (increasing or decreasing) variation, e.g., 20%, 15%, 10%, 5%, or 1%.

The wording "...represents a bond" used herein means that it is a chemical bond linker (i.e., that it is absent). For example, the term "X represents a bond" means that X is a bond linker. In other words, when X represents a bond, the L of the compound of Formula (I) is directly connected to phenyl in the core structure of the compound of Formula (I).

As used herein, the term "interrupted" of the wording "linear or branched alkylene is interrupted ... by..." used alone or in combination has the definition known in the art, i.e., can mean that there is a group as defined herein (e.g., a group selected from the group consisting of optionally substituted arylene, optionally substituted heteroarylene, or any combination thereof, as defined herein) inserted between any one or more pairs of adjacent carbon atoms in the main carbon chain backbone of the linear or branched alkylene. For example, the wording "the linear or branched alkylene is interrupted one or more times by one or more optionally substituted arylene" refers to that there are one or more (e.g., 1-30, 1-20, 1-15, 1-10, 1-8, 1-7, 1-6, 1-5, 1 -4, 1-3, 1-2, or 1) -arylene-groups inserted between any one or more pairs of two adjacent carbon atoms of the main backbone of the linear or branched alkylene, such that a linear or branched alkylene containing one or more (e.g., 1-10, 1-8, 1-7, 1-6, 1-5, 1-4, 1-3. 1-2, or 1) fragments "-CH₂-arylene-CH₂-" and/or one or more (e.g., 1-30, 1-20, 1-15, 1-10, 1-8, 1-7, 1-6, 1-5, 1-4, 1-3. 1-2, or 1) fragments "-CH₂-arylene-arylene-" is formed, e.g., "-(CH₂)₁₋₂₀-arylene-(CH₂)₁₋₂₀-", or "-(CH₂)₁₋₂₀-arylene-arylene- (CH₂)₁₋₂₀-", or "-(CH₂)₁₋₂₀-arylene-(CH₂)₁₋₂₀-arylene-(CH₂)₁₋₂₀".

Herein, bonds interrupted by wavy lines show the point of attachment of the depicted group to the rest of the molecule. For example, unless otherwise defined, when the moiety R-L- in formula (I) represents the following combined group depicted below, 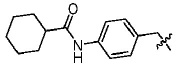 the methylene in the combined group is bonded to the group X of the compound of Formula (I). For example, unless otherwise specified, when the L group in Formula (I) represents the following group with two wave-broken bonds depicted herein, 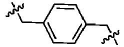 either of the two ends of the group may be connected to the R group, and the other end to X, and vice versa.

As used herein, the term "oxo" or "oxo group" refers to =O.

As used herein, the wordings "optionally substituted" and "unsubstituted or substituted" can be used interchangeably. The term "substituted" usually means that one or more hydrogen atoms in the structure referenced are replaced by the same or different specific substituents.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "Cₓ-C_{y} alkyl" or "C_{x-y} alkyl" (x and y each being an integer) refers to a linear or branched alkyl group containing from x to y carbon atoms. The term "C₁₋₁₀ alkyl" used alone or in combination in the present disclosure refers to a linear or branched alkyl group containing from 1 to 10 carbon atoms. The C₁₋₁₀ alkyl of the present disclosure is preferably C₁₋₉ alkyl, more preferably C₁₋₈ alkyl, still more preferably C₂₋₈ alkyl, even more preferably C₁₋₇ alkyl, even more preferably C₁₋₆ alkyl, C₁₋₃ alkyl, or C₁₋₄ alkyl. Representative examples include methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, and decyl. The term "C₁₋₃ alkyl" in the present disclosure refers to an alkyl group containing from 1 to 3 carbon atoms, and representative examples thereof include methyl, ethyl, n-propyl, and isopropyl. In the present invention, the "alkyl" is optionally substituted, and the substituent may optionally be one or more selected from the group consisting of halogen, hydroxyl, cyano, C₁₋₃ alkyl, C₁₋₃ alkoxy, trifluoromethyl, heterocyclyl, or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group is replaced with one or more halogens. The term "halogenated Cₓ₋c_{y} alkyl" or "halogenated C_{x-y} alkyl" (x and y are each an integer) refers to a linear or branched alkyl containing from x to y carbon atoms substituted with one or more halogens. The term "halogenated C₁₋₁₀ alkyl" used alone or in combination in the present invention refers to a linear or branched alkyl group containing from 1 to 10 carbon atoms substituted with one or more halogens. The halogenated C₁₋₁₀ alkyl group of the present invention is preferably halogenated C₁₋₉ alkyl group, more preferably halogenated C₁₋₈ alkyl group, still more preferably halogenated C₂₋₈ alkyl group, even more preferably halogenated C₁₋₇ alkyl group, halogenated C₁₋₆ alkyl, halogenated C₁₋₃ alkyl, or halogenated C₁₋₄ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halo-C₁₋₃ alkyl" of the present invention refers to an alkyl group containing from 1 to 3 carbon atoms substituted by one or more halogens, and its representative examples include halomethyl, haloethyl, halo-n-propyl and haloisopropyl.

As used herein, the term "alkylene" (which is used interchangeably with "alkylene chain"), used alone or in combination, refers to a linear or branched divalent saturated hydrocarbon group composed of carbon and hydrogen atoms. The term "Cₓ-C_{y} alkylene" or "C_{x-y} alkylene" (x and y each being an integer) refers to a linear or branched alkylene group containing from x to y carbon atoms. The C₁-C₄₀ alkylene in the present disclosure can optionally be C₁-C₃₃ alkylene, C₁-C₃₀ alkylene, C₁-C₂₉ alkylene, C₁-C₂₈ alkylene, C₁-C₂₇ alkylene, C₁-C₂₆ alkylene, C₁-C₂₅ alkylene, C₁-C₂₄ alkylene, C₁-C₂₃ alkylene, C₁-C₂₂ alkylene, C₁-C₂₁ alkylene, C₁-C₂₀ alkylene, C₁-C₁₉ alkylene, C₁-C₁₈ alkylene, C₁-C₁₇ alkylene, C₁-C₁₆ alkylene, C₁-C₁₅ alkylene, C₁-C₁₄ alkylene, C₁-C₁₃ alkylene, C₁-C₁₂ alkylene, C₁-C₁₁ alkylene, C₁-C₁₀ alkylene , C₁-C₉ alkylene, C₁-C₈ alkylene, C₁-C₇ alkylene, C₁-C₆ alkylene, C₁-C₅ alkylene, C₁-C₄ alkylene, C₁-C₃ alkylene, or C₁-C₂ alkylene. Representative examples include, but are not limited to, methylene, ethylene, propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, pentadecylene, hexadecylene, heptadecylene, octadecylene, nonadecylene, eicosylene, heneicosylene, docosylene, tricosylene, tetracosylene, pentacosylene, hexacosylene, peptacosylene, octacosylene, nonacosylene, and triacontylene. In the present disclosure, the "alkylene" is optionally substituted, and the substituent may optionally be one or more selected from C₁₋₃ alkyl, C₁₋₃ alkoxy, C₃₋₆ cycloalkyl, halogen, halogenated C₁₋₃ alkyl, hydroxyl, cyano, or any combination thereof.

As used herein, the term "heteroaryl" used alone or in combination refers to a 5- to 20-membered monocyclic or bicyclic aromatic ring group containing one or more (e.g., from 1 to 6, or from 1 to 5, or from 1 to 4, or from 1 to 3) heteroatoms independently selected from the group consisting of oxygen, nitrogen, and sulfur. Representative examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2a]pyridyl, 1H-pyrrolo[3,2-b]pyridyl, 1H-pyrrolo[2,3-b]pyridyl, 4H-fluoro[3,2b]pyrrolyl, pyrrolo[2,1-b]thiazolyl and imidazo[2,1-b]thiazolyl. The heteroaryl group may be unsubstituted or substituted. The substituted heteroaryl refers to heteroaryl substituted one or more times (e.g., 1-4, 1-3, or 1-2 times) by a substituent optionally selected from the group consisting of C₁₋₃ alkyl, C₁₋₃ alkoxy, C₃₋₆ cycloalkyl, halogenated C₁₋₃ alkyl, C₁₋₃ alkyl substituted by hydroxy, C₁₋₃ alkylamino, C₁₋₃ alkyl-NHC(O)-, halogen, hydroxyl, cyano, amino, or any combination thereof.

As used herein, the term "heteroarylene" used alone or in combination refers to a 5- to 20-membered monocyclic or bicyclic divalent aromatic ring group containing one or more (e.g., from 1 to 6, or from 1 to 5, or from 1 to 4, or from 1 to 3) heteroatoms independently selected from the group consisting of oxygen, nitrogen, and sulfur. Representative examples of heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2a]pyridylene, 1H-pyrrolo[3,2-b]pyridylene, 1H-pyrrolo[2,3-b]pyridylene, 4H-fluoro[3,2b]pyrrolylene, pyrrolo[2,1-b]thiazolylene and imidazo[2,1-b]thiazolylene. The heteroarylene group may be unsubstituted or substituted. A substituted heteroarylene refers to heteroarylene substituted one or more times (e.g., 1-4, 1-3, or 1-2 times) by a substituent optionally selected from the group consisting of C₁₋₃ alkyl, C₁₋₃ alkoxy, C₃₋₆ cycloalkyl, halogenated C₁₋₃ alkyl, C₁₋₃ alkyl substituted by hydroxy, C₁₋₃ alkylamino, C₁₋₃ alkyl-NHC(O)-, halogen, hydroxyl, cyano, amino, or any combination thereof.

As used herein, the term "aryl" used alone or in combination refers to a monovalent aromatic hydrocarbon group containing from 5 to 14 carbon atoms and optionally one or more fused rings, such as phenyl group, naphthyl group, or fluorenyl group. As used herein, the "aryl" is optionally substituted. A substituted aryl group refers to an aryl group optionally substituted one or more times (e.g., 1-4, 1-3, or 1-2 times) with a substituents. For example aryl is mono-, di-, or tri-substituted. The substituents can be selected from e.g., C₁₋₃ alkyl, C₁₋₃ alkoxy, trifluoromethyl, mercapto, cyano, halogen, amino, hydroxyl or any combination thereof.

As used herein, the term "arylene" used alone or in combination refers to a divalent aromatic hydrocarbon group containing from 5 to 14 carbon atoms and optionally one or more fused rings, such as phenylene, naphthylene, or fluorenylene. As used herein, the "arylene" is optionally substituted. A substituted arylene refers to an arylene group optionally substituted one or more times (e.g., 1-4, 1-3, or 1-2 times) with a substituents. For example arylene is mono-, di-, or tri-substituted. The substituents can be selected from e.g., C₁₋₃ alkyl, C₁₋₃ alkoxy, trifluoromethyl, mercapto, cyano, halogen, amino, hydroxyl or any combination thereof.

As used herein, the term "phenyl" used alone or in combination is optionally substituted. A substituted phenyl refers to phenyl optionally substituted with one or more (e.g., 1-4, 1-3 or 1-2) substituents, wherein the substituents are optionally selected from C₁₋₃ alkyl, C₁₋₃ alkoxy, C₃₋₆ cycloalkyl, halogenated C₁₋₃ alkyl, C₁₋₃ alkyl substituted by hydroxy, C₁₋₃ alkylamino, C₁₋₃ alkyl-NHC(O)-, halogen, hydroxyl, cyano, amino, or any combination thereof.

As used herein, the term "phenylene" used alone or in combination is optionally substituted. A substituted phenylene refers to phenylene optionally substituted with 1 to 3 substituents, wherein the substituents are optionally selected from C₁₋₃ alkyl, C₁₋₃ alkoxy, C₃₋₆ cycloalkyl, halogenated C₁₋₃ alkyl, halogen, hydroxyl, cyano, amino, or any combination thereof.

As used herein, the term "alkoxy" used alone or in combination refers to a linear or branched alkoxy group having the Formula of -O-alkyl. The alkyl of the alkoxy may optionally contain 1-10 carbon atoms. Representative examples of "alkoxy" include, but are not limited to, methoxy, ethoxy, propoxy, isopropoxy, n-butoxy, isobutoxy, tert-butoxy, pentoxy, 2-pentyloxy, isopentyloxy, neopentyloxy, hexyloxy, 2-hexyloxy, 3-hexyloxy, 3-methylpentyloxy, etc. The term "C₁-C₃ alkoxy" or "C₁₋₃ alkoxy" used alone or in combination refers to a linear or branched alkoxy group containing from 1 to 3 carbon atoms. Representative examples of C₁₋₃ alkoxy include, but are not limited to, methoxy, ethoxy, n-propoxy, and isopropoxy.

As used herein, the term "cycloalkyl", used alone or in combination, refers to a saturated or partially unsaturated (i.e., containing one or more double bonds, but not having a fully conjugated π-electron system) monocyclic or bicyclic or polycyclic cyclic hydrocarbon radical, which in some embodiments has from 3 to 20 carbon atoms (i.e., C₃₋₂₀ cycloalkyl), or from 3 to 15 carbon atoms (i.e., C₃₋₁₅ cycloalkyl), or from 3 to 12 carbon atoms (i.e., C₃₋₁₂ cycloalkyl), or from 3 to 11 carbon atoms (i.e., C₃₋₁₁ cycloalkyl), or from 3 to 10 carbon atoms (i.e., C₃₋₁₀ cycloalkyl), or from 3 to 8 carbon atoms (i.e., C₃₋₈ cycloalkyl), or from 3 to 7 carbon atoms (i.e., C₃₋₇ cycloalkyl), or from 3 to 6 carbon atoms (i.e., C₃₋₆ cycloalkyl). The term "cycloalkyl" includes monocyclic, bicyclic or tricyclic cyclic hydrocarbon radical having from 3 to 20 carbon atoms. Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Bicyclic and tricyclic cycloalkyl groups include bridged cycloalkyl, fused cycloalkyl and spiro-cycloalkyl groups such as, but not limited to, decalinyl, octahydropentalenyl, octahydro-1H-indenyl, spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) selected from C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, halogen, hydroxyl, cyano, amino, or any combination thereof. Examples of C₃₋₆ cycloalkyl includes, but is not limited to, cyclopropyl, cyclobutyl, cyclopentyl, cyclopentenyl, and cyclohexyl.

As used herein, the term "C_{x-y} spiro-cycloalkyl" (x and y each being an integer), used alone or in combination, refers to a spiro-cycloalkyl group containing from x to y carbon atoms. As used herein, the term "C₇₋₁₁ spiro-cycloalkyl", used alone or in combination, refers to a spiro-cycloalkyl group containing from 7 to 11 carbon atoms. The term "C₅₋₁₅ spiro-cycloalkyl" includes "C₇₋₁₁ spiro-cycloalkyl", and representative examples include, but are not limited to, spiro[3.3]heptyl, spiro[2.5]octyl, spiro[3.5]nonyl, spiro[4.4]nonyl, spiro[4.5]decyl, or spiro[5.5]undecyl. The "C₇₋₁₁ spiro-cycloalkyl" is optionally further substituted with one or more substituents selected from the group consisting of C₁₋₃ alkyl, halogenated C₁₋₃ alkyl, C₁₋₃ alkoxy, deuterated C₁₋₃ alkoxy, C₁₋₃ alkylamino, amino, oxo, halogen, hydroxyl, cyano, or any combination thereof.

As used herein, the term "C_{x-y} bridged cycloalkyl" (x and y each being an integer), used alone or in combination, refers to a bridged cycloalkyl group containing from x to y carbon atoms. As used herein, the term "C₇₋₁₁ bridged cycloalkyl", used alone or in combination, refers to bridged cycloalkyl containing from 7 to 11 carbon atoms. Representative examples of the term "C₇₋₁₁ bridged cycloalkyl" include, but are not limited to, adamantanyl, noradamantanyl, norbornyl (also named as bicyclo[2.2.1]heptyl by the IUPAC system). The "bridged cycloalkyl" is optionally substituted with 1 to 10 substituents selected from the group consisting of C₁₋₃ alkyl, deuterated C₁₋₃ alkyl, C₁₋₃ alkoxy, halogen, halogenated C₁₋₃ alkyl, C₁₋₃ alkylamino, amino, hydroxyl, cyano, oxo, or any combination thereof.

As used herein, the term "cycloalkylene" used alone or in combination refers to a saturated or partially unsaturated (i.e., containing one or more double bonds, but not having a fully conjugated π-electron system) divalent monocyclic or bicyclic or polycyclic cyclic hydrocarbon radical, which in some embodiments has from 3 to 20 carbon atoms (i.e., C₃₋₂₀ cycloalkylene), or from 3 to 15 carbon atoms (i.e., C₃₋₁₅ cycloalkylene), or from 3 to 12 carbon atoms (i.e., C₃₋₁₂ cycloalkylene), or from 3 to 11 carbon atoms (i.e., C₃₋₁₁ cycloalkylene), or from 3 to 10 carbon atoms (i.e., C₃₋₁₀ cycloalkylene), or from 3 to 8 carbon atoms ( i.e., C₃₋₈ cycloalkylene), or from 3 to 7 carbon atoms (i.e., C₃₋₇ cycloalkylene), or from 3 to 6 carbon atoms (i.e., C₃₋₆ cycloalkylene). The term "cycloalkylene" includes monocyclic, bicyclic or tricyclic cycloalkylene having from 3 to 20 carbon atoms. Representative examples of monocyclic cycloalkylene group include, but are not limited to, cyclopropylene, cyclobutylene, cyclopentylene, cyclopentenylene, cyclohexylene, cyclohexenylene, cycloheptylene, and cyclooctylene. The bicyclic and tricyclic cycloalkylene may include divalent bridged cyclic hydrocarbon group, divalent fused cyclic hydrocarbon group, or divalent spiro cyclic hydrocarbon group, for example, but are not limited to, decalinylene, octahydropentalenylene, octahydro-1H-indenylene, spiro-cycloalkylene, adamantanylene, nordamantanylene, bornylene, norbornylene or norcamphanylene (also named as bicyclo[2.2.1]heptanylene by IUPAC system). In the present disclosure, the "cycloalkylene" is optionally mono- or multi-substituted, and includes, but is not limited to, e.g., 2,2-, 2,3-, 2,4-, 2,5-, or 2,6-disubstituted cyclohexylene. The substituents of the substituted "cycloalkylene" is/are optionally one or more (such as 1-5, 1-4, 1-3, 1-2 or 1) selected from C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkylamino, halogen, hydroxyl, cyano, amino, or any combination thereof.

As used herein, the term "C_{x-y} spiro-cycloalkylene" (x and y each being an integer), used alone or in combination, refers to a spiro-cycloalkylene group containing from x to y carbon atoms. As used herein, the term "C₇₋₁₁ spiro-cycloalkylene", used alone or in combination, refers to a spiro-cycloalkylene group containing from 7 to 11 carbon atoms. The term "C₅₋₁₅ spiro-cycloalkylene" includes "C₇₋₁₁ spiro-cycloalkylene", and representative examples include, but are not limited to, spiro[3.3]heptylene, spiro[2.5]octylene, spiro[3.5]nonylene, spiro[4.4]nonylene, spiro[4.5]decylene, or spiro[5.5]undecylene. The "C₇₋₁₁ spiro-cycloalkylene" is optionally further substituted with one or more substituents selected from the group consisting of C₁₋₃ alkyl, halogenated C₁₋₃ alkyl, C₁₋₃ alkoxy, C₁₋₃ alkylamino, amino, oxo, halogen, hydroxyl, cyano, or any combination thereof.

As used herein, the term "heterocyclyl" or "heterocyclic group" used alone or in combination refers to a 3- to 20-membered saturated or partially unsaturated (i.e., containing one or more double bonds, but not having a fully conjugated π-electron system) monocyclic, bicyclic, or tricyclic cyclic hydrocarbon group containing one or more (e.g., from 1 to 5, or from 1 to 4, or from 1 to 3, or from 1 to 2, or 1) heteroatoms independently selected from sulfur, oxygen, and nitrogen. In some embodiments, "heterocyclyl" may preferably refer to a 3- to 15-membered (optionally 3- to 14-membered, 3- to 12-membered, 3- to 11-membered, 3- to 10-membered, 3- to 9-membered, 3- to 8-membered, 3- to 7-membered, 3- to 6-membered, or 3- to 5-membered) saturated or partially unsaturated (i.e., containing one or more double bonds, but not having a fully conjugated π-electron system) monocyclic cyclic hydrocarbon group containing one or more (e.g., from 1 to 5, or from 1 to 4, or from 1 to 3, or from 1 to 2, or 1) heteroatoms independently selected from sulfur, oxygen, and nitrogen. Representative examples of the heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, 1,4-diazacycloheptan-1-yl, 3,8-diazabicyclo[3.2.1]octan-3-yl, 2,5-diazabicyclo[2.2.2]octan-2-yl, and azaspirocycloalkyl (e.g., 3-azaspiro[5.5]undecane-3-yl). The heterocyclyl may be unsubstituted or substituted as explicitly defined (e.g., mono-, di-, tri-, or poly-substituted), and the substituents of the heterocyclyl can be preferably selected from C₁₋₃ alkyl, C₁₋₃ alkoxy, halogen, halogenated C₁₋₃ alkyl, C₁₋₃ alkyl substituted by hydroxy, C₁₋₃ alkyl substituted by NH₂, oxo, C₁₋₃ alkyl-NHC(O)-, hydroxy, cyano, oxo, C₁₋₃ alkylamino, amino, or any combination thereof.

As used herein, the term "heterocyclylene" used alone or in combination refers to a 3- to 20-membered saturated or partially unsaturated (i.e., containing one or more double bonds, but not having a fully conjugated π-electron system) monocyclic, bicyclic, or tricyclic bivalent cyclic hydrocarbon group containing one or more (e.g., from 1 to 5, or from 1 to 4, or from 1 to 3, or from 1 to 2, or 1) heteroatoms independently selected from sulfur, oxygen, and nitrogen. In some embodiments, "heterocyclylene" may refer to e.g., a 3- to 15-membered (optionally 3- to 14-membered, 3- to 12-membered, 3- to 11-membered, 3- to 10-membered, 3- to 9-membered, 3- to 8-membered, 3- to 7-membered, 3- to 6-membered, or 3- to 5-membered) saturated or partially unsaturated (i.e., containing one or more double bonds, but not having a fully conjugated π-electron system) monocyclic bivalent cyclic hydrocarbon group containing one or more (e.g., from 1 to 5, or from 1 to 4, or from 1 to 3, or from 1 to 2, or 1) heteroatoms independently selected from sulfur, oxygen, and nitrogen. Representative examples of the heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, diazacycloheptylene (e.g., 1,4-diazacycloheptanylene, 4,5-diazacycloheptanylene, 1,3-diazacycloheptanylene), 3,8-diazabicyclo[3.2.1]octanylene, 2,5-diazabicyclo[2.2.2]octanylene, and azaspirocycloalkylene (e.g., 3-azaspiro[5.5]undecaneylene). The heterocyclylene may be unsubstituted or substituted as explicitly defined, and the substituents of the heterocyclylene can be optionally selected from C₁₋₃ alkyl, C₁₋₃ alkoxy, halogen, halogenated C₁₋₃ alkyl, C₁₋₃ alkyl substituted by hydroxy, C₁₋₃ alkyl substituted by NH₂, oxo, C₁₋₃ alkyl-NHC(O)-, hydroxy, cyano, oxo, C₁₋₃ alkylamino, amino, or any combination thereof.

As used herein, the term "alkynylene", used alone or in combination, refers to a linear or branched divalent hydrocarbon group containing from 2 to 6 (e.g., from 2 to 5, from 2 to 4, preferably 2) carbon atoms and having one or more (e.g., from 1 to 3, from 1 to 2, or 1) carbon-carbon triple bonds. Representative examples of alkynylene include, but are not limited to, ethynylene, 1-propynylene, 1-butynylene, and 1,3-diynylene.

As used herein, the term "alkenylene", used alone or in combination, refers to a linear or branched divalent hydrocarbon group containing from 2 to 6 (e.g., from 2 to 5, from 2 to 4, from 2 to 3, or 2) carbon atoms and having one or more (e.g., from 1 to 3, from 1 to 2, or 1) carbon-carbon double bonds. Representative examples of alkenylene groups include, but are not limited to, vinylene (e.g., -CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, "bornylane" or "bornane" (also known as 1,7,7-trimethylbicyclo[2.2.1]heptane; camphane; bornylane) has a definition known to those skilled in the art and its structural formula is e.g., as follows: 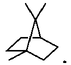 As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

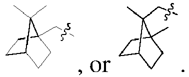

As used herein, "bicyclo[2.2.1]heptane" also known as "norbornane", has a structural formula as follow: 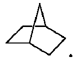 As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a structural formula as follow: 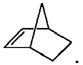 As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl.

As used herein, "adamantane" (also known as tricyclo[3.3.1.1^{3,7}]decane) has a definition known to those skilled in the art, and its structural formula is e.g., as follows: 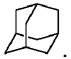 As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows: 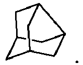 As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1^{3,7}]decan-1-amine; CAS No.: 768-94-5), with the following structural Formula:

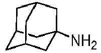

As used herein, "*p*-menthane" has a definition known to those skilled in the art, and its structural formula is e.g., as follows: 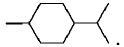 As used herein, "*p*-menthanyl" refers to a monovalent group of *p*-menthane, that is, the group remaining after any hydrogen in *p*-menthane is removed. Representative examples of "*p*-menthanyl" include, but are not limited to,

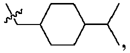

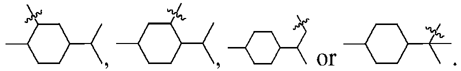

As used herein, "meta-menthane" has a definition known to those skilled in the art, and its structural formula is e.g., as follows: 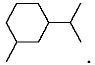 As used herein, "meta-menthanyl" refers to a monovalent group of meta-menthane, that is, the group remaining after any hydrogen in meta-menthane is removed. Representative examples of "meta-menthanyl" include, but are not limited to,

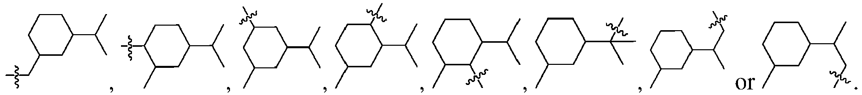

As used herein, "quinuclidine" (also known as 1-azabicyclo[2.2.2]octane) has a definition known to those skilled in the art, and its structural formula is e.g., as follows: 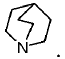 As used herein, "quinuclidinyl" refers to a monovalent group of quinuclidine, that is, the group remaining after any hydrogen in quinuclidine is removed. Representative examples of "quinuclidinyl" include, but are not limited to,

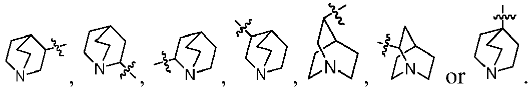

Salts or pharmaceutically acceptable salts, enantiomers, diastereoisomers, solvates, polymorphs of the compounds of Formula (I) of the present disclosure are also encompassed within the scope of the present invention.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers which have two or more chiral centers but which are non-mirror images. The diastereomers have different physical properties, such as melting point, boiling point, spectral properties and reactivity. The diastereomeric mixture can be separated by high-resolution analytical operations such as electrophoresis and chromatography, such as HPLC.

As used herein, the term "room temperature" refers to the ambient temperature, such as 20-30°C.

In all embodiments of the present disclosure, the salts or pharmaceutically acceptable salts of the compounds of Formula (I) refer to non-toxic inorganic or organic acid and/or base addition salts. Examples include: sulfate, hydrochloride, citrate, maleate, sulfonate, citrate, lactate, tartrate, fumarate, phosphate, dihydrogenphosphate, pyrophosphate, metaphosphate, oxalate, malonate, benzoate, mandelate, succinate, glycolate, or *p*-toluenesulfonate, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include, but are not limited to, sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

The term "treatment" or "treating" refers to the administration of the compound of Formula (I) or a pharmaceutically acceptable salt thereof according to the present disclosure, or the pharmaceutical composition containing, as an active ingredient, the compound of Formula I or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutically effective amount" of a compound of the present disclosure depends on the age, sex, and weight of the patient; the patient's current medical condition; the cancer or tumor progression of the patient being treated. Those skilled in the art will be able to determine appropriate dosages based on these and other factors.

It should be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see Jun Li ed., Clinical Pharmacology, 4th Edition, People's Medical Publishing House,China (2008)).

### Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:
- ACN: Acetonitrile
- Ac₂O: acetic anhydride
- AcOK: potassium acetate
- AIBN: Azobisisobutyronitrile
- Bipy: bipyridine
- BnCl: benzyl chloride
- Boc: tert-butoxycarbonyl
- BPO: dibenzoyl peroxide
- Cu(OAc)₂: copper acetate
- DCM: dichloromethane
- DIPEA: N,N-diisopropylethylamine
- DMF: N,N-dimethylformamide
- DMAP: N,N-dimethyl pyridine-4-amine
- DMSO: dimethyl sulfoxide
- EDCI: 1-(3-dimethylaminopropyl)-3-ethylcarbodiimide
- equiv or eq: equivalent
- ESI: electrospray ionization
- EtOAc: ethyl acetate
- Et₃N: triethylamine
- h: hour
- HPLC: high performance liquid chromatography
- HRMS: high-resolution mass spectrometry
- LC-MS: liquid chromatography-mass spectrometry
- LRMS: low resolution mass spectrometry
- LC: liquid chromatography
- MeOH: methanol
- MeCN: acetonitrile
- MW: microwave
- NMP: N-methylpyrrolidone
- ¹H NMR: H NMR spectroscopy
- -OMe: methoxy
- PhMe: toluene
- Py: pyridine
- rt: room temperature
- tBuONO: tert-butyl nitrite
- TEA: triethylamine
- TFA: trifluoroacetate
- THF: tetrahydrofuran
- TLC: thin layer chromatography
- TMS: tetramethylsilane

In the present disclosure, the ¹H NMR spectrum was recorded on a Bruker-500MHz nuclear magnetic resonance instrument, by using, as a solvent, CD₃OD (δ = 3.31 ppm) containing 0.1% TMS (as an internal standard); or using, as a solvent, CDCl₃ (δ = 7.26 ppm) containing 0.1% TMS (as an internal standard); or using, as a solvent, DMSO-*d*₆ (δ = 2.50 ppm) containing 0.03% TMS (as an internal standard). HRMS spectrum was recorded on an AB Triple 4600 mass spectrometer, and HPLC purity was measured on a SHIMADZU LC-30AP or Waters 1525 type instrument. Unless otherwise specified, all reactions were performed in the air atmosphere. The reactions were followed by TLC or LC-MS, intermediates were isolated and purified by column chromatography using an ISCO or Biotage, and the designed and synthesized target products were separated and purified by the Waters 2767 preparative HPLC.

Solvents and reagents are processed as follows:
The solvents used in the reaction such as DCM, DMF, anhydrous EtOH, and anhydrous MeOH were purchased from Chinese Sinopharm Group; Preparative grade CH₃CN and deionized water were used in HPLC preparation. Unless otherwise specified, other reaction substrates, reagents, medicines were commercially available.

### General synthetic methods

The compounds described herein and/or pharmaceutically acceptable salts thereof can be synthesized using commercially available raw materials by synthetic techniques known in the art. The synthetic schemes described below illustrate the preparation of most compounds. The starting materials or reagents used in each scheme can be commercially available or prepared by methods known to those skilled in the art. The salts, racemates, enantiomers, phosphates, sulfates, hydrochlorides and prodrug forms of the compounds of Formula (I) of the present disclosure can be prepared by those skilled in the art according to routine techniques in the art. In each of the schemes described below, the group R₁ corresponds to the R-L moiety of the compound of formula (I) of the present disclosure, or to the R group of the compound of formula (I) of the present disclosure.

### Synthesis Scheme 1:

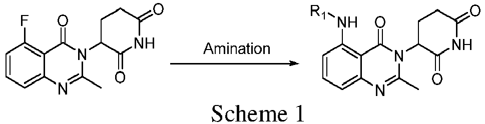

In Scheme 1, the group R₁ represents the R-L moiety of the compound of formula (I) according to the present disclosure.

A mixture of compound 3-(5-fluoro-2-methyl-4-oxoquinazolin-3(4H)-yl)piperidine-2,6-dione, corresponding amino compound and organic base is reacted under heating (for example, reacted for 2-4 hours, then purified by using preparative reverse-phase liquid chromatography and lyophilized) to obtain the final target compound.

### Synthesis Scheme 2:

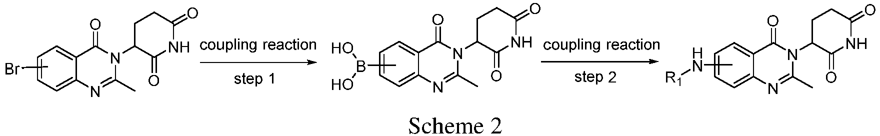

In Scheme 2, the group R₁ corresponds to the R-L moiety of the compound of formula (I) according to the present disclosure.

Step 1: a mixture of brominated 3-(2-methyl-4-oxoquinazolin-3(4H)-yl)piperidine-2,6-dione, bis(pinacolate)diboron, base and catalyst in organic solvent is reacted under reflux (such as reacted for 3h, then concentrated and subjected to a column chromatography) to obtain boric acid compound product which is directly used in the next step.

Step 2: a mixture of the boric acid compound obtained from step 1, corresponding amino compound, catalyst and base in organic solvent is reacted under reflux (in air atmosphere) (for example, reacted for 12 h, then purified by using preparative reverse-phase liquid chromatography and lyophilized) to obtain the final target compound.

### Synthesis Scheme 3:

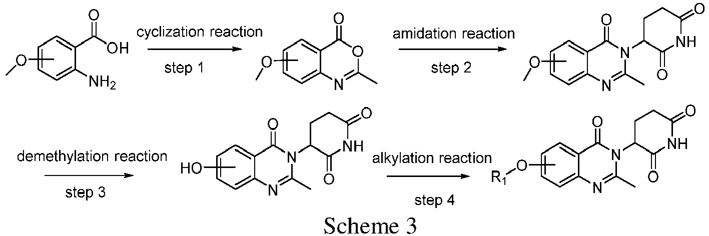

In Scheme 3, the group R₁ corresponds to the R-L moiety of the compound of formula (I) according to the present disclosure.

Methoxy-2-amino-benzoic acid undergoes cyclization reaction with acetic anhydride to obtain a cyclized compound, which subsequently undergoes amidation reaction with the compound 3-aminopiperidine-2,6-dione hydrochloride in the presence of base. The resulting product is treated with hydrochloric acid to obtain the target amidated compound. In step 3, the amidation compound obtained from step 2 undergoes demethylation reaction in the presence of boron tribromide, and the resulting product undergoes alkylation reaction in the presence of corresponding brominated compound and inorganic base in step 4 to obtain the final target compound.

For example, in an embodiment, the optional reaction conditions of Scheme 3 are as follows:
Step 1: to acetic anhydride is added methoxy-2-amino-benzoic acid. The mixture is reacted under reflux (for such as 4 h), and then concentrated to remove the acetic anhydride. The residue is directly used in the next step.
Step 2: the product obtained from step 1 and 3-aminopiperidine-2,6-dione hydrochloride are added into an alkaline solvent, and the resulting mixture is heated and reacted (for such as 2-4 h), and then concentrated to remove the solvent. The residue is washed with hydrochloric acid and ethyl acetate respectively. The resulting solid is stirred in methanol, and then filtered to obtain the corresponding compound product.
Step 3: to a mixture of the product obtained from step 2 in organic solvent is slowly added dropwise boron tribromide in an ice bath. The mixture is reacted overnight at room temperature and then concentrated and used directly in the next step.
Step 4: a mixture of the concentrate obtained from step 3, a corresponding brominated compound substrate and inorganic bases in organic solvent is stirred and reacted at room temperature or under heating (for such as 2h), and then the resulting mixture is subjected to a preparative reverse-phase liquid chromatography for purification and lyophilized to give the final target compound.

### Synthesis Scheme 4:

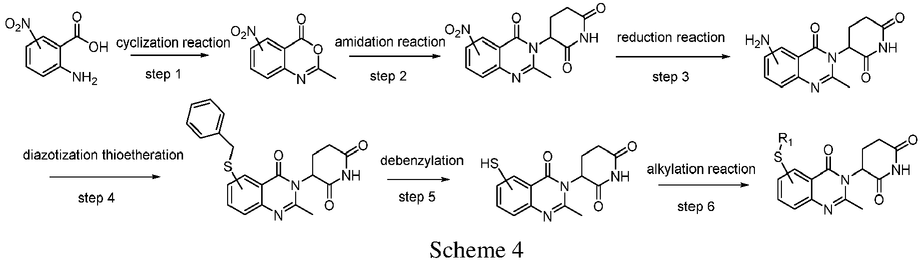

In Scheme 4, the group R₁ corresponds to the R-L moiety of the compound of formula (I) according to the present disclosure.

The compound nitro-2-aminobenzoic acid undergoes cyclization reaction with acetic anhydride to obtain the cyclized product, which subsequently undergoes amidation reaction with the compound 3-aminopiperidine-2,6-dione hydrochloride in the presence of base, and the resulting product is treated with hydrochloric acid to obtain the nitro intermediate compound. In step 3, the nitro intermediate compound obtained from step 2 is subject to reduction reaction, and the resulting reduction product is then subject to diazotization thioetherification reaction in step 4 according to the method known to those skilled in the art to obtain benzyl thio compound. In step 5, the benzyl thio compound obtained from step 4 is subject to debenzylaton to remove benzyl group according to a method known to those skilled in the art to obtain a thiophenol compound. In step 6, the thiophenol compound obtained from step 5 undergoes alkylation reaction in the presence of corresponding brominated compounds and inorganic bases (such as potassium carbonate) to obtain the target compound.

For example, in an embodiment, the optional reaction conditions of Scheme 4 are as follows:
Step 1: to nitro-2-aminobenzoic acid is added acetic anhydride. The mixture is reacted under reflux (for such as 2-4 h), and then concentrated, and the resulting concentrate is directly used in the next step.
Step 2: a mixture of the concentrate obtained from step 1 and 3-aminopiperidine-2,6-dione hydrochloride in an alkaline solvent is reacted under reflux (for such as 1 to 3 hours), and concentrated under reduced pressure. The resulting concentrate is then stirred in hydrochloric acid (for such as half an hour) and filtered. The resulting solid is washed with ethyl acetate, and then triturated with methanol and filtered to obtain the nitro compound.
Step 3: a mixture of the nitro compound and palladium hydroxide (catalytic amount) in methanol is stirred in hydrogen gas atmosphere (for such as 48 h), filtered, and sujected to column chromatography to obtain the reduction product.
Step 4: A reaction flask containing methanol and water is charged with sodium thiosulfate pentahydrate, benzyl bromide, copper sulfate pentahydrate (catalytic amount) and bipyridine (catalytic amount). The mixture is slowly warmed (for example, to 80°C) and stirred (for such as 2h). Then the reaction solution is cooled to room temperature, to which the reduction product from step 3 is added, followed by slowly addition dropwise tert-butyl nitrite. After addition, the reaction solution is warmed again (for example, to 80°C) and stirred (for scuh as 8 h). After the reaction was complete, the reaction solution was cooled to room temperature, diluted with water and extracted with ethyl acetate. The organic phases were combined, washed with water and saturated brine. Organic phase is dried over anhydrous sodium sulfate, and concentrated under reduced pressure to remove solvent. The resulting crude is subjected to column chromatography (eluent (v/v): petroleum ether/ethyl acetate=1:2) for purification to obtain benzyl thio compound.
Step 5: An egg-shaped flask was charged with anhydrous aluminum trichloride and anhydrous toluene, followed by slowly addition of the benzyl thio compound. After addition, the reaction mixture is stirred overnight at 35°C. After the reaction was complete, to the reaction mixture is slowly added 20% citric acid aqueous solution under stirring, and a large amount of off-white solids are precipitated out. The solids are filtrated, and the filter cake is washed with water and ethyl acetate respectively, and dried to obtain the target thiophenol compound.
Step 6: a mixture of the thiophenol compound obtained from step 5, corresponding brominated compound and potassium carbonate in organic solvent is stirred and reacted at room temperature or under heating (for such as 2h), and then the resulting mixture is subjected to a preparative reverse-phase liquid chromatography for purification and lyophilized to give the final target compound.

### Synthesis Scheme 5:

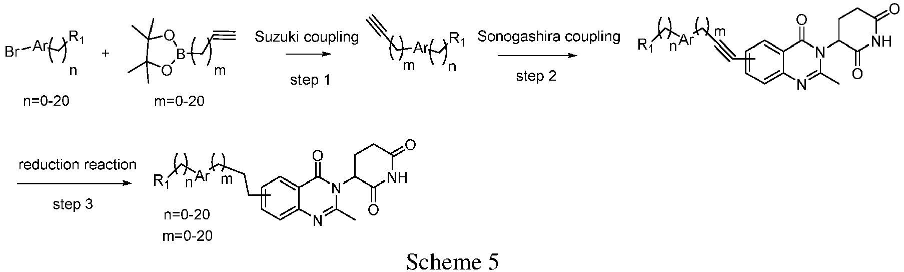

In Scheme 5, the group R₁ corresponds to the R of the compound of formula (I) according to the present disclosure, and Ar represents arylene or heteroarylene.

Brominated carboxylate compound undergos Suzuki coupling reaction with alkynyl borate compound in the presence of base and catalyst according to the method known to those skilled in the art to obtain alkynyl carboxylate compound. Then the alkynyl carboxylate compound and brominated 3-(2-methyl-4-oxoquinazolin-3(4H)-yl)piperidine-2,6-dione undergo Sonogashira coupling reaction in the presence of base and catalyst according to the method known to those skilled in the art to obtain the condensation product, which then undergos reduction reaction to obtain the target reduction product.

For example, in an embodiment, the optional reaction conditions of Scheme 5 are as follows:
Step 1: to a mixture of brominated carboxylate and alkynyl borate in dioxane are added water, base and catalyst. The resulting mixture is reacted under reflux (for such as 2h), cooled, filtered, and subjected to column chromatography to obtain alkynyl carboxylate, which is directly used in the next step.
Step 2: the alkynyl carboxylate compound and brominated 3-(2-methyl-4-oxoquinazolin-3(4H)-yl)piperidine-2,6-dione are added into tetrahydrofuran together with organic base and catalyst. The resulting mixture is reacted at room temperature or under heating (for such as 12h), filtered and then subjected to a preparative reverse-phase liquid chromatography for purification to obtain the target intermediate.
Step 3: to a mixture of the condensation product obtained from step 2 in methanol is added 10% Pd/C, and the resulting mixture is then stirred overnight in hydrogen gas atmosphere. The reaction mixture is filtered, and the filtrate is subjected to a preparative HPLC chromatography for separation and purification, and lyophilized to give the corresponding reduction target compound.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by changing the substituents and the like to obtain subsequent target compounds through methods well known to those skilled in the art.

### Examples

### Example 1: preparation of 3-(2-methyl-5-((4-(morpholinomethyl)benzyl)amino)-4-oxoquinazolin-3(4H)-yl)piperidine-2,6-dione (SIAIS264067)

The target compound SIAIS264067 was prepared with reference to the method of Scheme 1.

A mixture of 3-(5-fluoro-2-methyl-4-oxoquinazolin-3(4H)-yl)piperidine-2,6-dione (30 mg, 0.103 mmol), (4-(morpholinomethyl)phenyl)methylamine (23 mg, 1.1 eq) and diisopropylethylamine (25 µl, 1.5 eq) was reacted in microwave at 140 °C for 2 hours, then filtered, and subjected to preparative HPLC (eluent (v/v): acetonitrile/(water+0.05% HCl)=10%-100%) for separation. The collected fractions were rotary evaporated to remove acetonitrile, and lyophilized to give the final target compound SIAIS264067 (white solid, 18 mg, 36%). ¹H NMR (500 MHz, DMSO) *δ* 10.99 (s, 1H), 8.77 (t, *J* = 5.4 Hz, 1H), 7.44 (dd, *J* = 19.7, 11.6 Hz, 1H), 7.31 (brs, 4H), 6.66 (d, *J* = 7.8 Hz, 1H), 6.45 (d, *J* = 8.3 Hz, 1H), 5.20 (dd, *J* = 11.5, 5.7 Hz, 1H), 4.43 (d, *J* = 5.1 Hz, 2H), 3.57 (s, 4H), 3.43 (s, 2H), 2.83 (ddd, *J* = 16.3, 13.7, 5.4 Hz, 1H), 2.68 - 2.60 (m, 1H), 2.61 - 2.58 (m, 1H), 2.56 (s, 4H), 2.42 - 2.24 (m, 4H), 2.22 - 2.10 (m, 1H). ¹³C NMR (126 MHz, DMSO) *δ* 173.10, 170.10, 163.40, 154.54, 150.10, 149.17, 136.04, 129.76, 127.64, 112.75, 112.31, 106.56, 105.11, 56.72, 45.83, 31.05, 23.43, 21.50. HRMS (ESI) calcd for C₂₆H₃₀N₅O₄⁺ [M+H]⁺, 476.2292; found, 476.2306.

### Example 2: preparation of 3-(2-methyl-4-oxo-5-(((tetrahydro-2H-pyran-4-yl)methyl)amino)quinazolin-3(4H)-yl)piperidine-2,6-dione (SIAIS264066)

Referring to the methods of Schme 1 and example 1, the target compound SIAIS264066 was prepared under appropriate conditions that will be recognized by one skilled in the art. The target compound SIAIS264066 was obtained as a white solid (12 mg, 30%). ¹H NMR (500 MHz, DMSO) *δ* 11.00 (s, 1H), 8.43 (t, *J* = 5.6 Hz, 1H), 7.48 (t, *J* = 8.1 Hz, 1H), 6.63 (t, *J* = 8.6 Hz, 1H), 6.55 (t, *J* = 8.8 Hz, 1H), 5.18 (dd, *J* = 11.6, 5.7 Hz, 1H), 3.85 (dd, *J =* 11.3, 3.1 Hz, 2H), 3.29 (dd, *J* = 17.1, 6.3 Hz, 2H), 3.09 (dd, *J* = 15.8, 9.6 Hz, 2H), 2.91 - 2.78 (m, 1H), 2.63 (ddd, *J* = 26.1, 16.6, 8.7 Hz, 2H), 2.55 (s, 3H), 2.19 - 2.10 (m, 1H), 1.85 (ttd, *J* = 10.7, 6.9, 3.5 Hz, 1H), 1.61 (d, *J* = 12.9 Hz, 2H), 1.32 - 1.17 (m, 2H). ¹³C NMR (126 MHz, DMSO) *δ* 173.13, 170.16, 163.52, 154.47, 150.54, 149.22, 136.16, 111.85, 106.10, 104.86, 34.27, 31.10, 31.00, 23.74, 21.48. LC-MS (ESI) C₂₀H₂₅N₄O₄⁺ [M+H]⁺, found, 384.

### Example 3: preparation of 3-(2-methyl-4-oxo-5-((2-(phenylamino)ethyl)amino)quinazolin-3(4H)-yl)piperidine-2,6-dione (SIAIS264097)

Referring to the methods of Schme 1 and example 1, the target compound SIAIS264097 was prepared under appropriate conditions that will be recognized by one skilled in the art. The target compound SIAIS264097 was obtained as a white solid (6 mg, 14%). ¹H NMR (500 MHz, DMSO) *δ* 10.98 (s, 1H), 8.45 (s, 1H), 7.50 (t, *J* = 8.1 Hz, 1H), 7.07 (t, *J* = 7.8 Hz, 2H), 6.66 (d, *J* = 7.7 Hz, 1H), 6.61 (d, *J* = 7.9 Hz, 2H), 6.57 (d, *J* = 8.4 Hz, 1H), 6.54 (t, *J* = 7.3 Hz, 1H), 5.77 (s, 1H), 5.18 (dd, *J* = 11.6, 5.7 Hz, 1H), 3.38 (s, 2H), 3.26 (t, *J* = 6.2 Hz, 2H), 2.88 - 2.78 (m, 1H), 2.67 - 2.58 (m, 2H), 2.56 (s, 3H), 2.17 - 2.08 (m, 1H). ¹³C NMR (126 MHz, DMSO) *δ* 173.14, 170.09, 166.18, 163.23, 150.32, 149.27, 136.17, 129.30, 116.19, 112.48, 112.04, 105.94, 104.86, 56.59, 42.44, 42.22, 31.07, 23.65. HRMS (ESI) calcd for C₂₂H₂₄N₅O₃⁺ [M+H]⁺, 406.1874; found, 406.1873.

### Example 4: preparation of 3-(2-methyl-4-oxo-5-(phenethylamino)quinazolin-3(4H)-yl)piperidine-2,6-dione (SIAIS264103)

Referring to the methods of Schme 1 and example 1, the target compound SIAIS264103 was prepared under appropriate conditions that will be recognized by one skilled in the art. The target compound SIAIS264103 was obtained as a white solid (20 mg, 37%). ¹H NMR (500 MHz, DMSO) *δ* 11.04 (s, 1H), 8.42 (s, 1H), 7.54 (t, *J* = 8.1 Hz, 1H), 7.33 - 7.25 (m, 4H), 7.24 - 7.18 (m, 1H), 6.72 (d, *J* = 7.8 Hz, 1H), 6.65 (d, *J* = 8.4 Hz, 1H), 5.22 (dd, *J* = 11.5, 5.7 Hz, 1H), 3.42 (t, *J* = 7.5 Hz, 2H), 2.93 - 2.88 (m, 2H), 2.87 - 2.78 (m, 1H), 2.66 - 2.57 (m, 5H), 2.22 - 2.06 (m, 1H). ¹³C NMR (126 MHz, DMSO) *δ* 173.11, 169.88, 162.76, 150.24, 139.64, 136.62, 129.24, 128.83, 126.69, 106.78, 104.30, 56.76, 44.46, 34.94, 31.04, 23.03, 21.43. HRMS (ESI) calcd for C₂₂H₂₃N₄O₃⁺ [M+H]⁺, 391.1765; found, 391.1764.

### Example 5: preparation of 3-(2-methyl-4-oxo-5-((pyridin-2-ylmethyl)amino)quinazolin-3(4H)-yl)piperidine-2,6-dione (SIAIS264120)

Referring to the methods of Schme 1 and example 1, the target compound SIAIS264120 was prepared under appropriate conditions that will be recognized by one skilled in the art. The target compound SIAIS264120 was obtained as a white solid (6 mg, 15%). ¹H NMR (500 MHz, DMSO) *δ* 11.11 (s, 1H), 9.00 (s, 1H), 8.77 (d, *J* = 5.5 Hz, 1H), 8.28 (t, *J* = 7.7 Hz, 1H), 7.75 (d, *J* = 7.7 Hz, 2H), 7.53 (t, *J* = 8.1 Hz, 1H), 6.88 (t, *J* = 12.1 Hz, 1H), 6.59 (t, *J* = 14.1 Hz, 1H), 5.33 (dd, *J* = 11.6, 5.7 Hz, 1H), 4.86 (s, 2H), 2.95 - 2.82 (m, 1H), 2.74 (s, 3H), 2.69 - 2.56 (m, 2H), 2.30 - 2.15 (m, 1H). ¹³C NMR (126 MHz, DMSO) *δ* 173.25, 169.58, 162.05, 157.40, 155.79, 149.53, 143.28, 136.74, 125.18, 124.20, 114.01, 107.74, 104.79, 56.97, 45.18, 31.04, 22.44, 21.46. HRMS (ESI) calcd for C₂₀H₂₀N₅O₃⁺ [M+H]⁺, 378.1561; found, 378.1564.

### Example 6: preparation of 3-(5-((2-fluorobenzyl)amino)-2-methyl-4-oxoquinazolin-3(4H)-yl)piperidine-2,6-dione (SIAIS264123)

Referring to the methods of Schme 1 and example 1, the target compound SIAIS264123 was prepared under appropriate conditions that will be recognized by one skilled in the art. The target compound SIAIS264123 was obtained as a white solid (10 mg, 24%). ¹H NMR (500 MHz, DMSO) *δ* 10.99 (s, 1H), 8.75 (t, *J* = 5.8 Hz, 1H), 7.50 - 7.42 (m, 1H), 7.34 (dt, *J* = 21.4, 6.8 Hz, 2H), 7.27 - 7.19 (m, 1H), 7.16 (t, *J* = 7.4 Hz, 1H), 6.68 (d, *J* = 7.8 Hz, 1H), 6.47 (d, *J* = 8.3 Hz, 1H), 5.20 (dd, *J* = 11.5, 5.6 Hz, 1H), 4.50 (d, *J* = 5.8 Hz, 2H), 2.91 - 2.75 (m, 1H), 2.65 (ddd, *J* = 23.6, 14.4, 9.9 Hz, 2H), 2.57 (s, 3H), 2.23 - 2.08 (m, 1H). ¹³C NMR (126 MHz, DMSO) *δ* 173.25, 170.29, 163.31, 154.46, 149.87, 148.90, 136.02, 129.75, 126.18, 124.83, 115.63, 112.30, 106.40, 105.05, 56.74, 31.03, 23.43, 21.19. HRMS (ESI) calcd for C₂₁H₂₀FN₄O₃⁺ [M+H]⁺, 395.1514; found, 395.1516.

### Example 7: preparation of 3-(5-((4-(((1R,3R,SS)-adamantan-1-ylamino)methyl)benzyl)amino)-2-methyl-4-oxoquinazolin-3(4H)-yl)piperidine-2,6-dione (SIAIS264156)

Referring to the methods of Schme 1 and example 1, the target compound SIAIS264156 was prepared under appropriate conditions that will be recognized by one skilled in the art. The target compound SIAIS264156 was obtained as a white solid (15 mg, 26%). ¹H NMR (500 MHz, MeOD) *δ* 7.54 - 7.47 (m, 4H), 7.43 (t, *J* = 8.2 Hz, 1H), 6.73 (t, *J* = 10.7 Hz, 1H), 6.50 (d, *J* = 8.4 Hz, 1H), 5.22 (dd, *J* = 11.0, 5.7 Hz, 1H), 4.52 (s, 2H), 4.17 (s, 2H), 2.95 - 2.78 (m, 2H), 2.74 (dd, *J* = 21.3, 6.0 Hz, 1H), 2.68 (s, 3H), 2.31 - 2.21 (m, 4H), 2.05 (d, *J* = 17.3 Hz, 7H), 1.84 (d, *J* = 12.5 Hz, 3H), 1.77 (d, *J* = 12.5 Hz, 3H). ¹³C NMR (126 MHz, MeOD) *δ* 173.08, 170.03, 162.85, 156.43, 150.00, 140.43, 135.58, 130.67, 130.06, 127.92, 110.68, 106.92, 104.78, 57.65, 56.44, 46.02, 43.02, 37.93, 35.15, 30.19, 29.23, 21.43, 21.26. HRMS (ESI) calcd for C₃₂H₃₈N₅O₃⁺ [M+H]⁺, 540.2969; found, 540.2971.

### Example 8: preparation of 3-(5-((4-(((1R,2s,3S,5r)-adamantan-2-ylamino)methyl)benzyl)amino)-2-methyl-4-oxoquinazolin-3(4H)-yl)piperidine-2,6-dione (SIAIS264172)

Referring to the methods of Schme 1 and example 1, the target compound SIAIS264172 was prepared under appropriate conditions that will be recognized by one skilled in the art. The target compound SIAIS264172 was obtained as a white solid (20 mg, 17%). ¹H NMR (500 MHz, DMSO) δ 11.01 (s, 1H), 8.81 (s, 2H), 8.75 (s, 1H), 7.55 (d, *J* = 7.9 Hz, 2H), 7.44 (dd, *J* = 17.9, 8.1 Hz, 3H), 6.69 (d, *J* = 7.9 Hz, 1H), 6.46 (d, *J=* 8.5 Hz, 1H), 5.22 (dd, *J* = 11.4, 5.7 Hz, 1H), 4.47 (s, 2H), 4.17 (t, *J* = 5.3 Hz, 2H), 3.25 (s, 1H), 2.85 (dd, *J* = 23.4, 9.9 Hz, 1H), 2.67 - 2.54 (m, 2H), 2.60 (s, 3H), 2.19 (s, 3H), 2.07 (d, *J* = 13.1 Hz, 2H), 1.83 (d, *J* = 8.4 Hz, 4H), 1.73 - 1.60 (m, 4H), 1.56 (d, *J* = 13.1 Hz, 2H). HRMS (ESI) calcd for C₃₂H₃₈N₅O₃⁺ [M+H]⁺, 540.2969; found, 540.2969.

### Example 9: preparation of 3-(5-((benzo[d][1,3]dioxol-5-ylmethyl)amino)-2-methyl-4-oxoquinazolin-3(4H)-yl)piperidine-2,6-dione (SIAIS264177)

Referring to the methods of Schme 1 and example 1, the target compound SIAIS264177 was prepared under appropriate conditions that will be recognized by one skilled in the art. The target compound SIAIS264177 was obtained as a white solid (30 mg, 26%). ¹H NMR (500 MHz, DMSO) *δ* 10.99 (s, 1H), 8.69 (s, 1H), 7.45 (t, *J* = 8.1 Hz, 1H), 6.89 (d, *J* = 12.0 Hz, 1H), 6.85 (dd, *J* = 17.2, 7.9 Hz, 2H), 6.66 (d, *J* = 7.8 Hz, 1H), 6.49 (t, *J* = 14.2 Hz, 1H), 5.99 (s, 2H), 5.19 (dd, *J* = 11.5, 5.6 Hz, 1H), 4.33 (s, 2H), 2.83 (dd, *J* = 16.7, 13.6 Hz, 1H), 2.69 - 2.59 (m, 2H), 2.57 (s, 3H), 2.24 - 2.10 (m, 1H). HRMS (ESI) calcd for C₂₂H₂₁N₄O₅⁺ [M+H]⁺, 421.1506; found, 421.1513.

### Example 10: preparation of 3-(2-methyl-5-(((4-(morpholine-4-carbonyl)cyclohexyl)methyl)amino)-4-oxoquinazolin-3(4H)-yl)piperidine-2,6-dione (SIAIS313067)

Referring to the methods of Schme 1 and example 1, the target compound SIAIS313067 was prepared under appropriate conditions that will be recognized by one skilled in the art. The target compound SIAIS313067 was obtained as a white solid (18 mg, 51%). ¹H NMR (500 MHz, DMSO) *δ* 11.13 (s, 1H), 8.34 (s, 1H), 7.61 (t, *J* = 8.2 Hz, 1H), 6.82 (t, *J* = 13.5 Hz, 1H), 6.71 (d, *J* = 8.6 Hz, 1H), 5.32 (dd, *J* = 11.7, 5.6 Hz, 1H), 3.61 - 3.50 (m, 4H), 3.44 (d, *J* = 32.2 Hz, 4H), 3.11 (d, *J* = 6.6 Hz, 2H), 2.92 - 2.69 (m, 2H), 2.79 (s, 3H), 2.61 (dd, *J* = 12.2, 8.2 Hz, 2H), 2.30 - 2.14 (m, 1H), 1.79 (d, *J* = 11.4 Hz, 2H), 1.69 (d, *J* = 12.1 Hz, 2H), 1.60 (s, 1H), 1.35 (dd, *J =* 24.6, 11.8 Hz, 2H), 1.04 (dt, *J =* 27.5, 9.4 Hz, 2H). ¹³C NMR (126 MHz, DMSO) *δ* 173.87, 173.00, 169.43, 161.88, 150.78, 137.27, 129.57, 109.97, 108.09, 103.26, 66.90, 66.65, 57.07, 48.95, 45.84, 41.92, 36.46, 30.98, 30.00, 28.91, 21.65, 21.35. HRMS (ESI) calcd for C₂₆H₃₄N₅O₅⁺ [M+H]⁺, 496.2554; found, 496.2551.

### Example 11: preparation of N-((3s,5s,7s)-adamantan-1-yl)-4-(((3-(2,6-dioxopiperidin-3-yl)-2-methyl-4-oxo-3,4-dihydroquinazolin-5-yl)amino)methyl)cyclohexanecarboxamide (SIAIS313068)

Referring to the methods of Schme 1 and example 1, the target compound SIAIS313068 was prepared under appropriate conditions that will be recognized by one skilled in the art. The target compound SIAIS313068 was obtained as a white solid (20 mg, 51%). ¹H NMR (500 MHz, DMSO) *δ* 11.14 (s, 1H), 8.32 (s, 1H), 7.61 (t, *J=* 8.1 Hz, 1H), 7.11 (s, 1H), 6.82 (t, *J=* 13.5 Hz, 1H), 6.70 (d, *J* = 8.6 Hz, 1H), 5.32 (dd, *J* = 11.6, 5.6 Hz, 1H), 3.10 (d, *J* = 6.4 Hz, 2H), 2.92 - 2.81 (m, 1H), 2.79 (s, 3H), 2.66 - 2.55 (m, 2H), 2.29 - 2.16 (m, 1H), 2.09 - 2.00 (m, 1H), 1.98 (s, 3H), 1.89 (s, 6H), 1.77 (d, *J* = 12.2 Hz, 2H), 1.68 (d, *J* = 11.8 Hz, 2H), 1.64 - 1.52 (m, 7H), 1.30 (dd, *J* = 25.5, 13.0 Hz, 2H), 0.97 (q, *J* = 12.5 Hz, 2H). ¹³C NMR (126 MHz, DMSO) *δ* 174.95, 172.87, 169.36, 161.78, 150.62, 137.23, 108.08, 106.93, 103.02, 56.96, 50.75, 48.84, 44.95, 41.51, 36.37, 36.32, 30.86, 30.19, 29.24, 21.60, 21.34. HRMS (ESI) calcd for C₃₂H₄₂N₅O₄⁺ [M+H]⁺, 560.3231; found, 560.3230.

### Example 12: preparation of 3-(5-((4-(hydroxymethyl)benzyl)amino)-2-methyl-4-oxoquinazolin-3(4H)-yl)piperidine-2,6-dione (SIAIS313055)

Referring to the methods of Schme 1 and example 1, the target compound SIAIS313055 was prepared under appropriate conditions that will be recognized by one skilled in the art. The target compound SIAIS313055 was obtained as a white solid (150 mg, 18%). ¹H NMR (500 MHz, DMSO) δ 10.99 (s, 1H), 8.72 (s, 1H), 7.44 (t, *J* = 8.1 Hz, 1H), 7.32 - 7.26 (m, 4H), 6.66 (d, *J* = 7.9 Hz, 1H), 6.46 (d, *J* = 8.4 Hz, 1H), 5.20 (dd, *J* = 11.6, 5.6 Hz, 1H), 4.46 (s, 2H), 4.41 (s, 2H), 2.83 (td, *J* = 16.1, 5.1 Hz, 1H), 2.69 - 2.53 (m, 2H), 2.58 (s, 3H), 2.22 - 2.09 (m, 1H). ¹³C NMR (126 MHz, DMSO) δ 173.25, 170.11, 163.13, 149.98, 141.93, 137.53, 136.28, 127.53, 127.25, 106.85, 104.80, 98.34, 63.17, 56.72, 46.47, 31.13, 23.42, 21.39. HRMS (ESI) calcd for C₂₂H₂₃N₄O₄⁺ [M+H]⁺, 407.1714; found, 407.1713.

### Example 13: preparation of 3-(2-methyl-4-oxo-5-((4-(piperidin-1-ylmethyl)benzyl)amino)quinazolin-3(4H)-yl)piperidine-2,6-dione (SIAIS313069)

Referring to the methods of Schme 1 and example 1, the target compound SIAIS313069 was prepared under appropriate conditions that will be recognized by one skilled in the art. The target compound SIAIS313069 was obtained as a white solid (80 mg, 79%). ¹H NMR (500 MHz, MeOD) *δ* 7.65 (t, *J* = 8.2 Hz, 1H), 7.53 (q, *J* = 7.9 Hz, 4H), 6.80 (t, *J* = 8.9 Hz, 2H), 5.43 (dd, *J* = 10.5, 5.0 Hz, 1H), 4.63 (s, 2H), 4.30 (s, 2H), 3.45 (d, *J* = 12.1 Hz, 2H), 3.03 - 2.91 (m, 6H), 2.90 - 2.75 (m, 2H), 2.37 (d, *J* = 6.8 Hz, 1H), 1.95 (d, *J* = 14.6 Hz, 2H), 1.89 - 1.69 (m, 3H), 1.52 (q, *J* = 12.4 Hz, 1H). ¹³C NMR (126 MHz, MeOD) *δ* 172.45, 168.64, 161.59, 160.15, 150.63, 140.25, 138.13, 137.50, 131.52, 128.19, 127.72, 109.75, 103.64, 102.20, 59.94, 57.41, 52.59, 45.82, 29.99, 22.67, 21.30, 21.04, 19.13. HRMS (ESI) calcd for C₂₇H₃₂N₅O₃⁺ [M+H]⁺, 473.2500; found, 473.2507.

### Example 14: preparation of 3-(5-((4-((3,5-dimethylpiperidin-1-yl)methyl)benzyl)amino)-2-methyl-4-oxoquinazolin-3(4H)-yl)piperidine-2,6-dione (SIAIS313075)

Referring to the methods of Schme 1 and example 1, the target compound SIAIS313075 was prepared under appropriate conditions that will be recognized by one skilled in the art. The target compound SIAIS313075 was obtained as a white solid (58 mg, 27%). ¹H NMR (500 MHz, MeOD) *δ* 7.66 (t, *J* = 8.1 Hz, 1H), 7.55 (dd, *J* = 23.2, 7.2 Hz, 4H), 6.81 (t, *J* = 9.1 Hz, 2H), 5.44 (d, *J* = 6.1 Hz, 1H), 4.64 (s, 2H), 4.43 - 4.22 (m, 2H), 3.39 - 3.34 (m, 1H), 3.05 - 2.91 (m, 4H), 2.90 - 2.75 (m, 2H), 2.57 (dd, *J* = 23.8, 11.8 Hz, 2H), 2.38 (d, *J* = 6.7 Hz, 1H), 1.97 (s, 2H), 1.87 (d, *J* = 13.1 Hz, 1H), 1.15 (d, *J* = 7.3 Hz, 1H), 0.98 (d, *J* = 6.3 Hz, 6H), 0.94 - 0.81 (m, 1H). ¹³C NMR (126 MHz, MeOD) *δ* 172.46, 168.62, 161.75, 160.08, 150.65, 140.25, 137.90, 137.54, 131.74, 131.62, 128.14, 127.72, 109.83, 103.49, 102.14, 60.21, 57.55, 57.44, 45.81, 38.86, 30.00, 29.00, 21.05, 19.11, 17.35. LC-MS (ESI) C₂₉H₃₆N₅O₃⁺ [M+H]⁺, found, 502.

### Example 15: preparation of 3-(2-methyl-4-oxo-5-((4-(thiomorpholinomethyl)benzyl)amino)quinazolin-3(4H)-yl)piperidine-2,6-dione (SIAIS313074)

Referring to the methods of Schme 1 and example 1, the target compound SIAIS313074 was prepared under appropriate conditions that will be recognized by one skilled in the art. The target compound SIAIS313074 was obtained as a white solid (20 mg, 30%). ¹H NMR (500 MHz, MeOD) *δ* 7.66 (t, *J* = 8.2 Hz, 1H), 7.55 (dd, *J* = 19.1, 7.9 Hz, 4H), 6.85 - 6.69 (m, 2H), 5.43 (dd, *J* = 10.8, 5.7 Hz, 1H), 4.64 (s, 2H), 4.38 (s, 2H), 3.75 (d, *J* = 12.4 Hz, 2H), 3.25 (t, *J* = 12.2 Hz, 2H), 3.14 (t, *J* = 13.4 Hz, 2H), 2.97 (s, 2H), 2.92 (dd, *J* = 17.9, 12.8 Hz, 2H), 2.82 (dd, *J* = 27.7, 15.9 Hz, 4H), 2.42 - 2.31 (m, 1H). ¹³C NMR (126 MHz, MeOD) *δ* 172.42, 168.60, 161.75, 160.07, 150.65, 140.48, 137.89, 137.56, 131.73, 127.78, 127.54, 109.82, 103.46, 102.13, 60.56, 57.43, 53.50, 45.80, 29.97, 24.28, 21.01, 19.03. HRMS (ESI) calcd for C₂₆H₃₀N₅O₃S⁺ [M+H]⁺, 492.2064; found, 492.2073.

### Example 16: preparation of 3-(5-((4-(azepan-1-ylmethyl)benzyl)amino)-2-methyl-4-oxoquinazolin-3(4H)-yl)piperidine-2,6-dione (SIAIS313087)

Referring to the methods of Schme 1 and example 1, the target compound SIAIS313087 was prepared under appropriate conditions that will be recognized by one skilled in the art. The target compound SIAIS313087 was obtained as a white solid (60 mg, 26%). ¹H NMR (500 MHz, MeOD) *δ* 7.66 (t, *J* = 7.4 Hz, 1H), 7.58 (d, *J* = 6.7 Hz, 2H), 7.52 (d, *J* = 6.7 Hz, 2H), 6.81 (dd, *J* = 13.8, 7.9 Hz, 2H), 5.44 (d, *J* = 5.3 Hz, 1H), 4.63 (s, 2H), 4.37 (s, 2H), 3.50 - 3.40 (m, 2H), 3.25 - 3.13 (m, 2H), 2.98 (s, 3H), 2.95 - 2.75 (m, 3H), 2.38 (s, 1H), 1.95 (s, 2H), 1.86 (d, *J* = 37.0 Hz, 2H), 1.76 (s, 4H). ¹³C NMR (126 MHz, MeOD) *δ* 172.48, 168.63, 161.78, 160.08, 150.65, 140.16, 137.88, 137.58, 131.41, 128.96, 127.81, 109.86, 103.49, 102.16, 60.14, 57.46, 54.31, 45.84, 30.05, 26.11, 22.88, 21.14, 19.24. MS (ESI, m/z): 488 [M+H]⁺.

### Example 17: preparation of 3-(6-((4-(azepan-1-ylmethyl)benzyl)amino)-2-methyl-4-oxoquinazolin-3(4H)-yl)piperidine-2,6-dione (SIAIS355028)

The target compound SIAIS355028 was prepared with reference to the method of Scheme 2.

Step 1: a mixture of 3-(6-bromo-2-methyl-4-oxoquinazolin-3(4H)-yl)piperidine-2,6-dione (0.350, 1mmol), bis(pinacolate)diboron (0.5g, 2mmol), potassium acetate (0.196g, 2mmol) and [1,1 '-bis(diphenylphosphino)ferrocene]dichloropalladium (II) (73.1mg, 10mol %) in 1,4-dioxane was reacted under reflux for 3h, then concentrated and subjected to a column chromatography to obtain boric acid compound (3-(2,6-dioxopiperidin-3-yl)-2-methyl-4-oxo-3,4-dihydroquinazolin-6-yl)boronic acid, which was directly used in the next step.

Step 2: a mixture of the boric acid compound obtained from step 1 (0.22 g, 0.57 mmol), (4-(azepan-1-ylmethyl)phenyl)methanamine (0.248 g, 2 eq), copper acetate (103 mg, 1 eq), triethylamine (158 µl, 2 eq) and 3A molecular sieve in DCM was reacted under reflux in air atmosphere for 12 h, then filtered through Celite, and separated and purified by using preparative HPLC chromatography (eluent (v/v): acetonitrile/(water+0.05% HCl)=10%-100%). The collected fractions were rotary evaporated to remove acetonitrile, and lyophilized to give the final target compound (white solid, 21 mg, 7%). ¹H NMR (500 MHz, DMSO) *δ* 11.01 (d, *J* = 13.5 Hz, 1H), 10.44 (s, 1H), 7.58 (d, *J* = 7.4 Hz, 2H), 7.44 (m, 3H), 7.27 (t, *J* = 16.2 Hz, 1H), 6.91 (s, 1H), 5.27 (d, *J* = 56.8 Hz, 1H), 4.39 (s, 2H), 4.26 (d, *J* = 5.0 Hz, 2H), 3.25 (s, 3H), 3.00 (d, *J* = 7.5 Hz, 2H), 2.84 (t, *J* = 23.5 Hz, 1H), 2.66 - 2.55 (m, 3H), 2.13 (s, 1H), 2.00 (dd, *J* = 17.6, 6.7 Hz, 2H), 1.78 (d, *J* = 20.4 Hz, 4H), 1.68 - 1.49 (m, 4H). HRMS (ESI) calcd for C₂₇H₃₄N₅O₃⁺ [M+H]⁺, 488.2662; found, 488.2664.

### Example 18: preparation of 3-(5-((4-((adamantan-1-ylamino)methyl)-2-fluorobenzyl)amino)-2-methyl-4-oxoquinazolin-3(4H)-yl)piperidine-2,6-dione (SIAIS355049)

Referring to the methods of Schme 1 and example 1, the target compound SIAIS355049 was prepared under appropriate conditions that will be recognized by one skilled in the art. The target compound SIAIS355049 was obtained as a white solid (25mg, 14%). ¹H NMR (500 MHz, DMSO) *δ* 11.07 (s, 1H), 9.17 (s, 2H), 8.68 (s, 1H), 7.59 (d, *J* = 11.0 Hz, 1H), 7.52 (dd, *J* = 21.5, 13.5 Hz, 1H), 7.46 - 7.35 (m, 2H), 6.80 (d, *J* = 7.7 Hz, 1H), 6.57 (d, *J* = 8.1 Hz, 1H), 5.28 (d, *J* = 5.7 Hz, 1H), 4.54 (s, 2H), 4.09 (s, 3H), 2.85 (t, *J* = 14.1 Hz, 1H), 2.69 (s, 2H), 2.60 (d, *J* = 13.4 Hz, 2H), 2.22 (d, *J* = 23.2 Hz, 1H), 2.14 (s, 3H), 1.97 (s, 6H), 1.68 (d, *J* = 11.9 Hz, 3H), 1.60 (d, *J* = 11.7 Hz, 3H). HRMS (ESI) calcd for C₃₂H₃₇FN₅O₃⁺ [M+H]⁺, 558.2875; found, 558.2880.

### Example 19: preparation of 3-(5-(benzylthio)-2-methyl-4-oxoquinazolin-3(4H)-yl)piperidine-2,6-dione (SIAIS313034)

The target compound SIAIS313034 was prepared with reference to the method of Scheme 4.

Step 1: to 2-amino-6-nitrobenzoic acid (10 mmol) was added acetic anhydride (10 ml). The mixture was reacted at 200°C in microwave for 1.5 h, and then concentrated. The resulting concentrate was directly used in the next step.

Step 2: a mixture of the concentrate obtained from step 1 and 3-aminopiperidine-2,6-dione hydrochloride (1.64 g, 10 mmol) in pyridine (15 ml) was reacted at 140°C in microwave for 1.5 hours, and concentrated under reduced pressure. The resulting concentrate was then stirred in 1 M hydrochloric acid (30 ml) for half an hour, and filtered. The resulting solid was washed with ethyl acetate, and then triturated with methanol (20 ml), and filtered to obtain the nitro compound product.

Step 3: a mixture of the nitro compound (0.103 mmol) and palladium hydroxide (catalytic amount) in methanol was stirred in hydrogen gas atmosphere for 48 h, filtered, and sujected to column chromatography to obtain the reduction product.

Step 4: A 500 mL egg-shaped flask containing methanol (120 mL) and water (120 mL) was charged with sodium thiosulfate pentahydrate (0.53 g, 2.16 mmol), benzyl bromide (0.27 g, 2.16 mmol), copper sulfate pentahydrate (catalytic amount) and bipyridine (catalytic amount). The mixture was slowly warmed to 80°C and stirred for 2h. Then the reaction solution was cooled to room temperature, to which the reduction product from step 3 (0.30 mmol) was added, followed by slowly addition dropwise tert-butyl nitrite (0.047 g, 0.46 mmol). After addition, the reaction solution was warmed again to 80°C and stirred for 8 h. After the reaction was complete, the reaction solution was cooled to room temperature, diluted with water (200 mL) and extracted with ethyl acetate (2 × 200 mL). The organic phases were combined, washed with water (2 × 50 mL) and saturated brine (50 mL). Organic phase was dried over anhydrous sodium sulfate, and concentrated under reduced pressure to remove solvent. The resulting crude was subjected to column chromatography (eluent (v/v): petroleum ether/ethyl acetate=1:2) for purification to give the target benzyl thio compound (SIAIS313034) (white solid, 100mg, 23%). ¹H NMR (500 MHz, DMSO) *δ* 11.01 (s, 1H), 7.80 - 7.53 (m, 1H), 7.51 - 7.43 (m, 2H), 7.42 - 7.22 (m, 5H), 5.23 (dd, *J* = 11.3, 5.6 Hz, 1H), 4.19 (q, *J* = 12.2 Hz, 2H), 2.88 - 2.77 (m, 1H), 2.63 (s, 3H), 2.62 - 2.53 (m, 2H), 2.21 - 2.06 (m, 1H). HRMS (ESI) calcd for C₂₁H₂₀N₃O₃S⁺ [M+H]⁺, 394.1220; found, 394.1225.

### Example 20: preparation of 3-(5-((4-(azepan-1-ylmethyl)-2-fluorobenzyl)amino)-2-methyl-4-oxoquinazolin-3(4H)-yl)piperidine-2,6-dione (SIAIS355035)

Referring to the methods of Schme 1 and example 1, the target compound SIAIS355035 was prepared under appropriate conditions that will be recognized by one skilled in the art. The target compound SIAIS355035 was obtained as a white solid (24 mg, 25%). ¹H NMR (500 MHz, DMSO) δ 11.20 (s, 1H), 11.15 (s, 1H), 8.72 (s, 1H), 7.70 (d, *J* = 11.0 Hz, 1H), 7.60 (t, *J* = 8.1 Hz, 1H), 7.45 (d, *J* = 7.7 Hz, 1H), 7.39 (t, *J* = 7.5 Hz, 1H), 6.94 (d, *J* = 7.6 Hz, 1H), 6.66 (d, *J* = 8.4 Hz, 1H), 5.37 (dd, *J* = 11.4, 5.3 Hz, 1H), 4.60 (s, 2H), 4.28 (d, *J* = 4.5 Hz, 2H), 3.25 (s, 2H), 3.00 (dd, *J* = 17.0, 10.5 Hz, 2H), 2.89 (d, *J* = 13.4 Hz, 1H), 2.84 (s, 3H), 2.61 (t, *J* = 16.6 Hz, 2H), 2.25 (d, *J* = 5.4 Hz, 1H), 1.93 - 1.72 (m, 4H), 1.64 (s, 2H), 1.55 (s, 2H). ¹³C NMR (126 MHz, DMSO) δ 172.97, 169.39, 161.64, 159.48, 150.05, 143.37, 137.32, 132.49, 129.89, 127.97, 126.83, 123.93, 118.82, 108.24, 107.73, 103.68, 58.62, 57.13, 53.79, 30.94, 26.62, 22.75, 21.35. HRMS (ESI) calcd for C₂₈H₃₃FN₅O₃⁺ [M+H]⁺, 506.2562; found, 506.2560.

### Example 21: preparation of 3-(5-(((5-(azepan-1-ylmethyl)pyridin-2-yl)methyl)amino)-2-methyl-4-oxoquinazolin-3(4H)-yl)piperidine-2,6-dione (SIAIS355062)

Referring to the methods of Schme 1 and example 1, the target compound SIAIS355062 was prepared under appropriate conditions that will be recognized by one skilled in the art. The target compound SIAIS355062 was obtained as a white solid (5 mg, 4%). ¹H NMR (500 MHz, DMSO) δ 11.67 (s, 1H), 11.24 (s, 1H), 9.10 (d, *J* = 1.2 Hz, 1H), 8.97 (s, 1H), 8.63 (dd, *J* = 8.3, 1.4 Hz, 1H), 7.75 (d, *J* = 8.3 Hz, 1H), 7.67 (t, *J* = 8.2 Hz, 1H), 7.16 (d, *J* = 7.9 Hz, 1H), 6.80 (d, *J* = 8.6 Hz, 1H), 5.51 (dd, *J* = 11.6, 5.6 Hz, 1H), 4.91 (s, 2H), 4.50 (d, *J* = 5.2 Hz, 2H), 3.33 - 3.24 (m, 2H), 3.14 - 3.05 (m, 2H), 3.01 (s, 3H), 3.00 - 2.90 (m, 1H), 2.69 - 2.57 (m, 2H), 2.37 - 2.28 (m, 1H), 1.94 - 1.77 (m, 4H), 1.70 - 1.61 (m, 2H), 1.61 - 1.50 (m, 2H). ¹³C NMR (126 MHz, DMSO) δ 172.98, 169.04, 161.14, 160.57, 156.54, 149.80, 147.57, 145.54, 140.02, 137.92, 127.81, 123.84, 109.53, 106.18, 103.31, 57.34, 55.80, 53.89, 45.50, 30.89, 26.57, 22.81, 21.25, 20.32. HRMS (ESI) calcd for C₂₇H₃₃N₆O₃⁺ [M+H]⁺, 489.2609; found, 489.2607.

### Example 22: preparation of 3-(8-((4-(azepan-1-ylmethyl)benzyl)amino)-2-methyl-4-oxoquinazolin-3(4H)-yl)piperidine-2,6-dione (SIAIS355065)

Referring to the methods of Schme 2 and example 17, the target compound SIAIS355065 was prepared under appropriate conditions that will be recognized by one skilled in the art. The target compound SIAIS355065 was obtained as a white solid (14 mg, 28%). ¹H NMR (500 MHz, DMSO) δ 11.01 (s, 1H), 10.78 (s, 1H), 7.59 (s, 2H), 7.43 (s, 2H), 7.15 (d, *J* = 12.1 Hz, 2H), 6.71 (s, 1H), 5.34 - 5.21 (m, 1H), 4.53 (s, 2H), 4.25 (s, 3H), 3.25 (s, 2H), 2.99 (s, 2H), 2.85 (d, *J* = 14.8 Hz, 1H), 2.69 (t, *J* = 8.3 Hz, 3H), 2.67 - 2.56 (m, 2H), 2.18 (d, *J* = 5.4 Hz, 1H), 1.81 (s, 4H), 1.63 (s, 2H), 1.55 (s, 2H). ¹³C NMR (126 MHz, DMSO) δ 173.16, 170.06, 161.10, 153.12, 143.55, 141.51, 134.76, 131.87, 129.44, 127.72, 120.65, 112.16, 111.63, 59.36, 56.97, 53.68, 46.13, 31.10, 26.61, 23.99, 22.77, 21.42. HRMS (ESI) calcd for C₂₈H₃₄N₅O₃⁺ [M+H]⁺, 488.2656; found, 488.2658.

### Example 23: preparation of 3-(5-(((5-(((adamantan-1-yl)amino)methyl)pyridin-2-yl)methyl)amino)-2-methyl-4-oxoquinazolin-3(4H)-yl)piperidine-2,6-dione (SIAIS355068)

Referring to the methods of Schme 1 and example 1, the target compound SIAIS355068 was prepared under appropriate conditions that will be recognized by one skilled in the art. The target compound SIAIS355068 was obtained as a white solid (19 mg, 29%). ¹H NMR (500 MHz, DMSO) δ 11.16 (s, 1H), 9.56 (s, 2H), 8.94 (s, 1H), 8.39 (d, *J* = 7.0 Hz, 1H), 7.65 (d, *J* = 7.9 Hz, 1H), 7.59 (t, *J* = 8.0 Hz, 1H), 6.97 (d, *J* = 7.6 Hz, 1H), 6.65 (d, *J* = 8.3 Hz, 1H), 5.38 (s, 1H), 4.78 (s, 3H), 4.22 (s, 2H), 2.86 (m, 3H), 2.66 (t, *J* = 18.2 Hz, 2H), 2.26 (s, 1H), 2.15 (s, 3H), 2.00 (s, 6H), 1.65 (dd, *J* = 35.4, 10.3 Hz, 6H). HRMS (ESI) calcd for C₃₁H₃₇N₆O₃⁺ [M+H]⁺, 541.2922; found, 541.2924.

### Biological activity assay

Materials:
Halt proteosome and phosphatase inhibitors (Thermo Fisher)
Cell TITER BLUE detection kits (Promega Corporation)
Cell TITER GLO detection kits (Promega Corporation)
CCK8 (WST) reagent (DOJINDO LABORATORIES, Japan)
RPMI1640 (GIBICO Company)
Fetal bovine serum (FBS) (GIBICO Company)
Penicillin-Streptomycin (GIBICO Company)
SuperSignal West Pico Chemiluminescent Substrate (Thermo Fisher)
SuperSignal West Femto Maximum Sensitivity Substrate (Thermo Fisher)
Cycloheximide (Sigma)

### Antibodies:

Most antibodies were purchased from Cell Signaling Technology, including IKZF1 (#9034S), IKZF3(#15103S), CK1α(#ab108296). GAPDH antibody was purchased from Abcam Company.

### Cell culture

The tested cell lines used were: Multiple myeloma cell line MM.1S, human Burkitt's lymphoma cell line Daudi, human acute promyelocytic leukemia cell line NB4, human mantle cell lymphoma cell line JEKO-1, human B-cell lymphoma cell line SU-DHL-4, human mantle cell lymphoma cell line Mino, human diffuse large B-cell lymphoma cell line TMD8, human B-cell lymphoma cell line DOHH2, PBMCs cell and human diffuse large B-cell lymphoma cell line WSU-DLCL2, which were purchased from ATCC (American type Culture Collection) or Cell Bank/Stem Cell Bank, Chinese Academy of Sciences. The medium was RPMI1640 supplemented with 10% FBS (fetal calf serum) and 1% Penicillin-Streptomycin. The cells used were identified as correct cells by STR cells, and were negative for mycoplasma through routine inspections.

### Determination of half inhibitory concentration (IC₅₀) of the compounds

IC₅₀ values of the compounds of the present disclosure (including the compounds in Table 1 and compounds of examples 1-23) were measured using Cell Titer Blue, Cell Titer GLO, or WST reagent from Promega Company. Assay details are as follows: Cells were seeded in 100 µL RPMI1640 medium containing serum at a density of 15,000 cells/well. After 24h, the inoculated cells were treated with diluted commercial inhibitor and the compounds of the present disclosure to be tested after serial dilution. After the cells were treated with the compounds of the present disclosure to be tested for 72h, cell viability was determined after adding the cell viability detection kit listed above according to the reagent operating instructions. The negative control was DMSO, and the positive control was a commercial inhibitor, both of which were used to treat the cells through the same method as that of the compounds of the present disclosure. The growth inhibition of the compounds of the present disclosure on cells was plotted by Prism Graphpad software, and the IC₅₀ values of the compounds of the present disclosure were calculated therefrom. Results were shown in Table 2.

### Western Blotting assay

Tumor cells were plated in a 24-well plate at a cell seeding density of 3×10⁵ cells/mL, with 1mL culture media per well. After 24h, the cells were treated with different concentrations of the compounds of the present disclosure. After 16 hours, the cells were collected, and washed with PBS. The supernatant was discarded, and the cells were placed on ice, and treated with RIPA protein lysate containing Halt protease and phosphatase inhibitor. The lysate was centrifuged at 10000RPM at 4°C for 10 minutes, and the supernatant was collected. An equal amount of proteins were loaded in 4X SDS sample solution, denatured at 95°C for 5 minutes, and then freezed to -20°C or directly subjected to protein electrophoresis (4-15% gradient gel, Bio-rad). Electrophoresis apparatus and related components were purchased from Bio-rad company, and electrophoresis set at a constant pressure of 120V for 1h. Then transferring membrane was conducted by using PVDF (polyvinylidene fluoride) at 400mA for 1h on ice. Afterwards, the membranes were block for 30 minutes by using the TarKara Blocking Buffer at room temperature. Western blotting was conducted according to the antibody product manual of Cell Signaling Technology Company.

DC₅₀ value (the drug concentration required for degrading proteins by 50%, abbreviated as DC₅₀) reads method: comparing the gray values of the Western blotting bands for the drug treatment with the gray values of the Western blotting band for the DMSO control, and reading the drug concentration range corresponding to the gray value of the Western blotting bands for the drug treatment which is equal to half of the gray value of the Western blotting band for the DMSO control.

DC₅₀ value could also be calculated as follows: using software ImageJ to quantify the gray values of the Western blotting bands for the drug treatment, fitting the relationship curve between drug concentrations and gray values, and from the fitted curve, calculating the drug concentration corresponding to half of the gray value of the Western blotting band for the DMSO control.

### Evaluation of TNF-α Inhibition Effect

PBMC cells were cultured at 37°C in 5% CO₂ atmosphere, and then seeded in 96-well plates at 1×10⁷ cells/well. Compounds (including compounds in Table 1 and Examples 1-23 compounds) were dissolved in DMSO and diluted to corresponding concentrations so that the final concentration of DMSO added to the cell culture did not exceed 0.5%. Cells were incubated in medium with or without compounds for 1 h, then stimulated with lipopolysaccharide (LPS; 1 ng/ml), and continually cultured for 18-20 h. Then, the supernatant was collected, diluted with serum-free medium, and tested for TNF-α level by ELISA kit. IC₅₀ was then calculated by Graphpad Prism 7.0.

### Experimental result

The Compounds of the present invention in our research were developed based on immunomodulatory drugs. We studied the cell inhibition and protein degradation activities of the compounds of the present invention (including the compounds in Table 1 and Examples 1-23 compounds) designed on the basis of immunomodulatory drugs including Avadomide (CC-122). It was found that many compounds of the present invention were more active against multiple myeloma cancer cells MM.1S and B lymphoma cells Daudi than Avadomide. The compounds of the present invention could not only inhibit cancer cell proliferation, but also promote the degradation of IKZF, and inhibit the expression of TNF-α, and thus can be developed as a therapeutic drug for immune-related tumor patients. Detailed experiment results were shown below.

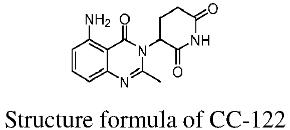

Proliferation inhibition of the compounds of the present invention based on Avadomide (CC-122) in tumor cells

We tested the activities of the compounds of the present invention (including the compounds in Table 1 and Examples 1-23 compounds) in multiple myeloma cancer cells MM.1S cell and B lymphoma cells Daudi through a dose-dependent manner. The cells were treated with the compounds of the present invention at 10 different successively decreasing concentrations (starting at the highest concentration of 10µM; 5-fold serial dilutions) for 72h, and then the cell viability determination was performed in accordance with CCK-8 reagent operating instructions. Results were shown in Table 2. It showed that the compounds of the present invention inhibited MM.1S and Daudi cell lines to different degrees, and some compounds of the present invention exhibited significantly stronger inhibitory effects as compared with CC-122.

**Table 2. IC₅₀ values (half inhibitory concentration) of the compounds of the present invention in tumor cells**

| Compounds | MM.1S/ IC₅₀(nM) | Daudi/ IC₅₀(nM)▪ |
|---|---|---|
| CC-122 | 21.76±4.3 | 158.1±9.48 |
| SIAIS264067 | 8.59±1.32 | 54.64±7.35 |
| SIAIS264103 | 87.28±5.18 | 826.35±37.26 |
| SIAIS264123 | 57.49±11.47 | 322.05±22.13 |
| SIAIS264156 | 9.64±0.83 | 44.76±19.62 |
| SIAIS264177 | 34.72±5.29 | 221.6±40.87 |
| SIAIS313068 | 83.7±2.79 | 492.15±33.16 |
| SIAIS313055 | 36.38±6.74 | 165.8±14.71 |
| SIAIS313069 | 21.47±5.84 | 129.2±18.77 |
| SIAIS313075 | 16.4±2.82 | 131.35±27.65 |
| SIAIS313074 | 10.55±1.95 | 58.73±22.37 |
| SIAIS313087 | 4.82±2.36 | 43.49±17.85 |
| SIAIS264172 | 2.52±0.68 | - |
| SIAIS355035 | 2.62±0.49 | 12.89±3.87 |
| SIAIS355049 | 5.61±2.41 | 82.89±25.95 |
| SIAIS355062 | 22.12±11.97 | 105.28±19.95 |
| SIAIS355068 | 37.74±12.06 | 138.67±21.97 |
| SIAIS355049 | 5.61±2.41 | 82.89±25.95 |

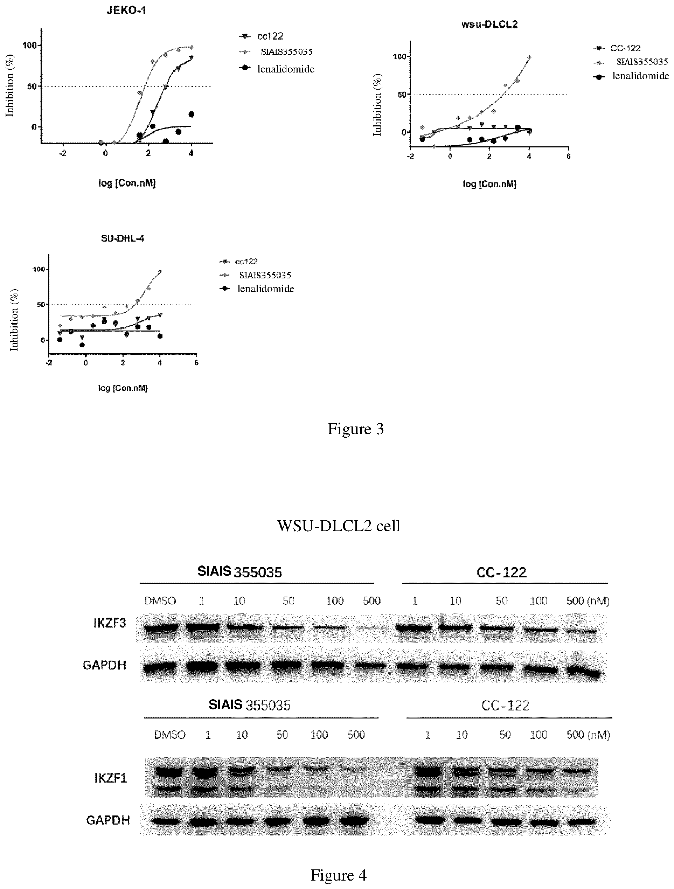

We further tested the activities of the compound SIAIS355035 of the present invention in human mantle cell lymphoma cell line Mino, human diffuse large B-cell lymphoma cell line TMD8, human B-cell lymphoma cell line DOHH2, human diffuse large B-cell lymphoma cell line WSU-DLCL2, human mantle cell lymphoma cell line JEKO-1, and human B-cell lymphoma cell line SU-DHL-4 through a dose-dependent manner. The cells were treated with the compound of the present invention to be tested at 10 different successively decreasing concentrations (starting at the highest concentration of 10µM; 5-fold serial dilutions) for 72h, and then the cell viability determination was performed in accordance with CCK-8 reagent operating instructions. Results were shown in Table 3 and Figure 3. It showed that the compound SIAIS355035 of the present invention can inhibit Mino, TMD8, DOHH2, WSU-DLCL2, JEKO-1 and SU-DHL-4 cell lines, and exhibite significantly stronger inhibitory effects as compared with CC-122 and lenalidomide.

**Table 4. IC₅₀ (half inhibitory concentration) of the compound SIAIS355035 of the present invention in tumor cells as compared with CC-122**

| Cell lines | IC₅₀(nM) | |
|---|---|---|
| | SIAIS355035 | CC-122 |
| MINO | 5.05 | 54.97 |
| WSU-DLCL2 | 163.509 | >1000 |
| DOHH2 | 306.671 | > 1000 |
| TMD8 | 103.099 | 492.16 |

### 1.2 IKZF protein degradation of the compounds of the present invention (half inhibitory concentration, DC₅₀)

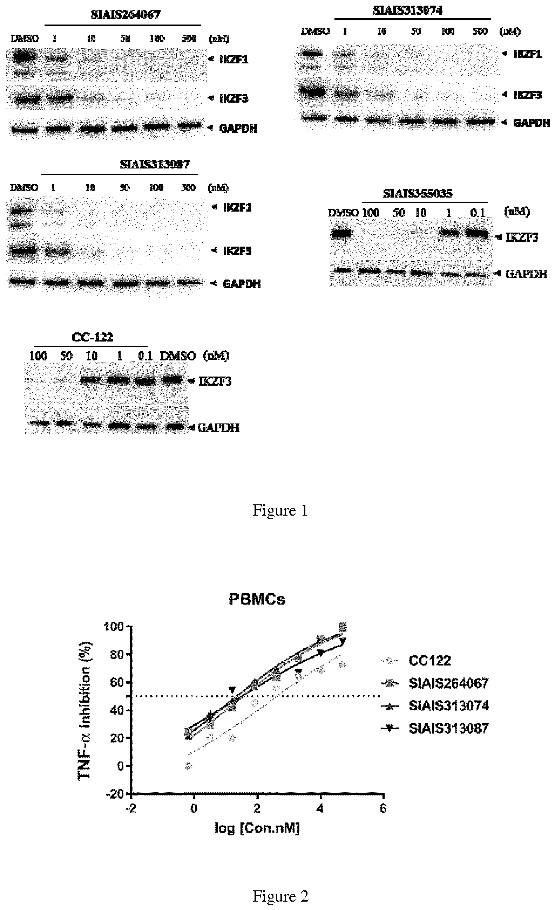

We selected and tested some compounds of the present invention (including the compounds in Table 1 and Examples 1-23 compounds) in MM.1S cell through western blotting assay in a dose-dependent manner. The studies have shown that these compounds can significantly degrade IKZF1/3 at effective concentrations as low to single-digit nanomolar levels or even picomolar levels. As shown in Figure 1, the compounds of the present invention (including Examples 1-23 compounds) exhibited significantly stronger IKZF protein degradation effects as compared with CC-122.

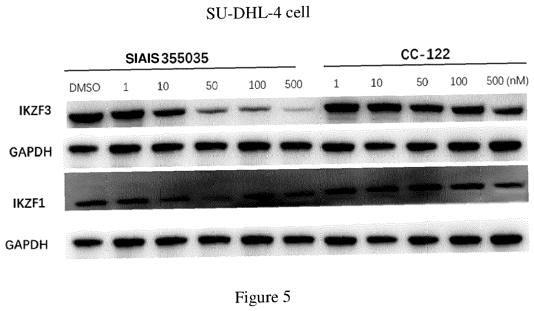

We further tested the activities of the compound SIAIS355035 of the present invention in human diffuse large B-cell lymphoma cell line WSU-DLCL2 and human B-cell lymphoma cell line SU-DHL-4 through western blotting assay in a dose-dependent manner. It showed that the compound SIAIS355035 can significantly degrade IKZF1/3 at effective concentration significantly lower than that of CC-122, and exhibite significantly stronger IKZF protein degradation effects as compared with CC-122 (Figures 4-5).

### 1.3 TNF-α Inhibition Effect of the compounds of the present invention

Down-regulation of TNF-α expression is an important way of anti-tumor effect of immunomodulators. The test results were shown in Figure 2. After treatment with CC-122 and the compounds of the present invention, the level of TNF-α was inhibited in a dose-dependent manner, and the compounds of the present invention SIAIS264067, SIAIS313074 and SIAIS313087 showed better inhibitory activities than CC-122.

The basic principles, main features and advantages of the present invention have been shown and described above. Those skilled in the art should understand that the present invention is not limited by the above-mentioned embodiments, and without departing from the spirit and scope of the present invention, the present invention can also undergo various changes and improvements, and these changes and improvements all fall within the scope of the present invention. The claimed scope of the present invention is defined by the appended claims and their equivalents.

## Claims

1. A compound of Formula (I) 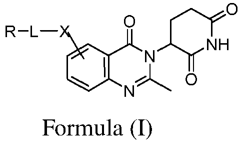 or a salt, an enantiomer, a stereoisomer, a solvate, or a polymorph thereof, wherein
X represents N(Rₐ₁), O or S, where Rₐ₁ represents H or C₁₋₃ alkyl;
L represents linear or branched C₁₋₃ alkylene optionally substituted by a substituent selected from the group consisting of halogen, hydroxyl, amino or any combination thereof; and
R represents:
NRₐ₇Rₐ₈, wherein Rₐ₇ is H or C₁₋₃ alkyl, and Rₐ₈ is phenyl optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; or
benzo[d][1,3]dioxolyl, tetrahydropyranyl, pyridyl or phenyl, said benzo[d][1,3]dioxolyl, tetrahydropyranyl, pyridyl and phenyl are each independently optionally substituted by a substituent selected from the group consisting of halogen, hydroxyl, mercapto, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)- or any combination thereof;
or
L represents Formula -Lₐ₂₋Lₐ₁₋-^{∗}, where Lₐ₁ represents linear or branched C₁₋₄₀ alkylene optionally substituted by a substituent selected from the group consisting of halogen, hydroxyl, amino or any combination thereof, Lₐ₂ represents optionally substituted C₃₋₁₅ cycloalkylene or optionally substituted C₅₋₁₅ arylene, wherein said C₃₋₁₅ cycloalkylene is optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)- or any combination thereof, and said C₅₋₁₅ arylene is optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof, and symbol * indicates the point of attachment to X; and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, or OS(O)₂Rₐ₂₈,
wherein Rₐ₁₆ and Rₐ₁₇ each independently represent H, optionally substituted cycloalkyl, or optionally substituted heterocyclyl;
Rₐ₉, Rₐ₁₁, Rₐ₂₁, and Rₐ₂₃ each independently represent H or C₁₋₃ alkyl; and
Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted cycloalkyl or optionally substituted heterocyclyl;
or
L represents linear or branched alkylene interrupted one or more times by one or more groups selected from the group consisting of: optionally substituted arylene, optionally substituted heteroarylene or any combination thereof, wherein said linear or branched alkylene is optionally substituted by a substituent selected from the group consisting of halogen, hydroxyl, amino or any combination thereof;
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted cycloalkyl, or optionally substituted heterocyclyl;
wherein Rₐ₇, Rₐ₈, Rₐ₉, Rₐ₁₀, Rₐ₁₆, Rₐ₁₇, Rₐ₂₁ and Rₐ₂₂ each independently represent H, optionally substituted linear or branched alkyl, optionally substituted cycloalkyl or optionally substituted heterocyclyl;
Rₐ₁₁ and Rₐ₂₃ each independently represent H or C₁₋₃ alkyl; and
Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched alkyl, optionally substituted cycloalkyl or optionally substituted heterocyclyl;
or
X represents alkynylene or alkenylene; and
L represents:
linear or branched alkylene interrupted one or more times by one or more groups selected from the group consisting of: optionally substituted arylene, optionally substituted heteroarylene or any combination thereof, wherein said linear or branched alkylene is optionally substituted by a substituent selected from the group consisting of halogen, hydroxyl, amino or any combination thereof; or
Formula -L_{b2}-L_{b1}-#, where L_{b1} represents optionally substituted C₅₋₁₅ arylene or optionally substituted C₅₋₁₅ heteroarylene, wherein said C₅₋₁₅ arylene and C₅₋₁₅ heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; L_{b2} represents optionally substituted linear or branched C₁₋₄₀ alkylene, and symbol # indicates the point of attachment to X; and
R represents NR_{b7}R_{b8}, C(O)NR_{b9}R_{b10}, N(R_{b11})C(O)R_{b12}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, optionally substituted cycloalkyl or optionally substituted heterocyclyl,
wherein R_{b7}, R_{b8}, R_{b9}, R_{b10}, R_{b16}, R_{b17}, R_{b21} and R_{b22} each independently represent H, optionally substituted linear or branched alkyl, optionally substituted cycloalkyl or optionally substituted heterocyclyl;
R_{b11} and R_{b13} each independently represent H or C₁₋₃ alkyl; and
R_{b12}, R_{b18}, R_{b19}, R_{b20}, R_{b24}, R_{b25}, R_{b26}, R_{b27}, and R_{b28} each independently represent optionally substituted linear or branched alkyl, optionally substituted cycloalkyl or optionally substituted heterocyclyl;
or
X represents a bond; and
L represents linear or branched alkylene interrupted one or more times by one or more groups selected from the group consisting of: optionally substituted arylene, optionally substituted heteroarylene or any combination thereof, wherein said linear or branched alkylene is optionally substituted by a substituent selected from the group consisting of halogen, hydroxyl, amino or any combination thereof; and
R represents NR_{c4}R_{c5}, C(O)NR_{c6}R_{c7}, N(R_{c8})C(O)R_{c9}, OR_{c13}, SR_{c14}, C(O)R_{c15}, OC(O)R_{c16}, C(O)OR_{c17}, S(O)₂NR_{c18}R_{c19}, N(R_{C20})S(O)₂R_{c21}, S(O)R_{c22}, S(O)₂R_{c23}, S(O)₂OR_{c24}, OS(O)₂R_{c25}, optionally substituted C₅₋₂₀ cycloalkyl, or optionally substituted C₅₋₂₀ heterocyclyl,
wherein R_{c8} and R_{c20} each independently represent H or C₁₋₃ alkyl;
R_{c4}, R_{c6}, and R_{c18} each independently represent H, optionally substituted linear or branched C₁₋₁₀ alkyl, optionally substituted cycloalkyl, optionally substituted aryl, optionally substituted heterocyclyl or optionally substituted heteroaryl; and
R_{c5}, R_{c7}, R_{c9}, R_{c13}, R_{c14}, R_{c15}, R_{c16}, R_{c17}, R_{c19}, R_{c21}, R_{c22}, R_{c23}, R_{c24}, and R_{c25} each independently represent optionally substituted C₅₋₂₀ cycloalkyl or optionally substituted C₅₋₂₀ heterocyclyl.

2. The compound of Formula (I) or a salt, an enantiomer, a stereoisomer, a solvate, or a polymorph thereof as claimed in claim 1, wherein the following compound is excluded:

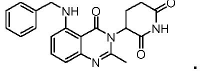

3. The compound of Formula (I) or a salt, an enantiomer, a stereoisomer, a solvate, or a polymorph thereof as claimed in claim 1 or 2, which is also a compound of Formula (Ia): 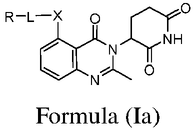 wherein X, R and L are as defined in claim 1.

4. The compound of Formula (I) or a salt, an enantiomer, a stereoisomer, a solvate, or a polymorph thereof as claimed in claim 1 or 2, which is also a compound of Formula (Ib): 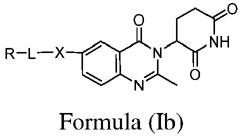 wherein X, R and L are as defined in claim 1.

5. The compound of Formula (I) or a salt, an enantiomer, a stereoisomer, a solvate, or a polymorph thereof as claimed in claim 1 or 2, which is also a compound of Formula (Ic): 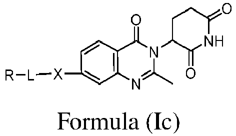 wherein X, R and L are as defined in claim 1.

6. The compound of Formula (I) or a salt, an enantiomer, a stereoisomer, a solvate, or a polymorph thereof as claimed in claim 1 or 2, which is also a compound of Formula (Id): 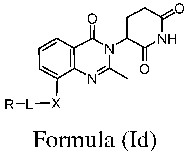 wherein X, R and L are as defined in claim 1.

7. The compound of Formula (I) or a salt, an enantiomer, a stereoisomer, a solvate, or a polymorph thereof as claimed in claim 1 or 2, wherein
X represents N(Rₐ₁), O or S, where Rₐ₁ represents H or C₁₋₃ alkyl;
L represents linear or branched C₁₋₃ alkylene optionally substituted by a substituent selected from the group consisting of halogen, hydroxyl, amino or any combination thereof; and
R represents:
NRₐ₇Rₐ₈, wherein Rₐ₇ is H or C₁₋₃ alkyl, and Rₐ₈ is phenyl optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; or
benzo[d][1,3]dioxolyl, tetrahydropyranyl, pyridyl or phenyl, said benzo[d][1,3]dioxolyl, tetrahydropyranyl, pyridyl and phenyl are each independently optionally substituted by a substituent selected from the group consisting of halogen, hydroxyl, mercapto, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)- or any combination thereof;
or
L represents Formula -Lₐ₂₋Lₐ₁₋-^{∗}, where Lₐ₁ represents linear or branched C₁₋₄₀ alkylene optionally substituted by a substituent selected from the group consisting of halogen, hydroxyl, amino or any combination thereof, Lₐ₂ represents optionally substituted C₃₋₁₅ cycloalkylene or optionally substituted C₅₋₁₅ arylene, wherein said C₃₋₁₅ cycloalkylene is optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)- or any combination thereof, and said C₅₋₁₅ arylene is optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof, and symbol * indicates the point of attachment to X; and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, or OS(O)₂Rₐ₂₈,
wherein Rₐ₁₆ and Rₐ₁₇ each independently represent H, optionally substituted C₃₋₂₀ cycloalkyl, or optionally substituted C₃₋₂₀ heterocyclyl;
Rₐ₉, Rₐ₁₁, Rₐ₂₁, and Rₐ₂₃ each independently represent H or C₁₋₃ alkyl; and
Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl;
or
L represents linear or branched C₂₋₄₀ alkylene interrupted one or more times by one or more groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof;
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted cycloalkyl, or optionally substituted heterocyclyl;
wherein Rₐ₇, Rₐ₈, Rₐ₉, Rₐ₁₀, Rₐ₁₆, Rₐ₁₇, Rₐ₂₁ and Rₐ₂₂ each independently represent H, optionally substituted linear or branched C₁₋₁₀ alkyl, optionally substituted C₃₋₂₀ cycloalkyl or optionally substituted C₃₋₂₀ heterocyclyl;
Rₐ₁₁ and Rₐ₂₃ each independently represent H or C₁₋₃ alkyl; and
Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
or
X represents C₂₋₆ alkynylene or C₂₋₆ alkenylene; and
L represents:
linear or branched C₂₋₄₀ alkylene interrupted one or more times by one or more groups selected from the group consisting of: optionally substituted C₅₋₂₀ arylene, optionally substituted C₅₋₂₀ heteroarylene or any combination thereof, wherein said linear or branched C₂₋₄₀ alkylene is optionally substituted by a substituent selected from the group consisting of halogen, hydroxyl, amino or any combination thereof; or
Formula -L_{b2}-L_{b1}-#, where L_{b1} represents optionally substituted C₅₋₁₅ arylene or optionally substituted C₅₋₁₅ heteroarylene, wherein said C₅₋₁₅ arylene and C₅₋₁₅ heteroarylene are each independently optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; L_{b2} represents optionally substituted linear or branched C₁₋₄₀ alkylene, and symbol # indicates the point of attachment to X; and
R represents NR_{b7}R_{b8}, C(O)NR_{b9}R_{b10}, N(R_{b11})C(O)R_{b12}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, optionally substituted C₃₋₂₀ cycloalkyl or optionally substituted C₃₋₂₀ heterocyclyl,
wherein R_{b7}, R_{b8}, R_{b9}, R_{b10}, R_{b16}, R_{b17}, R_{b21} and R_{b22} each independently represent H, optionally substituted linear or branched C₁₋₁₀ alkyl, optionally substituted C₃₋₂₀ cycloalkyl or optionally substituted C₃₋₂₀ heterocyclyl;
R_{b11} and R_{b13} each independently represent H or C₁₋₃ alkyl; and
R_{b12}, R_{b18}, R_{b19}, R_{b20}, R_{b24}, R_{b25}, R_{b26}, R_{b27}, and R_{b28} each independently represent optionally substituted linear or branched C₁₋₁₀ alkyl, optionally substituted C₃₋₂₀ cycloalkyl or optionally substituted C₃₋₂₀ heterocyclyl;
or
X represents a bond; and
L represents linear or branched C₂₋₄₀ alkylene interrupted one or more times by one or more groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof; and
R represents NR_{c4}R_{c5}, C(O)NR_{c6}R_{c7}, N(R_{c8})C(O)R_{c9}, OR_{c13}, SR_{c14}, C(O)R_{c15}, OC(O)R_{c16}, C(O)OR_{c17}, S(O)₂NR_{c18}R_{c19}, N(R_{c20})S(O)₂R_{c21}, S(O)R_{c22}, S(O)₂R_{c23}, S(O)₂OR_{c24}, OS(O)₂R_{c25}, optionally substituted C₅₋₂₀ cycloalkyl, or optionally substituted C₅₋₂₀ heterocyclyl,
wherein R_{c8} and R_{c20} each independently represent H or C₁₋₃ alkyl;
R_{c4}, R_{c6}, and R_{c18} each independently represent H, optionally substituted linear or branched C₁₋₁₀ alkyl, optionally substituted C₃₋₂₀ cycloalkyl, optionally substituted C₅₋₂₀ aryl, optionally substituted C₃₋₂₀ heterocyclyl or optionally substituted C₅₋₂₀ heteroaryl; and
R_{c5}, R_{c7}, R_{c9}, R_{c13}, R_{c14}, R_{c15}, R_{c16}, R_{c17}, R_{c19}, R_{c21}, R_{c22}, R_{c23}, R_{c24}, and R_{c25} each independently represent optionally substituted C₅₋₂₀ cycloalkyl or optionally substituted C₅₋₂₀ heterocyclyl.

8. The compound of Formula (I) or a salt, an enantiomer, a stereoisomer, a solvate, or a polymorph thereof as claimed in claim 1 or 2, wherein
X represents N(Rₐ₁), O or S, where Rₐ₁ represents H or C₁₋₃ alkyl; and
L represents linear or branched C₁₋₃ alkylene optionally substituted by a substituent selected from the group consisting of halogen, hydroxyl, amino or any combination thereof; and
R represents:
NRₐ₇Rₐ₈, wherein Rₐ₇ is H or C₁₋₃ alkyl, and Rₐ₈ is phenyl optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; or
benzo[d][1,3]dioxolyl, tetrahydropyranyl, pyridyl or phenyl, said benzo[d][1,3]dioxolyl, tetrahydropyranyl, pyridyl and phenyl are each independently optionally substituted by a substituent selected from the group consisting of halogen, hydroxyl, mercapto, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)- or any combination thereof.

9. The compound of Formula (I) or a salt, an enantiomer, a stereoisomer, a solvate, or a polymorph thereof as claimed in claim 1 or 2, wherein
X represents N(Rₐ₁), O or S, where Rₐ₁ represents H or C₁₋₃ alkyl; and
L represents Formula -Lₐ₂₋Lₐ₁₋-^{∗}, where Lₐ₁ represents linear or branched C₁₋₄₀ alkylene optionally substituted by a substituent selected from the group consisting of halogen, hydroxyl, amino or any combination thereof, Lₐ₂ represents optionally substituted C₃₋₁₅ cycloalkylene or optionally substituted C₅₋₁₅ arylene, wherein said C₃₋₁₅ cycloalkylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)- or any combination thereof, and said C₅₋₁₅ arylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof, and symbol * indicates the point of attachment to X; and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, or OS(O)₂Rₐ₂₈,
wherein Rₐ₁₆ and Rₐ₁₇ each independently represent H, optionally substituted C₃₋₂₀ cycloalkyl, or optionally substituted C₃₋₂₀ heterocyclyl;
Rₐ₉, Rₐ₁₁, Rₐ₂₁, and Rₐ₂₃ each independently represent H or C₁₋₃ alkyl; and
Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl.

10. The compound of Formula (I) or a salt, an enantiomer, a stereoisomer, a solvate, or a polymorph thereof as claimed in claim 1 or 2, wherein
X represents N(Rₐ₁), O or S, where Rₐ₁ represents H or C₁₋₃ alkyl; and
L represents linear or branched C₂₋₄₀ alkylene interrupted one or more times by one or more groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and each C₅₋₁₅ arylene and each C₅₋₁₅ heteroarylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)- or any combination thereof; and
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted C₃₋₂₀ cycloalkyl, or optionally substituted C₃₋₂₀ heterocyclyl;
wherein Rₐ₇, Rₐ₈, Rₐ₉, Rₐ₁₀, Rₐ₁₆, Rₐ₁₇, Rₐ₂₁ and Rₐ₂₂ are the same or different and each independently represent H, optionally substituted linear or branched C₁₋₁₀ alkyl, optionally substituted C₃₋₂₀ cycloalkyl or optionally substituted C₃₋₂₀ heterocyclyl;
Rₐ₁₁ and Rₐ₂₃ each independently represent H or C₁₋₃ alkyl; and
Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
wherein said linear or branched C₁₋₁₀ alkyl is optionally substituted by one or more substituents selected from the group consisting of halogen, hydroxyl, cyano, amino, C₁₋₃ alkoxy, C₁₋₃ alkylamino, C₁₋₃ alkyl-NHC(O)- or any combination thereof;
each C₃₋₂₀ cycloalkyl and each C₃₋₂₀ heterocyclyl is independently optionally substituted by one or more substituents selected from the group consisting of halogen, hydroxyl, mercapto, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

11. The compound of Formula (I) or a salt, an enantiomer, a stereoisomer, a solvate, or a polymorph thereof as claimed in claim 1 or 2, wherein
X represents C₂₋₆ alkynylene or C₂₋₆ alkenylene; and
L represents:
linear or branched C₂₋₄₀ alkylene interrupted one or more times by one or more groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and wherein said C₅₋₁₅ arylene and C₅₋₁₅ heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)- or any combination thereof; or
Formula -L_{b2}-L_{b1}-#, where L_{b1} represents optionally substituted C₅₋₁₅ arylene or optionally substituted C₅₋₁₅ heteroarylene, wherein said C₅₋₁₅ arylene and C₅₋₁₅ heteroarylene are each independently optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; L_{b2} represents linear or branched C₁₋₄₀ alkylene optionally substituted by a substituent selected from the group consisting of halogen, hydroxyl, amino or any combination thereof, and symbol # indicates the point of attachment to X; and
R represents NR_{b7}R_{b8}, C(O)NR_{b9}R_{b10}, N(R_{b11})C(O)R_{b12}, OR_{b16}, SR_{b17}, C(O)R_{b18}, OC(O)R_{b19}, C(O)OR_{b20}, S(O)₂NR_{b21}R_{b22}, N(R_{b23})S(O)₂R_{b24}, S(O)R_{b25}, S(O)₂R_{b26}, S(O)₂OR_{b27}, OS(O)₂R_{b28}, optionally substituted C₃₋₂₀ cycloalkyl or optionally substituted C₃₋₂₀ heterocyclyl,
wherein R_{b7}, R_{b8}, R_{b9}, R_{b10}, Rₐ₂₁ and R_{b22} are the same or different and each independently represent H, optionally substituted linear or branched C₁₋₁₀ alkyl, optionally substituted C₃₋₂₀ cycloalkyl or optionally substituted C₃₋₂₀ heterocyclyl;
R_{b16} and R_{b17} each independently represent H, optionally substituted linear or branched C₁₋₁₀ alkyl, optionally substituted C₃₋₂₀ cycloalkyl or optionally substituted C₃₋₂₀ heterocyclyl;
R_{b11} and R_{b23} each independently represent H or C₁₋₃ alkyl; and
R_{b12}, R_{b18}, R_{b19}, R_{b20}, R_{b24}, R_{b25}, R_{b26}, R_{b27}, and R_{b28} each independently represent optionally substituted linear or branched C₁₋₁₀ alkyl, optionally substituted C₃₋₂₀ cycloalkyl or optionally substituted C₃₋₂₀ heterocyclyl; and
wherein said linear or branched C₁₋₁₀ alkyl is optionally substituted by one or more substituents selected from the group consisting of halogen, hydroxyl, cyano, amino, C₁₋₃ alkoxy, C₁₋₃ alkylamino, C₁₋₃ alkyl-NHC(O)- or any combination thereof;
each C₃₋₂₀ cycloalkyl and each C₃₋₂₀ heterocyclyl is each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

12. The compound of Formula (I) or a salt, an enantiomer, a stereoisomer, a solvate, or a polymorph thereof as claimed in claim 1 or 2, wherein
X represents a bond; and
L represents linear or branched C₂₋₄₀ alkylene interrupted one or more times by one or more groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and said C₅₋₁₅ arylene and C₅₋₁₅ heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)- or any combination thereof; and
R represents NR_{c4}R_{c5}, C(O)NR_{c6}R_{c7}, N(R_{c8})C(O)R_{c9}, OR_{c13}, SR_{c14}, C(O)R_{c15}, OC(O)R_{c16}, C(O)OR_{c17}, S(O)₂NR_{c18}R_{c19}, N(R_{c20})S(O)₂R_{c21}, S(O)R_{c22}, S(O)₂R_{c23}, S(O)₂OR_{c24}, OS(O)₂R_{c25}, optionally substituted C₅₋₂₀ cycloalkyl, or optionally substituted C₅₋₂₀ heterocyclyl,
wherein R_{c8} and R_{c20} each independently represent H or C₁₋₃ alkyl;
R_{c4}, R_{c6}, and R_{c18} each independently represent H, optionally substituted linear or branched C₁₋₁₀ alkyl, optionally substituted C₃₋₂₀ cycloalkyl, optionally substituted C₅₋₂₀ aryl, optionally substituted C₃₋₂₀ heterocyclyl or optionally substituted C₅₋₂₀ heteroaryl; and
R_{c5}, R_{c7}, R_{c9}, R_{c13}, R_{c14}, R_{c15}, R_{c16}, R_{c17}, R_{c19}, R_{c21}, R_{c22}, R_{c23}, R_{c24}, and R_{c25} each independently represent optionally substituted C₅₋₂₀ cycloalkyl or optionally substituted C₅₋₂₀ heterocyclyl;
wherein said linear or branched C₁₋₁₀ alkyl is optionally substituted by one or more substituents selected from the group consisting of halogen, hydroxyl, cyano, amino, C₁₋₃ alkoxy, C₁₋₃ alkylamino, C₁₋₃ alkyl-NHC(O)- or any combination thereof;
each C₃₋₂₀ cycloalkyl and each C₃₋₂₀ heterocyclyl is independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof; and
each C₅₋₂₀ aryl and each C₅₋₂₀ heteroaryl is independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)-, optionally substituted C₆₋₂₀ aryl or any combination thereof, wherein said C₆₋₂₀ aryl is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, or any combination thereof.

13. The compound of Formula (I) or a salt, an enantiomer, a stereoisomer, a solvate, or a polymorph thereof as claimed in claim 1 or 2, wherein
X represents N(Rₐ₁), O or S, where Rₐ₁ represents H or C₁₋₃ alkyl;
L represents the following groups optionally substituted by a substituent selected from the group consisting of halogen, hydroxyl, amino or any combination thereof:
-CH₂-; -(CH₂)₂-; -CH₂-CH(CH₃)-; or -(CH₂)₃- ;
R represents:
NRₐ₇Rₐ₈, wherein Rₐ₇ is H or C₁₋₃ alkyl, and Rₐ₈ is phenyl optionally substituted by a substituent selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)- or any combination thereof; or
benzo[d][1,3]dioxolyl, tetrahydropyranyl, pyridyl or phenyl, said benzo[d][1,3]dioxolyl, tetrahydropyranyl, pyridyl and phenyl are each independently optionally substituted by a substituent selected from the group consisting of halogen, hydroxyl, mercapto, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)- or any combination thereof.

14. The compound of Formula (I) or a salt, an enantiomer, a stereoisomer, a solvate, or a polymorph thereof as claimed in claim 1 or 2, wherein
X represents N(Rₐ₁), O or S, where Rₐ₁ represents H or C₁₋₃ alkyl; and
L represents the group represented by the following formula:
-(CH₂)ₙ₁-C₅₋₁₅arylene-(CH₂)ₙ₂-^{∗∗}, or -(CH₂)ₙ₁-C₅₋₁₅heteroarylene-(CH₂)ₙ₂-^{∗∗}, wherein symbol ** indicates the point of attachment to X;
wherein n1 and n2 each independently represent an integer of 1, 2, 3, 4, 5, 6, 7, 8, 9, 10, 11, 12, 13, 14, 15, 16, 17, 18, 19, or 20; and
said C₅₋₁₅ arylene and C₅₋₁₅ heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof;
R represents NRₐ₇Rₐ₈, C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(O)Rₐ₂₅, S(O)₂Rₐ₂₆, S(O)₂ORₐ₂₇, OS(O)₂Rₐ₂₈, optionally substituted C₃₋₂₀ cycloalkyl, or optionally substituted C₃₋₂₀ heterocyclyl,
wherein Rₐ₇, Rₐ₈, Rₐ₉, Rₐ₁₀, Rₐ₁₆, Rₐ₁₇, Rₐ₂₁ and Rₐ₂₂ are the same or different and each independently represent H, optionally substituted linear or branched C₁₋₁₀ alkyl, optionally substituted C₃₋₂₀ cycloalkyl or optionally substituted C₃₋₂₀ heterocyclyl;
Rₐ₁₁ and Rₐ₂₃ each independently represent H or C₁₋₃ alkyl; and
Rₐ₁₂, Rₐ₁₈, Rₐ₁₉, Rₐ₂₀, Rₐ₂₄, Rₐ₂₅, Rₐ₂₆, Rₐ₂₇, and Rₐ₂₈ each independently represent optionally substituted linear or branched C₁₋₁₀ alkyl, optionally substituted C₃₋₂₀ cycloalkyl or optionally substituted C₃₋₂₀ heterocyclyl;
wherein said linear or branched C₁₋₁₀ alkyl is optionally substituted by one or more substituents selected from the group consisting of halogen, hydroxyl, cyano, amino, C₁₋₃ alkoxy, C₁₋₃ alkylamino, C₁₋₃ alkyl-NHC(O)- or any combination thereof; and
each C₃₋₂₀ cycloalkyl and each C₃₋₂₀ heterocyclyl is independently optionally substituted by one or more substituents selected from the group consisting of halogen, hydroxyl, mercapto, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, optionally substituted C₆₋₂₀ aryl or any combination thereof, wherein said C₆₋₂₀ aryl is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkylamino, or any combination thereof.

15. The compound of Formula (I) or a salt, an enantiomer, a stereoisomer, a solvate, or a polymorph thereof as claimed in claim 1 or 2, wherein
X represents N(Rₐ₁), O or S, where Rₐ₁ represents H or C₁₋₃ alkyl; and
L represents the group represented by the following formula:
-C₃₋₁₅cycloalkylene-(CH₂)₁₋₄₀-^{∗}, or -C₅₋₁₅arylene-(CH₂)₁₋₄₀-^{∗}, wherein symbol * indicates the point of attachment to X; and
wherein said C₃₋₁₅ cycloalkylene is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, or any combination thereof, and said C₅₋₁₅ arylene is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof; and
R represents C(O)NRₐ₉Rₐ₁₀, N(Rₐ₁₁)C(O)Rₐ₁₂, ORₐ₁₆, SRₐ₁₇, C(O)Rₐ₁₈, OC(O)Rₐ₁₉, C(O)ORₐ₂₀, S(O)₂NRₐ₂₁Rₐ₂₂, N(Rₐ₂₃)S(O)₂Rₐ₂₄, S(OORₐ₂₅, S(O)₂Rₐ₂₆, S(O)₂ORₐ₂₇, or OS(O)₂Rₐ₂₈,
wherein Rₐ₁₆ and Rₐ₁₇ are each independently represent H, optionally substituted C₃₋₂₀ cycloalkyl or optionally substituted C₃₋₂₀ heterocyclyl;
Rₐ₉, Rₐ₁₁, Rₐ₂₁ and Rₐ₂₃ each independently represent H or C₁₋₃ alkyl; and
Rₐ₁₀, Rₐ₁₂, Rₐ₁₈, Rₐ₁₉, Rₐ₂₀, Rₐ₂₂, Rₐ₂₄, Rₐ₂₅, Rₐ₂₆, Rₐ₂₇, and Rₐ₂₈ each independently represent optionally substituted C₃₋₂₀ cycloalkyl or optionally substituted C₃₋₂₀ heterocyclyl.

16. The compound of Formula (I) or a salt, an enantiomer, a stereoisomer, a solvate, or a polymorph thereof as claimed in claim 1 or 2, wherein
X represents C₂₋₆ alkynylene or C₂₋₆ alkenylene; and
L represents the group represented by the following formula:
-(CH₂)ₙ₁-C₅₋₁₅arylene-(CH₂)ₙ₂-^{∗∗}, or -(CH₂)ₙ₁-C₅₋₁₅heteroarylene-(CH₂)ₙ₂-^{∗∗}, wherein symbol ** indicates the point of attachment to X;
wherein n1 and n2 each independently represent an integer of 1, 2, 3, 4, 5, 6, 7, 8, 9, 10, 11, 12, 13, 14, 15, 16, 17, 18, 19, or 20; and
said C₅₋₁₅ arylene and C₅₋₁₅ heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof.

17. The compound of Formula (I) or a salt, an enantiomer, a stereoisomer, a solvate, or a polymorph thereof as claimed in claim 1 or 2, wherein
X represents C₂₋₆ alkynylene or C₂₋₆ alkenylene; and
L represents the group represented by the following formula:
-(CH₂)₁₋₄₀-C₅₋₁₅ arylene-#, or -(CH₂)₁₋₄₀-C₅₋₁₅ heteroarylene-#,
wherein said C₅₋₁₅ arylene and C₅₋₁₅ heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof; and
symbol # indicates the point of attachment to X.

18. The compound of Formula (I) or a salt, an enantiomer, a stereoisomer, a solvate, or a polymorph thereof as claimed in claim 1, wherein
X represents a bond; and
L represents:
-(CH₂)ₙ₁-C₅₋₁₅arylene-(CH₂)ₙ₂-^{∗∗}, or -(CH₂)ₙ₁-C₅₋₁₅heteroarylene-(CH₂)ₙ₂-^{∗∗}, wherein symbol ** indicates the point of attachment to X;
wherein n1 and n2 each independently represent an integer of 1, 2, 3, 4, 5, 6, 7, 8, 9, 10, 11, 12, 13, 14, 15, 16, 17, 18, 19, or 20; and
said C₅₋₁₅ arylene and C₅₋₁₅ heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof.

19. The compound of Formula (I) or a salt, an enantiomer, a stereoisomer, a solvate, or a polymorph thereof as claimed in any one of claims 14-18, wherein
in the formula of L, said C₅₋₁₅ heteroarylene includes furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzo[d][1,3]dioxolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene and imidazo[2,1-b]thiazolylene;
said C₅₋₁₅ arylene includes phenylene and naphthylene; and
said C₃₋₁₅ cycloalkylene includes cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, C₅₋₁₅ spiro-cycloalkylene, adamantanylene, noradamantanylene, bornylene, bicyclo[2.2.1]heptylene, and bicyclo[2.2.1]heptenylene;
wherein said C₃₋₁₅ cycloalkylene is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, or any combination thereof, and said C₅₋₁₅ arylene and C₅₋₁₅ heteroarylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof.

20. The compound of Formula (I) or a salt, an enantiomer, a stereoisomer, a solvate, or a polymorph thereof as claimed in claims 14, 16 or 18, wherein L represents:
-CH₂-phenylene-CH₂-^{∗∗}, -CH₂-phenylene-(CH₂)₂-^{∗∗}, -CH₂-phenylene-(CH₂)₃-^{∗∗}, -CH₂-phenylene-(CH₂)₄-^{∗∗}, -CH₂-phenylene-(CH₂)₅-^{∗∗}, -CH₂-phenylene-(CH₂)₆-^{∗∗}, -CH₂-phenylene-(CH₂)₇-^{∗∗}, - CH₂-phenylene-(CH₂)₈-^{∗∗}, -(CH₂)₂-phenylene-CH₂-^{∗∗}, -(CH₂)₂-phenylene-(CH₂)₂-^{∗∗}, -(CH₂)₂-phenylene-(CH₂)₃-^{∗∗}, -(CH₂)₂-phenylene-(CH₂)₄-^{∗∗}, -(CH₂)₂-phenylene-(CH₂)₅-^{∗∗}, -(CH₂)₂-phenylene-(CH₂)₆-^{∗∗}, -(CH₂)₂-phenylene-(CH₂)₇-^{∗∗}, -(CH₂)₂-phenylene-(CH₂)₈-^{∗∗}, -(CH₂)₃-phenylene-CH₂-^{∗∗}, -(CH₂)₃-phenylene-(CH₂)₂-^{∗∗}, -(CH₂)₃-phenylene-(CH₂)₃-^{∗∗}, -(CH₂)₃-phenylene-(CH₂)₄-^{∗∗}, -(CH₂)₃-phenylene-(CH₂)₅-^{∗∗}, -(CH₂)₃-phenylene-(CH₂)₆-^{∗∗}, -(CH₂)₃-phenylene-(CH₂)₇-^{∗∗}, -(CH₂)₃-phenylene-(CH₂)₈-^{∗∗}, -(CH₂)₄-phenylene-CH₂-^{∗∗}, -(CH₂)₄-phenylene-(CH₂)₂-^{∗∗}, - (CH₂)₄-phenylene-(CH₂)₃-^{∗∗}, -(CH₂)₄-phenylene-(CH₂)₄-^{∗∗}, -(CH₂)₄-phenylene-(CH₂)₅-^{∗∗}, -(CH₂)₄-phenylene-(CH₂)₆-^{∗∗}, -(CH₂)₄-phenylene-(CH₂)₇-^{∗∗}, -(CH₂)₄-phenylene-(CH₂)₈-^{∗∗}, -(CH₂)₅-phenylene-CH₂-^{∗∗}, -(CH₂)₅-phenylene-(CH₂)₂-^{∗∗}, -(CH₂)₅-phenylene-(CH₂)₃-^{∗∗}, -(CH₂)₅-phenylene-(CH₂)₄-^{∗∗}, -(CH₂)₅-phenylene-(CH₂)₅-^{∗∗}, -(CH₂)₆-phenylene-CH₂-^{∗∗}, -(CH₂)₆-phenylene-(CH₂)₂-^{∗∗}, -(CH₂)₆-phenylene-(CH₂)₃-^{∗∗}, -(CH₂)₆-phenylene-(CH₂)₄-^{∗∗}, -(CH₂)₆-phenylene-(CH₂)₅-^{∗∗}, -(CH₂)₆-phenylene-(CH₂)₆-^{∗∗}, -(CH₂)₇-phenylene-CH₂-^{∗∗}, -(CH₂)₇-phenylene-(CH₂)₂-^{∗∗}, -(CH₂)₇-phenylene-(CH₂)₃-^{∗∗}, -(CH₂)₇-phenylene-(CH₂)₄-^{∗∗}, -(CH₂)₇-phenylene-(CH₂)₅-^{∗∗}, -(CH₂)₇-phenylene-(CH₂)₆-^{∗∗}, -(CH₂)₈-phenylene-CH₂-^{∗∗}, -(CH₂)₈-phenylene-(CH₂)₂-^{∗∗}, -(CH₂)₈-phenylene-(CH₂)₃-^{∗∗}, - (CH₂)₈-phenylene-(CH₂)₄-^{∗∗}, -(CH₂)₈-phenylene-(CH₂)₅-^{∗∗}, -(CH₂)₈-phenylene-(CH₂)₆-^{∗∗}, -(CH₂)₈-phenylene-(CH₂)₇-^{∗∗}, -(CH₂)₈-phenylene-(CH₂)₈-^{∗∗}, -CH₂-pyridylene-CH₂-^{∗∗}, -CH₂-pyridylene-(CH₂)₂-^{∗∗}, -CH₂-pyridylene-(CH₂)₃-^{∗∗}, -CH₂-pyridylene-(CH₂)₄-^{∗∗}, -CH₂-pyridylene-(CH₂)₅-^{∗∗}, - CH₂-pyridylene-(CH₂)₆-^{∗∗}, -CH₂-pyridylene-(CH₂)₇-^{∗∗}, -CH₂-pyridylene-(CH₂)₈-^{∗∗}, -(CH₂)₂-pyridylene-CH₂-^{∗∗}, -(CH₂)₂-pyridylene-(CH₂)₂-^{∗∗}, -(CH₂)₂-pyridylene-(CH₂)₃-^{∗∗}, -(CH₂)₂-pyridylene-(CH₂)₄-^{∗∗}, -(CH₂)₂-pyridylene-(CH₂)₅-^{∗∗}, -(CH₂)₂-pyridylene-(CH₂)₆-^{∗∗}, -(CH₂)₂-pyridylene-(CH₂)₇-^{∗∗}, -(CH₂)₂-pyridylene-(CH₂)₈-^{∗∗}, -(CH₂)₃-pyridylene-CH₂-^{∗∗}, -(CH₂)₃-pyridylene-(CH₂)₂-^{∗∗}, -(CH₂)₃-pyridylene-(CH₂)₃-^{∗∗}, -(CH₂)₃-pyridylene-(CH₂)₄-^{∗∗}, -(CH₂)₃-pyridylene-(CH₂)₅-^{∗∗}, -(CH₂)₃-pyridylene-(CH₂)₆-^{∗∗}, -(CH₂)₃-pyridylene-(CH₂)₇-^{∗∗}, -(CH₂)₃-pyridylene-(CH₂)₈-^{∗∗}, -(CH₂)₄-pyridylene-CH₂-^{∗∗}, -(CH₂)₄-pyridylene-(CH₂)₂-^{∗∗}, -(CH₂)₄-pyridylene-(CH₂)₃-^{∗∗}, -(CH₂)₄-pyridylene-(CH₂)₄-^{∗∗}, -(CH₂)₄-pyridylene-(CH₂)₅-^{∗∗}, -(CH₂)₄-pyridylene-(CH₂)₆-^{∗∗}, -(CH₂)₄-pyridylene-(CH₂)₇-^{∗∗}, -(CH₂)₄-pyridylene-(CH₂)₈-^{∗∗}, -(CH₂)₅-pyridylene-CH₂-^{∗∗}, -(CH₂)₅-pyridylene-(CH₂)₂-^{∗∗}, -(CH₂)₅-pyridylene-(CH₂)₃-^{∗∗}, -(CH₂)₅-pyridylene-(CH₂)₄-^{∗∗}, -(CH₂)₅-pyridylene-(CH₂)₅-^{∗∗}, -(CH₂)₆-pyridylene-CH₂-^{∗∗}, -(CH₂)₆-pyridylene-(CH₂)₂-^{∗∗}, -(CH₂)₆-pyridylene-(CH₂)₃-^{∗∗}, -(CH₂)₆-pyridylene-(CH₂)₄-^{∗∗}, -(CH₂)₆-pyridylene-(CH₂)₅-^{∗∗}, -(CH₂)₆-pyridylene-(CH₂)₆-^{∗∗}, -(CH₂)₇-pyridylene-CH₂-^{∗∗}, -(CH₂)₇-pyridylene-(CH₂)₂-^{∗∗}, -(CH₂)₇-pyridylene-(CH₂)₃-^{∗∗}, -(CH₂)₇-pyridylene-(CH₂)₄-^{∗∗}, -(CH₂)₇-pyridylene-(CH₂)₅-^{∗∗}, , (CH₂)₇-pyridylene-(CH₂)₆-^{∗∗}, -(CH₂)₈-pyridylene-CH₂-^{∗∗}, -(CH₂)₈-pyridylene-(CH₂)₂-^{∗∗}, -(CH₂)₈-pyridylene-(CH₂)₃-^{∗∗}, -(CH₂)₈-pyridylene-(CH₂)₄-^{∗∗}, -(CH₂)₈-pyridylene-(CH₂)₅-^{∗∗}, -(CH₂)₈-pyridylene-(CH₂)₆-^{∗∗}, -(CH₂)₈-pyridylene-(CH₂)₇-^{∗∗}, -(CH₂)₈-pyridylene-(CH₂)₈-^{∗∗}, -CH₂-thiazolylene-CH₂-^{∗∗}, -CH₂-thiazolylene-(CH₂)₂-^{∗∗}, -CH₂-thiazolylene-(CH₂)₃-^{∗∗}, -CH₂-thiazolylene-(CH₂)₄-^{∗∗}, -CH₂-thiazolylene-(CH₂)₅-^{∗∗}, -CH₂-thiazolylene-(CH₂)₆-^{∗∗}, -CH₂-thiazolylene-(CH₂)₇-^{∗∗}, -CH₂-thiazolylene-(CH₂)₈-^{∗∗}, -(CH₂)₂-thiazolylene-CH₂-^{∗∗}, -(CH₂)₂-thiazolylene-(CH₂)₂-^{∗∗}, -(CH₂)₂-thiazolylene-(CH₂)₃-^{∗∗}, -(CH₂)₂-thiazolylene-(CH₂)₄-^{∗∗}, -(CH₂)₂-thiazolylene-(CH₂)₅-^{∗∗}, -(CH₂)₂-thiazolylene-(CH₂)₆-^{∗∗}, -(CH₂)₂-thiazolylene-(CH₂)₇-^{∗∗}, -(CH₂)₂-thiazolylene-(CH₂)₈-^{∗∗}, -(CH₂)₃-thiazolylene-CH₂-^{∗∗}, -(CH₂)₃-thiazolylene-(CH₂)₂-^{∗∗}, -(CH₂)₃-thiazolylene-(CH₂)₃-^{∗∗}, -(CH₂)₃-thiazolylene-(CH₂)₄-^{∗∗}, -(CH₂)₃-thiazolylene-(CH₂)₅-^{∗∗}, -(CH₂)₃-thiazolylene-(CH₂)₆-^{∗∗}, -(CH₂)₃-thiazolylene-(CH₂)₇-^{∗∗}, -(CH₂)₃-thiazolylene-(CH₂)₈-^{∗∗}, -(CH₂)₄-thiazolylene-CH₂-^{∗∗}, -(CH₂)₄-thiazolylene-(CH₂)₂-^{∗∗}, -(CH₂)₄-thiazolylene-(CH₂)₃-^{∗∗}, -(CH₂)₄-thiazolylene-(CH₂)₄-^{∗∗}, -(CH₂)₄-thiazolylene-(CH₂)₅-^{∗∗}, -(CH₂)₄-thiazolylene-(CH₂)₆-^{∗∗}, -(CH₂)₄-thiazolylene-(CH₂)₇-^{∗∗}, -(CH₂)₄-thiazolylene-(CH₂)₈-^{∗∗}, -(CH₂)₅-thiazolylene-CH₂-^{∗∗}, -(CH₂)₅-thiazolylene-(CH₂)₂-^{∗∗}, -(CH₂)₅-thiazolylene-(CH₂)₃-^{∗∗}, -(CH₂)₅-thiazolylene-(CH₂)₄-^{∗∗}, -(CH₂)₅-thiazolylene-(CH₂)₅-^{∗∗}, -(CH₂)₆-thiazolylene-CH₂-^{∗∗}, -(CH₂)₆-thiazolylene-(CH₂)₂-^{∗∗}, -(CH₂)₆-thiazolylene-(CH₂)₃-^{∗∗}, -(CH₂)₆-thiazolylene-(CH₂)₄-^{∗∗}, -(CH₂)₆-thiazolylene-(CH₂)₅-^{∗∗}, -(CH₂)₆-thiazolylene-(CH₂)₆-^{∗∗}, -(CH₂)₇-thiazolylene-CH₂-^{∗∗}, -(CH₂)₇-thiazolylene-(CH₂)₂-^{∗∗}, -(CH₂)₇-thiazolylene-(CH₂)₃-^{∗∗}, -(CH₂)₇-thiazolylene-(CH₂)₄-^{∗∗}, -(CH₂)₇-thiazolylene-(CH₂)₅-^{∗∗}, -(CH₂)₇-thiazolylene-(CH₂)₆-^{∗∗}, -(CH₂)₈-thiazolylene-CH₂-^{∗∗}, -(CH₂)₈-thiazolylene-(CH₂)₂-^{∗∗}, -(CH₂)₈-thiazolylene-(CH₂)₃-^{∗∗}, -(CH₂)₈-thiazolylene-(CH₂)₄-^{∗∗}, -(CH₂)₈-thiazolylene-(CH₂)₅-^{∗∗}, -(CH₂)₈-thiazolylene-(CH₂)₆-^{∗∗}, -(CH₂)₈-thiazolylene-(CH₂)₇-^{∗∗}, or -(CH₂)₈-thiazolylene-(CH₂)₈-^{∗∗}, wherein symbol ^{∗∗} indicates the point of attachment to X;
wherein said phenylene, pyridylene, and thiazolylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkylamino, C₁₋₃ alkyl-NHC(O)-, or any combination thereof.

21. The compound of Formula (I) or a salt, an enantiomer, a stereoisomer, a solvate, or a polymorph thereof as claimed in any one of claims 14, 16 or 18, wherein L represents:

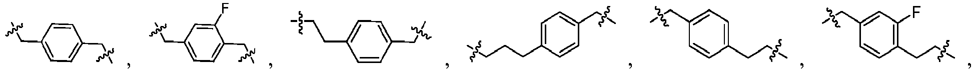

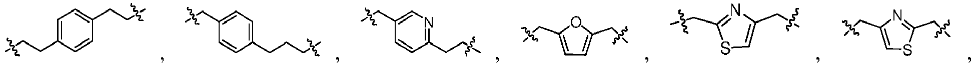

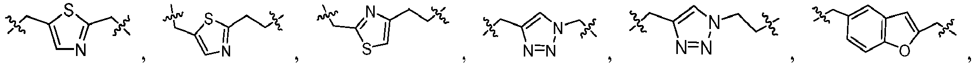

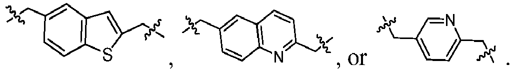

22. The compound of Formula (I) or a salt, an enantiomer, a stereoisomer, a solvate, or a polymorph thereof as claimed in claim 15, wherein L represents:
-cyclohexylene-CH₂-^{∗}, -cyclohexylene-(CH₂)₂-^{∗}, -cyclohexylene-(CH₂)₃-^{∗}, -cyclohexylene-(CH₂)₄-^{∗}, -cyclohexylene-(CH₂)₅-^{∗}, -cyclohexylene-(CH₂)₆-^{∗}, -cyclohexylene-(CH₂)₇-^{∗}, -cyclohexylene-(CH₂)₈-^{∗}, -cyclohexylene-(CH₂)₉-^{∗}, -cyclohexylene-(CH₂)₁₀-^{∗}, -cyclohexylene-(CH₂)₁₁-^{∗}, - cyclohexylene-(CH₂)₁₂-^{∗}, -phenylene-CH₂-^{∗}, -phenylene-(CH₂)₂-^{∗}, -phenylene-(CH₂)₃-^{∗}, - phenylene-(CH₂)₄-^{∗}, -phenylene-(CH₂)₅-^{∗}, -phenylene-(CH₂)₆-^{∗}, -phenylene-(CH₂)₇-^{∗}, - phenylene-(CH₂)₈-^{∗}, -phenylene-(CH₂)₉-^{∗}, -phenylene-(CH₂)₁₀-^{∗}, -phenylene-(CH₂)₁₁-^{∗}, or - phenylene-(CH₂)₁₂-^{∗}, wherein symbol ^{∗} indicates the point of attachment to X; and
wherein said cyclohexylene is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, oxo, C₁₋₃ alkyl-NHC(O)-, or any combination thereof; and said phenylene is optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof.

23. The compound of Formula (I) or a salt, an enantiomer, a stereoisomer, a solvate, or a polymorph thereof as claimed in claim 22, wherein L represents: 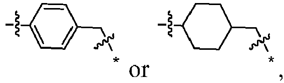 wherein symbol ^{∗} indicates the point of attachment to X.

24. The compound of Formula (I) or a salt, an enantiomer, a stereoisomer, a solvate, or a polymorph thereof as claimed in claim 17, wherein L represents:
-CH₂-phenylene-#, -(CH₂)₂-phenylene-#, -(CH₂)₃-phenylene-#, -(CH₂)₄-phenylene-#, -(CH₂)₅-phenylene-#, -(CH₂)₆-phenylene-#, -(CH₂)₇-phenylene-#, -(CH₂)₈-phenylene-#, -(CH₂)₉-phenylene-#, -(CH₂)₁₀-phenylene-#, -(CH₂)₁₁-phenylene-#, -(CH₂)₁₂-phenylene-#, -CH₂-pyridylene-#, -(CH₂)₂-pyridylene-#, -(CH₂)₃-pyridylene-#, -(CH₂)₄-pyridylene-#, -(CH₂)₅-pyridylene-#, -(CH₂)₆-pyridylene-#, -(CH₂)₇-pyridylene-#, -(CH₂)₈-pyridylene-#, -(CH₂)₉-pyridylene-#, -(CH₂)₁₀-pyridylene-#, -(CH₂)₁₁-pyridylene-#, -(CH₂)₁₂-pyridylene-#, -CH₂-thiazolylene-#, -(CH₂)₂-thiazolylene-#, -(CH₂)₃-thiazolylene-#, -(CH₂)₄-thiazolylene-#, -(CH₂)₅-thiazolylene-#, -(CH₂)₆-thiazolylene-#, -(CH₂)₇-thiazolylene-#, -(CH₂)₈-thiazolylene-#, -(CH₂)₉-thiazolylene-#, -(CH₂)₁₀-thiazolylene-#, -(CH₂)₁₁-thiazolylene-#, or -(CH₂)₁₂-thiazolylene-#, wherein symbol # indicates the point of attachment to X; and
wherein said phenylene, pyridylene, and thiazolylene are each independently optionally substituted by one or more substituents selected from the group consisting of halogen, hydroxyl, cyano, amino, C₁₋₃ alkyl, C₁₋₃ alkoxy, halogenated C₁₋₃ alkyl, C₁₋₃ alkylamino, C₁₋₃ alkyl-NHC(O)-, or any combination thereof.

25. The compound of Formula (I) or a salt, an enantiomer, a stereoisomer, a solvate, or a polymorph thereof as claimed in claim 24, wherein L represents: 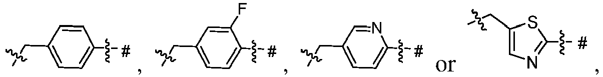 wherein symbol # indicates the point of attachment to X.

26. The compound of Formula (I) or a salt, an enantiomer, a stereoisomer, a solvate, or a polymorph thereof as claimed in claim 10, wherein
L represents linear or branched C₂₋₄₀ alkylene interrupted one or more times by one or more groups selected from the group consisting of: optionally substituted C₅₋₁₅ arylene, optionally substituted C₅₋₁₅ heteroarylene or any combination thereof, wherein said linear or branched C₂₋₄₀ alkylene is optionally substituted by a substituent selected from the group consisting of halogen, hydroxyl, amino or any combination thereof, and each C₅₋₁₅ arylene and each C₅₋₁₅ heteroarylene is optionally substituted by 1-3 substituents selected from the group consisting of halogen, hydroxyl, cyano, amino, C₁₋₃ alkyl, C₁₋₃ alkoxy, halogenated C₁₋₃ alkyl, C₁₋₃ alkyl substituted by hydroxy, C₁₋₃ alkyl substituted by NH₂, C₁₋₃ alkylamino, C₁₋₃ alkyl-NHC(O)- or any combination thereof; and
R represents NRₐ₇Rₐ₈, wherein Rₐ₇ and Rₐ₈ each independently represent:
H; or
methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, or decyl, which are optionally substituted by one or more substituents selected from the group consisting of halogen, hydroxyl, cyano, amino, mercapto, C₁₋₃ alkoxy, C₁₋₃ alkylamino, C₁₋₃ alkyl-NHC(O)- or any combination thereof; or
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 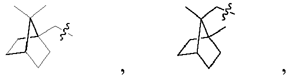 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

27. The compound of Formula (I) or a salt, an enantiomer, a stereoisomer, a solvate, or a polymorph thereof as claimed in claim 9 or 10, wherein R represents ORₐ₁₆, where Rₐ₁₆ represents:
H; or
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
*p*-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 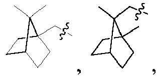 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

28. The compound of Formula (I) or a salt, an enantiomer, a stereoisomer, a solvate, or a polymorph thereof as claimed in claim 10, wherein R represents:
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are independently optionally substituted by one or more substituents selected from the group consisting of:
halogen, hydroxyl, mercapto, oxo, cyano, amino, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)-, optionally substituted phenyl or any combination thereof, wherein said optionally substituted phenyl is optionally substituted by a substituent selected from the group consisting of halogen, hydroxyl, amino, cyano, mercapto, C₁₋₃ alkyl, or any combination thereof.

29. The compound of Formula (I) or a salt, an enantiomer, a stereoisomer, a solvate, or a polymorph thereof as claimed in claim 9 or 10, wherein R represents:
C(O)NRₐ₉Rₐ₁₀, N(Rₐ₁₁)C(O)Rₐ₁₂, C(O)Rₐ₁₈, OC(O)Rₐ₁₉, C(O)ORₐ₂₀, S(O)₂NRₐ₂₁Rₐ₂₂, or N(Rₐ₂₃)S(O)₂Rₐ₂₄,
wherein Rₐ₉, Rₐ₁₁, Rₐ₂₁, and Rₐ₂₃ each independently represent H or C₁₋₃ alkyl; and
Rₐ₁₀, Rₐ₁₂, Rₐ₁₈, Rₐ₁₉, Rₐ₂₀, Rₐ₂₂ and Rₐ₂₄ each independently represent the following groups:
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, adamantan-10-yl, noradamantan-1-yl, noradamantan-2-yl, noradamantan-3-yl, noradamantan-4-yl, noradamantan-5-yl, noradamantan-6-yl, noradamantan-7-yl, noradamantan-8-yl, noradamantan-9-yl, cyclopropyl, cyclobutyl, cyclopentyl, cyclopentenyl, cyclohexyl, cyclohexenyl, cycloheptyl, cyclooctyl, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, C₇₋₁₁azaspirocycloalkyl, p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 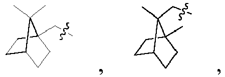 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl,
wherein the groups are optionally substituted by one or more substituents selected from the group consisting of halogen, hydroxyl, mercapto, oxo, cyano, amino, C₁₋₃ alkyl, halogenated C₁₋₃ alkyl, C₁₋₃ alkyl substituted by hydroxy, C₁₋₃ alkyl substituted by NH₂, C₁₋₃ alkoxy, C₁₋₃ alkylamino, C₁₋₃ alkyl-NHC(O)- or any combination thereof.

30. The compound of Formula (I) or a salt, an enantiomer, a stereoisomer, a solvate, or a polymorph thereof as claimed in claim 11, wherein R represents NR_{b7}R_{b8}, wherein R_{b7} and R_{b8} each independently represent:
H, or
methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, or decyl, which are optionally substituted by one or more substituents selected from the group consisting of halogen, hydroxyl, cyano, amino, mercapto, C₁₋₃ alkoxy, C₁₋₃ alkylamino, C₁₋₃ alkyl-NHC(O)- or any combination thereof; or
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 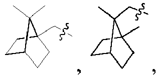 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

31. The compound of Formula (I) or a salt, an enantiomer, a stereoisomer, a solvate, or a polymorph thereof as claimed in claim 11, wherein R represents OR_{b16}, wherein R_{b16} represents:
H, or
methyl, ethyl, n-propyl, isopropyl, n-butyl, isobutyl, sec-butyl, tert-butyl, pentyl, isopentyl, neopentyl, tert-pentyl, hexyl, heptyl, octyl, nonyl, or decyl, which are optionally substituted by one or more substituents selected from the group consisting of halogen, hydroxyl, cyano, amino, mercapto, C₁₋₃ alkoxy, C₁₋₃ alkylamino, C₁₋₃ alkyl-NHC(O)- or any combination thereof; or
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
*p*-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 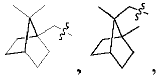 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

32. The compound of Formula (I) or a salt, an enantiomer, a stereoisomer, a solvate, or a polymorph thereof as claimed in claim 11, wherein R represents:
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are independently optionally substituted by one or more substituents selected from the group consisting of:
halogen, hydroxyl, mercapto, oxo, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)-, optionally substituted phenyl or any combination thereof, wherein said optionally substituted phenyl is optionally substituted by a substituent selected from the group consisting of halogen, hydroxyl, amino, cyano, mercapto, C₁₋₃ alkyl, or any combination thereof.

33. The compound of Formula (I) or a salt, an enantiomer, a stereoisomer, a solvate, or a polymorph thereof as claimed in claim 11, wherein R represents:
C(O)NR_{b9}R_{b10}, N(R_{b11})C(O)R_{b12}, C(O)R_{b18}, OC(O)R_{b19}, C(O)OR_{b20}, S(O)₂NR_{b21}R_{b22}, or N(R_{b23})S(O)₂R_{b24},
wherein R_{b9}, R_{b11}, R_{b21} and R_{b23} each independently represent H or C₁₋₃ alkyl; and
R_{b10}, R_{b12}, R_{b18}, R_{b19}, R_{b20}, R_{b22}, and R_{b24} each independently represent the following groups:
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, adamantan-10-yl, noradamantan-1-yl, noradamantan-2-yl, noradamantan-3-yl, noradamantan-4-yl, noradamantan-5-yl, noradamantan-6-yl, noradamantan-7-yl, noradamantan-8-yl, noradamantan-9-yl, cyclopropyl, cyclobutyl, cyclopentyl, cyclopentenyl, cyclohexyl, cyclohexenyl, cycloheptyl, cyclooctyl, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, C₇₋₁₁azaspirocycloalkyl, *p*-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 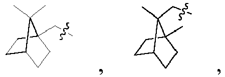 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl,
wherein the groups are each independently optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof.

34. The compound of Formula (I) or a salt, an enantiomer, a stereoisomer, a solvate, or a polymorph thereof as claimed in claim 8, wherein R represents:

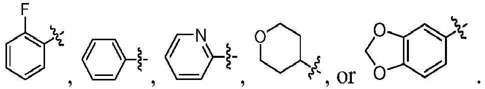

35. The compound of Formula (I) or a salt, an enantiomer, a stereoisomer, a solvate, or a polymorph thereof as claimed in claim 9, wherein R represents:
OH, N(CH₃)₂, N(CH₂CH₃)₂,

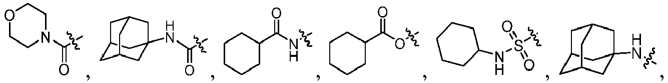

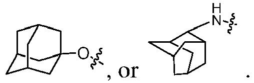

36. The compound of Formula (I) or a salt, an enantiomer, a stereoisomer, a solvate, or a polymorph thereof as claimed in claim 10 or 11, wherein R represents:
OH, N(CH₃)₂, N(CH₂CH₃)₂,

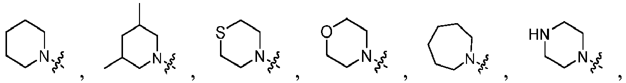

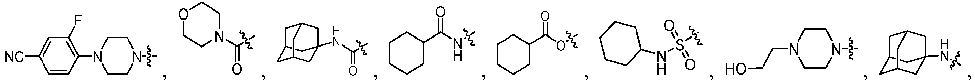

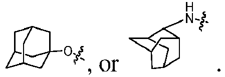

37. The compound of Formula (I) or a salt, an enantiomer, a stereoisomer, a solvate, or a polymorph thereof as claimed in claim 12, wherein R represents NR_{c4}R_{c5}, wherein
R_{c4} represents H or C₁₋₃ alkyl;
R_{c5} represents:
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 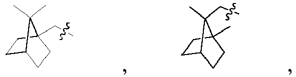 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

38. The compound of Formula (I) or a salt, an enantiomer, a stereoisomer, a solvate, or a polymorph thereof as claimed in claim 12, wherein R represents OR_{c13}, wherein R_{c13} represents
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, or adamantan-10-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
noradamantan-1-yl, noradamantan-2-yl, noradamantan-3-yl, noradamantan-4-yl, noradamantan-5-yl, noradamantan-6-yl, noradamantan-7-yl, noradamantan-8-yl, or noradamantan-9-yl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
cyclopropyl, cyclobutyl, cyclopentyl, cyclopentenyl, cyclohexyl, cyclohexenyl, cycloheptyl, or cyclooctyl, which are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof; or
*p*-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 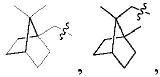 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl.

39. The compound of Formula (I) or a salt, an enantiomer, a stereoisomer, a solvate, or a polymorph thereof as claimed in claim 12, wherein R represents
azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, or C₇₋₁₁azaspirocycloalkyl, which are optionally substituted by one or more substituents selected from the group consisting of:
halogen, hydroxyl, mercapto, oxo, cyano, C₁₋₃ alkyl, halogenated C₁₋₃ alkyl, C₁₋₃ alkyl substituted by hydroxy, C₁₋₃ alkoxy, C₁₋₃ alkylamino, C₁₋₃ alkyl-NHC(O)-, or any combination thereof.

40. The compound of Formula (I) or a salt, an enantiomer, a stereoisomer, a solvate, or a polymorph thereof as claimed in claim 12, wherein R represents
C(O)NR_{c6}R_{c7}, N(R_{c8})C(O)R_{c9}, C(O)R_{c15}, OC(O)R_{c16}, C(O)OR_{c17}, S(O)₂NR_{c18}R_{c19}, or N(R_{c20})S(O)₂R_{c21},
wherein R_{c6}, R_{c8}, R_{c18} and R_{c20} each independently represent H or C₁₋₃ alkyl; and
R_{c7}, R_{c9}, R_{c15}, R_{c16}, R_{c17}, R_{c19}, and R_{c21} each independently represent the following groups:
adamantan-1-yl, adamantan-2-yl, adamantan-3-yl, adamantan-4-yl, adamantan-5-yl, adamantan-6-yl, adamantan-7-yl, adamantan-8-yl, adamantan-9-yl, adamantan-10-yl, noradamantan-1-yl, noradamantan-2-yl, noradamantan-3-yl, noradamantan-4-yl, noradamantan-5-yl, noradamantan-6-yl, noradamantan-7-yl, noradamantan-8-yl, noradamantan-9-yl, cyclopropyl, cyclobutyl, cyclopentyl, cyclopentenyl, cyclohexyl, cyclohexenyl, cycloheptyl, cyclooctyl, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, 1,4-diazacycloheptan-1-yl, 3,8-diazabicyclo[3.2.1]octan-3-yl, 2,5-diazabicyclo[2.2.2]octan-2-yl, C₇₋₁₁azaspirocycloalkyl, p-menthanyl, meta-menthanyl, quinuclidinyl, bicyclo[2.2.1]heptan-2-yl, bicyclo[2.2.1]heptan-3-yl, bicyclo[2.2.1]heptan-4-yl, bicyclo[2.2.1]heptan-5-yl, bicyclo[2.2.1]heptan-6-yl, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl, 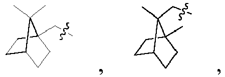 7,7-dimethyl-2-oxobicyclo[2.2.1]heptan-1-yl, 7,7-dimethyl-2-oxobicyclo[2.2.1]heptan-3-yl, 7,7-dimethyl-2-oxobicyclo[2.2.1]heptan-4-yl, 7,7-dimethyl-2-oxobicyclo[2.2.1]heptan-5-yl, 7,7-dimethyl-2-oxobicyclo[2.2.1]heptan-6-yl, bicyclo[2.2.1]hept-5-en-2-yl, bicyclo[2.2.1]hept-5-en-3-yl, or bicyclo[2.2.1]hept-5-en-7-yl,
wherein the groups are optionally substituted by one or more substituents selected from the group consisting of halogen, hydroxyl, mercapto, oxo, cyano, C₁₋₃ alkyl, halogenated C₁₋₃ alkyl, C₁₋₃ alkoxy, C₁₋₃ alkylamino, C₁₋₃ alkyl-NHC(O)- or any combination thereof.

41. The compound of Formula (I) or a salt, an enantiomer, a stereoisomer, a solvate, or a polymorph thereof as claimed in claim 12, wherein R represents

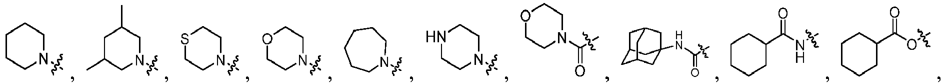

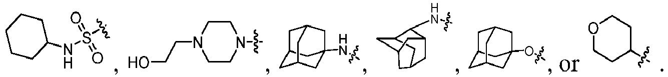

42. The compound of Formula (I) or a salt, an enantiomer, a stereoisomer, a solvate, or a polymorph thereof as claimed in claim 1, which is selected from:
3-(5-((benzo[d][1,3]dioxol-5-ylmethyl)amino)-2-methyl-4-oxoquinazolin-3(4H)-yl)piperidine-2,6-dione;
3-(2-methyl-4-oxo-5-(((tetrahydro-2H-pyran-4-yl)methyl)amino)quinazolin-3(4H)-yl)piperidine-2,6-dione;
3-(2-methyl-4-oxo-5-((pyridin-2-ylmethyl)amino)quinazolin-3(4H)-yl)piperidine-2,6-dione;
3-(5-((2-fluorobenzyl)amino)-2-methyl-4-oxoquinazolin-3(4H)-yl)piperidine-2,6-dione;
3-(5-((4-(((1R,3R,5S)-adamantan-1-ylamino)methyl)benzyl)amino)-2-methyl-4-oxoquinazolin-3(4H)-yl)piperidine-2,6-dione;
3-(5-((4-(((1R,2s,3S,5r)-adamantan-2-ylamino)methyl)benzyl)amino)-2-methyl-4-oxoquinazolin-3(4H)-yl)piperidine-2,6-dione;
3-(5-((4-((adamantan-2-yloxy)methyl)benzyl)amino)-2-methyl-4-oxoquinazolin-3(4H)-yl)piperidine-2,6-dione;
3-(5-((4-(hydroxymethyl)benzyl)amino)-2-methyl-4-oxoquinazolin-3(4H)-yl)piperidine-2,6-dione;
3-(2-methyl-4-oxo-5-((4-(piperidin-1-ylmethyl)benzyl)amino)quinazolin-3(4H)-yl)piperidine-2,6-dione;
3-(5-((4-((dimethylamino)methyl)benzyl)amino)-2-methyl-4-oxoquinazolin-3(4H)-yl)piperidine-2,6-dione;
3-(5-((4-((diethylamino)methyl)benzyl)amino)-2-methyl-4-oxoquinazolin-3(4H)-yl)piperidine-2,6-dione;
3-(5-((4-((3,5-dimethylpiperidin-1-yl)methyl)benzyl)amino)-2-methyl-4-oxoquinazolin-3(4H)-yl)piperidine-2,6-dione;
3-(2-methyl-4-oxo-5-((4-(thiomorpholinomethyl)benzyl)amino)quinazolin-3(4H)-yl)piperidine-2,6-dione;
3-(5-((4-(azepan-1-ylmethyl)benzyl)amino)-2-methyl-4-oxoquinazolin-3(4H)-yl)piperidine-2,6-dione;
3-(2-methyl-5-((4-(morpholinomethyl)benzyl)amino)-4-oxoquinazolin-3(4H)-yl)piperidine-2,6-dione;
4-(4-(4-(((3-(2,6-dioxopiperidin-3-yl)-2-methyl-4-oxo-3,4-dihydroquinazolin-5-yl)amino)methyl)benzyl)piperazin-1-yl)-3-fluorobenzonitrile;
3-(2-methyl-4-oxo-5-((2-(phenylamino)ethyl)amino)quinazolin-3(4H)-yl)piperidine-2,6-dione;
3-(2-methyl-4-oxo-5-(phenethylamino)quinazolin-3(4H)-yl)piperidine-2,6-dione;
3-(5-((4-((adamantan-1-ylamino)methyl)-2-fluorobenzyl)amino)-2-methyl-4-oxoquinazolin-3(4H)-yl)piperidine-2,6-dione;
3-(5-((2-fluoro-4-(piperidin-1-ylmethyl)benzyl)amino)-2-methyl-4-oxoquinazolin-3(4H)-yl)piperidine-2,6-dione;
3-(5-((4-((3,5-dimethylpiperidin-1-yl)methyl)-2-fluorobenzyl)amino)-2-methyl-4-oxoquinazolin-3(4H)-yl)piperidine-2,6-dione;
3-(5-((2-fluoro-4-(thiomorpholinomethyl)benzyl)amino)-2-methyl-4-oxoquinazolin-3(4H)-yl)piperidine-2,6-dione;
3-(5-((4-(azepan-1-ylmethyl)-2-fluorobenzyl)amino)-2-methyl-4-oxoquinazolin-3(4H)-yl)piperidine-2,6-dione;
3-(5-((2-fluoro-4-(morpholinomethyl)benzyl)amino)-2-methyl-4-oxoquinazolin-3(4H)-yl)piperidine-2,6-dione;
4-(4-(4-(((3-(2,6-dioxopiperidin-3-yl)-2-methyl-4-oxo-3,4-dihydroquinazolin-5-yl)amino)methyl)-3-fluorobenzyl)piperazin-1-yl)-3-fluorobenzonitrile;
3-(2-methyl-4-oxo-5-(((5-(piperidin-1-ylmethyl)pyridin-2-yl)methyl)amino)quinazolin-3(4H)-yl)piperidine-2,6-dione;
3-(5-(((5-((3,5-dimethylpiperidin-1-yl)methyl)pyridin-2-yl)methyl)amino)-2-methyl-4-oxoquinazolin-3(4H)-yl)piperidine-2,6-dione;
3-(2-methyl-4-oxo-5-(((5-(thiomorpholinomethyl)pyridin-2-yl)methyl)amino)quinazolin-3(4H)-yl)piperidine-2,6-dione;
3-(5-(((5-(azepan-1-ylmethyl)pyridin-2-yl)methyl)amino)-2-methyl-4-oxoquinazolin-3(4H)-yl)piperidine-2,6-dione;
3-(5-(((5-(((adamantan-1-yl)amino)methyl)pyridin-2-yl)methyl)amino)-2-methyl-4-oxoquinazolin-3(4H)-yl)piperidine-2,6-dione;
3-(2-methyl-5-(((5-(morpholinomethyl)pyridin-2-yl)methyl)amino)-4-oxoquinazolin-3(4H)-yl)piperidine-2,6-dione;
3-(5-(((5-((adamantan-1-ylamino)methyl)thiazol-2-yl)methyl)amino)-2-methyl-4-oxoquinazolin-3(4H)-yl)piperidine-2,6-dione;
3-(2-methyl-4-oxo-5-(((5-(piperidin-1-ylmethyl)thiazol-2-yl)methyl)amino)quinazolin-3(4H)-yl)piperidine-2,6-dione;
3-(5-(((5-(azepan-1-ylmethyl)thiazol-2-yl)methyl)amino)-2-methyl-4-oxoquinazolin-3(4H)-yl)piperidine-2,6-dione;
3-(2-methyl-5-(((5-(morpholinomethyl)thiazol-2-yl)methyl)amino)-4-oxoquinazolin-3(4H)-yl)piperidine-2,6-dione;
3-(2-methyl-5-(((4-(morpholine-4-carbonyl)cyclohexyl)methyl)amino)-4-oxoquinazolin-3(4H)-yl)piperidine-2,6-dione;
N-((3s,5s,7s)-adamantan-1-yl)-4-(((3-(2,6-dioxopiperidin-3-yl)-2-methyl-4-oxo-3,4-dihydroquinazolin-5-yl)amino)methyl)cyclohexanecarboxamide;
N-(4-(((3-(2,6-dioxopiperidin-3-yl)-2-methyl-4-oxo-3,4-dihydroquinazolin-5-yl)amino)methyl)phenyl)cyclohexanecarboxamide;
4-(((3-(2,6-dioxopiperidin-3-yl)-2-methyl-4-oxo-3,4-dihydroquinazolin-5-yl)amino)methyl)phenyl cyclohexanecarboxylate;
N-cyclohexyl-4-(((3-(2,6-dioxopiperidin-3-yl)-2-methyl-4-oxo-3,4-dihydroquinazolin-5-yl)amino)methyl)benzenesulfonamide;
3-(5-((4-((4-(2-hydroxyethyl)piperazin-1-yl)methyl)benzyl)amino)-2-methyl-4-oxoquinazolin-3(4H)-yl)piperidine-2,6-dione;
4-(4-(4-(((3-(2,6-dioxopiperidin-3-yl)-2-methyl-4-oxo-3,4-dihydroquinazolin-5-yl)amino)methyl)benzyl)piperazin-1-yl)-3-fluorobenzonitrile;
3-(2-methyl-4-oxo-6-((4-(piperidin-1-ylmethyl)benzyl)amino)quinazolin-3(4H)-yl)piperidine-2,6-dione;
3-(6-((4-((3,5-dimethylpiperidin-1-yl)methyl)benzyl)amino)-2-methyl-4-oxoquinazolin-3(4H)-yl)piperidine-2,6-dione;
3-(2-methyl-4-oxo-6-((4-(thiomorpholinomethyl)benzyl)amino)quinazolin-3(4H)-yl)piperidine-2,6-dione;
3-(6-((4-(azepan-1-ylmethyl)benzyl)amino)-2-methyl-4-oxoquinazolin-3(4H)-yl)piperidine-2,6-dione;
3-(2-methyl-6-((4-(morpholinomethyl)benzyl)amino)-4-oxoquinazolin-3(4H)-yl)piperidine-2,6-dione;
3-(2-methyl-4-oxo-8-((4-(piperidin-1-ylmethyl)benzyl)amino)quinazolin-3(4H)-yl)piperidine-2,6-dione;
3-(8-((4-((3,5-dimethylpiperidin-1-yl)methyl)benzyl)amino)-2-methyl-4-oxoquinazolin-3(4H)-yl)piperidine-2,6-dione;
3-(2-methyl-8-((4-(morpholinomethyl)benzyl)amino)-4-oxoquinazolin-3(4H)-yl)piperidine-2,6-dione;
3-(8-((4-(azepan-1-ylmethyl)benzyl)amino)-2-methyl-4-oxoquinazolin-3(4H)-yl)piperidine-2,6-dione;
3-(2-methyl-4-oxo-5-((4-(2-(piperidin-1-yl)ethyl)benzyl)amino)quinazolin-3(4H)-yl)piperidine-2,6-dione;
3-(2-methyl-4-oxo-5-((4-(3-(piperidin-1-yl)propyl)benzyl)amino)quinazolin-3(4H)-yl)piperidine-2,6-dione;
3-(2-methyl-4-oxo-5-((4-(piperidin-1-ylmethyl)phenethyl)amino)quinazolin-3(4H)-yl)piperidine-2,6-dione;
3-(5-((2-fluorobenzyl)oxy)-2-methyl-4-oxoquinazolin-3(4H)-yl)piperidine-2,6-dione;
3-(5-((4-((adamantan-1-ylamino)methyl)benzyl)oxy)-2-methyl-4-oxoquinazolin-3(4H)-yl)piperidine-2,6-dione;
3-(5-((4-(hydroxymethyl)benzyl)oxy)-2-methyl-4-oxoquinazolin-3(4H)-yl)piperidine-2,6-dione;
3-(2-methyl-4-oxo-5-((4-(piperidin-1-ylmethyl)benzyl)oxy)quinazolin-3(4H)-yl)piperidine-2,6-dione;
3-(5-((4-(azepan-1-ylmethyl)benzyl)oxy)-2-methyl-4-oxoquinazolin-3(4H)-yl)piperidine-2,6-dione;
3-(2-methyl-5-((4-(morpholinomethyl)benzyl)oxy)-4-oxoquinazolin-3(4H)-yl)piperidine-2,6-dione;
3-(5-((4-(azepan-1-ylmethyl)-2-fluorobenzyl)oxy)-2-methyl-4-oxoquinazolin-3(4H)-yl)piperidine-2,6-dione;
3-(5-((2-fluoro-4-(morpholinomethyl)benzyl)oxy)-2-methyl-4-oxoquinazolin-3(4H)-yl)piperidine-2,6-dione;
3-(2-methyl-4-oxo-5-((5-(piperidin-1-ylmethyl)pyridin-2-yl)methoxy)quinazolin-3(4H)-yl)piperidine-2,6-dione;
3-(5-((5-((3,5-dimethylpiperidin-1-yl)methyl)pyridin-2-yl)methoxy)-2-methyl-4-oxoquinazolin-3(4H)-yl)piperidine-2,6-dione;
3-(5-((5-(azepan-1-ylmethyl)pyridin-2-yl)methoxy)-2-methyl-4-oxoquinazolin-3(4H)-yl)piperidine-2,6-dione;
3-(2-methyl-5-((5-(morpholinomethyl)pyridin-2-yl)methoxy)-4-oxoquinazolin-3(4H)-yl)piperidine-2,6-dione;
3-(5-((5-((adamantan-1-ylamino)methyl)thiazol-2-yl)methoxy)-2-methyl-4-oxoquinazolin-3(4H)-yl)piperidine-2,6-dione;
3-(2-methyl-4-oxo-5-((5-(piperidin-1-ylmethyl)thiazol-2-yl)methoxy)quinazolin-3(4H)-yl)piperidine-2,6-dione;
3-(5-((5-(azepan-1-ylmethyl)thiazol-2-yl)methoxy)-2-methyl-4-oxoquinazolin-3(4H)-yl)piperidine-2,6-dione;
3-(2-methyl-5-((5-(morpholinomethyl)thiazol-2-yl)methoxy)-4-oxoquinazolin-3(4H)-yl)piperidine-2,6-dione;
3-(2-methyl-5-((4-(morpholine-4-carbonyl)cyclohexyl)methoxy)-4-oxoquinazolin-3(4H)-yl)piperidine-2,6-dione;
N-(4-(((3-(2,6-dioxopiperidin-3-yl)-2-methyl-4-oxo-3,4-dihydroquinazolin-5-yl)oxy)methyl)phenyl)cyclohexanecarboxamide;
3-(2-methyl-4-oxo-6-((4-(piperidin-1-ylmethyl)benzyl)oxy)quinazolin-3(4H)-yl)piperidine-2,6-dione;
3-(6-((4-(azepan-1-ylmethyl)benzyl)oxy)-2-methyl-4-oxoquinazolin-3(4H)-yl)piperidine-2,6-dione;
3-(2-methyl-6-((4-(morpholinomethyl)benzyl)oxy)-4-oxoquinazolin-3(4H)-yl)piperidine-2,6-dione;
3-(2-methyl-4-oxo-8-((4-(piperidin-1-ylmethyl)benzyl)oxy)quinazolin-3(4H)-yl)piperidine-2,6-dione;
3-(2-methyl-8-((4-(morpholinomethyl)benzyl)oxy)-4-oxoquinazolin-3(4H)-yl)piperidine-2,6-dione;
3-(8-((4-(azepan-1-ylmethyl)benzyl)oxy)-2-methyl-4-oxoquinazolin-3(4H)-yl)piperidine-2,6-dione;
3-(2-methyl-4-oxo-5-((4-(2-(piperidin-1-yl)ethyl)benzyl)oxy)quinazolin-3(4H)-yl)piperidine-2,6-dione;
3-(2-methyl-4-oxo-5-(4-(piperidin-1-ylmethyl)phenethoxy)quinazolin-3(4H)-yl)piperidine-2,6-dione;
3-(5-(benzylthio)-2-methyl-4-oxoquinazolin-3(4H)-yl)piperidine-2,6-dione;
3-(2-methyl-4-oxo-5-((pyridin-2-ylmethyl)thio)quinazolin-3(4H)-yl)piperidine-2,6-dione;
3-(5-((2-fluorobenzyl)thio)-2-methyl-4-oxoquinazolin-3(4H)-yl)piperidine-2,6-dione;
3-(5-((4-((adamantan-1-ylamino)methyl)benzyl)thio)-2-methyl-4-oxoquinazolin-3(4H)-yl)piperidine-2,6-dione;
3-(5-((4-(hydroxymethyl)benzyl)thio)-2-methyl-4-oxoquinazolin-3(4H)-yl)piperidine-2,6-dione;
3-(2-methyl-4-oxo-5-((4-(piperidin-1-ylmethyl)benzyl)thio)quinazolin-3(4H)-yl)piperidine-2,6-dione;
3-(5-((4-(azepan-1-ylmethyl)benzyl)thio)-2-methyl-4-oxoquinazolin-3(4H)-yl)piperidine-2,6-dione;
3-(2-methyl-5-((4-(morpholinomethyl)benzyl)thio)-4-oxoquinazolin-3(4H)-yl)piperidine-2,6-dione;
3-(5-((2-fluoro-4-(piperidin-1-ylmethyl)benzyl)thio)-2-methyl-4-oxoquinazolin-3(4H)-yl)piperidine-2,6-dione;
3-(5-((4-(azepan-1-ylmethyl)-2-fluorobenzyl)thio)-2-methyl-4-oxoquinazolin-3(4H)-yl)piperidine-2,6-dione;
3-(5-((2-fluoro-4-(morpholinomethyl)benzyl)thio)-2-methyl-4-oxoquinazolin-3(4H)-yl)piperidine-2,6-dione;
3-(2-methyl-4-oxo-5-(((5-(piperidin-1-ylmethyl)pyridin-2-yl)methyl)thio)quinazolin-3(4H)-yl)piperidine-2,6-dione;
3-(5-(((5-(azepan-1-ylmethyl)pyridin-2-yl)methyl)thio)-2-methyl-4-oxoquinazolin-3(4H)-yl)piperidine-2,6-dione;
3-(2-methyl-5-(((5-(morpholinomethyl)pyridin-2-yl)methyl)thio)-4-oxoquinazolin-3(4H)-yl)piperidine-2,6-dione;
3-(2-methyl-4-oxo-5-(((5-(piperidin-1-ylmethyl)thiazol-2-yl)methyl)thio)quinazolin-3(4H)-yl)piperidine-2,6-dione;
3-(5-(((5-(azepan-1-ylmethyl)thiazol-2-yl)methyl)thio)-2-methyl-4-oxoquinazolin-3(4H)-yl)piperidine-2,6-dione;
3-(2-methyl-5-(((5-(morpholinomethyl)thiazol-2-yl)methyl)thio)-4-oxoquinazolin-3(4H)-yl)piperidine-2,6-dione;
3-(2-methyl-4-oxo-6-((4-(thiomorpholinomethyl)benzyl)thio)quinazolin-3(4H)-yl)piperidine-2,6-dione;
3-(6-((4-(azepan-1-ylmethyl)benzyl)thio)-2-methyl-4-oxoquinazolin-3(4H)-yl)piperidine-2,6-dione;
3-(2-methyl-6-((4-(morpholinomethyl)benzyl)thio)-4-oxoquinazolin-3(4H)-yl)piperidine-2,6-dione;
3-(2-methyl-4-oxo-8-((4-(piperidin-1-ylmethyl)benzyl)thio)quinazolin-3(4H)-yl)piperidine-2,6-dione;
3-(8-((4-(azepan-1-ylmethyl)benzyl)thio)-2-methyl-4-oxoquinazolin-3(4H)-yl)piperidine-2,6-dione;
3-(2-methyl-4-oxo-5-((4-(2-(piperidin-1-yl)ethyl)benzyl)thio)quinazolin-3(4H)-yl)piperidine-2,6-dione;
3-(2-methyl-4-oxo-5-((4-(piperidin-1-ylmethyl)phenethyl)thio)quinazolin-3(4H)-yl)piperidine-2,6-dione;
3-(2-methyl-4-oxo-5-((4-(3-(piperidin-1-yl)propyl)benzyl)thio)quinazolin-3(4H)-yl)piperidine-2,6-dione;
3-(5-(4-(4-(azepan-1 -ylmethyl)phenyl)but-1 -yn-1 -yl)-2-methyl-4-oxoquinazolin-3(4H)-yl)piperidine-2,6-dione;
3-(5-((4-(azepan-1-ylmethyl)phenyl)ethynyl)-2-methyl-4-oxoquinazolin-3(4H)-yl)piperidine-2,6-dione;
3-(5-(4-(azepan-1-ylmethyl)phenethyl)-2-methyl-4-oxoquinazolin-3(4H)-yl)piperidine-2,6-dione;
3-(5-(4-(azepan-1-ylmethyl)-2-fluorophenethyl)-2-methyl-4-oxoquinazolin-3(4H)-yl)piperidine-2,6-dione;
3-(5-(4-(azepan-1-ylmethyl)-2-bromophenethyl)-2-methyl-4-oxoquinazolin-3(4H)-yl)piperidine-2,6-dione;
3-(5-(4-((adamantan-1-ylamino)methyl)phenethyl)-2-methyl-4-oxoquinazolin-3(4H)-yl)piperidine-2,6-dione;
3-(2-methyl-4-oxo-5-(4-(piperidin-1-ylmethyl)phenethyl)quinazolin-3(4H)-yl)piperidine-2,6-dione;
3-(2-methyl-5-(4-(morpholinomethyl)phenethyl)-4-oxoquinazolin-3(4H)-yl)piperidine-2,6-dione;
3-(2-methyl-4-oxo-5-(4-(thiomorpholinomethyl)phenethyl)quinazolin-3(4H)-yl)piperidine-2,6-dione;
3-(5-(2-fluoro-4-(morpholinomethyl)phenethyl)-2-methyl-4-oxoquinazolin-3(4H)-yl)piperidine-2,6-dione;
3-(2-methyl-4-oxo-5-(2-(5-(piperidin-1-ylmethyl)pyridin-2-yl)ethyl)quinazolin-3(4H)-yl)piperidine-2,6-dione;
3-(5-(2-(5-((3,5-dimethylpiperidin-1-yl)methyl)pyridin-2-yl)ethyl)-2-methyl-4-oxoquinazolin-3(4H)-yl)piperidine-2,6-dione;
3-(5-(2-(5-(azepan-1-ylmethyl)pyridin-2-yl)ethyl)-2-methyl-4-oxoquinazolin-3(4H)-yl)piperidine-2,6-dione;
3-(2-methyl-5-(2-(5-(morpholinomethyl)pyridin-2-yl)ethyl)-4-oxoquinazolin-3(4H)-yl)piperidine-2,6-dione;
3-(5-(2-(5-((((1R,3R,5S)-adamantan-1-yl)amino)methyl)thiazol-2-yl)ethyl)-2-methyl-4-oxoquinazolin-3(4H)-yl)piperidine-2,6-dione;
3-(2-methyl-4-oxo-5-(2-(5-(piperidin-1-ylmethyl)thiazol-2-yl)ethyl)quinazolin-3(4H)-yl)piperidine-2,6-dione;
3-(5-(2-(5-(azepan-1-ylmethyl)thiazol-2-yl)ethyl)-2-methyl-4-oxoquinazolin-3(4H)-yl)piperidine-2,6-dione;
3-(2-methyl-5-(2-(5-(morpholinomethyl)thiazol-2-yl)ethyl)-4-oxoquinazolin-3(4H)-yl)piperidine-2,6-dione;
3-(2-methyl-4-oxo-6-(4-(piperidin-1-ylmethyl)phenethyl)quinazolin-3(4H)-yl)piperidine-2,6-dione;
3-(6-(4-(azepan-1-ylmethyl)phenethyl)-2-methyl-4-oxoquinazolin-3(4H)-yl)piperidine-2,6-dione;
3-(2-methyl-6-(4-(morpholinomethyl)phenethyl)-4-oxoquinazolin-3(4H)-yl)piperidine-2,6-dione;
3-(2-methyl-4-oxo-8-(4-(piperidin-1-ylmethyl)phenethyl)quinazolin-3(4H)-yl)piperidine-2,6-dione;
3-(2-methyl-8-(4-(morpholinomethyl)phenethyl)-4-oxoquinazolin-3(4H)-yl)piperidine-2,6-dione;
3-(8-(4-(azepan-1-ylmethyl)phenethyl)-2-methyl-4-oxoquinazolin-3(4H)-yl)piperidine-2,6-dione;
3-(2-methyl-4-oxo-5-(4-(2-(piperidin-1-yl)ethyl)phenethyl)quinazolin-3(4H)-yl)piperidine-2,6-dione;
3-(2-methyl-4-oxo-5-(3-(4-(piperidin-1-ylmethyl)phenyl)propyl)quinazolin-3(4H)-yl)piperidine-2,6-dione;
3-(5-((4-((((1R,3R,SS)-adamantan-1-yl)amino)methyl)phenyl)ethynyl)-2-methyl-4-oxoquinazolin-3(4H)-yl)piperidine-2,6-dione;
3-(5-((4-(hydroxymethyl)phenyl)ethynyl)-2-methyl-4-oxoquinazolin-3(4H)-yl)piperidine-2,6-dione;
3-(2-methyl-4-oxo-5-((4-(piperidin-1-ylmethyl)phenyl)ethynyl)quinazolin-3(4H)-yl)piperidine-2,6-dione;
3-(2-methyl-5-((4-(morpholinomethyl)phenyl)ethynyl)-4-oxoquinazolin-3(4H)-yl)piperidine-2,6-dione;
3-(5-((4-(azepan-1-ylmethyl)-2-fluorophenyl)ethynyl)-2-methyl-4-oxoquinazolin-3(4H)-yl)piperidine-2,6-dione;
3-(5-((2-fluoro-4-(morpholinomethyl)phenyl)ethynyl)-2-methyl-4-oxoquinazolin-3(4H)-yl)piperidine-2,6-dione;
3-(2-methyl-4-oxo-5-((5-(piperidin-1-ylmethyl)pyridin-2-yl)ethynyl)quinazolin-3(4H)-yl)piperidine-2,6-dione;
3-(5-((5-((3,5-dimethylpiperidin-1-yl)methyl)pyridin-2-yl)ethynyl)-2-methyl-4-oxoquinazolin-3(4H)-yl)piperidine-2,6-dione;
3-(5-((5-(azepan-1-ylmethyl)pyridin-2-yl)ethynyl)-2-methyl-4-oxoquinazolin-3(4H)-yl)piperidine-2,6-dione;
3-(2-methyl-5-((5-(morpholinomethyl)pyridin-2-yl)ethynyl)-4-oxoquinazolin-3(4H)-yl)piperidine-2,6-dione;
3-(5-((5-((((1R,3R,5S)-adamantan-1-yl)amino)methyl)thiazol-2-yl)ethynyl)-2-methyl-4-oxoquinazolin-3(4H)-yl)piperidine-2,6-dione;
3-(2-methyl-4-oxo-5-((5-(piperidin-1-ylmethyl)thiazol-2-yl)ethynyl)quinazolin-3(4H)-yl)piperidine-2,6-dione;
3-(5-((5-(azepan-1-ylmethyl)thiazol-2-yl)ethynyl)-2-methyl-4-oxoquinazolin-3(4H)-yl)piperidine-2,6-dione;
3-(2-methyl-5-((5-(morpholinomethyl)thiazol-2-yl)ethynyl)-4-oxoquinazolin-3(4H)-yl)piperidine-2,6-dione;
3-(2-methyl-4-oxo-6-((4-(piperidin-1-ylmethyl)phenyl)ethynyl)quinazolin-3(4H)-yl)piperidine-2,6-dione;
3-(6-((4-(azepan-1-ylmethyl)phenyl)ethynyl)-2-methyl-4-oxoquinazolin-3(4H)-yl)piperidine-2,6-dione;
3-(2-methyl-6-((4-(morpholinomethyl)phenyl)ethynyl)-4-oxoquinazolin-3(4H)-yl)piperidine-2,6-dione;
3-(2-methyl-4-oxo-8-((4-(piperidin-1-ylmethyl)phenyl)ethynyl)quinazolin-3(4H)-yl)piperidine-2,6-dione;
3-(2-methyl-8-((4-(morpholinomethyl)phenyl)ethynyl)-4-oxoquinazolin-3(4H)-yl)piperidine-2,6-dione;
3-(2-methyl-4-oxo-5-((4-(thiomorpholinomethyl)phenyl)ethynyl)quinazolin-3(4H)-yl)piperidine-2,6-dione;
3-(8-((4-(azepan-1-ylmethyl)phenyl)ethynyl)-2-methyl-4-oxoquinazolin-3(4H)-yl)piperidine-2,6-dione;
3-(5-((4-(azepan-1-ylmethyl)-2-fluorophenyl)ethynyl)-2-methyl-4-oxoquinazolin-3(4H)-yl)piperidine-2,6-dione;
3-(2-methyl-4-oxo-5-((4-(2-(piperidin-1-yl)ethyl)phenyl)ethynyl)quinazolin-3(4H)-yl)piperidine-2,6-dione;
3-(2-methyl-4-oxo-5-(3-(4-(piperidin-1 -ylmethyl)phenyl)prop-1 -yn-1 -yl)quinazolin-3(4H)-yl)piperidine-2,6-dione;
3-(5-(3-(4-(azepan-1 -ylmethyl)phenyl)prop-1 -yn-1 -yl)-2-methyl-4-oxoquinazolin-3(4H)-yl)piperidine-2,6-dione;
3-(5-(3-(4-(azepan-1 -ylmethyl)-2-fluorophenyl)prop-1 -yn-1 -yl)-2-methyl-4-oxoquinazolin-3(4H)-yl)piperidine-2,6-dione;
3-(2-methyl-4-oxo-5-(3-(4-(thiomorpholinomethyl)phenyl)prop-1-yn-1-yl)quinazolin-3(4H)-yl)piperidine-2,6-dione; or
3-(2-methyl-5-(3-(4-(morpholinomethyl)phenyl)prop-1-yn-1-yl)-4-oxoquinazolin-3(4H)-yl)piperidine-2,6-dione.

43. A pharmaceutical composition comprising the compound of Formula (I) as claimed in any one of claims 1-42 or a pharmaceutically acceptable salt thereof, and at least one pharmaceutically acceptable carrier.

44. The pharmaceutical composition as claimed in claim 43, further comprising an additional therapeutic agent.

45. Use of the compound of Formula (I) or a pharmaceutically acceptable salt thereof as claimed in any one of claims 1 to 42 or the pharmaceutical composition as claimed in claim 43 or 44 for the manufacture of a medicament for the prevention and/or treatment of a disease or disorder associated with TNF-α.

46. The use as claimed in claim 45, wherein the disease or disorder associated with TNF-α is selected from the group consisting of: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, Unverricht Syndrome, acute liver failure, or diabetes.

47. The use as claimed in claim 45, wherein the disease or disorder associated with TNF-α is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, anemia associated with leukemia, acute myeloid leukemia (AML); lymphoma, including diffuse large B-cell lymphoma, non-Hodgkin's lymphoma, anaplastic lymphoma, anaplastic large cell lymphoma, lymphoma CD20 positive, mantle cell lymphoma, primary lymphoma, B-cell lymphoma, recurrent B-cell non-Hodgkin's lymphoma, recurrent diffuse large B-cell lymphoma, recurrent mediastinal (thymus) large B-cell, primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; inflammatory myofibroblastoma; colorectal cancer; brain glioma; astroblastoma; oophoroma; bronchial carcinoma; prostate cancer; breast cancer, including triple negative breast cancer, sporadic breast cancer and Cowden's disease patients; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial carcinoma; brain cancer; oral cancer; sarcoma, including rhabdomyosarcoma, various lipogenic tumors, Ewing's sarcoma/primitive neuroectodermal tumors (Ewing/PNETs), and smooth muscle sarcoma; Unverricht Syndrome; autoimmune diseases, including, e.g., rheumatoid arthritis; autoimmune encephalomyelitis; ankylosing spondylitis; psoriasis; systemic lupus erythematosus; multiple sclerosis; recurrent oral ulcer; Kawasaki disease; inflammatory diseases, including inflammatory bowel disease including Crohn's disease and ulcerative colitis, pneumonia, osteoarthritis, synovitis, systemic inflammatory response syndrome, airway inflammation, bronchitis; cerebral malaria; acquired immune deficiency syndrome (AIDS); COVID-19 novel coronavirus infection; septic shock; tuberculosis; sepsis syndrome; bacterial meningitis, chronic obstructive pulmonary disease; asthma; hemorrhagic shock; organ (including kidney, heart, lung) or tissue transplant rejection; diabetes; sarcoidosis; adult respiratory distress syndrome; congestive heart failure; myocardial infarction; multiple organ failure due to cachexia and septic shock; or acute liver failure.

48. The compound of Formula (I) or a pharmaceutically acceptable salt thereof as claimed in any one of claims 1 to 42, for use in the prevention and/or treatment of a disease or disorder associated with TNF-α.

49. The compound of Formula (I) or a pharmaceutically acceptable salt thereof as claimed in claim 48, wherein the disease or disorder associated with TNF-α is selected from the group consisting of: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, Unverricht Syndrome, acute liver failure, or diabetes.

50. The compound of Formula (I) or a pharmaceutically acceptable salt thereof as claimed in claim 48, wherein the disease or disorder associated with TNF-α is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, anemia associated with leukemia, acute myeloid leukemia (AML); lymphoma, including diffuse large B-cell lymphoma, non-Hodgkin's lymphoma, anaplastic lymphoma, anaplastic large cell lymphoma, lymphoma CD20 positive, mantle cell lymphoma, primary lymphoma, B-cell lymphoma, recurrent B-cell non-Hodgkin's lymphoma, recurrent diffuse large B-cell lymphoma, recurrent mediastinal (thymus) large B-cell, primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; inflammatory myofibroblastoma; colorectal cancer; brain glioma; astroblastoma; oophoroma; bronchial carcinoma; prostate cancer; breast cancer, including triple negative breast cancer, sporadic breast cancer and Cowden's disease patients; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial carcinoma; brain cancer; oral cancer; sarcoma, including rhabdomyosarcoma, various lipogenic tumors, Ewing's sarcoma/primitive neuroectodermal tumors (Ewing/PNETs), and smooth muscle sarcoma; Unverricht Syndrome; autoimmune diseases, including, e.g., rheumatoid arthritis; autoimmune encephalomyelitis; ankylosing spondylitis; psoriasis; systemic lupus erythematosus; multiple sclerosis; recurrent oral ulcer; Kawasaki disease; inflammatory diseases, including inflammatory bowel disease including Crohn's disease and ulcerative colitis, pneumonia, osteoarthritis, synovitis, systemic inflammatory response syndrome, airway inflammation, bronchitis; cerebral malaria; acquired immune deficiency syndrome (AIDS); COVID-19 novel coronavirus infection; septic shock; tuberculosis; sepsis syndrome; bacterial meningitis, chronic obstructive pulmonary disease; asthma; hemorrhagic shock; organ (including kidney, heart, lung) or tissue transplant rejection; diabetes; sarcoidosis; adult respiratory distress syndrome; congestive heart failure; myocardial infarction; multiple organ failure due to cachexia and septic shock; or acute liver failure.

51. A method for treating or preventing a disease or disorder associated with TNF-α, comprising administering to a subject a therapeutically effective amount of the compound of Formula (I) or a pharmaceutically acceptable salt thereof as claimed in any one of claims 1-42, or the pharmaceutical composition as claimed in claim 43 or 44.

52. The method as claimed in claim 51, wherein the disease or disorder associated with TNF-α is selected from the group consisting of: tumors, infectious diseases, inflammatory diseases, autoimmune diseases, anemia, hemorrhagic shock, transplant rejection, multiple organ dysfunction syndrome (MODS), sarcoidosis, adult respiratory distress syndrome, congestive heart failure, myocardial infarction, Unverricht Syndrome, acute liver failure, or diabetes.

53. The method as claimed in claim 51, wherein the disease or disorder associated with TNF-α is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, anemia associated with leukemia, acute myeloid leukemia (AML); lymphoma, including diffuse large B-cell lymphoma, non-Hodgkin's lymphoma, anaplastic lymphoma, anaplastic large cell lymphoma, lymphoma CD20 positive, mantle cell lymphoma, primary lymphoma, B-cell lymphoma, recurrent B-cell non-Hodgkin's lymphoma, recurrent diffuse large B-cell lymphoma, recurrent mediastinal (thymus) large B-cell, primary mediastinal (thymus) large B-cell, relapsed transformed non-Hodgkin's lymphoma, refractory B-cell non-Hodgkin's lymphoma, refractory diffuse large B-cell lymphoma, refractory primary mediastinal (thymus) large B-cell, refractory transformed non-Hodgkin's lymphoma; thyroid cancer; melanoma; lung cancer; inflammatory myofibroblastoma; colorectal cancer; brain glioma; astroblastoma; oophoroma; bronchial carcinoma; prostate cancer; breast cancer, including triple negative breast cancer, sporadic breast cancer and Cowden's disease patients; pancreatic cancer; neuroblastoma; extramedullary plasmacytoma; plasmacytoma; gastric cancer; gastrointestinal stromal tumor; esophageal cancer; large intestine adenocarcinoma; esophageal squamous cell carcinoma; liver cancer; renal cell carcinoma; bladder cancer; endometrial carcinoma; brain cancer; oral cancer; sarcoma, including rhabdomyosarcoma, various lipogenic tumors, Ewing's sarcoma/primitive neuroectodermal tumors (Ewing/PNETs), and smooth muscle sarcoma; Unverricht Syndrome; autoimmune diseases, including, e.g., rheumatoid arthritis; autoimmune encephalomyelitis; ankylosing spondylitis; psoriasis; systemic lupus erythematosus; multiple sclerosis; recurrent oral ulcer; Kawasaki disease; inflammatory diseases, including inflammatory bowel disease including Crohn's disease and ulcerative colitis, pneumonia, osteoarthritis, synovitis, systemic inflammatory response syndrome, airway inflammation, bronchitis; cerebral malaria; acquired immune deficiency syndrome (AIDS); COVID-19 novel coronavirus infection; septic shock; tuberculosis; sepsis syndrome; bacterial meningitis, chronic obstructive pulmonary disease; asthma; hemorrhagic shock; organ (including kidney, heart, lung) or tissue transplant rejection; diabetes; sarcoidosis; adult respiratory distress syndrome; congestive heart failure; myocardial infarction; multiple organ failure due to cachexia and septic shock; or acute liver failure.
